# FASCICULE DE BREVET EUROPEEN

(11) **EP 1 569 935 B1**
(45) Date de publication et mention de la délivrance du brevet: **20.08.2008**
(21) Numéro de dépôt: 03789523.2
(22) Date de dépôt: 28.11.2003
(51) Int. Cl.: C07D 471/18, C07D 495/18, C07D 471/08, A61K 31/55

(54) **COMPOSES HETEROCYCLIQUES, LEUR PREPARATION ET LEUR UTILISATION COMME MEDICAMENTS, NOTAMMENT COMME ANTI-BACTERIENS ET INHIBITEURS DE BETA-LACTAMASES**
HETEROZYKLISCHE VERBINDUNGEN, IHRE HERSTELLUNG UND IHRE THERAPEUTISCHE VERWENDUNG, BESONDERS ALS ANTIBAKTERIELLE UND BETA-LACTAMASE INHIBITOREN
HETEROCYCLIC COMPOUNDS, PREPARATION METHOD THEREOF AND USE OF SAME AS MEDICAMENTS, SUCH AS ANTI-BACTERIAL MEDICAMENTS AND BETA-LACTAMASE INHIBITORS

(30) Priorité: 06.12.2002 FR 0215428
(43) Date de publication de la demande: 07.09.2005
(73) Titulaire: NOVEXEL, 93230 Romainville (FR)
(72) Inventeur: LAMPILAS, Maxime, F-92210 Saint Cloud (FR); MUSICKI, Branislav, F-75002 Paris (FR); KLICH, Michel, F-93250 Villemomble (FR); ROWLANDS, David, Alan, F-78300 Poissy (FR)
(74) Mandataire: Hirsch & Associés
(86) Numéro de dépôt international: PCT/FR2003/003523
(87) Numéro de publication internationale: WO 2004/052891

(56) Documents cités:
- EP-A- 0 818 197
- WO-A-00/63187
- WO-A-02/10172
- WO-A-95/18129
- WO-A-02/100860
- FR-A- 2 676 230
- DATABASE CA [en ligne] CHEMICAL ABSTRACTS SERVICE, COLUMBUS, OHIO, US; TRIEBS, WILHELM ET AL: "Experiments for the preparation of azatropolones. I. Disubstituted 1-aza-4,5-cycloheptanedione and 5-azatropolone" retrieved from STN Database accession no. 56:53319 XP002259045 & JOURNAL FUER PRAKTISCHE CHEMIE (LEIPZIG) (1961), 14, 208-17,
- DATABASE CA [en ligne] CHEMICAL ABSTRACTS SERVICE, COLUMBUS, OHIO, US; PENNINGTON, F. C. ET AL: "Preparation and cyclization of substituted 1-anilino-3-halo-2- propanols and their conversion to indoles" retrieved from STN Database accession no. 63:62876 XP002259046 & JOURNAL OF ORGANIC CHEMISTRY (1965), 30(8), 2801-4,

## Description

L'invention concerne de nouveaux composés hétérocycliques, leur préparation et leur utilisation comme médicaments, notamment comme anti-bactériens.

Dans le journal J. Org. Chem., Vol. 37, No. 5, 1972, pages 697 à 699 est décrite notamment la préparation d'un dérivé bicyclique de formule brute C₁₀H₁₈N₂O.

Dans le journal J. Org. Chem., Vol. 45, No. 26, 1980, pages 5325-5326 est décrite notamment la préparation de dérivés bicycliques de formules brutes C₆H₉NO₂ et C₇H₁₁NO₂.

Dans la revue Chemical Reviews, 1983, vol. 83, No. 5, pages 549 à 555 est décrite notamment la préparation de dérivés bicycliques de formules brutes C₁₀H₁₈N₂O et C₇H₁₂N₂O.

Dans le journal Angew. Chem. Int. Ed. 2000, 39, n° 3, pages 625 à 628 est décrit notamment la préparation d'un composé de formule brute C₁₂H₁₂N₂O.

Aucune utilisation particulière dans le domaine thérapeutique de ces composés n'a été décrite dans ces documents.

Par ailleurs, la demande de brevet WO 2002 10172-A décrit des composés azabicycliques utiles dans le domaine thérapeutique, notamment antibactérien.

L'invention a pour objet les composés répondant à la formule (I) suivante : 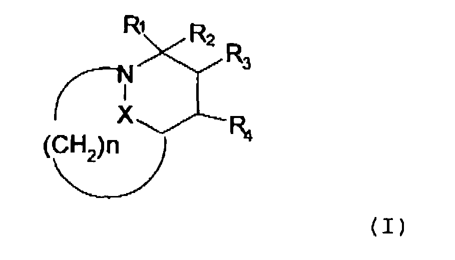 dans laquelle :
R1 représente un atome d'hydrogène, un radical COOH, COOR, CN, (CH₂)ₙ·R₅, CONR₆R₇ ou 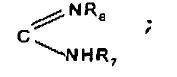
R est choisi dans le groupe constitué par un radical alkyle renfermant de 1 à 6 atomes de carbone, éventuellement substitué par un ou plusieurs atomes d'halogène ou par un radical pyridyle, un radical -CH₂-alkényle renfermant au total de 3 à 9 atomes de carbone, un groupe (poly)alkoxyalkyle renfermant 1 à 4 atomes d'oxygène et 3 à 10 atomes de carbone, un radical aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle renfermant de 1 à 6 atomes de carbone, alkoxy renfermant de 1 à 6 atomes de carbone ou par un ou plusieurs atomes d'halogène,
R₅ est choisi dans le groupe constitué par un radical COOH, CN, OH, NH₂, CO-NR₆R₇, COOR, OR, R étant défini comme ci-dessus,
R₆ et R₇ sont individuellement choisis dans le groupe constitué par un atome d'hydrogène, un radical alkyle renfermant de 1 à 6 atomes de carbone, alkoxy renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle,
n' est égal à 1 ou 2,
R₃ et R₄ forment ensemble un phényl ou un hétérocycle à caractère aromatique à 5 sommets renfermant 1 ou 2 héréroatomes choisis parmi l'azote, l'oxygène et le soufre, tel que défini ci-après:

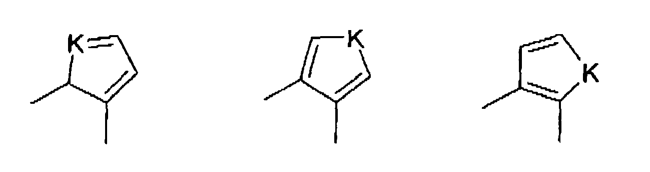

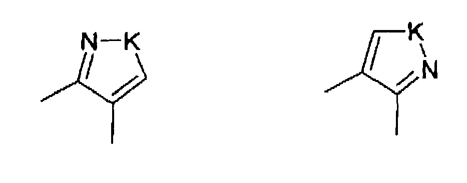

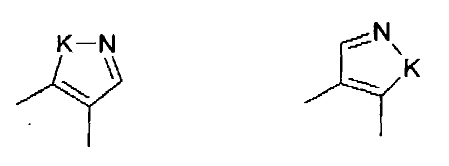

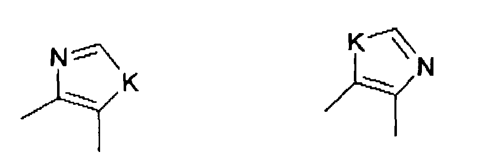

Avec K = NH, S, O
substitué par un groupement R', R' étant choisi dans le groupe constitué par les radicaux
-(O)ₐ-(CH₂)_{b}-(O)ₐ-CONR₆R₇, -(O)ₐ-(CH₂)_{b}-OSO₃H,-(O)ₐ-(CH₂)_{b}-SO₃H,
-(O)ₐ-SO₂R,-(O)ₐ-SO₂-CHal₃, -(O)ₐ-(CH₂)_{b}-NR₆R₇,
- (O)ₐ-(CH₂)_{b}-NH-COOR,-(CH₂)_{b}-COOH,-(CH₂)_{b}-COOR,-OR", OH,
-(CH₂)_{b}- phényle et -(CH₂)_{b} - hétérocycle à caractère aromatique à 5 ou 6 sommets renfermant de 1 à 4 hétéroatomes choisis parmi l'azote, l'oxygène et le soufre, le phényle et l'hétérocycle étant éventuellement substitués par un ou plusieurs halogènes, alkyle renfermant de 1 à 6 atomes de carbone, alkoxy renfermant de 1 à 6 atomes de carbone ou CF₃, R, R₆ et R₇ étant tels que définis précédemment, R" étant choisi dans le groupe constitué par les radicaux alkyles renfermant de 1 à 6 atomes de carbone substitués par un ou plusieurs radicaux hydroxy, hydroxy protégés, oxo, halogène ou cyano, a étant égal à O ou 1 et b étant un entier de 0 à 6, étant entendu que lorsque R' est OH, R₁ représente le radical CONR₆R₇ dans lequel R₆ ou R₇ est un alkoxy renfermant de 1 à 6 atomes de carbone et étant entendu que lorsque R' est - (O)ₐ-(CH₂)_{b}-NH-COOR, a et b ne prennent pas en même temps la valeur 0, et lorsque R' est -(CH₂)_{b}-COOH ou -(CH₂)_{b}-COOR b ne vaut pas 0,
R₂ représente un atome d'hydrogène
X représente un groupement divalent -C(O)-B- relié à l'atome d'azote par l'atome de carbone,
B représente un groupement divalent -NR₈-(CH₂)ₙ"- relié au carbonyle par l'atome d'azote, n" est égal à 0 ou 1 et R₈ est choisi dans le groupe constitué par un atome d'hydrogène, un radical OH, R, OR, Y, OY, Y₁ et OY₁, m étant égal à 0, 1 ou 2,
Y est choisi dans le groupe constitué par les radicaux COH, COR, COOR, CONH₂, CONHR, CONHOH, CONHSO₂R, CH₂COOH, CH₂COOR, CHF-COOH, CHF-COOR, CF₂-COOH, CF₂-COOR, CN, CH₂CN, CH₂CONHOH, CH₂CONHCN, CH₂-tétrazole, CH₂SO₃H, CH₂SO₂R, CH₂PO(OR)₂, CH₂PO(OR)(OH), CH₂PO(R)(OH) et CH₂PO (OH)₂,
Y₁ est choisi dans le groupe constitué par les radicaux SO₂R, SO₂NHCOH, SO₂NHCOR, SO₂NHCOOR, SO₂NHCONHR, SO₂NHCONH₂ et SO₃H,
n est égal à 1.

L'invention a également pour objet les sels de ces composés qui peuvent être obtenus avec des bases ou des acides minéraux ou organiques.

Il est clair que les composés selon l'invention se distinguent structurellement des composés de l'art antérieur cités plus haut.

Les atomes de carbone asymétriques contenus dans les composés de formule (I) peuvent indépendamment les uns des autres présenter la configuration R, S ou RS et l'invention a donc également pour objet les composés de formule (I) se présentant sous la forme d'énantiomères purs ou de diastéréoisomères purs ou sous la forme d'un mélange d'énantiomères notamment de racémates, ou de mélanges de diastéréoisomères.

Il résulte de ce qui précède que les substituants R₁, R₂, ou R₄ pris individuellement d'une part et X d'autre part peuvent être en position cis et/ou trans par rapport au cycle sur lequel ils sont fixés et que l'invention a donc pour objet les composés de formule (I) se présentant sous la forme d'isomères cis ou d'isomères trans ou de mélanges.

Par ailleurs, il est entendu que l'invention ne s'étend pas à des composés de formule (I) dans laquelle R' représente un groupement -(O)ₐ-(CH₂)_{b}-OSO₃H ou -(O)ₐ-(CH₂)_{b}-(O)ₐ-CONR₆R₇ dans lequel a est égal à 1 et b est égal à O.

Par radical alkyle renfermant de 1 à 6 atomes de carbone, on entend le radical méthyle, éthyle, propyle ou isopropyle, ainsi que butyle, pentyle ou hexyle, linéaire ou ramifié.

Par radical -CH₂-alkényle renfermant de 3 à 9 atomes de carbone, on entend par exemple le radical allyle, ou un radical butényle, pentényle ou hexényle.

Par radical aryle renfermant de 6 à 10 atomes de carbone, on entend un radical phényle ou naphtyle.

Par radical aralkyle renfermant de 7 à 11 atomes de carbone, on entend un radical benzyle, phénéthyle ou méthylnaphtyle.

Par radical alkoxy renfermant de 1 à 6 atomes de carbone, on entend notamment le radical méthoxy, éthoxy, propoxy, isopropoxy, butoxy, isobutoxy, sec-butoxy ou tert-butoxy.

Par radicaux hydroxy protégés, on entend des radicaux protégés par des groupes quelconques connus de l'homme du métier, mais plus particulièrement, pour des composés dihydroxy, par des groupes de type 1,3-dioxolanyl.

Par radical halogéno ou par atome d'halogène, on entend fluor, chlore, brome ou iode.

Par radical squarate, on entend le radical de formule :

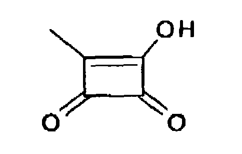

Parmi les sels d'acides des produits de formule (I), on peut citer entre autres, ceux formés avec les acides minéraux, tels que les acides chlorhydrique, bromhydrique, iodhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane et éthane sulfoniques, arylsulfoniques tels que les acides benzène et paratoluènesulfoniques.

Parmi les sels de bases des produits de formule (I), on peut citer, entre autres, ceux formés avec les bases minérales telles que, par exemple, l'hydroxyde de sodium, de potassium, de lithium, de calcium, de magnésium ou d'ammonium ou avec les bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine, ou encore les sels de phosphonium, tels que les alkyl-phosphonium, les aryl-phosphoniums, les alkyl-aryl-phosphonium, les alkényl-aryl-phosphonium ou les sels d'ammoniums quaternaires tels que le sel de tétra-n-butyl-ammonium.

Parmi les composés de formule (I), l'invention a notamment pour objet ceux dans laquelle R' est choisi dans le groupe constitué par les radicaux -(O)ₐ-(CH₂)_{b}-(O)ₐ-CONR₆R₇, -(O)ₐ-(CH₂)_{b}-OSO₃H, -(O)ₐ-(CH₂)_{b}-SO₃H, -(O)ₐ-SO₂R,-(O)ₐ-SO₂-CHal₃, -(O)ₐ-(CH₂)_{b}-NR₆R₇, (O)ₐ-CH₂)_{b}-NHCOOR, -(CH₂)_{b}-COOH, -(CH₂)_{b}-COOR, -OR", OH, -(CH₂)_{b}-phényle et -(CH₂)_{b}-hétérocycle à caractère aromatique à 5 ou 6 sommets renfermant de 1 à 4 hétéroatomes choisis parmi l'azote, l'oxygène et le soufre, le phényle et l'hétérocycle étant éventuellement substitués par un ou plusieurs halogènes, alkyle renfermant de 1 à 6 atomes de carobne, alkoxy renfermant de 1 à 6 atomes de carbone, alkoxy renfermant de 1 à 6 atomes de carbone, alkoxy renfermant de 1 à 6 atomes de arbone ou CF₃, R, R₆ et R₇ étant tels que définis à la revendication 1, R" étant choisi dans le groupe constitué par les radicaux allyles renfermant de 1 à 6 atomes de carbone substitués par un ou plusieurs radicaux hydroxy, oxo, halogène ou cyano, a étant égal à 0 ou 1 et b étant un entier de 0 à 6, étant entendu que:
(i) lorsque R' est OH ou OR" avec R" étant un radical alkyle renfermant de 1 à 6 atomes de carbone substitués par un ou plusieurs radicaux oxo, alors R₁ représente le radical CONR₆R₇ dans lequel R₆ ou R₇ est un alkoxy renfermant de 1 à 6 atomes de carbone, ou bien
(j) lorsque R' est un radical -(O)ₐ-(CH₂)_{b}-NR₆R₇, dans lequel R₆ et R₇ sont tels que définis à la revendication 1 alors a est égal à 0 ou 1 et b est un entier de 1 à 6 ou bien
(k) a est égal à 0 ou 1 et b est un entier de 0 à 6 lorsque R' est un radical -(O)ₐ-(CH₂)_{b}-NR₆R₇, dans lequel R₆ et R₇ sont individuellement choisis dans le groupe constitué par un radical alkyle renfermant de 1 à 6 atomes de carbone, alkoxy renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle, ou bien encore
(1) lorsque R' est choisi parmi le groupe constitué par les radicaux -(O)ₐ-(CH₂)_{b}-NH-COOR, -(CH₂)_{b}-COOH, - (CH₂)_{b}-COOR alors b est un entier de 1 à 6 et a vaut 0 ou 1 ;
les autres valeurs étant défini comme précédemment.

On préfère parmi les composés de formule (I) ceux dans lesquels R₃ et R₄ forment ensemble un phényl, un thiényle ou un pyrazolyle substitué par un substituant R' tel que défini précédemment .

Parmi les composés de formule (I), l'invention a notamment pour objet ceux dans lesquels R₁ est choisi dans le groupe constitué par l'atome d'hydrogène et les groupements COOCH₃, COOC₂H₅, CONH₂, CONHCH₃ et CONHOCH₃.

Parmi les composés de formule (I), l'invention a encore notamment pour objet ceux dans lesquels R₈ est un groupement OY dans lequel Y est choisi parmi les groupements CH₂COOH, CH₂COOR, CHF-COOH, CHF-COOR, CF2-COOH, CF2-COOR, CN, CH₂CN, CH₂CONHOH, CH₂CONHCN, CH₂tétrazole, CH₂tétrazole protégé, CH₂SO₃H, CH₂SO₂R, CH₂PO(OR)₂, CH₂PO(OR)(OH), CH₂PO(R)(OH) et CH₂PO(OH)₂ ou OY₁, dans lequel Y₁ est choisi parmi les groupements SO₂R, SO₂NHCOR, SO₂NHCOOR, SO₂NHCONHR et SO₃H, R étant tel que défini plus haut.

Parmi les composés de formule (I), l'invention a encore notamment pour objet ceux dans lesquels R' est choisi dans le groupe constitué par -O-CH₂-CHOH-CH₂OH, -CH₂-CH₂-NH₂,
-CH₂-COOC₂H₅, -CH₂-CH₂-Phényl, -CH₂-Phényl, -O-CO-NHPhényl, -O-CO-NHC₂H₅, -O-SO₂-CF₃, -O- (CH₂)₂-O-SO₃H, -CH₂-COOH, -CO-NH₂, -CO-NHPhényl, -CH₂-(p-OCH₃ Phényl) et Phényl éventuellement substitué par CH₃, C₂H₅, F et CF₃.

Parmi les composés de formule (I), l'invention a tout particulièrement pour objet les composés dont les noms suivent :
• le sel de triéthylammonium de 5,6-dihydro-6-oxo-N²-phényl-5(sulfooxy)-4*H*-4,7-méthanopyrazolo[3,4-e][1,3]diazépine-2,8(8*H*)-dicarboxamide,
• le sel de sodium de 4,5,6,8-tétrahydro-6-oxo-5-(sulfooxy)-1*H*-4,7-méthanopyrazolo[3,4-e][1,3]diazépine - 1-carboxamide,
• le sel de sodium de 1,4,5,8-tétrahydro-1-[(4-méthoxyphényl)méthyl]-5-(sulfooxy)-6*H*-4,7-méthanopyrazolo[3,4-e][1,3]diazépin-6-one,
• le sel de sodium de trans-4,5,6,8-tétrahydro-2-(2-méthylphényl)-6-oxo-5-(sulfooxy)-4,7-méthano-7*H-*thiéno[2,3-e][1,3]diazépine-8-carboxamide,
• le sel de sodium de trans-4,5,6,8-tétrahydro-6-oxo-5-(sulfooxy)-2-[2-(trifluorométhyl)phényl]-4,7-méthano-7*H-*thiéno[2,3-e][1,3]diazépine-8-carboxamide,
• le sel de sodium de trans-2-(2-éthylphényl)-4,5,6,8-tétrahydro-6-oxo-5-(sulfooxy)-4,7-méthano-7*H*-thiéno[2,3-e][1,3]diazépine-8-carboxamide,
• le sel de sodium de trans-8-(2,3-dihydroxypropoxy)-1,2,3,5-tétrahydro-3-oxo-2-(sulfooxy)-1,4-méthano-4*H*-2,4-benzodiazépine-5-carboxamide,
• le sel de sodium de trans-3-(4-fluorophényl)-4,6,7,8-tétrahydro-6-oxo-7-(sulfooxy)-5,8-méthano-5*H*-thiéno[2,3-e][1,3]diazépine-4-carboxylate d'éthyle,
• le sel de sodium de trans-2,5,6,8-tétrahydro-6-oxo-2-phényl-5-(sulfooxy)-4*H*-4,7-méthanopyrazolo[3,4-e][1,3]diazépine-8-carboxamide,
• le sel de sodium de 1,4,5,8-tétrahydro-1-phényl-5-(sulfooxy)-6*H*-4,7-méthanopyrazolo[3,4-e][1,3]diazépin-6-one,
• le sel de sodium de trans-4,5,6,8-tétrahydro-6-oxo-1-phényl-5(sulfooxy)-1*H*-4,7-méthanopyrazolo[3,4-e][1,3] diazépine-8-carboxamide,
• le sel de triéthylammonium de trans-2,5,6,8-tétrahydro-6-oxo-2-(phénylméthyl)-5-(sulfooxy)-4_{H}-4,7-méthanopyrazolo[3,4-e][1,3]diazépine-8-carboxylate de méthyle,
• le sel de triéthylammonium de trans-4,5,6,8-tétrahydro-6-oxo-1-(2-phényléthyl)-5-(sulfooxy)-1_{H}-4,7-méthanopyrazolo[3,4-e][1,3]diazépine-8-carboxylate de méthyle,
• le sel de triéthylammonium de trans-4,5,6,8-tétrahydro-8-(méthoxycarbonyl)-6-oxo-5-(sulfooxy)-1*H*-4,7-méthanopyrazolo[3,4-e][1,3]diazépine-1-acétate d'éthyle,
• le sel de triéthylammonium de trans-5,6-dihydro-8-(méthoxycarbonyl)-6-oxo-5-(sulfooxy)-4*H*-4,7-méthanopyrazolo[3,4-e][1,3]diazépine-2(8*H*)-acétate d'éthyle,
• le sel de di-(triéthylammonium) de l'acide trans-5,6-dihydro-8-(méthoxycarbonyl)-6-oxo-5-sulfooxy-4*H*-4,7-méthanopyrazolo[3,4-e][1,3]diazépine-2(8*H*)-acétique,
• le sel de pyridinium de trans-1-(aminocarbonyl)-4,5,6,8-tétrahydro-6-oxo-5-(sulfooxy)-1*H*-4,7-méthanopyrazolo[3,4-e][1,3]diazépine-8-carboxylate de méthyle,
• le sel de pyridinium de trans-2-(aminocarbonyl)-2,5,6,8-tétrahydro-6-oxo-5-(sulfooxy)-4*H*-4,7-méthanopyrazolo[3,4-e][diazépine-8-carboxylate de méthyle,
• le sel de sodium de trans-2-(4-fluorophényl)-4,5,6,8-tétrahydro-6-oxo-5-(sulfooxy)-4,7-méthano-7*H*-thiéno[2,3-e][1,3]diazépine-8-carboxylate de méthyle,
• le sel de sodium de trans-2(aminocarbonyl)-4,5,6,8-tétrahydro-6-oxo-5-(sulfooxy)-4,7-méthano-7*H*-thiéno[2,3-e][1,3]diazépine-8-carboxylate de méthyle,
• le sel de sodium de trans-1,2,3,5-tétrahydro-3-oxo-9-[[(phénylamino)carbonyl]oxy]-2-(sulfooxy)-1,4-méthano-4*H-*2,4-benzodiazépine-5-carboxylate d'éthyle,
• le sel de sodium de trans-1,2,3,5-tétrahydro-N-méthoxy-8-[(2 méthoxyéthoxy)méthoxy]-3-oxo-2-(sulfoxy)-1,4-méthano-4H-2,4-benzodiazépine-5-carboxamide,
• le sel de sodium de trans-1,2,3,5-tétrahydro-3-oxo-8-[[(phénylamino)carbonyl]oxy]-2-(sulfooxy)-1,4-méthano-4*H-*2,4-benzodiazépine-5-carboxylate d'éthyle,
• le sel de sodium de trans-8-[[(éthylamino)carbonyl]oxy]-1,2,3,5-tétrahydro-3-oxo-2-(sulfooxy)-1,4-méthano-4*H*-2,4-benzodiazépine-5-carboxylate d'éthyle,
• le sel de sodium de trans-1,2,3,5-tétrahydro-3-oxo-2-(sulfooxy)-8-[[trifluorométhyl)sulfonyl]oxy]-1,4-méthano-4*H*-2,4-benzodiazépine-5-carboxylate d'éthyle,
• le sel disodique de trans-1,2,3,5-tétrahydro-3-oxo-2-(sulfooxy)-8-[2-(sulfooxy)éthoxy]-1,4-méthano-4*H*-2,4-benzodiazépine-5-carboxamide,
• le sel de sodium de trans-8-[(2,2-diméthyl-1,3-dioxolan-4-yl)méthoxy]-1,2,3,5-tétrahydro-3-oxo-2-(sulfooxy)-1,4-méthano-4*H*-2,4-benzodiazépine-5-carboxamide.
• Sel de triéthylammonium de trans-2,5,6,8-tétrahydro-6-oxo-2-(2-phényléthyl)-5-(sulfooxy)-4*H*-4,7-méthanopyrazolo[3,4-e][1,3]diazépine-8-carboxylate de méthyle.

Un autre objet de l'invention est un procédé permettant la préparation des composés de formule (I).

Ce procédé se caractérise en ce qu'il comporte :
a) une étape au cours de laquelle on fait réagir, avec un agent de carbonylation, le cas échéant en présence d'une base, un composé de formule (II) : 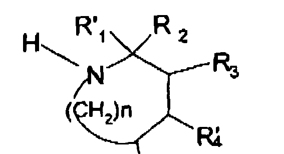 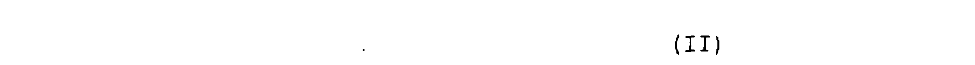 dans laquelle :
   R'₁ représente un atome d'hydrogène, un radical CN, COOH protégé, COOR₉, (CH₂)_{n'}R'₅, CONR₆R₇, 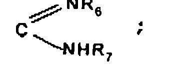
   R₉ est choisi dans le groupe constitué par un radical alkyle renfermant de 1 à 6 atomes de carbone, éventuellement substitué par un ou plusieurs atomes d'halogène ou par un radical pyridyle, un radical -CH₂-alkényle renfermant au total de 3 à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical NO₂, OH protégé, NH₂ protégé, alkyle renfermant de 1 à 6 atomes de carbone, alkoxy renfermant de 1 à 6 atomes de carbone ou par un ou plusieurs atomes d'halogène,
   R'₅ est choisi dans le groupe constitué par un radical OH protégé, CN, NH₂ protégé, CO-NR₆R₇, COOH protégé, COOR₉, OR₉, R₉ étant défini comme ci-dessus,
   n', R₆ et R₇ sont tels que définis ci-dessus,
   R₃ et R'₄ forment ensemble un phényl ou un hétérocycle à caractère aromatique à 5 sommets tel que défini précédemment et éventuellement substitué par un groupement R₁₀, R₁₀ étant choisi dans le groupe constitué par un atome d'hydrogène et les radicaux alkyle renfermant de 1 à 6 atomes de carbone substitué par un ou plusieurs radicaux hydroxy, oxo, halogène ou cyano, ou par un radical alkényle renfermant de 2 à 6 atomes de carbone, halogèno, OH protégé, -OR, OR", R" étant tel que défini précédemment, -(CH₂)_{b}-phényle ou -(CH₂)_{b}-hétérocycle à caractère aromatique, éventuellement substitué, tel que défini précédemment ;
   R₂ représente un atome d'hydrogène,
   ZH représente un groupement HNR'₈-(CH₂)_{n"}-, n" est tel que défini ci-dessus et R'₈ représente un atome d'hydrogène, un radical R₉, OH protégé, OR₉, Y', OY', Y'₁, OY'₁, R₉ étant défini comme précédemment, et m étant égal à 0, 1 ou 2,
   Y' est choisi dans le groupe constitué par les radicaux COH, COR₉, COOR₉, CONH₂, CONHR₉, CONHSO₂R₉, CH₂COOR₉, CH₂tétrazole protégé, CH₂SO₂R₉, CH₂PO(OR₉)₂, CONHOH protégé, CH₂COOH protégé, CH₂CONHOH protégé, CH₂SO₃ protégé, CH₂PO(OR)(OH) protégé, CH₂PO(R) (OH) protégé et CH₂PO(OH)₂ protégé,
   Y'₁ est choisi dans le groupe constitué par les radicaux SO₂R₉, SO₂NHCOH, SO₂NHCOR₉, SO₂NHCOOR₉, SO₂NHCONH₂, SO₂NHCONHR₉ et SO₃H protégé,
   et n est égal à 1 ;
   en vue d'obtenir un composé intermédiaire de formule : 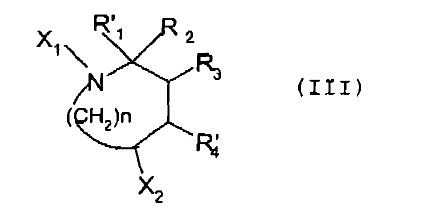 dans laquelle :
   R'₁, R₂, R₃, R'₄ et n ont les mêmes significations que ci-dessus et soit X₁ est un atome d'hydrogène et X₂ représente un groupement -Z-CO-X₃, X₃ représentant le reste de l'agent de carbonylation, à savoir un reste Cl, OCCl₃, OAr, OCH₂Ar, OAlk, OAlkenyl, OCOOAlk et imidazolyl, soit X₂ est un groupement -ZH et X₁ représente un groupement CO-X₃, X₃ étant défini comme ci-dessus ;
b) une étape au cours de laquelle on cyclise l'intermédiaire obtenu précédemment, en présence d'une base ;
   et en ce que :
c) le cas échéant, l'étape a) est précédée et/ou l'étape b) est suivie de l'une ou de plusieurs des réactions suivantes, dans un ordre approprié :
   - protection des fonctions réactives ;
   - déprotection des fonctions réactives;
   - estérification ;
   - saponification ;
   - sulfatation ;
   - phosphatation ;
   - amidification ;
   - acylation ;
   - sulfonylation ;
   - alkylation ;
   - formation d'un groupe urée ;
   - réduction d'acides carboxyliques ;
   - réduction de cétones et d'aldéhydes en alcools ;
   - salification ;
   - échange d'ions ;
   - dédoublement ou séparation de diastéréoisomères ;
   - oxydation de sulfure en sulfoxyde et/ou sulfone ;
   - oxydation d'aldéhyde en acide ;
   - oxydation d'alcool en cétone ;
   - halogénation ou déshalogénation ;
   - carbamoylation ;
   - carboxylation ;
   - introduction d'un groupe azido ;
   - réduction d'un azido en amine ;
   - réactions de couplage d'halogénures ou de triflates aromatiques ou hétéroaromatiques ou d'azotes hétérocycliques avec des acides aryl ou hétéroaryl boroniques ;
   - réactions de couplage d'halogénures ou de triflates aromatiques ou hétéroaromatiques avec des réactifs stannylés ;
   - hydrogénation de doubles liaisons ;
   - dihydroxylation de doubles liaisons ;
   - cyanuration.

Comme agent de carbonylation, on peut mettre en oeuvre un réactif tel que le phosgène, le diphosgène, le triphosgène, un chloroformiate d'aryle tel que le chloroformiate de phényle ou de p-nitrophényle, un chloroformiate d'aralkyle tel que chloroformiate de benzyle, un chloroformiate d'alkyle ou d'alkényle tel que le chloroformiate de méthyle ou d'allyle, un dicarbonate d'alkyle tel que le dicarbonate de tert-butyle, le carbonyl-diimidazole et leurs mélanges.

La réaction a lieu de préférence en présence d'une base ou d'un mélange de bases qui neutralise l'acide formé. Elle peut notamment être une amine telle que la triethylamine, la diisopropyléthylamine, la pyridine, la diméthylaminopyridine. Toutefois, on peut également opérer en utilisant le produit de départ de formule II comme base. On en utilise alors un excès.

Le cas échéant, le produit de formule II est mis en oeuvre sous la forme d'un sel d'acide, par exemple un chlorhydrate ou un trifluoroacétate.

Comme base dans l'étape b), on peut également utiliser les amines, ou encore les hydrures, les alcoolates, les amidures ou carbonates de métaux alcalins ou alcalino-terreux.

Les amines peuvent être choisies par exemple dans la liste ci-dessus.

Comme hydrure on peut notamment utiliser l'hydrure de sodium ou de potassium.

Comme alcoolate de métal alcalin, on utilise de préférence le t-butylate de potassium.

Comme amidure de métal alcalin on peut notamment utiliser le bis(triméthylsilyl) amidure de lithium.

Comme carbonate, on peut notamment utiliser le carbonate ou bicarbonate de sodium ou de potassium.

Le cas échéant, l'intermédiaire de formule III peut être obtenu sous forme d'un sel d'acide généré lors de la réaction de carbonylation et notamment un chlorhydrate. Il est ensuite mis en oeuvre dans la réaction de cyclisation sous cette forme.

Le cas échéant, la cyclisation peut être effectuée sans isolement de l'intermédiaire de formule III.

Les réactions mentionnées à l'étape c) sont d'une manière générale des réactions classiques, bien connues de l'homme du métier.

Les fonctions réactives qu'il convient, le cas échéant, de protéger sont les fonctions acides carboxyliques, amines, amides et hydroxy.

La protection de la fonction acide est notamment effectuée sous forme d'esters d'alkyle, d'esters allyliques, de benzyle, benzhydryle ou p-nitrobenzyle.

La déprotection est effectuée par saponification, hydrolyse acide, hydrogénolyse, ou encore clivage à l'aide de complexes solubles du Palladium O.

La protection des amines, des azotes hétérocycliques et des amides est notamment effectuée, selon les cas, sous forme de dérivés benzylés ou tritylés, sous forme de carbamates, notamment d'allyle, benzyle, phényle ou tertbutyle, ou encore sous forme de dérivés silylés tels que les dérivés tertbutyle diméthyl, triméthyl, triphényl ou encore diphényl tertbutyl-silyle, ou de dérivés phénylsulfonylalkyle ou cyanoalkyle.

La déprotection est effectuée, selon la nature du groupement protecteur, par le sodium ou le lithium dans l'ammoniac liquide, par hydrogénolyse ou à l'aide de complexes solubles du Palladium O, par action d'un acide, ou par action du fluorure de tétrabutylammonium ou de bases fortes telles que l'hydrure de sodium ou le t.butylate de potassium.

La protection des hydroxylamines est effectuée notamment sous forme d'éthers de benzyle ou d'allyle.

Le clivage des éthers est effectué par hydrogénolyse ou à l'aide de complexes solubles du Palladium O.

La protection des alcools et des phénols est effectuée de manière classique, sous forme d'éthers, d'esters ou de carbonates. Les éthers peuvent être des éthers d'alkyle ou d'alkoxyalkyle, de préférence des éthers de méthyle ou de méthoxyéthoxyméthyle, des éthers d'aryle ou de préférence d'aralkyle, par exemple de benzyle, ou des éthers silylés, par exemple les dérivés silylés cités plus haut. Les esters peuvent être n'importe quel ester clivable connu de l'homme du métier et de préférence l'acétate, le propionate ou le benzoate ou p-nitrobenzoate. Les carbonates peuvent être par exemple des carbonates de méthyle, tertbutyle, allyle, benzyle ou p-nitrobenzyle.

La déprotection est effectuée par les moyens connus de l'homme du métier, notamment la saponification, l'hydrogénolyse, le clivage par des complexes solubles du Palladium O, l'hydrolyse en milieu acide ou encore, pour les dérivés silylés, le traitement par le fluorure de tétrabutylammmonium.

Des exemples de protections et déprotections des fonctions réactives sont fournis dans la partie expérimentale.

La réaction de sulfatation est effectuée par action des complexes SO₃-amines tels que SO₃-pyridine ou SO₃-diméthylformamide, en opérant dans la pyridine, le sel formé, par exemple le sel de pyridine, pouvant ensuite être échangé par exemple par un sel d'une autre amine, d'un ammonium quaternaire ou d'un métal alcalin. Des exemples sont fournis dans la partie expérimentale.

La réaction de phosphatation est effectuée par exemple par action d'un chlorophosphate tel que le diméthyl, dibenzyl ou diphényl chlorophosphate.

La réaction d'amidification est effectuée au départ de l'acide carboxylique à l'aide d'un agent d'activation tel qu'un chloroformiate d'alkyle, l'EDCI ou le BOP par action de l'ammoniaque ou d'une amine ou alkoxylamine appropriée ou de leurs sels d'acides. Des exemples sont fournis ci-après dans la partie expérimentale.

Les réactions d'acylation et de sulfonylation sont effectuées sur les alcools, les amines ou les azotes hétérocycliques, par action selon les cas, d'un halogènure ou d'un anhydride d'acide carboxylique ou d'acide sulfonique approprié, le cas échéant en présence d'une base. Plusieurs exemples sont fournis ci-après dans la partie expérimentale.

La réaction d'alkylation, laquelle dans le contexte est entendue au sens large et concerne l'introduction de groupements alkyles substitués diversement comme décrit plus haut, notamment dans la définition de R', est effectuée par action sur les dérivés hydroxylés, les énolates d'esters ou de cétones, les amines ou les azotes hétérocycliques, selon les cas, d'un sulfate d'alkyle ou d'un halogènure d'alkyle ou d'alkyle substitué. Des illustrations sont fournies ci-après dans la partie expérimentale.

La réduction d'acides en alcools peut être effectuée par action d'un borane ou via un anhydride mixte intermédiaire, par action d'un borohydrure alcalin. L'anhydride mixte est préparé par exemple à l'aide d'un chloroformiate d'alkyle. La réduction de cétone ou d'aldéhyde en alcool est de préférence effectuée par action de borohydrure de sodium. Des illustrations sont fournies dans la partie expérimentale.

La déshydratation d'amide en nitrile peut intervenir dans les conditions des réactions de carbonylation et cyclisation.

L'oxydation des sulfures en sulfoxyde et/ou sulfone peut être effectuée par action d'un peracide tel que l'acide métachloroperbenzoïque ou perphtalique ou de tout autre réactif connu de l'homme du métier.

La salification par les acides est le cas échéant réalisée par addition d'un acide en phase soluble au composé. La salification par les bases peut concerner les composés comportant une fonction acide et notamment les composés comportant une fonction carboxy, ceux comportant une fonction sulfooxy ou dérivée de l'acide phosphorique ou ceux comportant un hétérocycle à caractère acide.

Dans le cas d'une fonction carboxy, on opère par addition d'une base appropriée telle que celles citées précédemment. Dans le cas d'une fonction sulfooxy ou dérivée de l'acide phosphorique, on obtient directement le sel de pyridinium lors de l'action du complexe S03-pyridine et on obtient les autres sels à partir de ce sel de pyridinium. Dans l'un ou l'autre cas, on peut encore opérer par échange d'ions sur résine. Des exemples de salifications par les acides ou par les bases et y compris d'hétérocycle à caractère acide, figurent ci-après dans la partie expérimentale.

L'oxydation d'aldéhyde en acide peut être effectuée par action du permanganate de potassium ou du chlorite de sodium.

L'oxydation d'alcool en cétone peut être effectuée par action du chlorochromate de pyridinium.

Par halogénation on entend introduction d'un substituant halogéné à partir d'un hydroxy ou halogénation directe d'un cycle aromatique ou hétéroaromatique. Selon le cas, la réaction peut par exemple être mise en oeuvre par action d'iode ou en présence de triphénylphosphine, par action de brome dans l'acide acétique ou encore d'iode en présence de C₆H₅I(OCOCF₃)₂, ou encore par réaction d'un réactif halogéné électrophile tel que le N-fluorosulfonylimide en présence d'une base forte. De tels réactifs sont connus de l'homme du métier et des exemples figurent ci-après dans la partie expérimentale.

La déshalogénation peut être effectuée par hydrogénolyse.

La réaction de carbamoylation peut être réalisée par la mise en oeuvre d'un chloroformiate ou du diphosgène puis d'une amine ou, le cas échéant, d'ammoniac, ou encore par la mise en oeuvre d'un isocyanate approprié.

La réaction de carboxylation peut être réalisée par action d'un alkyllithié puis de gaz carbonique ou d'un chloroformiate.

L'introduction d'un groupe azido peut être effectuée par exemple par action d'azoture de sodium sur un intermédiaire de type mésylate ou iodo.

La réduction d'un groupe azide peut être effectuée par action de trialkyl ou triarylphosphine.

La réaction de couplages d'halogénures aromatiques avec des dérivés de l'étain est effectuée par une méthode dite de Stille. Une illustration est donnée dans la partie expérimentale.

L'hydrogénation de doubles liaisons, lesquelles peuvent être des liaisons carbone-carbone ou carbone-azote sont effectuées par les méthodes connues de l'homme du métier.

La dihydroxylation de double liaison carbone-carbone est effectuée notamment par action de tétroxide d'osmium.

Le clivage des diols est de préférence réalisé par le périodate de sodium.

L'introduction d'un cyano est réalisée par substitution nucléophile à l'aide d'un cyanure alcalin.

Des illustrations de ces réactions figurent ci-après dans la partie expérimentale.

La séparation des énantiomères et diastéréoisomères peut être réalisée selon les techniques connues de l'homme du métier, notamment la chromatographie.

Outre via les procédés décrits précédemment, des composés de formule (I) peuvent bien entendu être obtenus par des méthodes qui utilisent au départ un composé de formule (II) dans laquelle R'1, R'2, R3, R'4 et ZH ont les valeurs qui conduisent directement (sans transformation) à celles des composés que l'on souhaite préparer. Le cas échéant, celles de ces valeurs qui renfermeraient des fonctions réactives telles que mentionnées plus haut sont alors protégées, la déprotection intervenant à l'issue de l'étape de cyclisation b ou à tout autre moment opportun dans la synthèse. Les protections et déprotections sont alors réalisées comme décrit ci-dessus.

De telles méthodes sont fournies ci-après dans la partie expérimentale.

L'invention a encore pour objet un procédé selon ce qui précède, selon lequel le composé de formule (II) dans laquelle ZH représente un groupement HNR'₈-(CH₂)ₙ"- dans lequel n" est égal à O, est obtenu par un procédé caractérisé en ce que l'on traite un composé de formule (IV) : 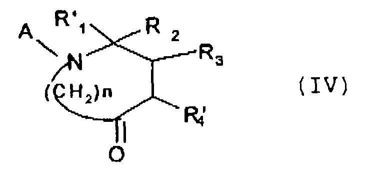 dans laquelle R'₁, R₂ et n sont définis comme précédemment, R₃ et R'₄ ont les valeurs définies précédemment ou bien des valeurs précurseurs des valeurs définies précédemment et A représente un atome d'hydrogène ou un groupement protecteur de l'azote, par un agent de réduction, pour obtenir un composé de formule (V) : 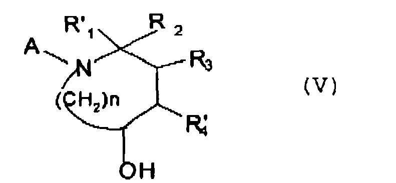 dans laquelle A, R'₁, R₂, R₃, R'₄ et n conservent leur signification précitée, dans lequel, le cas échéant, l'on remplace le groupement OH par un groupe partant, pour obtenir un composé de formule (VI) : 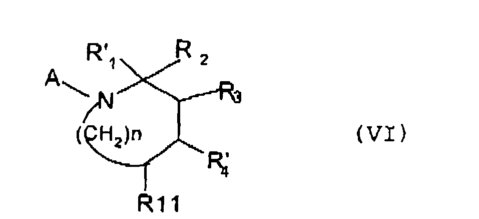 dans laquelle A, R'₁, R₂, R₃, R'₄ et n conservent leur signification précitée et R₁₁ représente un groupe partant, puis traite par un composé de formule Z₁H₂ dans laquelle Z₁ représente un groupement divalent -NR'₈-, R'₈ conservant la signification précitée, pour obtenir un composé de formule (VIII) ou (VIII') : 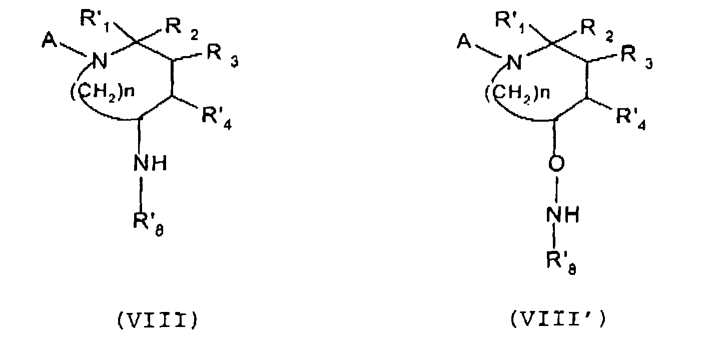 dans laquelle A, R'₁, R₂, R₃, R'₁, n" et R'₈ sont définis comme précédemment, puis, le cas échéant, par un agent de déprotection de l'atome d'azote approprié, et en ce que, le cas échéant, l'on soumet l'intermédiaire de formule (IV), (V), (VIII) ou (VIII'), à une ou plusieurs des réactions décrites à l'étape c) du procédé décrit plus haut, dans un ordre approprié.

L'invention a encore pour objet un procédé selon ce qui précède, selon lequel le composé de formule (II) dans laquelle ZH représente un groupement NHR'₈-(CH₂)ₙ"- dans lequel n" est égal à 0 est obtenu par un procédé caractérisé en ce que l'on traite un composé de formule (IV) telle que définie précédemment, par un composé de formule H₂NR'₆, pour obtenir un composé de formule (VII) : 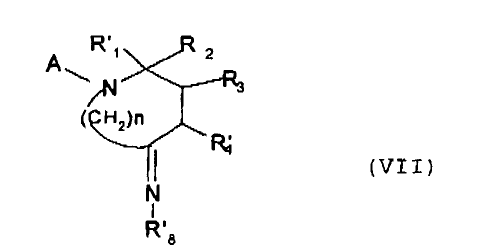 dans laquelle A, R'₁, R₂, n et R'₈ sont définis comme précédemment et R₃ et R₄ ont les valeurs définies précédemment ou bien des valeurs précurseurs des valeurs définies précédemment, que l'on fait réagir avec un agent de réduction pour obtenir un composé de formule (VIII) : 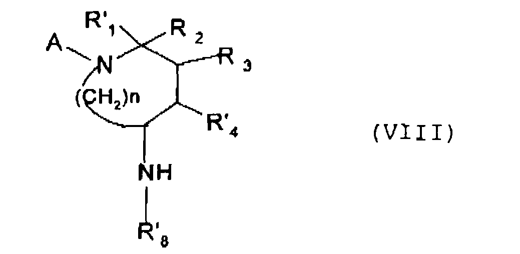 dans laquelle A, R'₁, R₂, R₃, R'₄, n" et R'₈ sont définis comme précédemment, que l'on traite, le cas échéant, par un agent de déprotection de l'atome d'azote approprié, et en ce que, le cas échéant, l'on soumet l'intermédiaire de formule (VII) ou (VIII) à une ou plusieurs des réactions décrites à l'étape c) du procédé décrit plus haut, dans un ordre approprié.

Les composés de formule (II) dans laquelle ZH représente un groupement HO-(CH₂)_{n"} dans lequel n" est égal à 1 peuvent être obtenus selon les méthodes décrites par exemple par S. Shiotani et al. Chem. Pharm. Bull. 15(1)88-93 (1967) (composé "IV" p. 89) ou encore par N, Itoh. Chem. Pharm. Bull 16 (3)455-470 (1968) (composé "XVIII" p. 461) en utilisant un composé de départ approprié. Les composés de formule (II) dans laquelle ZH représente un groupement NHR'₈-(CH₂)_{n"} dans lequel n" est égal à 1 peuvent être obtenus au départ des composés ci-dessus par un procédé identique à celui qui est décrit plus haut pour la préparation des composés dans lesquels n" = 0.

Le groupement protecteur de l'azote est notamment l'un de ceux qui sont cités plus haut.

L'agent de réduction est notamment un borohydrure alcalin.

Le groupe partant est notamment un phosphate ou un sulfonate, par exemple un mésylate ou un tosylate, obtenu par action de chlorure de sulfonyle correspondant en présence d'une base, ou un halogène, plus particulièrement un chlore, un brome ou un iode, obtenu par exemple par action du chlorure de thionyle ou de P(C₆H₅)₃CBr₄ ou PBr₃ ou, dans le cas d'un atome d'iode, par action d'un iodure alcalin sur un sulfonate.

L'agent de déprotection est notamment l'un de ceux mentionnés plus haut.

L'agent de réduction que l'on fait agir sur le composé de formule (VII) est notamment un cyano ou un acétoxyborohydrure de sodium.

Pour des raisons pratiques ou liées à la nature des réactions impliquées, il peut être nécessaire ou souhaitable d'effectuer sur les intermédiaires de formule (IV), (V) ou (VII) une ou plusieurs des réactions décrites à l'étape c) du procédé défini plus haut. Il est entendu que ces réactions sont effectuées dans les conditions qui ont été définies précédemment.

Les produits de formule générale (I) possèdent une très bonne activité antibiotique sur les bactéries gram(+) telles que les staphylocoques ou les streptocoques. Leur efficacité sur les bactéries gram(-) notamment sur les entérobactéries est particulièrement notable.

Ces propriétés rendent aptes lesdits produits ainsi que leurs sels d'acides et de bases pharmaceutiquement acceptables à être utilisés comme médicaments dans le traitement des affections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érysipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

Les composés de formule générales (I) sont par ailleurs doués de propriétés inhibitrices de béta-lactamases, et présentant par là de l'intérêt dans la lutte contre les maladies infectieuses ou la prévention de celles-ci, sous forme d'association avec divers composés antibiotiques de type β-lactamines, afin de renforcer leur efficacité dans la lutte contre les bactéries pathogènes productrices de β-lactamases.

Il est bien connu que l'inactivation enzymatique des antibiotiques de type β-lactamines, que ce soit des composés de type pénicillines ou céphalosporines, dans le traitement des infections bactériennes est un obstacle pour ce type de composés. Cette inactivation consiste en un processus de dégradation des β-lactamines et constitue l'un des mécanismes pour lesquels les bactéries peuvent devenir résistantes aux traitements. Il est donc souhaitable de parvenir à contrer ce processus enzymatique en associant à l'agent antibactérien de type β-lactamines un agent susceptible d'inhiber l'enzyme. Lorsqu'un inhibiteur de β-lactamase est utilisé en combinaison avec un antibiotique de type β-lactamines, il peut donc renforcer son efficacité contre certains microorganismes.

La présente invention a donc également pour objet, à titre de médicaments et notamment de médicaments destinés au traitement des infections bactériennes chez l'homme ou l'animal et de médicaments destinés à inhiber la production des β-lactamases par les bactéries pathogènes, les composés de formule (I) tels que définis ci-dessus ainsi que leurs sels avec les acides et les bases pharmaceutiquement acceptables, et notamment parmi ceux-ci, ceux dans lesquels R' est choisi dans le groupe constitué par les radicaux -(O)ₐ-(CH₂)_{b}-(O)ₐ-CONR₆R₇, - (O)ₐ-(CH₂)_{b}-OSO₃H, -(O)ₐ-(CH₂)_{b}-SO₃H, -(O)ₐ-SO₂R,-(O)ₐ-SO₂-CHal₃, -(O)ₐ-(CH₂)_{b}-NR₆R₇, (O)ₐ-CH₂)_{b}-NHCOOR, -(CH₂)_{b}-COOH, -(CH₂)_{b}-COOR, -OR", OH, -(CH₂)_{b}-phényle et -(CH₂)_{b}-hétérocycle à caractère aromatique à 5 ou 6 sommets renfermant de 1 à 4 hétéroatomes choisis parmi l'azote, l'oxygène et le soufre, le phényle et l'hétérocycle étant éventuellement substitués par un ou plusieurs halogènes, alkyle renfermant de 1 à 6 atomes de carobne, alkoxy renfermant de 1 à 6 atomes de carbone, alkoxy renfermant de 1 à 6 atomes de carbone, alkoxy renfermant de 1 à 6 atomes de arbone ou CF₃, R, R₆ et R₇ étant tels que définis à la revendication 1, R" étant choisi dans le groupe constitué par les radicaux alkyles renfermant de 1 à 6 atomes de carbone substitués par un ou plusieurs radicaux hydroxy, oxo, halogène ou cyano, a étant égal à 0 ou 1 et b étant un entier de 0 à 6, étant entendu que:
(i) lorsque R' est OH ou OR" avec R" étant un radical alkyle renfermant de 1 à 6 atomes de carbone substitués par un ou plusieurs radicaux oxo, alors R₁ représente le radical CONR₆R₇ dans lequel R₆ ou R₇ est un alkoxy renfermant de 1 à 6 atomes de carbone, ou bien
(j) lorsque R' est un radical -(O)ₐ-(CH₂)_{b}-NR₆R₇, dans lequel R₆ et R₇ sont tels que définis à la revendication 1 alors a est égal à 0 ou 1 et b est un entier de 1 à 6 ou bien
(k) a est égal à 0 ou 1 et b est un entier de 0 à 6 lorsque R' est un radical -(O)ₐ-(CH₂)_{b}-NR₆R₇, dans lequel R₆ et R₇ sont individuellement choisis dans le groupe constitué par un radical alkyle renfermant de 1 à 6 atomes de carbone, alkoxy renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle, ou bien encore
(1) lorsque R' est choisi parmi le groupe constitué par les radicaux -(O)ₐ-(CH₂)_{b}-NH-COOR, -(CH₂)_{b}-COOH, -(CH₂)_{b}-COOR alors b est un entier de 1 à 6 et a vaut 0 ou 1 ;
les autres valeurs étant défini comme précédemment.

L'invention a tout particulièrement pour objet, à titre de médicaments, les produits de formule (I) dans lesquels R₃ et R₄ forment ensemble un phényle ou un thiényle ou un pyrazolyle substitué par un substituant R' tel que défini précédemment.

Parmi, ces derniers, on cite en particulier ceux dans lesquels R₁ est choisi dans le groupe constitué par l'atome d'hydrogène et les groupements COOCH₃, COOC₂H₅, CONH₂, CONHCH₃ et CONHOCH₃.

Parmi les composés de formule (I), l'invention a encore notamment pour objet, à titre de médicaments, les produit de formule (I) dans lesquels R₈ est un groupement OY dans lequel Y est choisi parmi les groupements CH₂COOH, CH₂COOR, CHF-COOH, CHF-COOR, CF2-COOH, CF2-COOR, CH₂CONHOH, CH₂CONHCN, CH₂ tétrazole, CH₂ tétrazole protégé, CH₂SO₃H, CH₂SO₂R, CH₂PO(OR)₂, CH₂PO(OR)(OH), CH₂PO(R)(OH) et CH₂PO(OH)₂ ou OY₁, dans lequel Y₁ est choisi parmi les groupements SO₂R, SO₂NHCOR, SO₂NHCOOR, SO₂NHCONHR et SO₃H, R étant tel que défini plus haut, ainsi que ceux dans lesquels R' est choisi dans le groupe constitué par - O-CH₂-CHOH-CH₂OH, -CH₂-CH₂NH₂, -CO-NH₂, -CO-NHPhényl, -CH₂-(pOCH₃-Phényl) et Phényl éventuellement substitué par CH₃, C₂H₅, F et CF₃.

Parmi les composés de formule (I), l'invention a tout particulièrement pour objet, à titre de médicaments, les composés dont les noms suivent :
• le sel de triéthylammonium de 5,6-dihydro-6-oxo-N²-phényl-5(sulfooxy)-4*H*-4,7-méthanopyrazolo[3,4-e][1,3]diazépine-2,8(8*H*)-dicarboxamide,
• le sel de sodium de 4,5,6,8-tétrahydro-6-oxo-5-(sulfooxy)-1*H*-4,7-méthanopyrazolo[3,4-e][1,3]diazépine - 1-carboxamide,
• le sel de sodium de 1,4,5,8-tétrahydro-1-[(4-méthoxyphényl)méthyl]-5-(sulfooxy)-6*H*-4,7-méthanopyrazolo[3,4-e][1,3]diazépin-6-one,
• le sel de sodium de trans-4,5,6,8-tétrahydro-2-(2-méthylphényl)-6-oxo-5-(sulfooxy)-4,7-méthano-7*H-*thiéno[2,3-e][1,3]diazépine-8-carboxamide,
• le sel de sodium de trans-4,5,6,8-tétrahydro-6-oxo-5-(sulfooxy)-2-[2-(trifluorométhyl)phényl]-4,7-méthano-7*H-*thiéno[2,3-e][1,3]diazépine-8-carboxamide,
• le sel de sodium de trans-2-(2-éthylphényl)-4,5,6,8-tétrahydro-6-oxo-5-(sulfooxy)-4,7-méthano-7*H*-thiéno[2,3-e][1,3]diazépine-8-carboxamide,
• le sel de sodium de trans-8-(2,3-dihydroxypropoxy)-1,2,3,5-tétrahydro-3-oxo-2-(sulfooxy)-1,4-méthano-4*H*-2,4-benzodiazépine-5-carboxamide,
• le sel de sodium de trans-3-(4-fluorophényl)-4,6,7,8-tétrahydro-6-oxo-7-(sulfooxy)-5,8-méthano-5*H*-thiéno[2,3-e][1,3]diazépine-4-carboxylate d'éthyle,
• le sel de sodium de trans-2,5,6,8-tétrahydro-6-oxo-2-phényl-5-(sulfooxy)-4*H*-4,7-méthanopyrazolo[3,4-e][1,3]diazépine-8-carboxamide,
• le sel de sodium de 1,4,5,8-tétrahydro-1-phényl-5-(sulfooxy)-6*H*-4,'7-méthanopyrazolo[3,4-e][1,3]diazépin-6-one,
• le sel de sodium de trans-4,5,6,8-tétrahydro-6-oxo-1-phényl-5(sulfooxy)-1*H*-4,7-méthanopyrazolo[3,4-e][1,3] diazépine-8-carboxamide,
• le sel de triéthylammonium de trans-2,5,6,8-tétrahydro-6-oxo-2-(phénylméthyl)-5-(sulfooxy)-4*H*-4,7-méthanopyrazolo[3,4-e][1,3]diazépine-8-carboxylate de méthyle,
• le sel de triéthylammonium de trans-4,5,6,8-tétrahydro-6-oxo-1-(2-phényléthyl)-5-(sulfooxy)-1H-4,7-méthanopyrazolo[3,4-e][1,3]diazépine-8-carboxylate de méthyle,
• le sel de triéthylammonium de trans-4,5,6,8-tétrahydro-8-(méthoxycarbonyl)-6-oxo-5-(sulfooxy)-1H-4,7-méthanopyrazolo[3,4-e][1,3]diazépine-1-acétate d'éthyle,
• le sel de triéthylammonium de trans-5,6-dihydro-8-(méthoxycarbonyl)-6-oxo-5-(sulfooxy)-4H-4,7-méthanopyrazolo[3,4-e][1,3]diazépine-2(8H)-acétate d'éthyle,
• le sel de di-(triéthylammonium) de l'acide trans-5,6-dihydro-8-(méthoxycarbonyl)-6-oxo-5-sulfooxy-4H-4,7-méthanopyrazolo[3,4-e][1,3]diazépine-2(8H)-acétique,
• le sel de pyridinium de trans-1-(aminocarbonyl)-4,5,6,8-tétrahydro-6-oxo-5-(sulfooxy)-1H-4,7-méthanopyrazolo[3,4-e][1,3]diazépine-8-carboxylate de méthyle,
• le sel de pyridinium de trans-2-(aminocarbonyl)-2,5,6,8-tétrahydro-6-oxo-5-(sulfooxy)-4H-4,7-méthanopyrazolo[3,4-e][diazépine-8-carboxylate de méthyle,
• le sel de sodium de trans-2-(4-fluorophényl)-4,5,6,8-tétrahydro-6-oxo-5-(sulfooxy)-4,7-méthano-7H-thiéno[2,3-e][1,3]diazépine-8-carboxylate de méthyle,
• le sel de sodium de trans-2(aminocarbonyl)-4,5,6,8-tétrahydro-6-oxo-5-(sulfooxy)-4,7-méthano-7H-thiéno[2,3-e][1,3]diazépine-8-carboxylate de méthyle,
• le sel de sodium de trans-1,2,3,5-tétrahydro-3-oxo-9-[[(phénylamino)carbonyl]oxy]-2-(sulfooxy)-1,4-méthano-4H-2,4-benzodiazépine-5-carboxylate d'éthyle,
• le sel de sodium de trans-1,2,3,5-tétrahydro-N-méthoxy-8-[(2 méthoxyéthoxy)méthoxy]-3-oxo-2-(sulfoxy)-1,4-méthano-4H-2,4-benzodiazépine-5-carboxamide,
• le sel de sodium de trans-1,2,3,5-tétrahydro-3-oxo-8-[[(phénylamino)carbonyl]oxy]-2-(sulfooxy)-1,4-méthano-4H-2,4-benzodiazépine-5-carboxylate d'éthyle,
• le sel de sodium de trans-8-[[(éthylamino)carbonyl]oxy]-1,2,3,5-tétrahydro-3-oxo-2-(sulfooxy)-1,4-méthano-4H-2,4-benzodiazépine-5-carboxylate d'éthyle,
• le sel de sodium de trans-1,2,3,5-tétrahydro-3-oxo-2-(sulfooxy)-8-[[trifluorométhyl)sulfonyl]oxy]-1,4-méthano-4H-2,4-benzodiazépine-5-carboxylate d'éthyle,
• le sel disodique de trans-1,2,3,5-tétrahydro-3-oxo-2-(sulfooxy)-8-[2-(sulfooxy)éthoxy]-1,4-méthano-4H-2,4-benzodiazépine-5-carboxamide,
• le sel de sodium de trans-8-[(2,2-diméthyl-1,3-dioxolan-4-yl)méthoxy]-1,2,3,5-tétrahydro-3-oxo-2-(sulfooxy)-1,4-méthano-4H-2,4-benzodiazépine-5-carboxamide,
• Sel de triéthylammonium de trans-2,5,6,8-tétrahydro-6-oxo-2-(2-phényléthyl)-5-(sulfooxy)-4*H*-4,7-méthanopyrazolo[3,4-e][1,3]diazépine-8-carboxylate de méthyle.

L'antibiotique de type β-lactamines auquel peut-être associé le composé de formule (I) peut être choisi dans le groupe constitué par les pénames, les pénèmes, les carbapénèmes, les céphèmes, les carbacéphèmes, les oxacéphèmes, les céphamycines et les monobactames.

Par β-lactamines, on entend par exemple les pénicillines telles que amoxicilline, ampicilline, azlocilline, mezlocilline, apalcilline, hetacilline, bacampicilline, carbenicilline, sulbenicilline, ticarcilline, piperacilline, azlocilline, mecillinam, pivmecillinam, methicilline, ciclacilline, talampicilline, aspoxicilline, oxacilline, cloxacilline, dicloxacilline, flucloxacilline, nafcilline ou pivampicilline, les céphalosporines telles que céphalothine, céphaloridine, céfaclor, céfadroxile, céfamandole, céfazoline, céphalexine, céphradine, ceftizoxime, céfoxitine, céphacétrile, céfotiam, céfotaxime, cefsulodine, céfopérazone, ceftizoxime, cefménoxime, cefmétazole, céphaloglycine, céfonicide, céfodizime, cefpirome, ceftazidime, ceftriaxone, cefpiramide, cefbupérazone, cefozopran, céfépime, céfoselis, céfluprenam, céfuzonam, cefpimizole, cefclidine, céfixime, ceftibutène, cefdinir, cefpodoxime axétil, cefpodoxime proxétil, ceftéram pivoxil, céfétamet pivoxil, cefcapène pivoxil ou cefditoren pivoxil, céfuroxime, céfuroxime axétil, loracarbacef, latamoxef, les carbapénèmes tels que imipénème, méropénème, biapénème ou panipénème et les monobactames tels que l'aztréonam et le carumonam, ainsi que leur sels.

Les composés de formule (I) ou leurs sels pharmaceutiquement acceptables, peuvent être administrés en même temps que la prise d'antibiotiques de type β-lactamines, ou séparément, de préférence après celle-ci. Cela peut s'effectuer sous forme d'un mélange des deux principes actifs ou sous forme d'une association pharmaceutique des deux principes actifs séparés.

La posologie des composés de formule (I) et de leurs sels pharmaceutiquement acceptables peut bien entendu varier dans de larges limites et doit naturellement être adaptée, dans chaque cas particulier, aux conditions individuelles et à l'agent pathogène à combattre. En général, pour une utilisation dans le traitement des infections bactériennes, la dose journalière peut être comprise entre 0,250 g et 10 g par jour, par voie orale chez l'homme, avec le produit décrit à l'exemple 24 ou 45 ou encore comprise entre 0,25 g et 10 g par jour par voie intramusculaire ou intraveineuse. Pour une utilisation comme inhibiteur de β-lactamase, une dose journalière chez l'homme pouvant aller de 0,1 à environ 10 g peut convenir.

Par ailleurs, le rapport de l'inhibiteur de β-lactamase de formule (I) ou du sel pharmaceutiquement acceptable de celui-ci à l'antibiotique de type β-lactamines peut également varier dans de larges limites et doit être adapté, dans chaque cas particulier, aux conditions individuelles. En général, un rapport allant d'environ 1:20 à environ 1:1 devrait être indiqué.

Les médicaments antibiotiques ou inhibiteurs de β-lactamases tels que définis plus haut sont mis en oeuvre sous forme de compositions pharmaceutiques en mélange avec un excipient pharmaceutique inerte, organique ou minéral, adapté au mode d'administration recherché, et l'invention a également pour objet les compositions pharmaceutiques renfermant comme principe actif, au moins un des composés de l'invention tels que définis plus haut et les compositions contenant comme principe actif, au moins un des composés de l'invention tels que définis plus haut et au moins un médicament de type β-lactamines.

Ces compositions peuvent être administrées par voie buccale, rectale, parentérale, notamment intramusculaire, ou par voie locale en application topique sur la peau et les muqueuses.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'un lyophilisat destiné à être dissout extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 10 g par jour, par voie orale chez l'homme, avec le produit décrit à l'exemple 1 ou encore comprise entre 0,25 g et 10 g par jour par voie intramusculaire ou intraveineuse.

Les produits de formule (I) peuvent également être utilisés comme désinfectants des instruments chirurgicaux.

L'invention a enfin pour objet, à titre de produits industriels nouveaux et notamment à titre de produits intermédiaires nécessaires à la préparation des produits de formule (I) :
- les produits de formule (III) telle que définie précédemment dans laquelle R₃ et R'₄ sont tels que définis précédemment, les groupements protecteurs apparaissent dans les définitions ci-dessous étant choisis comme suit :
   - pour une fonction acide, les restes d'esters d'alkyle, d'allyle, de benzyle, de benhydryle et de p-nitrobenzyle,
   - pour OH, les restes d'éthers d'alkyle, d'alkoxyalkyle, d'aryle et d'aralkyle, les restes d'éthers silylés choisi parmi les éthers de tertbutyldiméthyl, triméthyl, triphényl et diphényltertbutyl-silyle, les restes d'esters clivables choisis parmi l'acétate, le propionate, le benzoate et le p-nitrobenzoate, les retes de carbonates de méthyle, terbutyle, allyle, benzyle et p-nitrobenzyle,
   - pour NH₂, les dérivés benzylés et tritylés, les carbamates d'allyle, de benzyle, phényle et tertbutyle, les dérivés silylés choisis parmi les dérivés tertbutyle diméthyl, triméthyl, triphényl et diphényl tertbutyl-silyle, et les dérivés phénylsulfonylalkyle et cyanoalkyle,
   - pour les hydroxylamines, les éthers de benzyle et d'allyle, ainsi que leurs sels avec les acides et notamment leurs chlorhydrates et trifluoroacétates ;
- les produits de formule (III) telle que définie précédemment, dans laquelle R'₁ représente un radical CONR₆R₇ dans lequel R₆ ou R₇ représente un radical alkoxy renfermant de 1 à 6 atomes de carbone, l'ensemble des autres valeurs étant définies comme précédemment, ainsi que leurs sels avec les acides et notamment leurs chlorhydrates et trifluoroacétates ;
- les produits de formule (II) telle que définie précédemment, dans laquelle R₃ et R'₄ et l'ensemble des autres substituants sont tels que définis précédemment, ainsi que leurs sels avec les acides et notamment leurs chlorhydrates et trifluoroacétates ;
- les produits de formule (II) telle que définie précédemment, dans laquelle R'₁ représente un radical CONR₆R₇ dans lequel R₆ ou R₇ représente un radical alkoxy renfermant de 1 à 6 atomes de carbone, l'ensemble des autres valeurs étant définies comme précédemment, ainsi que leurs sels avec les acides et notamment leurs chlorhydrates et trifluoroacétates ;
- les produits de formules (IV), (V), (VI), (VII), (VIII) et (VIII') telles que définies précédemment, dans lesquelles A représente un atome d'hydrogène ou un groupement protecteur de l'azote choisi parmi les dérivés benzylés ou tritylés, les carbamates d'allyle, de benzyle, phényle ou tertbutyle, ou encore les dérivés silylés choisis parmi terbutyle diméthyl, triméthyl, triphéyl et diphényl terbutyl-silyle, ou les dérivés phéylsulfonylalkyle ou cyanoalkyle, R₁₁ représente un groupe partant choisi parmi le groupe des phosphate, sulfonate et halogène et R₃ et R'₄ tels que définis précédemment , et l'ensemble des autres valeurs sont définis comme précédemment, ainsi que leurs sels avec un acide et notamment leurs chlorhydrates et trifluoroacétates ;
- les produits de formules (IV), (V), (VI), (VII), (VIII) et (VIII') telles que définies précédemment, dans lesquelles R'₁ représente un radical CONR₆R₇ dans lequel R₆ ou R₇ représente un radical alkoxy renfermant de 1 à 6 atomes de carbone, l'ensemble des autres valeurs étant définies comme dans les formules (IV), (V), (VI), (VII), (VIII) et (VIII'), ainsi que leurs sels avec un acide et notamment leurs chlorhydrates et trifluoroacétates.

Les produits de formule (IV) sont préparables par exemple selon des méthodes fournies ci-après dans la partie expérimentale.

Les exemples suivants illustrent l'invention .

### EXEMPLES

Dans la description qui précède ainsi que dans les exemples qui suivent les abréviations suivantes ont été utilisées :
- DEAD: : azo-dicarboxylate de diéthyle
- TEA: : triéthylamine
- DMAP: : 4-diméthylamino-pyridine
- EDCI: : 1-(3-diméthylamino-propyl)-3-éthylcarbo-diimide chlorhydrate
- THF: : tétrahydrofuranne
- AcOEt: : acétate d'éthyle
- DMF: : N,N-diméthylformamide
- AIBN: : 2,2'-azo-bis-isobutyronitrile
- M: : masse molaire moléculaire
- SM: : spectrométrie de masse
- EI: : impact électronique
- SIMS: : secondary ion mass spectrometry
- FAB: : fast atom bombardement
- BOP: : benzotriazol-1-yloxytripyrolidinophosphonium hexafluorophosphate
- HOBt: : 1-hydroxybenzotriazole hydrate
- DBU: : diazabicycloundécène
- (BOC)₂O :: dicarbonate de t-butyle
- NaBH₃CN :: cyanoborohydrure de sodium
- DMSO: : diméthylsulfoxyde
- DIEA: : diisopropylethyldiamine
- ClMEM: : chlorure de 2-méthoxyéthoxyméthyle
- TMSCN: : cyanure de triméthylsilyle
- BOC-ON :: 2-(terbutoxycarbonyloxyimino)-2-phénylacétonitrile

### Example 1

### Trans-4,5,6,8-tétrahydro-6-oxo-5-(phénylméthoxy)-1H-4,7-méthanopyrazolo[3,4-e] [1,3]diazépine-8-carboxylate de méthyle.

### Stade A1:

### Préparation du [2-(phénylthio)éthyl]hydrazine.

### Etape 1

Dans un ballon placé sous atmosphère d'azote, on introduit 100 g de 2-bromoéthylphényl sulfure dissous dans 1 l. d'éthanol et on ajoute 184,2 g d'hydrate d'hydrazine. On chauffe à 100°C toute une nuit. Puis, une fois la réaction terminée, on distille le solvant sous pression réduite à 80°C-90°C. Puis, on ajoute au milieu 65 g de carbonate de potassium et 1 1. de chlorure de méthylène. On agite pendant 15 minutes, puis on extrait la phase organique avec 2 x 500 ml d'eau, puis la phase organique est séchée sur sulfate de magnésium, filtrée et évaporée sous pression réduite. On reprend ensuite le résidu dans 750 ml d'un mélange éthanol/eau auquel on ajoute goutte à goutte 12 ml d'acide sulfurique concentré. Le produit cristallise et le précipité est filtré puis rincé avec une solution éthanol/eau, 80/20 puis à l'éther. Le produit est ensuite séché sous pression réduite.

On obtient 81,84 g de sel d'hemisulfate de [2-(phénylthio)éthyl]-hydrazine, de formule brute, C₈H₁₂N₂S +1/2 de H₂SO₄. (M = 217,3 g).

Le rendement obtenu est de 81%.

### Etape 2

On dissout 79,7 g du produit obtenu à l'étape 1 précédente, dans 1,9 l de dichlorométhane. Puis on ajoute 400 ml de soude 1N, et on agite fortement. On extrait ensuite la phase organique au dichlorométhane, puis on sèche la phase organique sur mgSO₄ et l'on évapore sous pression réduite.

On obtient avec un rendement quantitatif le [2-(phenylthio)éthyl]-hydrazine de formule brute C₈H₁₂N₂S (M = 168,26 g).

Le rendement obtenu est de 89%.

### Stade A2

On met en suspension 56,0 g (de 3,5-dzoxo-1-pipéridinecarboxylate de 1,1-diméthyléthyle de formule brute C₁₀R₁₅NO₄ (préparé par un procédé analogue à celui décrit dans Heterocycles, 22, 2769-2773, (1984), en remplaçant le chloroformiate de méthyle par (BOC)₂O. On ajoute à température ambiante 55,5 ml de N,N-diméthylformamide diméthylacétal à 95%.On agite pendant une demi-heure à 80°C puis pendant 3 heures à 50°C.On évapore le solvant sous pression réduite.

On obtient ainsi 79 g de 4-[(diméthylamino)méthylène]-3,5-dioxo-1-pipéridinecarboxylate de 1,1-diméthyléthyle de formule brute C₁₃H₂₀N₂O₄.

Le rendement correspondant est de 99%.

### Stade B

On dissout 79 g du produit obtenu au stade A2 dans 1 litre de méthanol absolu, puis on ajoute 54.5 g de [2-(phénylthio)éthyl]-hydrazine.On agite pendant 3 heures 30 à température ambiante.On évapore le solvant sous pression réduite et on purifie par chromatographie sur silice en éluant avec un mélange dichlorométhane/AcOEt 1/1.

On obtient le composé 1,4,5,7-tétrahydro-4-oxo-1-[2-(phénylthio)éthyl]-6*H*-pyrazolo[3,4-*c*]pyridine-6-carboxylate de 1,1-diméthyléthyle, de formule brute C₁₉H₂₃N₃O₃S (M = 373,48 g).

Le rendement obtenu correspondant est de 57,6%.

### Stade C

Dans un ballon placé sous atmosphère d'azote, on dissout 74,5 g de la cétone obtenue au stade précédent B dans 372,5 ml de méthanol, puis on refroidit avec un bain de glace et on introduit par petites fractions, en 20 minutes, 7,58 g de borohydrure de sodium.

On laisse revenir à la température ambiante en deux heures, puis on ajoute successivement du dichlorométhane et une solution aqueuse d'acide tartrique à 10%. On agite vigoureusement puis on décante et on réextrait au dichlorométhane. On réunit les phases organiques et on les sèche sur du sulfate de magnésium, on filtre et on évapore le solvant du filtrat sous pression réduite.On reprend le résidu du dichlorométhane, on filtre et on évapore à nouveau le solvant sous pression réduite.

On obtient 72,5 g du composé 1,4,5,7-tétrahydro-4-hydroxy-1-[2- (phénylthio) éthyl]-6H-pyrazolo[3,4-c]pyridine-6-carboxylate de 1,1-diméthyléthyle, de formule brute C₁₉H₂₅N₃O₃S (M = 375, 49 g).

Le rendement correspondant est de 97%.

### Stade D

On dissout 75 g du produit obtenu au stade précédent C dans 1 1. de dichlorométhane à 0°C. On ajoute 118 g d'acide méthachloroperbenzoïque à 70%, puis on agite 1h30 à température ambiante. On ajoute au milieu réactionnel 1,5 l. de dichlorométhane et 1,6 1. de thiosulfate de sodium 0,5N. Après extraction de la phase organique, on la relave avec 1 1. de thiosulfate de sodium puis avec 1,5 l, de NaHCO₃ et enfin avec 1,5 1, d'eau. Les phases aqueuses sont alors à nouveau réextraites avec du dichlorométhane, puis les différentes phases organiques sont rassemblées, séchées sur sulfate de magnésium, filtrées, puis le solvant est évaporé sous pression réduite.

le produit brut est recristallisé dans l'éther isopropylique pour donner 81 g de composé 1,4,5,7-tétrahydro-4-hydroxy-1-[2-[1-propénylsulfonyl]éthyl]-6*H*-pyrazolo [3,4-*c*]pyridine-6-carboxylate de 1,1-diméthyléthyle de formule brute C₁₉H₂₅N₃O₅S (M = 407,49 g).

Le rendement correspondant est de 99%.

### Stade E

Dans un ballon placé sous atmosphère inerte et à une température de -30°C, on dissout 57,2 g du produit obtenu au stade précédent D, dans 572 ml de THF anhydre. Puis, on ajoute 337 ml d'une solution de tert-butylate de potassium 1M dans le THF. On agite pendant 1 heure puis on ajoute au milieu réactionnel 20 ml d'acide acétique. On ajoute ensuite une solution aqueuse de bicarbonate de NaHCO₃ et NaCl, puis on extrait la phase organique par de l'acétate d'éthyle plusieurs fois. Les phases organiques sont séchées sur sulfate de magnésium, filtrées et le solvant évaporé sous pression réduite. On récupère 57,4 g de produit brut qui est ensuite purifié sur silice en éluant avec un mélange dichlorométhane/acétone, 4/6.

On obtient après évaporation du solvant 38,6 g de 1,4,5,7-tétrahydro-4-hydroxy-6*H*-pyrazolo[3,4-*c*]pyridine-6-carboxylate de 1,1-diméthyléthyle, de formule brute C₁₁H₁₇N₃O₃ (M = 239,28 g).

Le rendement correspondant est de 84%.

### Stade F

Dans un ballon placé sous atmosphère d'azote, on dissout 35 g du produit obtenu au stade précédent E, dans 890 ml de dichlorométhane. On ajoute 49,77 g de (Ph)₃CCl puis on refroidit la solution à -30°C avec un mélange carboglace/acétone, On ajoute ensuite 24,7 ml de triéthylamine, et on agite le milieu réactionnel en laissant revenir à température ambiante pendant 4h30. Puis, après évaporation du solvant sous pression réduite, on verse le résidu dans 1,6 1. d'acétate d'éthyle et on lave avec 1,8 1. d'eau. La phase organique est ensuite séchée sur sulfate de magnésium, filtrée, et le solvant évaporé sous pression réduite. Le résidu est repris dans l'éther éthylique et le précipité obtenu est lavé dans du pentane. Après séchage, on obtient 62,5 g de 2,4,5,7-tétrahydro-4-hydroxy-2-(triphénylméthyl)-6*H*-pyrazolo [3,4-*c*]pyridine-6-carboxylate de 1,1-diméthyléthyle, de formule brute C₃₀H₃₁N₃O₃ (M = 481,60 g).

Le rendement est de 99%.

### Stade G

Dans un ballon placé sous atmosphère d'azote, on dissout 26,6 g de l'alcool obtenu au stade précédent F dans 230 ml de THF, puis on refroidit à -78°C et on introduit 81 ml de terbutyllithium en solution 1,7 M dans le pentane.

On laisse réagir 15 minutes à -78°C, et on introduit du gaz carbonique en excès pendant 10 minutes, puis on laisse revenir à la température ambiante.

Le mélange réactionnel est hydrolysé par addition de 100 ml d'eau et 300 ml d'acétate d'éthyle puis acidifié à pH = 4 par addition d'acide formique. La phase aqueuse est extraite plusieurs fois à l'acétate d'éthyle puis la phase organique est séchée sur mgSO₄, filtrée et le solvant évaporé sous pression réduite pour donner 29,8 g de produit brut. Ce dernier est dissout dans 300 ml d'éther puis extrait par 3 x 200 ml d'une solution saturée de NaHCO₃ La phase aqueuse est acidifiée à pH = 4 par addition d'acide formique puis extrait par l'acétate d'éthyle. Après séchage sur mgSO4, filtration et évaporation sous pression réduite on obtient 14,8 g d'acide de formule brute
C₃₁H₃₁N₃O₅ (M = 525,61 g).

Les 14,8 g du composé obtenu sont ensuite estérifiés en présence de 3,7 g de K₂CO₃ et 4,9 ml de sulfate diméthylique. On agite pendant 1 heure à température ambiante puis on ajoute 7,4 ml de triéthylamine. Après 40 minutes, on ajoute 300 ml d'acétate d'éthyle et 200 ml d'eau. Après agitation, on décante puis on réextrait la phase aqueuse à l'acétate d'éthyle. Les phases organiques sont lavées avec une solution d'eau saturée en NaCl. Les phases organiques sont ensuite séchées sur sulfate de magnésium et le solvant est évaporé sous pression réduite.

On obtient 11,23 g de 2,4,5,7-tétrahydro-4-hydroxy-2-(triphénylméthyl)-6*H*-pyrazolo[3,4-*c*]pyridine-6,7-dicarboxylate de 6-(1,1-diméthyléthyle) et de 7-méthyle, de formule brute C₃₂H₃₃N₃O₅ (M = 739, 64 g).

Le rendement correspondant sur les deux étapes est de 42%.

### Stade H

Dans un ballon sous atmosphère d'argon, on solubilise 1g du produit obtenu au stade précédent G dans 50 ml de dichlorométhane. On ajoute 2,8 g de triéthylamine puis 4,8 g de (CH₃SO₂)₂ Odilués dans 1 ml de dichlorométhane. On agite une heure à -70°C puis on ajoute 0,68 g de O-benzylhydroxylamine. On agite encore 10 minutes à -78°C, 1h20 à -50°C, et enfin à 0°C pendant toute la nuit. On laisse encore une heure à 20°C, puis on ajoute du dichlorométhane et on lave la phase organique avec une solution d'acide tartrique puis une solution aqueuse de NaCl et enfin d'eau pure. On sèche la phase organique sur mgSO₄, on filtre et on évapore le solvant sous pression réduite. On obtient 11,9 g de produit qui est purifié sur silice en éluant avec un mélange d'éther de pétrole/acétate d'éthyle, 8/2.

On obtient 8,9 g de 2,4,5,7-tétrahydro-4-[(phénylméthoxy)amino]-2-(triphénylméthyl)-6*H*-pyrazolo[3,4-*c*]pyridine-6,7-dicarboxylate de 6-(1,1-diméthyléthyle) et de 7-méthyle, de formule brute C₃₉H₄₀N₄O₅ (M = 644,78 g).

Le rendement correspondant est de 75%.

### Stade I

Dans un ballon maintenu à 0°C, on dissout 10 g du produit obtenu au stade précédent H dans 70 ml d'acétate d'éthyle. On ajoute 35 ml d'une solution saturée d'acide chlorhydrique dans l'acétate d'éthyle puis on agite pendant 3 heures. Après évaporation du solvant, on obtient le dichlorhydrate de 4,5,6,7-tétrahydro-4-[(phénylméthoxy)amino]-2*H*-pyrazolo[3,4-*c*]pyridine-7-carboxylate de méthyle brut.

### Stade J

Le produit brut obtenu au stade I est repris dans l'eau. Puis on lave la phase aqueuse avec de l'acétate d'éthyle. La phase aqueuse est ensuite amenée à pH = 10 avec une solution d'ammoniaque à 20% puis extraite trois fois avec de l'acétate d'éthyle. Après séchage sur sulfate de magnésium, filtration, et évaporation du solvant sous pression réduite, on obtient 4,21 g de 4, 5, 6,7-tétrahydro-4-[(phénylméthoxy)amino]-2*H-*pyrazolo[3,4-*c*]pyridine-7-carboxylate de méthyle, de formule brute C₁₅H₁₈N₄O₃ (M = 302,34 g).

Le rendement correspondant aux deux étapes I et J est de 89,7%.

### Stade K

Dans un ballon placé sous atmosphère d'argon et refroidit par un bain de glace, on introduit 9,24 g du produit obtenu au stade précédent I, 3 litres d'acétonitrile et 12,3 ml de TEA. On agite pendant 2 minutes et on introduit ensuite 1,85 ml de diphosgène. On agite la solution à 20°C pendant 1 heure. On dilue à l'AcOEt et on lave avec une solution d'acide tartrique à 10% puis à l'eau.On sèche la phase organique sur du sulfate de magnésium et on évapore le solvant sous pression réduite.

Le produit brut est dissout dans 500 ml de dichlorométhane avec 0,5 ml de DBU. Après 10 minutes de contact, le mélange réactionnel est lavé par une solution d'acide tartrique à 10% puis à l'eau. Après évaporation du solvant sous pression réduite.

On obtient 9,6 g de trans-4,5,6,8-tétrahydro-6-oxo-5-(phénylméthoxy)-1H-4,7-méthanopyrazolo[3,4-e][1,3]diazépine-8-carboxylate de méthyle, de formule brute C16H16N4O4 (M = 328,33 g).

Le rendement correspondant est de 95%.

### Stade L

Dans un ballon maintenu à 0°C sous atmosphère d'azote, on dissout 0,2 g du produit obtenu au stade précédent K dans 2 ml de DMF, et on ajoute 0,115 g de bromure de benzyle puis 0,032 g de NaH. On agite pendant 10 minutes à 0°C puis on laisse revenir à la température ambiante. Au bout d'une heure, la réaction est terminée. On verse le milieu réactionnel dans une solution aqueuse de NaCl et on extrait deux fois à l'acétate d'éthyle. Puis la phase organique est séchée sur sulfate de magnésium, filtrée et le solvant est évaporé sous pression réduite. On obtient 231 mg de produit qui est purifié sur colonne de silice en éluant avec un mélange chlorure de méthylène/ acétate d'éthyle/triaéthylamine 95/0,5/0,1%. On obtient après évaporation du solvant 70 mg du composé trans-2,5,6,8-tétrahydro-6-oxo-5-(phénylméthoxy)-2-(phénylméthyl)-4*H*-4,7-méthanopyrazolo[3,4-*e*][1,3]diazépine-8-carboxylate de méthyle, de formule brute C₂₃H₂₂N₄O₄ (M = 418, 46 g).

Le rendement correspondant est de 27%.

### Stade M

On dissout 65 mg du produit obtenu au stade précédent dans 1 ml de MeOH puis on ajoute 29 mg de palladium sur charbon à 10% et on place sous atmosphère d'hydrogène en agitant fortement. Lorsque le produit de départ est consommé, on filtre le catalyseur et on évapore le solvant sous pression réduite.

On obtient 47 mg du composé trans-2,5,6,8-tétrahydro-5-hydroxy-6-oxo-2-(phénylméthyl)-4*H*-4,7-méthanopyrazolo[3,4-*e*][1,3]diazépine-8-carboxylate de méthyle, de formule brute C₁₆H₁₆N₄O₄ (M = 328,33 g).

### Stade N

On dissout 0,047 g du produit obtenu au stade précédent M dans 1 ml de pyridine contenant quelques grains de tamis moléculaire 4Å. Puis on ajoute 0,068 g du complexe pyridine-SO₃. On agite une nuit à température ambiante. On filtre ensuite le tamis que l'on rince avec de l'eau puis avec du chlorure de méthylène. Après évaporation des solvants co-évaporés avec du toluène, on obtient 114 mg de produit brut qui sont purifiés sur silice en éluant avec un mélange chlorure de méthylène/éthanol/triéthylamine 90/10/0,1%. On obtient 41 mg du composé de sel de triéthylammonium de trans-2,5,6,8-tétrahydro-6-oxo-2-(phénylméthyl)-5-(sulfooxy)-4*H*-4,7-méthanopyrazolo[3,4-*e*][1,3]diazépine-8-carboxylate de méthyle, de formule brute C₂₂H₃₁N₅O₇S ( M = 509,58).

Le rendement obtenu sur les deux stades M et N est de 52%.

RMN du proton :
DMSO-d₆ à 300 MHz (déplacement chimique et multiplicité) : 1,12 (sl) : (CH₃CH₂)₃N ; 3, 05 (1) : (CH₃CH₂)₃N ; 3,45 (m) : N-CH₂-CH ; 4,75 (m) : -)N-CH₂-CH ; 3,73 (s) : CH₃OOC-CH; 4,98 (s) : CH₃OOC-CH; 5,28 (sl) : N-CH₂-φ ;7,22 à 7,39 (m) : H aromatique ; 7,87 (s) : N-CH.

LC/SM (électrospray négatif) : conditions générales
Colonne kromasil C18 4,6 x 250 mm, 5 µ Four à 30°C
Débit = 1ml/min Vᵢₙⱼ = 15 µl
Détection λ = 200-400 mm
SM/ESP mode+/- CV = 50V
Eluant : A = H₂O ( 0,1% HCO₂H)
B = CH₃CN

### Graduent :

| temps | A% | B% |
|---|---|---|
| 0,00 | 80,0 | 20,0 |
| 15,00 | 50,0 | 50,0 |
| 25,00 | 20,0 | 80,0 |
| 40,00 | 80,0 | 20,0 |
| 50,00 | 80,0 | 20,0 |

LC/SM (électrospray négatif) m/z :TR = 9,70 min M⁻ = 407.

### Exemple 2

### Sel de triéthylammonium de trans-4,5,6,8-tétrahydro-6-oxo-1-(phényléthyl)-5-(sulfooxy)-1H-4,7-methanopyrazolo[3,9-e][1,3]diazépine-8-carboxylate de méthyle

### Stade A

Dans un ballon placé sous atmosphère d'azote à 0°C, on dissout 0,1 g du produit obtenu au stade K de l'Exemple 1 dans 0,8 ml de DMF. On ajoute 0,062 g de 2-bromophényléthane puis 0,015 g de NaH. On agite pendant 15 minutes à 0°C et on laisse revenir le milieu réactionnel à température ambiante. On agite le milieu pendant 6 heures, puis on additionne une solution aqueuse saturée en NaCl et on extrait la phase aqueuse plusieurs fois avec de l'acétate d'éthyle. Après séchage de la phase organique sur sulfate de magnésium, on filtre et on évapore le solvant sous pression réduite. Le produit brut obtenu est purifié sur silice en éluant avec un mélange chlorure de méthylène/acétone/triéthylamine, 98/2/0,1%. Après évaporation du solvant, on obtient 28,8 mg soit un rendement de 21,5% du composé isomère A, trans-4,5,6,8-tétrahydro-6-oxo-5-(phénylméthoxy)-1-(phényléthyl)-1*H*-4,7-méthanopyrazolo[3,4-*e*][1,3]diazépine-8-carboxylate de méthyle, de formule brute C₂₄H₂₄N₄O₄ (432,46 g) et on obtient 37 mg soit un rendement de 28% en isomère B, trans-4,5,6,8-tétrahydro-6-oxo-5-(phénylméthoxy)-2- (2-phényléthyl)-1*H*-4,7-méthanopyrazolo[3,4-*e*][1,3]diazépine-8-carboxylate de méthyle de formule brute C₂₄H₂₄N₄O₄ (M = 432,48 g).

### Stade B

Dans un ballon placé sous atmosphère d'azote, on dissout 0,028 g de l'isomère A obtenu au stade A précédent dans 1 ml de méthanol puis on ajoute 0,0168 g de palladium sur charbon à 10%. On place sous atmosphère d'hydrogène. Au bout de 2 heures, la réaction est terminée et le milieu réactionnel est filtré et le solvant est évaporé sous pression réduite. On obtient 13,8 mg de trans-4,5,6,8-tétrahydro-5-hydroxy-6-oxo-1-(phényléthyl)-1*H*-4,7-méthanopyrazolo[3,4-*e*][1,3]diazépine-8-carboxylate de méthyle, de formule brute C₁₇H₁₈N₄O₄ (M = 342,36 g).

Le rendement correspondant est de 62%.

### Stade C

Dans un ballon, on dissout 0,0130 g du produit obtenu au stade B précédent dans 1ml de pyridine et on ajoute 0,018 g de complexe pyridine-SO₃. On agite une nuit à température ambiante puis on filtre le mélange réactionnel et on rince avec un mélange chlorure de méthylène/eau. Après évaporation, on obtient 19 mg de produit brut qui sont purifiés par chromatographie sur silice en éluant avec un mélange chlorure de méthylène/éthanol/triéthylamine, 95/15/0,1%. On obtient 6,6 mg du sel de triéthylammonium de trans-4,5,6,8-tétrahydro-6-oxo-1-(phényléthyl)-5-(sulfooxy)-1*H*-4,7-methanopyrazolo-[3,4-*e*][1,3]diazépine-8-carboxylate de méthyle, de formule brute C₂₃H₃₃N₅O₇S, (M = 523,61 g).

Le rendement correspondant est de 20%.

LC/SM (électrospray négatif), m/z :
TR = 13,02 min [MH]⁻ = 421 et [2M+Na - 2H]⁻ = 865.

### Exemple 3

### Sel de triéthylammonium de trans-2,5,6,8-tétrahydro-6-oxo-2-(2-phényléthyl)-5-(sulfooxy)-4H-4,7-méthanopyrazolo[3,4-e][1,3]diazépine-8-carboxylate de méthyle

### Stade A

On procède comme au Stade M de l'Exemple 1 avec 0,037 g de l'isomère B obtenu au Stade A de l'Exemple 2, 0,022 g de palladium sur charbon, et 0,5 ml de méthanol. On obtient 28 mg du composé Trans-2,5,6,8-tétrahydro-5-hydroxy-6-oxo-2-(2-phényléthyl)-4*H*-4,7-méthanopyrazolo[3,4-*e*][1,3]diazépine-8-carboxylate de méthyle de formule brute C₁₇H₁₈N₄O₄ (342,36 g).

Le rendement correspondant est de 96%.

### Stade B

On procède comme dans l'Exemple 1 au Stade M avec 0,028 g du produit obtenu au stade précédent A, 0,039 g du complexe pyridine-SO₃ et 1 ml de pyridine. On obtient 20 mg du sel de triéthylammonium de trans-2,5,6,8-tétrahydro-6-oxo-2-(2-phényléthyl)-5-(sulfooxy)-4*H*-4,7-méthanopyrazolo[3,4-*e*][1,3]diazépine-8-carboxylate de méthyle de formule brute C₂₃H₃₃N₅O₇S, (M = 523,61 g).

Le rendement correspondant est de 47%.

LC/SM (électrospray négatif), m/z :
TR = 11,87 min [2M⁻ + Na - 2H]⁻ = 865- et [M]⁻ = 421⁻

### Exemple 4

### Sel de triéthylammonium de trans-4,5,6,8-tétrahydro-8-(méthoxycarbonyl)-6-oxo-5-(sulfooxy)-1H-4,7-méthanopyrazolo[3,4-e][1,3]diazépine-1-acétate d'éthyle.

### Stade A

Dans un ballon, on dissout 0,2 g du produit obtenu au Stade K de l'Exemple 1 dans 2 ml d'acétonitrile. On ajoute 0,150 µl de bromoacétate d'éthyl et 240 µl de DIEA. On chauffe à 50°C pendant 24 heures puis on extrait le milieu réactionnel à l'acétate d'éthyle, on lave la phase organique avec une solution aqueuse d'acide tartrique à 10% puis avec une solution aqueuse de NaCl. On sèche la phase organique sur sulfate de magnésium, on filtre et on évapore le solvant sous pression réduite. On obtient 266 mg du produit brut qui sont purifiés par chromatographie sur silice en éluant avec un mélange chlorure de méthylène/acétone/triéthylamine, 95/0,5/0,1%. On obtient 37 mg de l'isomère A, trans-4,5,6,8-tétrahydro-8-(méthoxycarbonyl)-6-oxo-5-(phénylméthoxy)-1*H*-4,7-méthanopyrazolo[3,4-*e*][1,3]diazépine-1-acétate d'éthyle, de formule brute C₂₀H₂₂N₄O₆ (M = 414,42 g).

Le rendement correspondant est de 11,8%.

On obtient aussi 88 mg de l'isomère B, trans-5,6-dihydro-8-(méthoxycarbonyl)-6-oxo-5-(phénylméthoxy)-4*H*-4,7-méthanopyrazolo[3,4-*e*][1,3]diazépine-2(8*H*)-acétate d'éthyle, de formule brute C₂₀H₂₂N₄O₅ (412,42 g).

Le rendement correspondant est de 28%.

### Stade B

On procède comme au Stade M de l'Exemple 1 avec 0,020 g de l'isomère A obtenu au stade précédent, 0,01 g de palladium sur charbon et 0,5 ml de méthanol. On obtient 24,2 mg du composé trans-4,5,6,8-tétrahydro-5-hydroxy-8-(méthoxycarbonyl)-6-oxo-1*H*-4,7-méthanopyrazolo[3,4-*e*][1,3]diazépine-1-acétate d'éthyle, de formule brute C₁₃H₁₆N₄O₆ (324,30 g).

Le rendement correspondant est de 83%.

### Stade C

On procède comme au Stade N de l'Exemple 1 avec 0,024 g du produit obtenu au stade précédent, 0,035 g du complexe pyridine-SO₃ et 1 ml de pyridine. On obtient 28,8 mg du composé de sel de triéthylammonium de trans-4,5,6,8-tétrahydro-8-(méthoxycarbonyl)-6-oxo-5-(sulfooxy)-1*H*-4,7-méthanopyrazolo[3,4-*e*][1,3]diazépine-1-acétate d'éthyle, de formule brute C₁₉H₃₁N₅O₉S, (505,55).

Le rendement correspondant est de 77%.

LC/SM (électrospray négatif), m/z :
TR = 6,06 min [M]⁻ = 403.

RMN du proton :
CDCl₃ à 300 MHz et 60°C (déplacement chimique et multiplicité) :
1,28 (t) : CH₃-CH₂-O-CO ; 4,20 (q) : CH₃-CH₂-O-CO ; 5,06 et 4,96 (AB): O-CO-CH₂-N ; 3,84 (s) : CH₃-O-CO ; 5,32 (s) : CH₃-O-CO-CH-N ; 3,45 (d) et 3,82 (dd) : N-CH₂-CH-N; 5,00 (d) : N-CH₂-CH-N ; 7,62 (s) : N=CH-C ; 1,28 (t) : CH₃-CH₂-N ; 3,15 (q) : CH₃-CH₂-N.

### Exemple 5

### Sel de triéthylammonium de trans-5,6-dihydro-8-(méthoxycarboayl)-6-oxo-5-(sulfooxy)-4H-4,7-méthanopyrazolo[3,4-e][1,3]diazépine-2(8H)-acétate d'éthyle.

### Stade A

On procède comme au Stade M de l'Exemple 1 avec 0,067 g de l'isomère B obtenu au Stade A de l'Exemple 4. 0,038 g de palladium sur charbon et 0,4 ml de méthanol. On obtient 62 mg du composé trans-5,6-dihydro-8-(méthoxycarbonyl)-6-oxo-5-(phénylméthoxy)-4*H*-4,7,méthanopyrazolo[3,4-e][1,3]diazépine-2(8*H*)-acétate d'éthyle, de formule brute C₁₃H₁₆N₄O₆ (324,30 g).

Le rendement correspondant est de 90%.

### Stade B

On procède comme au Stade M de l'Exemple 1 avec 0,062 g du produit obtenu au stade précédent, 0,091 g du complexe pyridine-SO₂ et 2 ml de pyridine. On obtient 79 mg du composé de sel de triéthylammonium de trans-5,6-dihydro-8-(méthoxycarbonyl)-6-oxo-5-(sulfooxy)-4*H*-4,7-méthanopyrazolo[3,4-*e*][1,3]diazépine-2(8*H*)-acétate d' éthyle, de formule brute C₁₉H₃₁N₅O₉S, (M = 505,55 g).

Le rendement correspondant est de 82%.

LC/SM (électrospray négatif), m/z :
TR = 5,64 min M⁻ = 403.

RMN du proton : CDCl₃ (déplacement chimique et multiplicité) :
1,28 (t) : CH₃-CH₂OCO ; 4,22 (q) : CH₃-CH₂-OCO ; 4,90 et 4,80 (AB): CH₃-CH₂-OCO-CH2-N ; 3,85 (s) : CH₃O-CO ; 5,26 (s) : CH₃-O-CO-CH-N ; 3,63 (d) et 3,82 (dd) : N-CH₂-CH-N ; 4,99 (dl) : N-CH₂-CH-C= ; 7,53 (s) : C=CH-N.

### Exemple 6

### Sel de triéthylammonium de 2,5,6,8-tétrahydro-6-oxo-2-[(phénylamino)carbonyl]-5-(sulfooxy)-4H-4,7-méthanopyrazolo[3,4-e][1,3]diazépine-8-carboxylate de méthyle

### Stade A

Dans un ballon, on dissout 0,3 g du composé obtenu au Stade K de l'Exemple 1 dans 5 ml de CH₂Cl₂. On ajoute 0,109 g de phénylisocyanate. Puis on agite le milieu réactionnel pendant 1 heure. Après évaporation, on obtient un produit brut qui est purifié par chromatographie sur silice en éluant avec un mélange chlorure de méthylène/acétate d'éthyle/triéthylamine, 99/1/0,1%. On 176 mg de l'isomère A de trans-4,5,6,8-tétrahydro-6-oxo-1-[(phénylamino)carbonyl]-5-(phénylméthoxy)-1*H*-4,7-méthanopyrazolo[3,4-*e*][1,3]diazépine-8-carboxylate de méthyle, et 63,5 mg de l'isomère B de trans-2,5,6,8-tétrahydro-6-oxo-2-[(phénylamino)carbonyl]-5-(phénylméthoxy)-4*H*-4,7-méthanopyrazolo[3,4-*e*][1,3]diazépine-8-carboxylate de méthyle, de formule brute C₂₃H₂₁N₅O₅ (M = 447,45 g).

Les rendements correspondants en isomères A et B sont de 36% et de 14%.

### Stade B

On procède comme au Stade M de l'Exemple 1, avec 0,054 g de l'isomère B obtenu au stade précédent, 0,008 g de palladium sur charbon, 5 ml de méthanol, 1 ml de THF. On obtient 44 mg du composé trans-2,5,6,8-tétrahydro-5-hydroxy-oxo-2-[(phénylamino)carbonyl]-4*H*-4,7-méthanopyrazolo[3,4-*e*][1,3]diazépine-8-carboxylate de méthyle, de formule brute C₁₆H₁₅N₅O₅ (357,33 g).

Le rendement correspondant est quantitatif.

### Stade C

On procède comme au Stade M de l'Exemple 1 avec 40 mg du produit obtenu au stade précédent, 62m g du complexe pyridine-SO₃ et 3 ml de pyridine. On obtient 21 mg du composé de sel de triéthylammonium de 2,5,6,8-tétrahydro-6-oxo-2-[(phénylamino)carbonyl]-5-(sulfooxy)-4*H*-4,7-méthanopyrazolo[3,4-*e*][1,3]diazépine-8-carboxylate de méthyle, de formule brute C₂₂H31N6O₈S (M = 532,59 g).

Le rendement correspondant est de 35%.

LC/SM (électrospray négatif), m/z : MH⁻ - 436.

RMN du proton dans DMSO-d₆ à 300 MHz, déplacement chimique et multiplicité :
3,80 : CH₃-O-CO ; 5,17 (s) : CH₃-O-CO=CH-N ; 3,48 (d) et 3,59 (dd) : N-CH₂-CH-N ; 4,91 (d) : N-CH₂-CH-C= ; 8,41 (s) : C=CH-N ; 7, 71 (d), 7,36 (t), 7, 14 (t) pour H aromatiques ; 10,40 (s) : NH.

### Exemple 7

### Sel de triéthylammonium de 5,6-dihydro-6-oxo-N2-phényl-5-(sulfooxy)-4H-4,7-méthanopyrazolo[3,4-e][1,3]diazépine-2,8(8H)-dicarboxamide

### Stade A

Dans un ballon, on dissout 0, 445 g du produit de l'isomère B obtenu au Stade A de l'Exemple 8 dans 10 ml de dioxanne. Puis on ajoute 10 ml d'eau, puis 0,995 ml de soude normale. On ajoute au milieu réactionnel une solution aqueuse de NaH₂PO₄. On extrait deux fois avec de l'acétate d'éthyle. La phase organique est séchée sur sulfate de magnésium, filtrée et le solvant est évaporé sous pression réduite. On obtient 460 mg du composé trans-acide 2,5,6,8-tétrahydro-6,oxo-2-[(phénylamino)carbonyl]-5-(phénylméthoxy)-4H-4,7-méthanopyrazolo[3,4-e][1,3]diazépine-8-carboxylique, de formule brute C₂₂H₁₉N₅O₅ (M = 433,43 g).

Le rendement est quantitatif.

### Stade B

Dans un ballon placé sous atmosphère d'azote, on dissout 0,16 g du produit obtenu au stade précédent dans 5 ml de DMF. On ajoute successivement 0,239 g de BOP, 0,073 g de HOBt 0,039 g de NH₄Cl et 0,139 ml de DIEA. Après 2H30 d'agitation, on ajoute de l'acétate d'éthyle et on lave la phase organique avec de l'eau. La phase organique est ensuite lavée successivement avec une solution d'acide tartrique à 10%, une solution de NaHCO₃, une solution tampon de pH 7, et une solution aqueuse de NaCl. La phase organique est séchée sur sulfate de magnésium puis filtrée et le solvant est évaporé sous pression réduite. On obtient un produit 160 mg de produit brut qui est repris dans de l'éther pour donner 100 mg du composé trans-5,6,dihydro-6-oxo-N2-phényl-5-(phénylméthoxy)-4H-4,7-méthanopyrazolo[3,4-e][1,3]diazépine-2,8(8H)-dicarboxamide, de formule brute C₂₂H₂ON₆O₄ (M = 432,44 g).

Le rendement correspondant est de 64%.

### Stade C

On procède comme au Stade M de l'Exemple 1 avec 0,09 g du produit obtenu au stade précédent B. 0,018 g de palladium sur charbon, 2 ml de THF, 2 ml de méthanol et 1 ml d'acétate d'éthyle. On obtient 74 mg du composé trans-5,6-dihydro-6-oxo-N₂-phényl-5-(phénylméthoxy)-4H-4,7-méthanopyrazolo[3,4-e][1,3]diazépine-2,8(8H)-dicarboxamide, de formule brute C₁₅H₁₄N₆O₄ (M = 342,32 g).

Le rendement est quantitatif.

### Stade D

On procède comme au Stade M de l'Exemple 1 avec 0,079 g du produit obtenu au stade précédent. 0,110 g du complexe pyridine-SO₃ et 2 ml de pyridine. On obtient 26 mg du composé de sel de triéthylammonium de 5,6-dihydro-6-oxo-N2-phényl-5-(sulfooxy)-4H-4,7-méthanopyrazolo[3,4-e][1,3]diazépine-2,8(8H)-dicarboxamide, de formule brute C₂₀H₁₉N₇O₇S (M = 501,48 g).

Le rendement correspondant est de 25%.

LC/SM (électrospray négatif), m/z : M⁻ = 421.

### Exemple 8

### Sel de triéthylammonium de trans-2,5,6,8-tétrahydro-6-oxo-2-(phénylsulfonyl)-5-(sulfooxy)-4H-4,7-méthanopyrazolo[3,4-e][1,3]diazépirie-8-carboxylate de méthyle

### Stade A

Dans un ballon placé sous atmosphère d'argon, on dissout 0,30 g du produit obtenu au Stade K de l'Exemple 1 dans 5 ml de dichlorométhane, On ajoute 0,19 ml de TEA, 0,242 g de chlorure de phénylsulfonyle. Au bout d'une heure, le milieu réactionnel est lavé avec une solution aqueuse de NaH₂PO₄ et la phase organique est séparée puis séchée sur sulfate de magnésium et filtrée. Le solvant est évaporé sous pression réduite pour donner 850 mg de produit brut. Le produit est purifié par chromatographie sur silice en éluant avec un mélange CH2Cl2/AcoEt/TEA 95/5/0,1. On obtient 128 mg du composé trans-2,5,6,8-tétrahydro-6-oxo-5-(phénylméthoxy)-2-(phénylsulfonyl)-4H-4,7-méthanopyrazolo[3,4-e][1,3]diazépine-8-carboxylate de méthyle, de formule brute C₂₂H₂₀N₄O₆S (M = 468,49 g).

Le rendement correspondant est de 29%.

### Stade B

On procède comme au Stade M de l'Exemple 1 avec 0,125 g du produit obtenu au stade précédent, 0,156 g de palladium sur charbon, 3 ml de THF et 3 ml de méthanol. On obtient 98 mg du composé trans-2,5,6,8-tétrahydro-5-hydroxy-6-oxo-2-(phénylsulfonyl)-4H-4,7-méthanopyrazolo[3,4-e][1,3]diazépine-8-carboxylate de méthyle, de formule brute C₁₅H₁₄N₄O₆S (M = 378,37 g).

Le rendement correspondant est quantitatif.

### Stade C

On procède comme au Stade M de l'Exemple 1 avec 0,079 g du produit obtenu au stade précédent, 0,10 g du complexe pyridine-SO₃ et 3 ml de pyridine. On obtient 3,6 mg du composé de sel de triéthylammonium de trans-2,5,6,8-tétrahydro-6-oxo-2-(phénylsulfonyl)-5-(sulfooxy)-4H-4,7-méthanopyrazolo[3,4-e][1,3]diazépine-8-carboxylate de méthyle, de formule brute C₂₀H₁₉N₅O₉S₂ (M = 547,53 g).

Le rendement correspondant est de 3,5%.

LC/SM (électrospray négatif), m/z : M⁻ = 457.

### Exemple 9

### Sel de di-(triéthylammonium) de l'acide trans-5,6-dihydro-8-(méthoxycarbonyl)-6-oxo-5-sulfooxy-4H-4,7-méthanopyrazolo[3,4-e][1,3]diazépine-2(8H)-acétique

### Stade A

Dans un ballon, on dissout 2,0 g du produit obtenu au Stade K de l'Exemple 1 dans 20 ml d'acétonitrile sec. On ajoute 3,1 ml de DIEA puis 1,9 ml de bromoacétate d'allyle. On agite une nuit à 50°C puis on ajoute de l'acétate d'éthyle. On lave le milieu réactionnel avec une solution d'acide tartrique à 10% puis avec une solution aqueuse de NaCl. La phase organique est séchée sur sulfate de magnésium, filtrée et le solvant est évaporé sous pression réduite. Le produit brut obtenu est purifié par chromatographie sur silice en éluant avec un mélange heptane/acétate d'éthyle/triéthylamine, 1/1/0,1%. On obtient 1,48 g du composé trans-5,6-dihydro-8-(méthoxycarbonyl)-6-oxo-5-(phénylméthoxy)-4H-4,7-méthanopyrazolo[3,4-e][1,3]diazépine-2(8H)-acétate de 2-propényle, de formule brute C₂₁H₂₂N₄O₆ (M = 426,43 g).

Le rendement correspondant est de 57%.

### Stade B

Dans un ballon, on dissout 0,084 g du produit obtenu au stade précédent dans 1 ml de dichlorométhane. On ajoute 2 mg de Pd(PPh₃)₄ et 0,043 g de Ph SiH₃. Au bout d'une heure d'agitation, on agite à nouveau 3,5 mg de Pd(PPh₃)₄ dans 1 ml de chlorure de méthylène. Le mélange réactionnel est ensuite évaporé puis repris dans un mélange THF/eau. On ajoute de l'acétate d'éthyle et du NaH₂PO₄, puis on extrait la phase organique ; celle-ci est lavée avec de l'eau puis séchée sur sulfate de magnésium, filtrée et le solvant est évaporé sous pression réduite. On obtient 90,8 mg du composé trans-acide 5,6-dihydro-8-(méthoxycarbonyl)-6-oxo-5-(phénylméthoxy)-4H-4,7-méthanopyrazolo[3,4-e][1,3]diazépine-2(8H)-acétique, de formule brute C₁₈H₁₈N₄O₆ (M = 386,37 g). Le produit brut est utilisé tel quel au stade suivant.

### Stade C

On procède comme au Stade M de l'Exemple 1 avec 0,090 g du produit brut obtenu au stade précédent, 0,036 g de palladium sur charbon et 3 ml d'éthanol. On obtient 77 mg du composé trans-acide 5,6-dihydro-5-hydroxy-8-(méthoxycarbonyl)-6-oxo-4H-4,7-méthanopyrazolo[3,4-e][1,3]diazépine-2(8H)-acétique, de formule brute C₁₁H₁₂N₄O₆ (M = 296,24 g).

Le rendement est quantitatif.

### Stade D

On procède comme au Stade M de l'Exemple 1 avec 0,070 g du produit obtenu au stade précédent, 0,113 g du complexe pyridine-SO₃ et 1 ml de pyridine. Le produit brut est purifié sur résine XAD4 en éluant par un gradient eau/acétone 100/0, 95/5, 50/50. On obtient 14 mg du composé de sel de di-(pyridinium) de l'acide trans-5,6-dihydro-8-(méthoxycarbonyl)-6-oxo-5-sulfooxy-4H-4,7-méthanopyrazolo[3,4-e][1,3]diazépine-2(8H)-acétique, de formule brute C₁₇H₂₇N₅O₉S, C₆H₁₆N (M = 477,50 g).

Le rendement correspondant est de 13%.

LC/SM (électrospray négatif), m/z : TR = 3,12 min M⁻ = 375.

### Exemple 10

### Sel de pyridinium de trans-1-(aminocarbonyl)-4,5,6,8-tétrahydro-6-oxo-5-(sulfooxy)-1H-4,7-méthanopyrazolo[3,4-e][1,3]diazépine-8-carboxylata de méthyle

### Stade A

Dans un ballon placé sous atmosphère d'argon à 0°C on dissout 0,188 g du produit obtenu au Stade K de l'Exemple 1 dans 30 ml de dichlorométhane. On ajoute 70 µl de diphosgène puis au bout d'une 1H15 on ajoute 0,49 ml d'une solution aqueuse concentrée d'ammoniaque. Après une heure, on ajoute au milieu réactionnel du chlorure de méthylène et on lave la phase organique avec une solution aqueuse de NaH₂PO₄ puis avec une solution aqueuse de NaCl. Les phases organiques sont séchées sur sulfate de magnésium, filtrées et le solvant est évaporé sous pression réduite. Le produit brut obtenu est purifié par chromatographie sur silice en éluant avec un mélange chlorure de méthylène/acétate d'éthyle/triéthylamine, 8/2/0,1%. Puis en éluant ensuite avec un mélange dichlorométhane/acétate d'éthyle/triéthylamine, 7/3/0,1%. Après évaporation des fractions, on obtient 32 mg de l'isomère A de composé trans-1-(aminocarbonyl),4,5,6,8-tétrahydro-6-oxo-5-(phénylméthoxy)-1*H*-4,7-méthanopyrazolo[3,4-*e*][1,3]diazépine-8-carboxylate de méthyle, avec un rendement de 15% et 79 mg de l'isomère B du composé trans-2-(aminocarbonyl)-2,5,6,8-tétrahydro-6-oxo-5-(phénylméthoxy)-4*H*-4,7-méthanopyrazolo[3,4-*e*][1,3]diazépine-8-carboxylate de méthyle avec un rendement de 37%. Les formules brutes des isomères sont C₁₇H₁₇N₅O₅ (M = 371,36 g).

### Stade B

On procède comme au Stade M de l'Exemple 1 avec 30 mg de l'isomère A obtenu au stade précédent, 9 mg de palladium sur charbon, 2 ml de méthanol et 0,5 ml d'eau. On obtient 22 mg du composé trans-1-(aminocarbonyl)-4,5,6,8-tétrahydro-5-hydroxy-6-oxo-1*H*-4,7-méthanopyrazolo[3,4-*e*][1,3]diazépine-8-carboxylate de méthyle, de formule brute C₁₀H₁₁N₅O₅ (M = 281,23 g).

Le rendement est quantitatif.

### Stade C

On procède comme au Stade M de l'Exemple 1 avec 0,022 g du produit obtenu au stade précédent, 0,037 g du complexe pyridine-SO₃ et 2 ml de pyridine. Le produit brut est purifié sur résine XAD4 avec un gradient eau/acétone. On obtient 13 mg du composé de sel de pyridinium de trans-1-(aminocarbonyl)-4,5,6,8-tétrahydro-6-oxo-5-(sulfooxy)-1*H*-4,7-méthanopyrazolo[3,4-*e*][1,3]diazépine-8-carboxylate de méthyle, de formule brute C₁₅H₁₆N₆O₈S, (M = 440,39 g).

Le rendement est de 64%.

LC/SM (électrospray négatif) : TR = 4,20 min MH⁻ = 360.

### Exemple 11

### Sel de pyridinium de trans-2-(aminocarbonyl)-2,5,6,8-tétrahydro-6-oxo-5-(sulfooxy)-4H-4,7-méthanopyrazolo[3,4-e][1,3]diazépine-8-carboxylate de méthyle

### Stade A

On procède comme au Stade M de l'Exemple 1 avec 0,079 g de l'isomère B obtenu au Stade A de l'Exemple 10, 0,010 g de palladium sur charbon, 4 ml de méthanol et 0,5 ml d'eau. On obtient 54 mg de composé trans-2-(aminocarbonyl)-2,5,6,8-tétrahydro-5-hydroxy-6-oxo-4*H*-4,7-méthanopyrazolo[3,4, *e*][1,3]diazépine-8-carboxylate de méthyle, de formule brute C₁₀H₁₁N₅O₅ (M = 281,23 g).

Le rendement correspondant est de 98%.

### Stade B

On procède comme au Stade M de l'Exemple 1 avec 0,059 g du produit obtenu au stade précédent, 0,1 g du complexe pyridine-SO₃, et 3 ml de pyridine. Le produit brut est purifié sur résine XAD4 avec un gradient eau/acétone. On obtient 40 mg du composé de sel de pyridinium de trans-2-(aminocarbonyl)-2,5,6,8-tétrahydro-6-oxo-5-(sulfooxy)-4*H*-4,7-méthanopyrazolo[3,4-*e*][1,3]diazépine-8-carboxylate de méthyle, de formule brute C₁₆H₁₅N₆O₆S, (M = 440,39 g).

Le rendement correspondant est de 45%.

LC/SM (électrospray négatif) : TR = 3,63 min MH⁻ = 360.

### Exemple 12

### Sel de sodium de 2-(4,5,6,8-tétrahydro-6-oxo-5-(sulfooxy)-1H-4,7-méthanopyrazolo[3,4-e][1,3]diazépin-1-yl)éthyl]-carbamate de 1,1-diméthpléthyle

### Stade A

Dans un ballon placé sous atmosphère inerte, on dissout 50 g de BOC-NH-NH₂ dans 250 ml de DMF anhydre. On refroidit à -10°C, puis on ajoute par petites fractions 16,5 g d'hydrure de sodium à 50% dans l'huile.

On ajoute ensuite du bromure de propylène et on laisse sous agitation pendant une nuit à température ambiante. Puis, on ajoute lentement de l'eau et une solution à 1M d'hydrogénophosphate de sodium, puis 200 ml d'un mélange AcOEt/heptane 2/1 puis on extrait et on sèche la phase organique sur du sulfate de magnésium.

On filtre et on évapore ensuite le solvant sous pression réduite. On purifie le produit brut obtenu sur silice en éluant avec un mélange dichlorométhane/AcOEt 95/5.

On recueille ainsi 24 g de 2-(2-propényl)-hydrazinecarboxylate de 1,1-diméthyléthyle pur.

Le rendement correspondant est de 76%.

### Stade B

On dissout 24 g du produit obtenu au stade A dans 80 ml d'AcOEt.

On refroidit à 0°C, puis on ajoute 332 ml d'une solution d'acide chlorhydrique 5,5 N dans l'AcOEt. On agiLe pendant 1 heure 30 à température ambiante, puis on filtre et on lave à l'éther.

On obtient ainsi 15 g de (2-propényl)-hydrazine de formule brute C₃H₈N₂, 2HCl sous la forme de cristaux blancs.

Le rendement correspondant est de 84%.

### Stade C

On dissout 11 g du produit de formule brute C₁₄H₂₁N₃O₄ obtenu au stade A2 de l'Exemple 1 dans 130 ml d'éthanol.

On ajoute 6,51 g du produit obtenu au stade B et 11,33 g de carbonate de potassium.

On agite la suspension pendant 45 minutes, puis on évapore l'éthanol sous pression réduite. On solubilise le résidu dans l'AcOEt, puis on lave la phase organique à l'eau, on la sèche ensuite sur du sulfate de magnésium, on filtre et on évapore le solvant sous pression réduite.

On obtient ainsi 10,8 g de 3,5-dioxo-4-[[2-(2-propényl)hydrazino]méthylène]-1-pipéridinecarboxylate de 1,1-diméthyléthyle de formule brute C₁₄H₂₁N₃O₃ (M = 295,34 g).

Le rendement correspondant est de 80%.

### Stade D

On dissout 10,8 g du produit obtenu au stade C dans 120 ml de toluène.

On ajoute 1 g d'acide p-toluène sulfonique monohydraté et on chauffe à reflux pendant une heure.

On laisse refroidir, on verse dans de l'AcOEt, on lave la phase organique à l'eau et on la sèche sur du sulfate de magnésium. On évapore le solvant sous pression réduite.

On obtient ainsi 8,5 g de produit brut que l'on purifie par chromatographie sur silice en éluant avec un mélange heptane/AcOEt 2/1.

On recueille ainsi 7,5 g de 4,7-dihydro-4-oxo-1-(2-propényl)-1H-pyrazolo[3,4-c]pyridine-6(5H)-carboxylate de 1,1-diméthyléthyle de formule brute C₁₄H₁₉N₃O₃ (M = 277,33 g).

Le rendement correspondant est de 74%.

### Stade E

Dans un ballon, on introduit 7,5 g du produit obtenu au stade D. On ajoute ensuite 4,74 g de O-benzylhydroxylamine puis 150 ml de pyridine. On agite pendant 1 heure à 20°C. On évapore ensuite le solvant sous pression réduite puis on dilue avec du dichlorométhane, on lave avec une solution aqueuse d'acide tartrique à 10%, puis à l'eau déminéralisée. On sèche ensuite la phase organique sur du sulfate de sodium et on évapore le solvant sous pression réduite. Le produit brut obtenu est ensuite chromatographié sur silice en éluant avec un mélange dichlorométhane/AcOEt 95/5 pour donner 9,72 g de 4,7-dihydro-4-[(phénylméthoxy)imino]-1-(2-propényl)-1H-pyrazolo[3,4-c]pyridine-6(5H)-carboxylate de 1,1-diméthyléthyle de formule brute C₂₁H₂₆N₄O₃ (M = 382,47 g).

Le rendement correspondant est de 90%.

### Stade F

On introduit 9,2 g du produit obtenu au stade E dans 750 ml de méthanol.On refroidit vers 0-5°C, puis on ajoute 24,2 g de NaBH₃CN et 36,51 ml d'éthérate de trifluorure de bore.On agite pendant 30 minutes en maintenant à 0-5°C, puis on laisse la température revenir à 20°C et on agite à cette température pendant 30 minutes. On verse ensuite le milieu réactionnel dans de l'eau saturée en hydrogénocarbonate de sodium. On agite pendant 45 minutes, puis on décante, on lave la phase organique à l'eau déminéralisée et on la sèche sur du sulfate de sodium. On évapore ensuite le solvant sous pression réduite pour obtenir le produit brut que l'on purifie par chromatographie sur silice en éluant avec du dichlorométhane contenant 2% d'acétone.

On obtient ainsi 5,3 g de 4,7-dihydro-4-[(phénylméthoxy)amino]-1-(2-propényl)-1H-pyrazolo[3,4-c]pyridine-6(5H)-carboxylate de 1,1-diméthyléthyle de formule brute C₂₁H₂₈N₄O₃ (M= 384,48 g).

Le rendement correspondant est de 60%.

### Stade G

On procède comme indiqué au stade I de l'Exemple 1 avec 5,25 g du produit obtenu au stade F et une solution de chlorure d'hydrogène 5,5 N. Sur le produit obtenu, on procède comme indiqué au stade J de l'exemple 1.

On obtient ainsi 3,95 g de N-(phénylméthoxy)-1-(2-propényl)-4,5,6,7-tétrahydxo-1H-pyrazolo[3,4-c]pyridin-4-amine de formule brute C₁₆H₂₀N₄O (M = 284,36 g).

Le rendement correspondant est de 90%.

### Stade H

On procède comme indiqué au stade K de l'Exemple 1 avec 3,8 g du produit obtenu au stade G, 4,2 ml de TEA et 0,8 ml de diphosgène.

On obtient ainsi 2,5 g de 5-(phénylméthoxy)-1-(2-propényl)-4,5,7,8-tétrahydro-4,7-méthano-pyrazolo[3,4-e][1,3]diazépin-6(1H)-one de formule brute C₁₇H₁₈N₄O₂ (M = 310,36 g).

Le rendement correspondant est de 68%.

### Stade I

6 g (19,33 mmoles) du produit obtenu au stade H précédent sont mis en solution dans 180 ml de THF, 180 ml de tert-butanol et 60 ml d'eau. On introduit 3,92 g (29 mmoles) de N-oxyde de N-méthyle-morpholine puis 2,98 ml (0,579 mmole) de tétraoxyde d'osmium. On agite 54 heures à température ambiante. Après évaporation du THF, le milieu est repris avec une solution aqueuse 1M de NaH₂PO₄. On extrait avec un mélange acétate d'éthyle/heptane à 20% puis au dichlorométhane/chlorure de méthylène et THF. Après séchage de la phase organique sur mgSO₄ puis évaporation des solvants sous pression réduite, on obtient 6,16 g de 1-(2,3-dihydroxypropyl)-1,4,5,8-tétrahydro-5-(phénylméthoxy)-6H-4,7-méthanopyrazolo[3,4-e][1,3]diazépin-6-one, de formule brute C₁₇H₂₀N₄O₄ (M = 344,37 g).

Le rendement correspondant obtenu est de 93%.

### Stade J

6,13 g (17,8 mmoles) du produit obtenu au stade précédent I est dissous dans 140 ml de THF. Puis on ajoute 45 ml de méthanol suivi de 45 ml d'eau. La solution obtenue est refroidie à 0°C. Puis on ajoute 6,08 g de métapériodate de sodium. On agite pendant 2 heures en laissant remonter la température à 20°C. Au bout de 2 heures, on rajoute 1,52 g de métapériodate de sodium et on agite à nouveau pendant 40 minutes. Une fois la réaction terminée, on ajoute 260 ml d'une solution aqueuse de NaH₂PO₄ 1M, puis on sature la solution avec du NaCl solide et on extrait au THF et avec un mélange acétate d'éthyle/heptane à 30%. La phase organique est lavée avec une solution aqueuse de NaH₂PO₄ saturée puis séchée sur mgSO₄. Après évaporation du solvant sous pression réduite on obtient 9,98 g de 4,5,6,8-tétrahydro-6-oxo-5-(phénylméthoxy)-1H-4,7-méthanopyrazolo[3,4-*e*][1,3]diazépine-1-acétaldéhyde, de formule brute C₁₆H₁₆N₄O₄ (M = 342,39 g).

Le rendement obtenu est quantitatif.

### Stade K

On dissout 5,6 g du produit obtenu au stade précédent J dans 100 ml d'éthanol puis on ajoute à 0°C 2,71 g de NaBH₄ par portions. On agite 2 heures à 0°C puis on évapore l'éthanol, on rajoute de la glace, du chlorure de méthylène et petit à petit une solution aqueuse 1M de NaH₂PO₄. Le dégagement gazeux est important. Puis on extrait la phase aqueuse avec du chlorure de méthylène et on procède à un lavage de la phase organique avec une solution de thiosulfate pour éliminer les résidus de NaIO₄. Après séchage de la phase organique sur mgSO₄, on évapore les solvants sous pression réduite.

On obtient un résidu solide qui est cristallisé dans un mélange d'éther éthylique et d'isopropanol. Après filtration, on obtient 3,45 g de 1,4,5,8-tétrahydro-1-(2-hydroxyéthyl)-5-(phénylméthoxy)-6H-4,7-méthano-pyrazolo[3,4-*e*][1,3]diazépin-6-one, de formule brute C₁₆H₁₈N₄O₃ (M = 314,35 g.

Le rendement correspondant est de 62%.

### Stade L

On dissout 1,35 g (4,29 mmoles) du produit obtenu au stade précédent K dans 50 ml de THF. Puis on ajoute à température ambiante, 0,69 ml de pyridine, suivi de 1,46 g de triphénylphosphine. On ajoute 1,42 g d'iode par portions puis après 2 heures, on rajoute 200 mg d'iode, 220 mg de triphénylphosphine et 0,13 ml de pyridine. On verse dans le milieu par une solution de NaH₂PO₄ puis on extrait par un mélange acétate d'éthyle/heptane et on lave la phase organique avec une solution aqueuse saturée de NaCl. Après évaporation des solvants sous pression réduite de la phase organique, on obtient 1,6 g de produit brut qui est purifié par chromatographie liquide en éluant avec un mélange dichlorométhane/acétonitrile à 10%. On obtient 1,60 g du produit 1,4,5,8-tétrahydro-1-(2-iodoéthyl)-5-(phénylméthoxy)-6H-4,7-méthanopyrazolo[3,4-*e*][1,3]diazépin-6-one, de formule brute C₁₇H₁₇IN₄O₂ (M = 424,24 g).

Le rendement obtenu est de 88%.

### Stade M

On dissout 408 mg du produit obtenu au stade précédent L dans 4 ml de DMF. On agite la solution à température ambiante en présence de 128 mg d'azoture de sodium. On agite pendant 5 heures puis on traite la solution par une solution aqueuse de NaH2PO4 et on extrait par un mélange acétate d'éthyle/heptane 1/1. On lave la phase organique avec une solution aqueuse saturée de NaCl puis on évapore les solvants sous pression réduite. On obtient 328 mg du composé 1-(2-azidoéthyl)-1,4,5,8-tétrahydro-5-(phénylméthoxy)-6*H*-4,7-méthanopyrazolo[3,4-*e*][1,3]diazépin-6-one de formule brute C₁₆H₁₇N₇O₂ (M = 339,36 g).

Le rendement correspondant est quantitatif.

### Stade N

On dissout 323 mg du produit obtenu au stade précédent dans 10 ml de THF anhydre. On refroidit la solution à 0°C puis on ajoute 300 mg de triphénylphosphine par portions. On agite le milieu réactionnel à température ambiante pendant 16 heures. Une fois la réaction terminée, on ajoute 345 µl d'eau déminéralisée et on agite pendant plusieurs heures. Après traitement, on obtient le composé 1-(2-aminoéthyl)-1,4,5,8-tétrahydro-5-(phénylméthoxy)-6*H*-4,7-méthanopyrazolo[3,4-*e*][1,3]diazépin-6-one de formule brute C₁₆H₁₉N₅O₂ (M = 313,36 g).

### Stade O

On dissout le composé obtenu au stade précédent dans 5 ml de THF anhydre. On ajoute 280 µl de triéthylamine puis 182 mg de Boc anhydride en solution dans 0,5 ml de THF. On agite la réaction pendant 2 heures puis on lave le milieu réactionnel par NaH₂PO₄, on l'extrait avec un mélange d'acétate d'éthyle contenant 20% d'heptane. La phase organique est séchée sur sulfate de magnésium, filtrée puis le solvant est évaporé sous pression réduite. Le résidu huileux obtenu est repris dans l'éther et trituré avec du pentane. Après filtration du Pφ₃O précipité, le filtrat est évaporé puis purifié par chromatographie sur silice en éluant avec un mélange toluène alcool isopropylique à 18 à 15%. On obtient après évaporation 362 mg du composé [2-[4,5,6,8-tétrahydro-6-oxo-5-(phénylméthoxy)-1*H*-4,7-méthanopyrazolo[3,4-*e*][1,3]diazépin-1-yl]éthyl]-carbamate de 1,1-dimthyléthyle de formule brute C₂₁H₂₇N₅O₄ (M = 413,48 g).

Le rendement correspondant est de 81%.

### Stade P

On procède comme au Stade M de l'Exemple 1 avec 339 mg du produit obtenu au stade précédent, 750 mg de palladium sur charbon dans 17 ml de mélange éthanol/acide acétique (1 goutte d'acide acétique pour 1 ml d'éthanol). On obtient 281 mg de composé [2-(4,5,6,8-tétrahydro-5-hydroxy-6-oxo-1*H*-4,7-méthanopyrazolo[3,4-*e*][1,3]diazépin-1-yl)éthyl]-carbamate de 1,1-diméthyléthyle de formule brute C₁₄H₂₈N₅O₄ (M = 323,35 g).

Le rendement obtenu est quantitatif.

### Stade Q

On procède comme au stade M de l'exemple 1 avec le produit brut obtenu au stade précédent, 400 mg du complexe pyridine-SO₃ et 6 ml de pyridine. On obtient les sels de pyridinium du produit attendu.

### Stade R

On reprend le sel de pyridinium obtenu au stade précédent dans une solution aqueuse contenant 10% de THF. On passe la solution sur 90 g de résine Dowex préalablement activée avec de la soude. Les fractions sont ensuite lyophilisées et on obtient 60 mg de produit brut qui est purifié dans de l'acétone. Après évaporation, le résidu est redissous dans 1 ml d'eau déminéralisée puis fixé. On obtient 255 mg de composé de sel de sodium de 2-(4,5,6,8-tétrahydro-6-oxo-5-(sulfooxy)-1*H*-4,7-méthanopyrazolo[3,4-*e*][1,3]diazépin-1-yl)éthyl]-carbamate de 1,1-diméthyléthyle de formule brute C₁₄H₂₀N₅O₇S Na (M = 425,20 g).

Le rendement obtenu est de 73%.

RMN du proton, DMSO-d₆, 300 MHz (déplacement chimique et multiplicité) :
1,38 (s) : 0-C-(CH₃)₃ ; 3,07 (d) et 3,49 (dd) : N-CH₂-CH ; 4,67 (d) : N-CH₂-CH ; 3,94 (m) : N-CH₂-CH₂-NH ; 3,20 (m) : N-CH₂-CH₂-NH ; 6, 95 (tl) : N-CH₂-CH₂-NH ; 4,26 et 4,33 : N-CH₂-C= ; 7,39 (s) : N=CH.

LC/SM (électrospray négatif), m/z : M⁻ = 402,1, (2M + Na⁺)⁻ = 827,2.

### Exemple 13

### Sel de sodium de 1-(2-aminoéthyl)-1,4,5,8-tétrahydro-5-(sulfooxy)-6H-4,7-méthanopyrazolo[3,4-e][1,3]diazépin-6-one

### Stade A

On dissout 140 mg du produit obtenu au Stade R de l'Exemple 12 dans 2 ml d'acide trifluoroacétique. La solution d'acide trifluoroacétique est préalablement refroidie à 0°C. On agite pendant 10 minutes à 0°C puis on évapore l'acide trifluoroacétique. Le résidu est traité plus d'une fois par ajout de toluène et celui-ci est évaporé afin d'éliminer l'acide trifluoroacétique résiduel. Le résidu est lavé par un mélange H₂O/THF à 10% puis séché sous vide. On obtient 90 mg du composé de sel de sodium de 1-(2-aminoéthyl)-1,4,5,8-tétrahydro-5-(sulfooxy)-6*H*-4,7-méthanopyrazolo[3,4-*e*][1,3]diazépin-6-one de formule brute C₉H₁₂N₅O₅S Na (M = 325,28 g).

RMN du proton, DMSO-d₆, 300 MHz, déplacement chimique et multiplicité.

3,09 (d) et 3,52 (dd) : N-CH₂-CH ; 4,72 (d) : N-CH₂-CH ; 4,38 (sl) : N-CH₂-C= ; 3,22 (tl), 4,13 (m) : N-CH₂-CH₂-NH₂ ; 7,86 (sl) : N-CH₂-CH₂-NH₂ ; 7,51 (s) : N=CH.

LC/SM (électrospray négatif), m/z : M⁻ = 302,1 g, (2M⁻ + H⁺)⁻ = 605,0 g et (2M⁻ + Na⁺)⁻ = 627,1 g.

### Exemple 14

### Sel de sodium de 1-[2-[(aminocarbonyl)oxy]éthyl]-1,4,5,8-tétrahydro-5-(sulfooxy)-6H-4,7-méthanopyrazolo [3,4-e][1,3]diazépin-6-one

### Stade A

On dissout 0,172 g du composé obtenu au Stade K de l'Exemple 12 dans du dichlorométhane. On ajoute à 0°C 122 mg de DMAP et 202 mg de p-NO₂ PhO-COCl. On agite une heure à 0°C sous atmosphère d'argon puis on évapore à sec et on solubilise le résidu dans du DMF. On fait ensuite barboter 20 secondes du NH₃ gazeux et on agite pendant 5 minutes. La suspension obtenue de couleur jaune est versée dans de l'acétate d'éthyle et lavée plusieurs fois avec une solution aqueuse de NaHCO₃ à 10%. La phase organique est évaporée et le résidu est réextrait par du THF. Après évaporation du THF, le résidu est repris dans l'acétate d'éthyle et on obtient 80 mg de cristaux blancs du composé 1-[2-[(aminocarbonyl) oxy]éthyl]-1,4,5,8-tétrahydro-5-(phénylméthoxy)-6*H*-4,7-méthanopyrazolo[3,4-e][1,3] diazépin-6-one de formule brute C₁₇H₁₉N₉O₄ (M = 357, 37 g).

Le rendement correspondant est de 81%.

### Stade B

On procède comme au Stade M de l'Exemple 1 avec 0,070 g du produit obtenu au stade précédent et 1,5 ml d'acide acétique. On obtient 52 mg du produit 1-[2-[(aminocarbonyl)oxy]éthyl]-1,4,5,8-tétrahydro-5-hydroxy-6*H*-4,7-méthanopyrazolo[3,4-e] [1,3]diazépin-6-one de formule brute C₁₀H₁₃N₅O₄ (M = 267,25 g).

Le rendement est quantitatif.

### Stade C

On procède comme au stade M de l'Exemple 1 avec 0,052 g du produit obtenu au stade précédent, 0,070 g du complexe pyridine-SO₃ et 2 ml de pyridine. On obtient le sel de pyridinium du composé attendu, celui-ci est ensuite traité comme au Stade R de l'Exemple 12 sur résine Dowex et on l'obtient 62 mg sous forme de cristaux jaunes du composé de sel de sodium de 1-[2-[(aminocarbonyl)oxy]éthyl]-1,4,5,8-tétrahydro-5-(sulfooxy)-6*H*-4,7-méthanopyrazolo[3,4-e][1,3]diazépin-6-one de formule brute C₁₀H₁₂N₅O₇S Na (M = 346,30 + 22,99 g).

Le rendement obtenu est de 78%.

LC/SM (électrospray négatif), m/z : M⁻ 346,1
RMN du proton, DMSO-d₆, 300 MHz, déplacement chimique et multiplicité :
3,11 (d) et 3,49 (dd) : N-CH₂-CH ; 4,68 (d) : N-CH₂-CH ; 4,12 (m) : N-CH₂-CH₂-O ; 6,54 (sl) : NH₂ et 7,39 (s) : N=CH.

### Exemple 15

### Sel de sodium de 4,5,6,8-tétrahydro-6-oxo-5-(sulfooxy)-1H-4,7-méthanopyrazolo[3,4-e][1,3]diazépine-1-acétamide

### Stade A

On dissout 0,54 g du produit obtenu au Stade J de l'Exemple 12 dans de l'acétonitrile. On ajoute à 0°C une solution de 30 mg de NaH₂PO₄ dans 0,3 ml d'eau. Puis on ajoute une solution 0,189 ml d'une solution de H₂O₂ à 30% et enfin, on ajoute goutte à goutte pendant 30 minutes une solution de 0,22 g de NaO₂Cl dans 2 ml d'eau. On laisse sous agitation à température ambiante pendant 4 heures. On ajoute ensuite une solution aqueuse de NaHCO₃ et on extrait plusieurs fois à l'acétate d'éthyle. La phase aqueuse est traitée par NaHSO₄ aqueux puis extraite par un mélange acétate d'éthyle-THF. La phase organique est séchée sur sulfate de magnésium, filtrée et après évaporation du solvant, on obtient 0,290 mg sous forme de cristaux blancs du composé acide 4,5,6,8-tétrahydro-6-oxo-5-(phénylméthoxy)-1*H*-4,7,méthanopyrazolo[3,4-*e*][1,3]diazépine-1-acétique de formule brute C₁₆H₁₆N₄O₄ (M = 328,33 g).

Le rendement correspondant est de 52%.

### Stade B

On dissout 0,29 g du composé obtenu au stade précédent dans 32 ml de DMF. On ajoute 0,59 g de BOP puis 0,188 g de HOBT. On agite 5 minutes puis on ajoute 0,099 g de NH₄Cl puis 0,64 ml de diisopropyléthylamine. On agite deux heures à température ambiante puis on verse le milieu réactionnel dans de l'acide chlorhydrique 0,1 N et on extrait avec du THF. La phase organique est lavée par une solution de NHCO₃ puis la phase organique est séchée sur sulfate de magnésium et le solvant est évaporé sous pression réduite. On obtient un produit brut qui est repris dans le méthanol. On obtient 0,066 g sous forme de cristaux blancs de composé 4,5,6,8-tétrahydro-6-oxo-5-(phénylméthoxy)-1*H*-4,7-méthanopyrazolo[3,4-*e*][1,3]diazépine-1-acétamide, de formule brute C₁₆H₁₇N₅O₃ (M = 327,35 g).

Le rendement correspondant est de 23%.

### Stade C

On procède comme au Stade M de l'Exemple 1 avec 56 mg du produit obtenu au stade précédent, 1 ml d'acide acétique et 20 mg de palladium sur charbon. On obtient 48 mg sous forme de cristaux blancs du composé 4,5,6,8-tétrahydro-5-hydroxy-6-oxo-1*H*-4,7-méthanopyrazolo[3,4-*e*][1,3]diazépine-1-acétamide de formule brute C₉H₁₁N₅O₃ (M = 237,22 g).

Le rendement correspondant est quantitatif.

### Stade D

On procède comme au Stade M de l'Exemple 1 avec 0,048 g du composé obtenu au stade précédent, 1,5 ml de pyridine et 0,1 g du complexe pyridine-SO₃. On obtient 33 mg du sel de pyridine attendu.

### Stade E

On procède comme au Stade R de l'Exemple 12 et l'on obtient le sel de sodium du composé attendu de sel de sodium de 4,5,6,8-tétrahydro-6-oxo-5-(sulfooxy)-1*H*-4,7-méthanopyrazolo[3,4-e][1,3]diazépine-1-acétamide de formule brute C₉H₁₀N₅O₆ SNa (M = 316,27 g + 23 g).

LC/SM (électrospray négatif) M⁻ = 316.

RMN du proton, DMSO-d₆, 300 MHz, déplacement chimique et multiplicité :
3,07 (d), 3,49 (dd) : N-CH₂-CH ; 4,68 (d) : N-CH₂-CH ; 4,23 et 4,37 : N-CH₂-C= ; 4,59 et 4,67 : CH₂-CO-NH₂ ; 7,36 ,(s) N=CH.

### Exemple 16

### Sel disodique de 1,4,5,8-tétrahydro-5-(sulfooxy)-1-[2-(sulfooxy)éthyl]-6H-4,7-méthanopyrazolo[3,4,-e][1,3]diazépin-6-one

### Stade A

On solubilise 0,080 g du produit obtenu au Stade K de l'Exemple 12 dans 1 ml d'éthanol et 0,5 ml de THF. On ajoute 20 mg de palladium sur charbon à 10% et on place le milieu sous hydrogène. Après 4 heures d'agitation à température ambiante, on filtre le catalyseur et on évapore à sec la solution. On obtient 0,059 g d'une résine qui est utilisée telle quelle pour l'étape suivante.

### Stade B

On solubilise 0,059 mg de la résine obtenue au stade précédent dans 2 ml de pyridine en présence de 0,250 g de complexe pyridine-SO₃. Après une nuit d'agitation à température ambiante, on évapore à sec la solution et le résidu est filtré sur résine Dowex préalablement préparée à la soude, l'élution s'effectue avec un mélange eau-THF, 90/10. Après évaporation à sec de la fraction, on reprend le résidu dans le méthanol puis dans l'éther. On obtient 0,70 g sous forme de cristaux jaunes du sel disodique de 1,4,5,8-tétrahydro-5-(sulfooxy)-1-[2-(sulfooxy)éthyl]-6*H*-4,7-méthanopyrazolo[3,4-e][1,3]diazépin-6-one de formule brute C₉H₁₀N₄O₉S₂ 2NA (M = 382,33 + 46 g).

Le rendement obtenu est de 65%.

RMN du proton, DMSO-d₆, 300 MHz, déplacement chimique et multiplicité :
3,02 (d), 3,47 (dd) : N-CH₂-CH ; 4,67 (d) : N-CH₂-CH ; 3,95 (m), 4, 13 (m) : N-CH₂-CH₂-O ; 4,32 et 4,41 : N-CH₂-C- ; 7, 32 (s) : N=CH.

LC/SM (électrospray négatif) (M²⁻ + Na)⁻ = 405,0 g. (M²⁻ + H)⁻ = 383,1 g.

### Exemple 17

### Sel de sodium de 4,5,6,8-tétrahydro-6-oxo-5-(sulfooxy)-1H-4,7-méthanopyrazolo[3,4-e][1,3]diazépine-1-carboxamide

### Stade A

On dissout 1,6 g du produit obtenu au stade L de l'exemple 12 dans 16 ml de DMF anhydre. On ajoute 260 mg de cyanure de potassium et on agite à température ambiante pendant 20 heures. On lave le milieu réactionnel à l'eau puis on extrait par un mélange acétate d'éthyle/heptane à 20%. On sèche la phase organique sur sulfate de magnésium puis après évaporation des solvants sous pression réduite, on obtient un résidu brut qui est purifié par chromatographie sur silice en éluant avec tout d'abord du dichlorométhane puis un mélange dichlorométhane/méthanol à 10%. Après évaporation des fractions contenant le produit attendu, on obtient 1,20 g de 4,5,6,8-tétrahydro-6-oxo-5-(phénylméthoxy)-1*H*-4,7-méthanopyrazolo[3,4-*e*][1,3]diazépine-1-propanenitrile, de formule brute C₁₇H₁₇N₅O₂ (M = 323,36 g).

Le rendement correspondant est de 98%.

### Stade B

On dissout 2,15 g du produit obtenu au stade précédent dans 20 ml de DMF anhydre. On refroidit la solution à 0°C puis on ajoute 290 mg de NaH à 50% dans l'huile. On agite pendant 3H30 à 0°C. On traite la solution par NaH₂PO₄ et on extrait avec un mélange acétate d'éthyle/heptane à 20%. Les phases organiques sont séchées sur sulfate de magnésium, filtrées et le solvant est évaporé sous pression réduite. On obtient 1,19 g de composé 1,4,5,8-tétrahydro-5-(phénylméthoxy)-6*H*-4,7-méthanopyrazolo[3,4-*e*][1,3]diazépin-6-one de formule brute C₁₄H₁₄N₄O₂ (M = 270,29 g).

Le rendement correspondant est de 42%.

### Stade C

On prépare une solution refroidie à 0°C de 451 mg du produit obtenu au stade précédent dans 45 ml de chlorure de méthylène anhydre. A cette solution, on ajoute 700 µl de triéthylamine puis 201 µl de diphosgène toujours à 0°C. On agite pendant 2,5 heures à 0°C puis on fait barboter de l'ammoniac pendant 20 minutes à 0°C. Le milieu réactionnel est traité par NaH₂PO₄ puis le milieu est évaporé à sec. Le résidu est trituré dans l'éther isopropylique et le pentane puis est isolé par filtration. Le résidu solide est purifié par chromatographie sur silice en éluant avec un mélange chlorure de méthylène/méthanol, 90/10. On obtient 200 mg du composé 4,5,6,8-tétrahydro-6-oxo-5-(phénylméthoxy)-1*H*-4,7-méthanopyrazolo[3,4-*e*][1,3]diazépine-1-carboxamide de formule brute C₁₅H₁₅N₅O₃ (M = 313,32 g).

Le rendement correspondant est de 38%.

### Stade D

On procède comme au Stade M de l'Exemple 1, avec 190 mg du produit obtenu au stade précédent, 475 mg de palladium sur charbon et 10 ml d'éthanol en présence de 1% d'acide acétique. On obtient 150 mg de composé 4,5,6,8-tétrahydro-5-hydroxy-6-oxo-1*H*-4,7-méthanopyrazolo[3,4-*e*][1,3]diazépine-1-carboxamide.

Le rendement correspondant est quantitatif.

### Stade E

On procède comme au Stade M de l'Exemple 1 avec 150 mg du produit obtenu au stade précédent, 427 mg du complexe pyridine-SO₃ et 3 ml de pyridine. On obtient 130 mg du sel de pyridinium attendu.

### Stade F

On procède comme au Stade R de l'Exemple 12 à partir de 130 mg du sel de pyridinium obtenu au stade précédent en présence de résine Dowex. On obtient 130 mg du composé sel de sodium de 4,5,6,8-tétrahydro-6-oxo-5-(sulfooxy)-1*H*-4,7-méthanopyrazolo[3,4-*e*][1,3]diazépine-1-carboxamide de formule brute C₆H₈N₅O₆S, Na (M = 325,24 g).

LC/SM (électrospray négatif) : m/z :
MH⁻ = 302 ; MH⁻ - CONH₂ = 259
RMN du proton, DMSO-d₆, 300 MHz, déplacement chimique et multiplicité :
3,22 (d), 3,48 (dd), 3,25 (d) et 3,56 (dd) : N-CH₂-CH ; 4,73 (d) et 4,79 (d) : N-CH₂-CH ; 4,22 et 4,36 (AB), 4,45 et 4,54 (AB) : N-CH₂=C ; 7,76(sl), 7,84(sl), 7,90(sl), 7,94(sl) : =OCNH₂ ; 7,69(s), 8,16(s) : N=CH-.

### Exemple 18

### Sel de sodium de 1,4,5,8-tétrahydro-1-[(4-méthoxyphényl)méthyl]-5-(sulfooxy)-6H-4,7-méthanopyrazolo[3,4-e][1,3]diazépin-6-one

### Stade A

On procède comme au Stade C de l'Exemple 12 avec 7,8 g du produit obtenu au Stade A de l'exemple 1, 6,03 g du produit obtenue au Stade Al de l'exemple 1, 2,93 g de NaHCO₃ et 100 ml d'éthanol. On obtient 8,74 g sous forme de poudre beige du composé 4-[[2-[(4-méthoxyphényl)méthyl]hydrazino]méthylène]-3,5-dioxo-1-pipéridinecarboxylate de 1,1-diméthyle de formule brute C₁₉H₂₅N₃O₅ (M = 375,43 g).

### Stade B

On procède comme au Stade D de l'Exemple 12 avec 8,74 g du produit obtenu au stade précédent, 800 mg d'acide paratoluène sulfonique et 250 ml de toluène. On obtient 5,30 g du composé 1,4,5,7-tétrahydro-1-[(4-méthoxyphényl)méthyl]-4-oxo-6*H-*pyrazolo[3,4-*c*]pyridine-6-carboxylate 1,1-diméthyléthyle de formule brute C₁₉H₂₃N₃O₄ (M = 357,41 g).

Le rendement correspondant est de 63%.

### Stade C

On procède comme au Stade E de l'Exemple 12 avec 5,3 g du produit obtenu au stade précédent, 1,79 g de chlorhydrate de O-benzylhydroxylamine et 5 ml de pyridine. On obtient 6,26 g de composé 1,4,5,7-tétrahydro-1-[(4-méthoxyphényl)méthyl]-4-[(2-propényloxy)imino]-6*H*-pyrazolo[3,4-*c*]pyridine-6-carboxylate de 1,1-diméthyléthyle, de formule brute C₂₂H₂₈N₄O₄ (M = 412,49 g).

Le rendement correspondant est quantitatif.

### Stade D

On procède comme au Stade F de l'Exemple 12 avec 300 mg du produit obtenu au stade précédent, 735 mg de NaBH₃CN, 920 µl de Et₂OBF₃ et 15 ml de méthanol. On obtient 55 mg du composé 1,4,5,7-tétrahydro-1-[(4-méthoxyphényl)méthyl]-4-[(2-propényloxy)amino]-6*H*-pyrazolo[3,4-*c*]pyridine-6-carboxylate de 1,1-diméthyléthyle de formule brute C₂₂H₃₀N₄O₄ (M = 414,51 g).

Le rendement est de 58%.

### Stade E

On procède comme au Stade G de l'Exemple 12 avec 3,28 g du produit obtenu au stade précédent, 10 ml d'acétate d'éthyle, 14,2 ml d'un mélange acétate d'éthyle/ acide chlorhydrique, et 11,8 ml de soude 2N. On obtient 2,34 g du composé 4,5,6,7-tétrahydro-1-[(4-méthoxyphényl)méthyl]-4-[(2-propényloxy)amino]-1*H*-pyrazolo[3,4-*c*]pyridine de formule brute C₁₇H₂₂N₄O₂ (M = 314,39 g).

Le rendement correspondant est de 94%.

### Stade F

On procède comme au Stade H de l'Exemple 12 avec 2,29 g du produit obtenu au stade précédent, 800 ml d'acétonitrile, 439 µl de diphosgène et 2,1 ml de triéthylamine. On obtient 1,79 g de composé 1,4,5,8-tétrahydro-1-[(4-méthoxyphényl)méthyl]-5-(2-propényloxy)-6*H*-4,7-méthanopyrazolo[3,4-*e*][1,3]diazépin-6-one de formule brute C₁₈H₂₀N₄O₃ (M = 340,39 g).

Le rendement correspondant est de 72%.

### Stade G

Dans un ballon placé sous atmosphère d'argon, on dissout 1,5 g du produit obtenu au stade précédent dans 30 ml de dichlorométhane. Puis on introduit successivement 504 µl d'acide acétique et 2,6 g de Pd (PPh3)4. On agite pendant 45 minutes à température ambiante puis on ajoute 30 ml de pyridine anhydre suivi de 2,8 g du complexe pyridine-SO3. On agite 2 heures à température ambiante. On évapore à sec le milieu réactionnel et on le reprend plusieurs fois avec du toluène pour entraîner la pyridine para-azéotrope. Le résidu est repris dans le chlorure de méthylène, lavé à l'eau, séché sur sulfate de magnésium puis évaporé à sec. Le résidu est purifié par chromatographie sur silice en éluant avec du chlorure de méthylène pur puis un mélange de chlorure de méthylène/acétone, 98/2, puis en éluant chlorure de méthylène/acétone, 92/8, et enfin en éluant chlorure de méthylène/acétone/triéthylamine, 0,6%. On obtient après évaporation des fractions, 2,22 g du sel de phosphonium attendu.

### Stade H

On procède comme au Stade R de l'Exemple 12 avec le sel de phosphonium obtenu au stade précédent et 500 g de résine Dowex. On obtient 1,29 g de sel de sodium de 1,4,5,8-tétrahydro-1-[(4-méthoxyphényl)méthyl]-5-(sulfooxy)-6*H*-4,7-méthanopyrazolo[3,4-*e*][1,3]diazépin-6-one de formule brute C₁₅H₁₅N₄O₆S, Na (M = 402,36 g).

Le rendement correspondant est de 77%.

LC/SM (électrospray négatif) : m/z :
M⁻ = 379,1 ; (2M+Na)⁻ = 781,2

RMN du proton, DMSO-d₆, 300 MHz, déplacement chimique et multiplicité :
3,09(d), 3,45(dd) : N-CH₂-CH ; 4,67(d) ; N-CH₂-CH ; 3,73(s) : CH₃-O-Ph ; 4,19 et 4,29 (AB) : N-CH₂-CN=C ; 5,07 et 5,14 (AB) : N-CH₂-Ph ; 6,88 et 7,13 (AA'BB') les 4 H aromatiques ; 7,39(s) N=CH.

### Exemple 19

### Sel de sodium de trans-2-(4-fluorophényl)-4,5,6,8-tétrahydro-6-oxo-5-(sulfooxy)-4,7-méthano-7H-thiéno[2,3-e][1,3]diazépine-8-carboxylate de méthyle.

### Stade A

On dissout 48,14 g (0,281 mole) d'alpha-amino-2-thiophèneacétate de méthyle de formule brute C₇H₁₉NO₂S (préparé à partir de l'acide alpha-aminothiophène acétique commercial selon une technique analogue à celle décrite dans J. Med. Chem., 26, 1267-1277 (1983)) dans 930 ml d'acétonitrile.

On ajoute 38,8 g de carbonate de potassium (0,281 mole) puis 55,5 ml de BrCH₂CO₂tBu (0,337 mole).

On chauffe à 70°C pendant 6 heures et demie, puis on laisse revenir à 20°C et on élimine les insolubles par filtration. On concentre partiellement sous pression réduite, on reprend avec 550 ml d'AcOEt, on lave avec de l'eau, puis avec une solution saturée de chlorure de sodium. On sèche la phase organique sur du sulfate de sodium, on filtre et on évapore le solvant sous pression réduite.

On obtient ainsi 90 g de alpha-[[[(1,1-diméthyléthoxy)carbonyl]méthyl]amino]-2-thiophéneacétate de méthyle de formule brute C₁₃H₁₉NO₄S (M = 285,36 g).

### Stade B

Dans un ballon placé sous atmosphère d'argon, on introduit 90 g du produit obtenu au stade A, 620 ml de THF anhydre, 61,7 ml de diisopropyléthylamine.

On refroidit vers 0-5°C, puis on ajoute 25,2 ml de chloroformiate de méthyle. On laisse en contact 1 heure 30 à 20°C.On dilue ensuite à l'AcOEt, puis on lave avec une solution aqueuse d'acide tartrique à 10% et à l'eau déminéralisée.

On sèche ensuite la phase organique sur du sulfate de magnésium, on filtre et on évapore le solvant sous pression réduite. On recueille 70,9 g de alpha-[[[(1,1-diméthyléthoxy)carbonyl]méthyl](méthoxycarbonyl)amino]-2-thiophéneacétate de méthyle de formule brute C₁₅H₂₁NO₆S (M = 343,40 g).

Le rendement correspondant aux stades A et B est de 73,4%.

### Stade C

Dans un ballon, on introduit 70 g du produit obtenu au stade B puis on refroidit vers 0-5°C et on ajoute 900 ml de mélange acide trifluoroacétique/dichlorométhane l/1.On laisse en contact à 20°C pendant 1 heure.

On obtient 75 g d'un produit brut qui est purifié de la manière suivante.On introduit les 75 g de produit brut dans 300 ml d'éther, puis on ajoute à 20°C, goutte à goutte, 33 ml de cyclohexylamine (0,29 mole).

Le sel ayant précipité est filtré et lavé 2 fois avec 50 ml d'éther.

Le produit obtenu est redissout dans 200 ml d'eau, puis on ajoute à 20°C, goutte à goutte, 36 ml d'acide chlorhydrique 6N, puis on décante, on extrait la phase aqueuse 2 fois avec 300 ml d'AcOEt.

On réunit les phases aqueuses et on les lave avec de l'eau, puis avec une solution de chlorure de sodium saturé.

On filtre et on sèche la phase organique sur sulfate de magnésium.

On évapore le solvant sous pression réduite.

On obtient ainsi 59,95 g de alpha-[(carboxyméthyl)(méthoxycarbonyl)amino]-2-thiophéneacétate de méthyle de formule brute C₁₁H₁₃NO₆S (M = 287,29 g).

Le rendement correspondant est quantitatif.

### Stade D

Dans un ballon équipé d'une agitation magnétique, d'un réfrigérant et d'une garde de chlorure de calcium, on introduit 49,76 g de l'acide obtenu au stade C puis 57 ml de SOCl₂.

On chauffe à 70°C et on maintient pendant 4 heures.

On évapore à sec sous pression réduite.

On obtient ainsi 44,5 g de 2,5-dioxo-alpha-(2-thiényl)-3-oxazolidineacétate de méthyle de formule brute C₁₀H₉NO₅S (M = 255,25 g). Le rendement est quantitatif.

### Stade E

Dans un ballon placé sous atmosphère d'azote, on introduit 44,5 g du produit obtenu au stade D et 500 ml de dichlorométhane.

On ajoute ensuite 92,3 g de chlorure d'aluminium.

On garde sous agitation pendant une nuit à 20°C, puis on dilue au dichlorométhane et amène à pH 8-9 par ajout d'une solution d'acide tartrique et d'ammoniaque en refroidissant. On dilue ensuite avec 1 l d'eau et 1 l de dichlorométhane.

On décante, on extrait à plusieurs reprises au dichlorométhane, on rassemble les phases organiques et on les sèche sur sulfate de sodium.

On évapore ensuite le solvant sous pression réduite.

On obtient ainsi 32,5 g de 4-oxo-4,5,6,7-tétrahydro-thiéno[2,3-c]pyridine-7-carboxylate de méthyle de formule brute C₉H₉NO₃S (M = 211,24 g).

Le rendement correspondant est de 88%.

### Stade F

Dans un ballon, on introduit 30 g du produit obtenu au stade E et 360 ml de THF.

On refroidit à 0°C, puis on ajoute 93 g de (BOC)₂O et on laisse réagir pendant 2 heures 30 à 20°C.

On dilue ensuite à l'AcOEt, lave avec une solution aqueuse d'acide tartrique à 10%, puis à l'eau déminéralisée.

On sèche ensuite la phase organique sur du sulfate de magnésium.

On évapore le solvant sous pression réduite et on purifie ensuite par chromatographie sur silice.

On obtient ainsi 27,91 g de 4,5-dihydro-4-oxo-thiéno[2,3-c]pyridine-6(7H),7-dicarboxylate de 6-(1,1-diméthyléthyle) et de 7-méthyle de formule brute C₁₄H₁₇NO₅S (M = 311,36 g).

Le rendement correspondant est de 63%.

### Stade G

Dans un ballon placé sous atmosphère d'azote et refroidi par un bain de glace, on introduit 50 g du produit obtenu au stade F (67,1 mmoles) et 1500 ml de méthanol. On ajoute ensuite 1,6 g de NaBH₄. On agite tout en laissant revenir à 20°C pendant 30 minutes.On dilue ensuite avec 225 ml de dichlorométhane, on lave avec une solution aqueuse d'acide tartrique à 10%, puis à l'eau déminéralisée, on sèche la phase organique sur du sulfate de sodium.On évapore le solvant sous pression réduite.On obtient 51,4 g de 4,7-dihydro-4-hydroxy-thiéno[2,3-c]pyridine-6,7(5H)-dicarboxylate de 6-(1,1-diméthyléthyle) et de 7-méthyle, de formule brute C₁₄H₁₅NO₅S (M = 313,38 g).

### Stade H

Dans un ballon placé sous atmosphère d'argon, on introduit 59,7 du produit obtenu au stade G et 583 ml de dichlorométhane.

On refroidit vers 0-5°C, puis on ajoute successivement 39,3 ml de TEA et 48,7 g de (CH₃SO₂)₂O. On laisse revenir la température à 20°C et on garde sous agitation pendant 1h20 à 20°C.

On dilue au dichlorométhane, puis on lave avec une solution aqueuse d'acide tartrique à 10% et à l'eau déminéralisée. On sèche la phase organique sur du sulfate de sodium.

On évapore ensuite le solvant sous pression réduite. On ajoute 68,9 g de benzyl-O-NH₂, puis on laisse en contact à 0-5°C pendant 72 heures.

On dilue ensuite au dichlorométhane et on lave avec une solution aqueuse d'acide tartrique à 10% puis à l'eau déminéralisée.

On sèche la phase organique sur du sulfate de sodium et on évapore le solvant sous pression réduite.

L'extrait sec obtenu est purifié par chromatographie sur silice en éluant avec un mélange dichlorométhane/AcOEt 98/2.

On obtient 47,0 g de 4,7-dihydro-4-[(phénylméthoxy)amino]-thiéno[2,3-c]pyridine-6,7(5H)-dicarboxylate de 6-(1,1-diméthyléthyle) et de 7-méthyle, de formule chimique C₂₁H₂₆O₅N₂S (M = 418,515 g).

Le rendement correspondant est de 60,2%.

### Stade I

On dissout 47 g du produit obtenu au stade H dans 79 ml d'AcOEt et on refroidit à 0°C.

On ajoute 261 ml d'une solution saturée de HCl gazeux dans l'acétate. On laisse en contact pendant 1 heure à 20°C.

On évapore le solvant sous pression réduite puis on cristallise le produit dans l'éther éthylique.

On obtient 44,12 g de chlorhydrate de 4,5,6,7-tétrahydro-4-[phénylméthoxy)amino]-thiéno[2,3-c]pyridine-7-carboxylate de méthyle, de formule brute C₁₆H₂₀N₂O₃S₂Cl₂ (M = 391,318 g).

### Stade J

On place les 44,1 g du produit obtenu du stade I en suspension dans 1000 ml de dichlorométhane.On ajoute 35 ml d'une solution d'ammoniaque concentrée. On décante, on lave à l'eau déminéralisée et on sèche la phase organique sur du sulfate de sodium.On évapore ensuite le solvant sous pression réduite.

On obtient 34,6 g de 4,5,6,7-tétrahydro-4-[(phénylméthoxy)amino]-thiéno[2,3-c]pyridine-7-carboxylate de méthyle, de formule brute C₁₆H₁₈N₂O₃S (M = 318,4 g).

Le rendement correspondant est de 96,7%.

### Stade K

Dans un ballon placé sous atmosphère d'argon et refroidit par un bain de glace, on introduit 34,1 g du produit obtenu du stade J, 8,8 l d'acétonitrile et 30,8 ml de TEA.

On agite pendant 2 minutes et on introduit ensuite 6,5 ml de diphosgène.

On agite la solution à 20°C pendant 1 heure.

On dilue à l'AcOEt et on lave avec une solution d'acide tartrique à 10% puis à l'eau.

On sèche la phase organique sur du sulfate de magnésium et on évapore le solvant sous pression réduite.

Le produit brut est dissout dans 1000 ml de dichlorométhane avec 1,2 ml de DBU. Après 10 minutes de contact, le mélange réactionnel est lavé par une solution d'acide tartrique à 10% puis à l'eau. Après évaporation du solvant sous pression réduite, on obtient un produit brut qui est purifié par chromatographie pour donner 37,2 g de trans 4,5,6,8-tétrahydro-6-oxo-5-(phénylméthoxy)-4,7-méthano-7H-thiéno[2,3-e][1,3]diazépine-8-carboxylate de méthyle, de formule brute C₁₇H₁₆N₂O₄S (M = 344,39 g).

Le rendement correspondant est de 80%.

### Stade L

Dans un ballon placé sous atmosphère d'argon, on dissout 211 mg de produit obtenu au stade précédent dans 2,2 ml d'acide acétique, puis on ajoute lentement 1,7 ml d'eau. Le milieu est refroidi à 3°C puis on ajoute lentement 31,5 µl de brome en solution dans 0,85 ml d'acide acétique. On agite 45 minutes jusqu'à l'apparition d'un important précipité crème. On introduit ensuite la suspension dans 20 ml d'une solution aqueuse à 0,5N de thiosulfate de sodium. On extrait à l'acétate d'éthyle et on lave la phase organique à plusieurs reprises avec une solution aqueuse saturée de bicarbonate de sodium puis avec une solution aqueuse saturée de chlorure de sodium. On sèche la phase organique sur mgSO₄, on filtre et on évapore le solvant sous pression réduite. On obtient 293 mg de produit brut qui sont purifiés par chromatographie sur colonne de silice en éluant avec un mélange chlorure de méthylène/acétate d'éthyle/triéthylamine 95/5/0,5. Après évaporation du solvant, on obtient 222 mg de composé trans-2-bromo-4,5,6,8-tétrahydro-6-oxo-5-(phénylméthoxy)-4,7-méthano-7*H*-thiéno[2,3-*e*][1,3]diazépine-8-carboxylate de méthyle de formule brute C₁₇H₁₅BrN₂O₄S (M = 423,29 g).

Le rendement correspondant est de 85,6%.

### Stade M

Dans un ballon placé sous atmosphère d'argon, on dissout 226 mg du produit obtenu au stade précédent dans 11,5 ml de toluène préalablement dégazé par barbotage d'argon. On ajoute ensuite 112 mg d'acide 4-fluorophényl-boronique. Puis 54 mg de Pd (PPh₃)₄. On ajoute ensuite 2,17 ml d'une solution aqueuse 2N de Na₂CO₃. On porte ensuite la solution au reflux pendant 4h30. On refroidit le milieu réactionnel et on le verse dans l'eau puis on extrait à l'acétate d'éthyle. La phase organique est lavée avec une solution aqueuse saturée en chlorure de sodium et séchée sur sulfate de magnésium. Le solvant est évaporé sous pression réduite et on obtient 126,1 mg de produit brut qui est purifié par chromatographie sur silice en éluant avec un mélange chlorure de méthylène/acétate d'éthyle/triéthylamine, 96/4/0,1%. On obtient après évaporation du solvant 211 mg de composé trans-2-(4-fluorophényl)-4,5,6,8-tétrahydro-6-oxo-5-(phénylméthoxy)-4,7-méthano-7*H*-thiéno[2,3-e][1,3]diazépine-8-carboxylate de méthyle de formule brute C₂₃H₁₉FN₂O₄S (M = 438,48 g).

Le rendement correspondant est de 90%.

### Stade N

Dans un ballon, on dissout 221,5 mg du produit obtenu au stade précédent dans 26,6 ml d'éthanol. On ajoute 221,5 mg de palladium sur charbon à 10% et on purge le milieu sous vide et on sature en hydrogène. Après 1h45 d'agitation, on filtre le catalyseur puis on évapore le solvant sous pression réduite. On obtient après évaporation du solvant 163,5 mg sous forme de cristaux de couleur crème du composé trans-2-(4-fluorophényl)-4,5,6,8-tétrahydro-5-hydroxy-6-oxo-4,7-méthano-7*H*-thiéno[2,3-*e*][1,3]diazépine-8-carboxylate de méthyle de formule brute C₁₆H₁₃FN₂O₄S (M = 348,35 g).

Le rendement correspondant est de 93%.

### Stade O

Dans un ballon, on dissout 176,4 mg du produit obtenu au stade précédent dans 2 ml de pyridine. On introduit ensuite 241 mg du complexe pyridine-SO₃ et on agite à température ambiante pendant 16h30. La solution jaune obtenue est purifiée par chromatographie sur silice en éluant avec un mélange chlorure de méthylène/méthanol 90/10. Après évaporation des fractions, on obtient 554,5 mg de composé sel de pyridinium de trans-2-(4-fluorophényl)-4,5,6,8-tétrahydro-6-oxo-5-(sulfooxy)-4,7-méthano-7H-thiéno[2,3-e][1,3]diazépine-8-carboxylate de méthyle de formule brute C₂₁H₁₈FN₃O₇N₂ (M = 507,51 g).

Le produit brut est purifié après passage au sel de sodium.

### Stade P

On procède à l'échange de sel en faisant passer 554,5 mg du sel de pyridinium obtenu au stade précédent sur 58 g de résine Dowex préalablement préparée avec une solution de soude aqueuse 2N. Le produit déposé sur la colonne de résine Dowex est élué par de l'eau contenant 10% de THF. Après avoir assemblée les fractions et évaporé le solvant sous pression réduite, on effectue une lyophylisation et on obtient 332,8 mg qui est purifié par empatage dans le méthanol puis dans l'éther éthylique. On obtient finalement 175,2 mg de composé sel de sodium de trans-2-(4-fluorophényl)-4,5,6,8-tétrahydro-6-oxo-5-(sulfooxy)-4,7-méthano-7*H*-thiéno[2,3-*e*][1,3]diazépine-8-carboxylate de méthyle de formule brute C₁₆H₁₂FO₇N₂S₂,Na (M = 450,40 g).

Le rendement correspondant est de 79%.

Spectre RMN, D₂O à 300 MHz, déplacement chimique et multiplicité :
3,89 (s) ; CH₃-O-CO ; 5,49 (s) : CH₃-O-CO-CHN ; 3,52 (t) et 3,79 (d1) : N-CH₂-CH-N ; 4,89 (sl) : N-CH₂-CH-N; 7,11 et 7,51 : H aromatiques ; 7,23 (s) : CH=C=S.

LC/SM (électrospray négatif), m/z : M⁻ = 427et (2M+Na)⁻ =877.

### Exemple 20

### Sel de sodium de trans- 4,5,6,8-tétrahydro-6-oxo-2-(3-pyridinyl)-5-(sulfooxy)-4,7-méthano-7H-thiéno[2,3-e][1,3]diazépine-8-carboxamide

### Stade A

On procède comme au stade A de l'Exemple 7 avec 2,5 g du produit obtenu au Stade L de l'Exemple 19, 11,2 ml de dioxanne, 11,2 ml d'eau, 5,95 ml de soude aqueuse 1N. On obtient 1,82 g de composé trans-acide 2-bromo-4,5,6,8-tétrahydro-6-oxo-5-(phénylméthoxy)-4,7-méthano-7*H*-thiéno[2,3-e][1,3]diazépine-8-carboxylique de formule brute C₁₆H₁₃BrO₄N₂S (M = 409,27 g).

Le rendement correspondant est de 75,7%.

### Stade B

On procède comme au Stade B de l'Exemple 7 avec 1,82 g de produit obtenu au stade précédent, 21 ml de DMF, 2,95 g de BOP, 0,9 g de HOBT, 476,5 mg de NH₄Cl, 3,1 ml de DIPEA. On obtient 870 mg du composé trans-2-bromo-4,5,6,8-tétrahydro-6-oxo-5-(phénylméthoxy)-4,7-méthano-7*H*-thiéno[2,3-e][1,3]diazépine-8-carboxamide de formule brute C₁₆H₁₄BrN₃O₃S (M = 408, 28 g).

Le rendement correspondant est de 48%.

### Stade C

Dans un ballon placé sous atmosphère d'argon, on dissout 305,5 mg de produit obtenu au stade précédent dans 24 ml de 1,4-dioxanne, puis on ajoute à la solution 413 mg de 3-tri-N-butylstanylpyridine et 86,4 mg de Pd (PPh₃)₄. On chauffe la solution à 100°C pendant 6 heures puis on rajoute à nouveau 86,4 mg de Pd (PPh₃)₄. On agite à nouveau à 100°C pendant 17 heures, le solvant est évaporé sous pression réduite. L'extrait sec est repris dans 50 ml d'acétate d'éthyle auxquels on rajoute 50 ml d'une solution aqueuse de KF. La phase aqueuse est à nouveau extraite à l'acétate d'éthyle et lavée avec une solution aqueuse saturée de chlorure de sodium, les phases organiques sont rassemblées, séchées sur sulfate de magnésium et le solvant est évaporé sous pression réduite. On obtient 590 mg de produit brut qui est purifié par chromatographie sur colonne de silice en éluant avec un mélange chlorure de méthylène/méthanol/triéthylamine, 95/5/0,1%. On obtient 92,4 mg de composé trans-4,5,6,8-tétrahydro-6-oxo-5-(phénylméthoxy)-2-(3-pyridinyl)-4,7-méthano-7*H*-thiéno[2,3-*e*][1,3]diazépine-8-carboxamide de formule brute C₂₁H₁₈O₃N₄S (M = 406,47 g).

Le rendement correspondant est de 30%.

### Stade D

On procède comme au Stade M de l'Exemple 1 avec 55 mg du produit obtenu au stade précédent, 15 ml d'éthanol et 55 mg de palladium sur charbon en présence d'hydrogène. On obtient 26 mg de composé trans-4,5,6,8-tétrahydro-5-hydroxy-6-oxo-2-(3-pyridinyl)-4,7-méthano-7*H*-thiéno[2,3-*e*][1,3]diazépine-8-carboxamide de formule brute C₁₄H₁₂O₃N₄S (M = 316,34 g).

Le rendement correspondant est de 62%.

### Stade E

On procède comme au Stade N de l'Exemple 1 avec 26,6 mg du produit obtenu au stade précédent, 0,34 ml de pyridine et 40,1 mg du complexe pyridine-SO₃. On obtient 71,5 mg sous forme d'huile jaune du composé sel de pyridinium de trans- 4,5,6,8-tétrahydro-6-oxo-2-(3-pyridinyl)-5-(sulfooxy)-4,7-méthano-7*H-*thiéno[2,3-*e*][1,3]diazépine-8-carboxamide de formule brute C₁₉H₁₇O₆N₅S₂ (M = 475,05 g).

### Stade F

On procède comme au Stade R de l'Exemple 12 avec 10 g de résine Dowex, 71,5 mg du produit obtenu au stade précédent et 0,5 ml d'eau à 10% en THF. On obtient 26,4 g sous forme de poudre crème du composé sel de sodium de trans- 4,5,6,8-tétrahydro-6-oxo-2-(3-pyridinyl)-5-(sulfooxy)-4,7-méthano-7*H-*thiéno[2,3-*e*][1,3]diazépine-8-carboxamide de formule brute C₁₄H₁₁O₆M₄S₂,Na (M = 418,38 g).

Le rendement correspondant est de 75%.

### Exemple 21

### Sel de sodium de trans-2-(aminocarbonyl)-4,5,6,8-tétrahydro-6-oxo-5-(sulfooxy)-4,7-méthano-7H-thiéno[2,3-e][1,3]diazépine-8-carboxylate de méthyle.

### Stade A

Dans un ballon placé sous atmosphère d'argon, on dissout 1 g du produit au Stade L de l'Exemple 19 dans 80 ml de toluène préalablement dégazé par barbotage d'argon. On ajoute ensuite 1,12 g de vinyl tributyl-stanone, puis 272 mg de Pd(PPh₃)₄. On porte la suspension à 100°C et on agite pendant une heure. Après 40 minutes d'agitation, on refroidit le milieu réactionnel à température ambiante et on évapore le solvant sous pression réduite. On reprend le résidu par 120 ml d'acétate d'éthyle et 120 ml d'une solution aqueuse de KF. Après extraction, la phase organique est lavée avec une solution aqueuse saturée de chlorure de sodium, puis séchée sur sulfate de magnésium et le solvant est évaporé sous pression réduite. On obtient 1,67 g de produit brut qui est purifié par chromatographie sur silice en éluant avec un mélange chlorure de méthylène/acétate d'éthyle/ triéthylamine 95/5/0,1%. On obtient 405,7 mg de composé trans-2-éthényl-4,5,6,8-tétrahydro-6-oxo-5-(phénylméthoxy)-4,7-méthano-7*H-*thiéno[2,3-*e*][1,3]diazépine-8-carboxylate de méthyle de formule brute C₁₉H₁₈N₂O₄S (M = 370,43 g).

Le rendement correspondant est de 46%.

### Stade B

Dans un ballon on dissout 513 mg du produit obtenu au stade précédent, 8,2 ml de THF, 4,1 ml d'eau et 8,2 ml de tert-butanol. A la solution obtenue précédemment, on ajoute 220 µl de O_{S}O₄ en solution à 5% dans l'eau et 888 mg de NaIO₄. On obtient une suspension que l'on agite à température ambiante pendant 1 heure. Puis on verse le milieu réactionnel dans de l'eau et on extrait à l'acétate d'éthyle. La phase organique est lavée à l'eau puis avec une solution aqueuse saturée de chlorure de sodium et séchée sur sulfate de magnésium. Après évaporation sous pression réduite des solvants, on obtient 534,6 mg de produit brut qui est purifié par chromatographie sur colonne de silice en éluant avec un mélange chlorure de méthylène/acétate d'éthyle/ triéthylamine, 90/10/0,1%. On obtient 286,6 mg de composé trans-2-formyl-4,5,6,8-tétrahydro-6-oxo-5-(phénylméthoxy)-4,7-méthano-7H-thiéno[2,3-*e*][1,3]diazépine-8-carboxylate de méthyle, de formule brute C₁₈H₁₆N₂O₅S (M = 372,40 g).

Le rendement correspondant est de 55,5%

### Stade C

On dissout 297 mg du produit obtenu au stade précédent dans 30 ml d'acétone. On ajoute ensuite 189 mg de KMnO₄ en poudre puis 30 ml d'eau. On agite la suspension à température ambiante pendant 1H30, puis on rajoute 30 ml d'acétone et on agite à nouveau la suspension pendant 30 minutes. Après évaporation de l'acétone sous pression réduite, on dilue le milieu réactionnel à l'eau et on acidifie avec 2 ml d'une solution aqueuse HCl 1N. On extrait à l'acétate d'éthyle et on lave la phase organique avec une solution aqueuse saturée en chlorure de sodium. On sèche la phase organique sur sulfate de magnésium et on évapore le solvant sous pression réduite. On obtient 311,8 mg de composé trans-4,5,6,8-tétrahydro-6-oxo-5-(phénylméthoxy)-4,7-méthano-7*H*-thiéno[2,3-*e*][1,3]diazépine-2,8-dicarboxylate de 8-méthyle de formule brute C₁₆H₁₆N₂O₆S (M = 388,40 g).

Le rendement est quantitatif.

### Stade D

On procède comme au Stade B de l'Exemple 7 avec 69 mg du produit obtenu au stade précédent, 0,84 ml de DMF, 117,8 mg de BOP, 36 mg de HOBT, 19 mg de NH₄Cl et 0,124 ml du DIPEA. On obtient 44,6 mg d'un composé trans-2-(aminocarbonyl)-4,5,6,8-tétrahydro-6-oxo-5-(phénylméthoxy)-4,7-méthano-7*H*-thiéno[2,3-*e*][1,3]diazépine-8-carboxylate de méthyle de formule brute C₁₈H₁₇N₅O₃S (M = 387,41 g).

Le rendement correspondant est de 64,8%.

### Stade E

On procède comme au Stade M de l'Exemple 1 avec 44,6 mg de produit obtenu au stade précédent, 4,5 ml d'éthanol et 44,6 mg de catalyseur palladium sur charbon à 10%. On obtient 24,3 mg de composé trans-2-(aminocarbonyl)-4,5,6,8-tétrahydro-5-hydroxy-6-oxo-4,7-méthano-7H-thiéno[2,3-e][1,3]diazépine-8-carboxylate de méthyle de formule brute C₁₁H₁₁O₅N₃S (M = 297,29 g).

Le rendement correspondant est de 71%.

### Stade F

On procède comme au Stade M de l'Exemple 1 avec 24,3 mg du produit obtenu au stade précédent, 0,32 ml de pyridine et 39 mg du complexe pyridine-SO₃. On obtient 74,6 mg du composé sel de pyridinium de trans-2-(aminocarbonyl)-4,5,6,8-tétrahydro-6-oxo-5-(sulfooxy)-4,7-méthano-7*H*-thiéno[2,3-*e*][1,3]diazépine-8-carboxylate de méthyle de formule brute C₁₆H₁₆O₈N₄S₂ (M = 456,45 g).

Le produit brut est ensuite transformé en sel de sodium.

### Stade G

On procède comme au Stade R de l'Exemple 12 avec 74,6 mg du produit obtenu au stade précédent, 10 g de résine Dowex. On obtient 19,9 mg de composé sel de sodium de trans-2-(aminocarbonyl)-4,5,6,8-tétrahydro-6-oxo-5-(sulfooxy)-4,7-méthano-7*H*-thiéno[2,3-*e*][1,3]diazépine-8-carboxylate de méthyle de formule brute C₁₁H₁₀O₈N₃S₂,Na (M = 399,34 g).

Le rendement correspondant est de 61%.

LC/SM (électrospray négatif), m/z : M⁻ = 376.

RMN du proton, D₂O à 300 MHz, déplacement chimique et multiplicité :
3,63 (d) et 3,82 (dd) et 3,66 (d) et 3,98 (dd) : N-CH₂-CH ; 4, 95 (d) et 5,01 (d) : N-CH₂-CH ; 5,64 (s) : CH=C-O-OCH₃ ; 7, 67 (s) et 7,70 (s) : H du furane ; 3,91 (s), 3,93 (s) : CH₃-O-CO.

### Exemple 22

### Sel de sodium de trans-4,5,6,8-tétrahydro-6-oxo-2-[[[2-(4-pyridinyl)éthyl]amino]carbonyl]-5-(sulfooxy)-4,7-méthano-7H-thiéno[2,3-e][1,3]diazépine-8-carboxylate de méthyle

### Stade A

On procède comme au Stade B de l'Exemple 7 avec 196 mg du produit obtenu au Stade C de l'Exemple 21, 2,24 ml de DMF, 335 mg de BOP, 102 mg de HOBT, 123 g de 4,2-aminoéthyl pyridine et 176 µl de DIPEA. On obtient 203 mg de produit trans-4,5,6,8-tétrahydro-6-oxo-5-(phénylméthoxy)-2-[[[2-(4-pyridinyl)éthyl]amino]carbonyl]-4,7-méthano-7*H*-thiéno[2,3-*e*][1,3]diazépine-8-carboxylate de méthyle de formule brute C₂₅H₂₄O₅N₄S (M = 492,56 g).

Le rendement correspondant est de 70,7%.

### Stade B

On procède comme au Stade M de l'Exemple 1 avec 50 mg de produit obtenu au stade précédent, 4,5 ml d'éthanol, 50 mg de palladium sur charbon à 10%. On obtient 36,8 mg de composé trans-4,5,6,8-tétrahydro-5-hydroxy-6-oxo-2-[[[2-(4-pyridinyl)éthyl]amino]carbonyl]-4,7-méthano-7*H*-thiéno[2,3-*e*][1,3]diazépine-8-carboxylate de méthyle de formule brute C₁₈H₁₈O₅N₄S (M = 402,43 g).

Le rendement correspondant est de 98%.

### Stade C

On procède comme au Stade M de l'Exemple 1 avec 36,8 mg du produit obtenu au stade précédent, 0,36 ml de pyridine et 43,7 mg de complexe pyridine-SO₃. On obtient 93,7 mg de composé sel de pyridinium de trans-4,5,6,8-tétrahydro-6-oxo-2-[[[2-(4-pyridinyl)éthylamino] carbonyl]-5-(sulfooxy)-4,7-méthano-7*H-*thiéno[2,3-*e*][1,3]diazépine-8-carboxylate de méthyle de formule brute C₂₅H₂₃O₈N₅S₂ (M = 561,59 g).

Le produit brut est ensuite transformé en sel de sodium.

### Stade D

On procède comme au Stade R de l'Exemple 12 avec 93,7 mg du produit obtenu au stade précédent, 11 g de résine Dowex. Le lyophilisat est obtenu après passage sur résine Dowex et dissous dans 1 ml d'eau puis est fixé sur une colonne de 54 ml de résine DIAION HP20. On élue le produit successivement avec un mélange eau/acétone 95/5, puis avec un mélange acétone/eau 10/90, acétone/eau 20/80. Après évaporation des fractions obtenues dans l'élution acétone/eau 20/80, on obtient 12,2 mg de composé sel de sodium de trans-4,5,6,8-tétrahydro-6-oxo-2-[[[2-(4-pyridinyl)éthyl]amino]carbony]-5-(sulfooxy)-4,7-méthano-7*H*-thiéno[2,3-*e*][1,3]diazépine-8-carboxylate de méthyle de formule brute C₁₈H₁₇O₆N₄S₂,Na (M = 504,48 g).

Le rendement correspondant est de 23%.

LC/SM (electrospray négatif) m/z : M⁻ = 481

### Exemple 23

### Sel de sodium de 4,5,6,8-tétrahydro-6-oxo-N²-[2-(4-pyridinyl)éthyl]-5-(sulfooxy)- 4,7-méthano-7H-thiéno[2,3-e][1,3]diazépine-2,8-dicarboxamide

### Stade A

On procède comme au Stade A de l'Exemple 7 avec 126 mg du produit obtenu au Stade A de l'Exemple 22, 1,74 ml de dioxanne, 0,64 ml d'eau et 281 µl de soude aqueuse 1N. On obtient 124 mg de composé trans-acide-4,5,6,8-tétrahydro-6-oxo-5-(phénylméthoxy)-2-[[[2-(4-pyridinyl)éthyl]amino]carbonyl]-4,7-méthano-7*H*-thiéno[2,3-*e*][1,3]diazépine-8-carboxylique de formule brute C₂₄H₂₂O₅N₄S (M = 478,53 g).

Le rendement correspondant est quantitatif.

### Stade B

On procède comme au Stade B de l'Exemple 7 avec 124 mg du produit obtenu au stade précédent, 162 mg de BOP, 51,8 mg de HOBT, 27,4 mg de NH₄Cl, 178 µl de DIPEA. On obtient 35,8 mg de composé trans-4,5,6,8-tétrahydro-6-oxo-5-(phénylméthoxy)-*N²*-[2-(4-pyridinyl)éthyl]-4,7-méthano-7*H*-thiéno[2,3-*e*][1,3]diazépine-2,8-dicarboxamide de formule brute C₂₄H₂₃O₄N₅S (M = 477,55 g).

Le rendement correspondant est de 29,3%.

### Stade C

On procède comme au stade M de l'Exemple 1 avec 36,4 mg du produit obtenu au stade précédent, 4 ml d'éthanol absolu, 72,8 mg de palladium sur charbon. On obtient 19,6 mg de composé trans-4,5,6,8-tétrahydro-5-hydroxy-6-oxo-*N*²-[2-(4-pyridinyl)éthyl]-4,7-méthano-7*H*-thiéno[2,3-*e*][1,3]diazépine-2,8-dicarboxamide de formule brute C₁₇H₁₇O₄N₅S (M = 396,42 g).

Le produit brut est utilisé sans purification au stade suivant.

### Stade D

On procède comme au Stade M de l'Exemple 1 avec 19,6 mg du produit obtenu au stade précédent, 0,20 ml de pyridine et 24 g de complexe pyridine-SO₃. On obtient 53,6 mg du composé sel de pyridinium de 4,5,6,8-tétrahydro-6-oxo-*N²*-[2-(4-pyridinyl)éthyl]-5-(sulfooxy)- 4,7-méthano-7*H*-thiéno[2,3-*e*][1,3]diazépine-2,8-dicarboxamide de formule brute C₂₂H₂₂O₇N₆S₂ (M = 546,58 g).

Le produit brut est transformé en sel de sodium.

### Stade E

On procède comme au Stade R de l'Exemple 4 avec 53,8 mg du produit obtenu au stade précédent, et 6,02 g de résine Dowex. Le lyophilisat obtenu est purifié sur résine DIAION HP20et l'on obtient 3,3 mg du composé sel de sodium de 4,5,6,8-tétrahydro-6-oxo-*N*²-[2-(4-pyridinyl)éthyl]-5-(sulfooxy)- 4,7-méthano-7*H*-thiéno[2,3-*e*][1,3]diazépine-2,8-dicarboxamide de formule brute C₁₇H₁₆O₇N₅S₂ (M = 489,46 g).

Le rendement correspondant est de 13,3%.

LC/SM (electrospray negatif) : m/z : M⁻ = 466

### Exemple 24

### Sel de sodium de trans-4,5,6,8-tétrahydro-2-(2-méthylphényl)-6-oxo-5-(sulfooxy)- 4,7-méthano-7H-thiéno[2,3-a][1,3]diazépine-8-carboxamide

### Stade A

Dans un ballon de 60 ml muni d'une agitation magnétique, on dissout sous argon, 5,09 g de 4,5,6,8-tétrahydro-6-oxo-5-(phénylméthoxy) -4, 7-méthano-7*H*-thiéno[2,3-*e*][1,3]diazépine-8-carboxylate de méthyle préparé au stade K de l'exemple 19 dans 100 ml CCl₄, on refroidit au bain de glace, puis on ajoute 1,87 g (7,4 mmoles) d'iode, 3,813 g de PhI(OCOCF₃)₂. On laisse revenir à 20°C. Après 2 à 3 h d'agitation à 20°C, on verse la solution dans une solution aqueuse 0,5N de thiosulfate de sodium, on extrait à l'acétate d'éthyle, puis on lave de nouveau avec une solution aqueuse de thiosulfate de sodium à 0,5N, on lave avec une solution aqueuse saturée de chlorure de sodium, puis avec une solution de tampon phosphate, pH = 7,0, et enfin avec une solution aqueuse saturée de chlorure de sodium. Après séchage sur mgSO₄ et évaporation du solvant sous pression réduite, on obtient le produit brut qui est purifié par chromatographie sur silice en éluant avec un mélange CH₂Cl₂/acétate d'éthyle (95/5), TEA = 0,1%.

On obtient 4,88 g de 4,5,6,8-tétrahydro-2-iodo-6-oxo-5-(phénylméthoxy)-4,7-méthano-7*H*-thiéno[2,3-*e*][1,3]diazépine-8-carboxylate de méthyle, de formule brute C₁₇H₁₅N₂IO₄S (M = 470,29 g).

Le rendement est de 70%.

### Stade B

On procède comme au Stade M de l'Exemple 19 avec 250 mg du produit obtenu au stade précédent, 11,5 ml de toluène, 108,4 mg d'acide méthyl-2-phénylboronique, 61,43 mg de Pd(PPh₃)₄ et 2,15 ml d'une solution aqueuse de Na₂CO₃ 2N. On obtient 229 mg du composé trans-4,5,6,8-tétrahydro-2-(2-méthylphényl)-6-oxo-5-(phénylméthoxy)-4,7-méthano-7*H-*thiéno[2,3-*e*][1,3]diazépine-8-carboxylate de méthyle de formule brute C₂₄H₂₂N₂O₄S (M = 434,52 g).

Le rendement correspondant est de 99%.

### Stade C

On procède comme au Stade A de l'Exemple 7 avec 265,4 mg de produit obtenu au stade précédent, 3,5 ml de dioxanne, 1,45 ml d'eau et 0,64 ml de soude aqueuse 1N. On obtient 235,5 mg de composé trans-acide 4,5,6,8-tétrahydro-2-(2-méthylphényl)-6-oxo-5-(phénylméthoxy)- 4,7-méthano-7*H*-thiéno[2,3-*e*][1,3]diazépine-8-carboxylique de formule brute C₂₃H₂₀N₂O₄S (M = 420, 49 g).

Le rendement correspondant est de 92%.

### Stade D

On procède comme au Stade B de l'Exemple 7 avec 232 mg de produit obtenu au stade précédent, 3 ml de DMS, 365 mg de BOP, 111,6 mg de HOBT, 59 mg de NH₄Cl, 383 µl de DIPEA. On obtient 196,1 mg de composé trans-4,5,6,8-tétrahydro-2-(2-méthylphényl)-6-oxo-5-(phénylméthoxy)- 4,7-méthano-7*H-*thiéno[2,3-*e*][1,3]diazépine-8-carboxamide de formule brute C₂₃H₂₁N₃O₃S, (M = 420,49 g).

Le rendement correspondant est de 85%.

### Stade E

On procède comme au Stade M de l'Exemple 1 avec 177 mg du produit obtenu au stade précédent, 35 ml d'éthanol, 17 ml de THF et 177 mg de palladium sur charbon à 30%. On obtient 123 mg du composé trans-4,5,6,8-tétrahydro-5-hydroxy-2-(2-méthylphényl)-6-oxo 4,7-méthano-7*H*-thiéno[2,3-*e*][1,3]diazépine-8-carboxamide de formule brute C₁₆H₁₅N₃O₃S (M = 329,38 g).

Le rendement correspondant est de 83%.

### Stade F

On procède comme au stade N de l'Exemple 1 avec 123 mg de produit obtenu au stade précédent, 2 ml de pyridine, 174 mg de complexe SO₃-pyridine. On obtient 100 mg de composé sel de triéthylammonium de trans-4,5,6,8-tétrahydro-2-(2-méthylphényl)-6-oxo-5-(sulfooxy)- 4,7-méthano-7*H*-thiéno[2,3-*e*] [1,3]diazépine-8-carboxamide de formule brute C₂₂H₃₀O₆N₄S₂ (M = 510,63 g).

Le rendement correspondant est de 52,4%.

### Stade G

On procède comme au Stade R de l'Exemple 12 avec 100 mg du produit obtenu au stade précédent et 30 g de résine Dowex. On obtient 76,8 mg de composé sel de sodium de trans-4,5,6,8-tétrahydro-2-(2-méthylphényl)-6-oxo-5-(sulfooxy)- 4,7-méthano-7*H*-thiéno[2,3-*e*][1,3]diazépine-8-carboxamide de formule brute C₁₆H₁₄O₆N₃S₂,Na (M = 431,42 g).

Le rendement correspondant est de 90%.

LC/SM (électrospray négatif) M⁻ = 408,2 g ; (2M+Na)⁻ =839.

RMN du proton, D₂O, 300 MHz, déplacement chimique et multiplicité :
2,41 (s) : CH₃-φ ; 3,50 (d), 3,83 (dd) : N-CH₂-CH ; 4,95 (d) : N-CH₂-CH ; 5,38 (s) : CH-CO-N ; 7,15 (s) : SC=CH ; 7,26 à 7,50 (m) : H aromatiques.

### Exemple 25

### Sel de sodium de trans-4,5,6,8-tétrahydro-6-oxo-5-(sulfooxy)-2-[2-(trifluorométhyl)phényl]-4,7-méthano-7H-thiéno[2,3-e][1,3]diazépine-8-carboxamide

### Stade A

On procède comme au Stade M de l'Exemple 19 avec 250 mg du produit obtenu au Stade A de l'Exemple 24, 11 ml de toluène, 151 mg d'acide trifluorométhyl-2-phénylboronique, 61 mg de Pd(PPh₃)₄, et 2,15 ml d'une solution aqueuse de Na₂CO₃ 2M. On obtient 199 mg de composé trans-4,5,6,8-tétrahydro-6-oxo-5-(phénylméthoxy)-2-[2-(trifluorométhyl)phényl]-4,7-méthano-7*H-*thiéno[2,3-*e*][1,3]diazépine-8-carboxylate de méthyle de formule brute C₂₄H₁₉N₂O₄SF₃ (M = 488,49 g).

Le rendement correspondant est de 76,5%.

### Stade B

On procède comme au Stade A de l'Exemple 7 avec 230 mg du produit obtenu au stade précédent, 5 ml de dioxanne, 1,45 ml d'eau, et 0,5 ml de soude aqueuse 1N. On obtient 199 mg de composé trans-acide 4,5,6,8-tétrahydro-6-oxo-5-(phénylméthoxy)-2-[2-(trifluorométhyl)phényl]-4,7-méthano-7*H-*thiéno[2,3-*e*][1,3]diazépine-8-carboxylique de formule brute C₂₃H₁₇N₂O₄SF₃ (M = 474,46 g).

Le rendement correspondant est de 89,2%.

### Stade C

On procède comme au Stade B de l'Exemple 7 avec 196,2 mg du produit obtenu au stade précédent, 2,5 ml de DMF, 274,3 mg de BOP, 83,8 mg de HOBT, 44,2 mg de NH₄Cl, et 288 µl de DIPEA. On obtient 99,1 mg de composé t rans-4,5,6,8-tétrahydro-6-oxo-5-(phénylméthoxy)-2-[2-(trifluorométhyl)phényl]-4,7-méthano-7*H-*thiéno[2,3-*e*][1,3]diazépine-8-carboxamide de formule brute C₂₃H₁₈N₃O₃SF₃ (M = 473,48 g).

Le rendement correspondant est de 50,6%.

### Stade D

On procède comme au Stade M de l'Exemple 1 avec 84,3 mg du produit obtenu au stade précédent, 16,8 ml de méthanol, 8,4 ml de THF, et 84,3 mg de palladium sur charbon à 30%. On obtient 74,7 mg du composé trans-4,5,6,8-tétrahydro-5-hydroxy-6-oxo-2-[2-(trifluorométhyl)phényl]-4,7-méthano-7*H*-thiéno[2,3-*e*][1,3]diazépine-8-carboxamide de formule brute C₁₆H₂₂N₃O₃SF₃ (M = 383,35 g).

Le rendement correspondant est de 98%.

### Stade E

On procède comme au Stade M de l'Exemple 1 avec 74,7 mg de produit obtenu au stade précédent, 1 ml de pyridine et 92,8 mg du complexe pyridine-SO₃. La purification sur silice du produit brut s'effectue comme au Stade M de l'Exemple 1 en éluant avec un mélange chlorure de méthylène/éthanol/triéthylamine 60/40/0,5. On obtient 73,5 mg de composé sel de triéthylammonium de trans-4,5,6,8-tétrahydro-6-oxo-5-(sulfooxy)-2-[2-(trifluorométhyl)phényl]-4,7-méthano-7*H-*thiéno[2,3-*e*][1,3]diazépine-8-carboxamide de formule brute C₂₂H₂₇O₆N₄S₂F₃ (M = 564,61 g).

Le rendement correspondant est de 66,8%.

### Stade F

On procède comme au Stade R de l'Exemple 12 avec 73,5 mg du produit obtenu au stade précédent, et 20 g de résine Dowex. On élue avec de l'eau contenant 20% de THF et on obtient 35,2 mg du composé sel de sodium de trans-4,5,6,8-tétrahydro-6-oxo-5-(sulfooxy)-2-[2-(trifluorométhyl)phényl]-4,7-méthano-7*H-*thiéno[2,3-*e*][1,3]diazépine-8-carboxamide de formule brute C₁₆H₁₁O₆N₃S₂F₃Na (M = 485,99 g).

Le rendement correspondant est de 55,6%.

LC/SM (électrospray négatif), M⁻ = 462 ; (2M+Na)⁻= 947.

RMN du proton, D₂O, 300 MHz, déplacement chimique et multiplicité :
3,52 (dl) et 3,85 (dd) : N-CH₂-CH ; 4,44 (d) : N-CH₂-CH ; 5,39 (s) : CH-CO-N ; 7,18 (s) : S-C=CH ; 7,54 à 7,72 (m) et 7,87 (dl) : les H aromatiques.

### Exemple 26

### Sel de sodium de trans-2-(2-éthylphényl)-4,5,6,8-tétrahydro-6-oxo-5-(sulfooxy)-4,7-méthano-7H-thiéno[2,3-e][1,3]diazépine-8-carboxamide

### Stade A

On procède comme au Stade M de l'Exemple 19 avec 315 mg du produit obtenu au Stade A de l'Exemple 24, 14,5 ml de toluène, 150,63 mg de 2-éthylbenzène ou d'acide éthyl-2-phenylboronique, 77,4 mg de Pd(PPh₃)₄ et 2,7 ml d'une solution aqueuse de Na₂CO₃ 2M. On obtient 274,2 mg du composé trans-2-(2-éthylphényl)-4,5,6,8-tétrahydro-6-oxo-5-(phénylméthoxy)-4,7-méthano-7*H*-thiéno[2,3-*e*][1,3]diazépine-8-carboxylate de méthyle de formule brute C₂₅H₂₄N₂O₄S (M = 448,54 g).

Le rendement correspondant est de 91,2%.

### Stade B

On procède comme au Stade A de l'Exemple 7 avec 270,8 mg du produit obtenu au stade précédent, 5 ml de dioxanne, 1,45 ml d'eau, 0,63 ml de soude aqueuse 1N. On obtient 237,5 mg de composé trans-acide 2-(2-éthylphényl)-4,5,6,8-tétrahydro-6-oxo-5-(phénylméthoxy)-4,7-méthano-7*H*-thiéno[2,3-*e*][1,3]diazépine-8-carboxylique de formule brute C₂₄H₂₂N₂O₄S (M = 434,52 g).

Le rendement correspondant est de 90,5%.

### Stade C

On procède comme au Stade B de l'Exemple 7 avec 234,2 mg de produit obtenu au stade précédent, 3,3 ml de DMF, 357,58 mg de BOP, 109,2 mg de HOBT, 57,7 mg de NH₄Cl et 375,5 µl de DIPEA. On obtient 210 mg de composé trans-2-(2-éthylphényl)-4,5,6,8-tétrahydro-6-oxo-5-(phénylméthoxy)-4,7-méthano-7*H*-thiéno[2,3-e][1,3]diazepine-8-carboxamide de formule brute C₂₄H₂₃N₃O₃S (M = 433,53 g).

Le rendement correspondant est de 90%.

### Stade D

On procède comme au Stade M de l'Exemple 1 avec 191,1 mg de produit obtenu au stade précédent, 38,2 ml de méthanol, 19,1 ml de THF et 191,1 mg de palladium sur charbon à 30%. On obtient 107,2 mg du composé trans-2-(2-éthylphényl)-4,5,6,8-tétrahydro-5-hydroxy-6-oxo-4,7-méthano-7*H*-thiéno[2,3-e][1,3]diazepine-8-carboxamide de formule brute C₁₇H₁₇N₃O₃S (M = 343,41 g).

Le rendement correspondant est de 70,8%.

### Stade E

On procède comme au Stade N de l'Exemple 1, avec 107,2 mg du produit obtenu au stade précédent, 149 mg du complexe pyridine-SO₃.

Le produit brut obtenu est purifié par chromatographie sur silice en éluant avec un mélange chlorure de méthylène/éthanol/triéthylamine 60/40/0,5. On obtient 106,3 mg du composé sel de triéthylammonium de trans-2-(2-éthylphényl)-4,5,6,8-tétrahydro-6-oxo-5-(sulfooxy)-4,7-méthano-7*H-*thiéno[2,3-e][1,3]diazépine-8-carboxamide de formule brute C₂₃H₃₂O₆N₄S₂ (M=524,66 g).

Le rendement correspondant est de 76,6%.

### Stade F

On procède comme au Stade R de l'Exemple 12 avec 106,3 mg du produit obtenu au stade précédent et 31 g de résine Dowex. L'élution s'effectue à l'eau contenant 20% de THF. On obtient 81,1 mg de composé sel de sodium de trans-2-(2-éthylphényl)-4,5,6,8-tétrahydro-6-oxo-5-(sulfooxy)-4,7-méthano-7*H-*thiéno[2,3-e][1,3]diazépine-8-carboxamide de formule brute C₁₇H₁₆O₆N₃S₂Na (M = 445,45 g).

Le rendement correspondant est de 89,8%.

LC/SM (électrospray négatif) M⁻ = 422,3 ; (2M+Na)⁻= 867.

RMN du proton, D₂0, 300 MHz, déplacement chimique et multiplicité :
1, 13 (t) : CH₃-CH₂-φ ; 2,74 (q) : CH₃-CH₂-φ ; 3,62 (dl) et 3,84 (dd) : N-CH₂-CH ; 4,94 (d) : N-CH₂-CH ; 5,39 (s) : CH-CO-N ; 7,11 (s) : S-C=CH ; 7,33 (m) et 7,42 (m) : les H aromatiques.

### Exemple 27

### Trans-1,2,3,5-tétrahydro-9-hydroxy-3-oxo-2-(phénylméthoxy)-1,4-méthano-4H-2,4-benzodiazépine-5-carboxylate d'éthyle

### Stade A

On dissout 50 g de 3-méthoxy-benzaldéhyde dans 245 ml d'acide acétique. A 0°C, on ajoute goutte à goutte 22 ml de brome, on agite à température ambiante pendant 4 heures, puis on laisse une nuit à température ambiante. On ajoute 300 ml d'eau à la solution et le produit attendu cristallise. Après filtration et lavage à l'eau et séchage, on obtient 68 g de 2-bromo-5-méthoxy-benzaldéhyde, de formule brute C₆H₇Br.O₂ (M = 114 g).

Le rendement correspondant est de 87%.

### Stade B

On dissout 30 g du produit obtenu à l'étape précédente B dans 200 ml de dichlorométhane. On ajoute à 0°, 0,4 g de ZnI₂ puis goutte à goutte 20,86 ml de TMSCN. On agite pendant 1h30, puis on verse le mélange dans une solution aqueuse saturée en bicarbonate de sodium. Après décantation, la phase organique est séchée sur sulfate de magnésium et le solvant est évaporé sous pression réduite. Le produit brut est solubilisé dans 9 ml d'éthanol et 6 ml d'acide chlorhydrique concentré. Après chauffage à reflux pendant 1 h, on verse le milieu réactionnel dans une solution saturée en NaHCO₃, puis on extrait avec de l'acétate d'éthyle. Après évaporation du solvant sous pression réduite, on obtient 41 g de 2-bromo-α-hydroxy-5-méthoxy-benzèneacétate d'éthyle, de formule brute C₁₁H₁₃BrO₄ (M = 288 g).

Le rendement correspondant est de 63%.

### Stade C

Dans un ballon placé sous atmosphère d'azote, on introduit 23,5 g du produit obtenu à l'étape précédente dans 250 ml de dichlorométhane. On ajoute 100 ml de TEA, 1,05 g de DMAP. On refroidit à 0°C puis on introduit 6,31 ml de chlorure de mésyle. On agite 1 heure à 0°C.

On verse ensuite le milieu réactionnel sur un mélange d'eau et de dichlorométhane. On extrait 2 fois avec du dichlorométhane, on lave 2 fois à l'eau, on réunit les phases organiques et on les sèche sur du sulfate de sodium, puis on évapore le solvant sous pression réduite.

On obtient 32 g sous forme d'huile du composé attendu de formule brute C₁₇H₂₄N.BrO₅ (M = 401 g).

### Stade D

On procède comme indiqué au stade C de l'Exemple 21 avec, 130 ml de DMF, 16 g de glycinate de tert-butyle et 9,66 ml de lutidine.

Dans un ballon placé sous atmosphère d'azote, on introduit 30 g du produit obtenu au stade précédent C et 130 ml de DMF. On ajoute 9,66 ml de 2,6-lutidine et 16 g de tertbutyl glycinate. On porte à 80°C pendant 6 heures. On laisse revenir à température ambiante et on verse dans un mélange de glace et d'éther sulfurique. On extrait une fois à l'éther.On lave 4 fois à l'eau la phase éthérée.On sèche la phase organique sur du sulfate de sodium, puis on la filtre et on évapore le solvant sous pression réduite. On entraîne au toluène.Le produit brut est repris à l'AcOEt, on lave avec une solution aqueuse d'acide tartrique à 10% puis deux fois à l'eau et ensuite avec une solution aqueuse saturée d'hydrogénocarbonate de sodium.

On sèche la phase organique sur du sulfate de sodium, on filtre et on évapore le solvant sous pression réduite.On obtient 15,3 g sous forme d'huile de 2-bromo-α-[[2-(1,1-diméthyléthoxy)-2-oxoéthyl]amino]-5-méthoxy-benzèneacétate d'éthyle, de formule brute C₁₇H₂₄BrNO₅ (M = 402,29 g).

Le rendement correspondant est de 47%.

### Stade E

Dans un ballon refroidi par un bain de glace, on introduit 6 g du produit obtenu au stade D, 2,2 ml de triéthylamine et 60 ml de dichlorométhane.

On refroidit à 0°C et on ajoute et 2,4 ml d'anhydride trifluoroacétique.

On laisse en contact pendant 2 heures 30.

On verse ensuite le milieu réactionnel sur un mélange glace/ammoniaque/dichlorométhane. On lave à l'eau, extrait au dichlorométhane. On sèche les phases organiques sur du sulfate de sodium. On filtre et on évapore le solvant sous pression réduite.

On obtient 6,05 g de 2-bromo-α-[[2-(1,1-diméthyléthoxy)-2-oxoéthyl](trifluoroacétyl)amino]-5-méthoxy-benzèneacétate d'éthyle, de formule brute C₁₉H₂₃BrF₃NO₆ (M = 498,30 g).

Le rendement correspondant est de 81%.

### Stade F

Dans un ballon placé sous atmosphère d'azote, on introduit 6,05 g du produit obtenu au stade E et 30 ml de dichlorométhane.

On refroidit à 0°C et on introduit rapidement 30 ml d'acide trifluoroacétique.

On laisse remonter à température ambiante puis on laisse sous agitation pendant 4 heures.

On évapore le solvant sous pression réduite.

Le produit est dissout dans de l'AcOEt, lavé successivement par une solution diluée d'ammoniaque puis par une solution aqueuse saturée en NaH₂PO₄.

On sèche ensuite les phases organiques sur du sulfate de sodium, on filtre et on évapore le solvant sous pression réduite.

On obtient 5,2 g sous forme de cristaux blancs de 2-bromo-α-[[(carboxyméthyl) (trifluoroacétyl)amino]-5-méthoxy-benzèneacétate d'éthyle, de formule brute C₁₅H₁₅BrF₃NO₆ (M = 442,19 g).

Le rendement correspondant est de 97%.

### Stade G

Etape 1 - Préparation du chlorure d'acide

61 g du produit obtenu au stade F est solublisé dans 120 ml de SOCl₂. On chauffe à 80°C pendant 2h puis on évapore à sec.

### Etape 2

Le chlorure d'acide est solubilisé dans 300 ml de CH₃NO₂. Puis on ajoute par fractions 76 g de chlorure d'aluminium et on agite une nuit à température ambiante. Puis on verse le milieu réactionnel dans un mélange hepthane/acétate d'éthyle et on lave la phase organique avec NaH₂PO₄ 1M. Après séchage de la phase organique sur mgSO₄ et évaporation du solvant sous pression réduite, on purifie le résidu obtenu par chromatographie sur silice en éluant avec un mélange heptane/acétate éthyle 4 :1. On obtient un résidu qui est cristallisé dans un mélange pentane/éther.

On obtient 31 g de 8-bromo-1,2,3,4-tétrahydro-5-hydroxy-4-oxo-2-(trifluoroacétyl)-1-isoquinoléinecarboxylate d'éthyle, de formule brute C₁₄H₁₁BrF₃NO₅ (M = 409,15 g),

Le rendement correspondant est de 55%.

### Stade H

On dissout 30 g du produit obtenu au stade précédent G dans 250 ml d'éthanol. Puis on ajoute 3 g de palladium sur charbon à 10% en poids et 21,1 ml de triéthylamine. Le milieu est mis sous pression d'hydrogène. Après 1h30, on filtre le catalyseur puis le filtrat est versé dans un mélange heptane/acétate d'éthyle, la phase organique est lavée avec une solution aqueuse de NaH₂PO₄ 1M, puis séchée sur sulfate de magnésium et le solvant est évaporé sous pression réduite. Le produit brut obtenu est purifié sur silice en éluant avec un mélange heptane/acétate d'éthyle 4/1.

On obtient 22,2 g de 1,2,3,4-tétrahydro-5-hydroxy-4-[(phénylméthoxy)imino]-2-(trifluoroacétyl)-1-isoquinoléinecarboxylate d'éthyle de formule brute C₁₄H₁₂F₂NO₅ (M = 331,25 g).

Le rendement correspondant est de 92%.

### Stade I

On procède comme au Stade E de l'exemple 12 avec 24,4 g du produit obtenu au stade précédent, 250 ml de pyridine, et 15,5 g de chlorhydrate de O-benzylhydroxylamine. On obtient 25,6 g de composé 1,2,3,4-tétrahydro-5-hydroxy-4-[(phénylméthoxy)imino]-2-(trifluoroacétyl)-1-isoquinoléinecarboxylate d'éthyle de formule brute C₂₁H₁₉F₃N₂O₅ (M = 436,39).

Le rendement correspondant est de 80%.

### Stade J

On dissout 25,7 g du produit obtenu au stade précédent dans 300 ml d'acétone. On ajoute à la solution 90 g de K₂CO₃ et 17,3 ml de bromure d'allyle. On chauffe une nuit à reflux puis on verse la solution dans un mélange heptane/acétate d'éthyle 1/2. Et on lave à l'eau. La phase organique est ensuite lavée avec une solution de NaH₂PO₄, puis séchée sur sulfate de magnésium. Après évaporation du solvant sous pression réduite, on obtient 30 g de produit brut qui est purifié par chromatographie sur colonne de silice en éluant avec un mélange heptane/ acétate d'éthyle 4/1. Après une seconde purification par chromatographie sur silice en éluant avec un mélange heptane/acétate d'éthyle 8/1, on obtient 24,8 g sous forme d'huile jaune du composé 1,2,3,4-tétrahydro-4-[(phénylméthoxy)imino]-5-(2-propényloxy)-2-(trifluoroacétyl)-1-isoquinoléinecarboxylate d'éthyle de formule brute C₂₄H₂₃F₃N₂O₅ (M = 476,47).

Le rendement correspondant est de 87%.

### Stade K

On dissout 25,2 g du produit obtenu au stade précédent dans 300 ml d'éthanol. On fait barboter à 0°C du NH₃ gazeux pendant 5 minutes puis on agite la solution à température ambiante pendant 3 heures. On traite la solution en ajoutant de l'acétate d'éthyle et la phase organique est lavée avec une solution aqueuse de NaH₂PO₄ puis séchée sur sulfate de magnésium. Après évaporation des solvants sous pression réduite et un entraînement au toluène, on obtient 21,2 g sous forme d'une huile jaune du composé 1,2,3,4-tétrahydro-4-[(phénylméthoxy)imino]-5-(2-propényloxy)-1-isoquinoléinecarboxylate d'éthyle de formule brute C₂₂H₂₄N₂O₄ (M = 300,45 g).

Le rendement est quantitatif.

### Stade L

On dissout 22,5 g du produit obtenu au stade précédent dans 300 ml de THF. On ajoute 1,1 équivalent de réactif Boc₂-O. On agite pendant deux heures à température ambiante puis on verse le milieu réactionnel dans un mélange heptane/acétate d'éthyle 1/2 et on lave le mélange avec une solution d'acide tartrique à 10%. La phase organique est ensuite séchée sur sulfate de magnésium et le solvant est évaporé sous pression réduite. On obtient un résidu qui est purifié par chromatographie sur colonne de silice en éluant avec un mélange heptane/acétate d'éthyle 5/1. Après évaporation du solvant, on obtient 19,3 g sous forme de cristaux blanc du composé 3,4-dihydro-4-[(phénylméthoxy)imino]-5-(2-propényloxy)-1,2(1*H*)-isoquinoléinedicarboxylate de 2-(1,1-diméthyléthyle) et de 1-éthyle de formule brute C₂₇H₃₂N₂O₆ (M = 480,57),

### Stade M

On procède comme au Stade F de l'Exemple 12 avec 19,3 g du produit obtenu au stade précédent, 250 ml de méthanol, 40,5 g de NaBH₃CN, 59 ml d'éthérate trifluorure de bore. On obtient 21 g de composé 3,4-dihydro-4-[(phénylméthoxy)amino]-5-(2-propényloxy)-1,2(1*H*)-isoquinoléinedicarboxylate de 2-(1,1-diméthyléthyle) et de 1-éthyle de formule brute C₂₇H₃₄N₂O₆ (M = 482,58 g).

Le rendement correspondant est de 79%.

### Stade N

On procède comme aux Stades I et J de l'Exemple 1 avec 15,3 g du produit obtenu au stade précédent, 10 ml d'acétate d'éthyle, 83 ml d'une solution HCl dans l'acétate d'éthyle. Puis 130 ml de chlorure de méthylène et 35 ml de soude aqueuse 2N. On obtient 12,1 g sous forme d'huile incolore du composé 1,2,3,4-tétrahydro-4-[(phénylméthoxy)aminol-5-(2-propényloxy)-1-isoquinoléinecarboxylate d'éthyle de formule brute C₂₂H₂₆N₂O₄ (M = 382,46 g).

Le rendement est quantitatif.

### Stade O

On procède comme au Stade K de l'Exemple 1 avec 12,1 g du produit obtenu au stade précédent, 9 ml de triéthylamine et 2 ml de diphosgène et 31 d'acétonitrile. On obtient 7 g du composé trans-1,2,3,5-tétrahydro-3-oxo-2-(phénylméthoxy)-9-(2-propényloxy)-1,4-méthano-4*H*-2,4-benzodiazépine-5-carboxylate d'éthyle de formule brute C₂₃H₂₄N₂O₅ (M = 408,48 g).

Le rendement est de 55%.

### Stade P

Dans un ballon placé sous atmosphère d'argon, on dissout 4,1 g du composé obtenu au stade précédent dans 50 ml de toluène. On ajoute 231 mg de Pd(PPh₃)₄ et 630 µl d'acide acétique. Puis on ajoute goutte à goutte à 0°C pendant 10 minutes 3,49 ml de Bu₃SnH. On agite le milieu réactionnel à la température ambiante pendant deux heures. On ajoute ensuite au milieu réactionnel 200 ml de CH₃CN et le milieu est extrait trois fois avec 60 ml de pentane. La solution d'acétonitrile est ensuite évaporé et le résidu est purifié par chromatographie sur silice en éluant avec un mélange heptane/acétate d'éthyle 2/1 puis 1/1. On obtient 3,45 g du composé 1,2,3,4-tétrahydro-5-hydroxy-4-[(phénylméthoxy)imino]-2-(trifluoroacétyl)-1-isoquinoléinecarboxylate d'éthyle de formule brute C₂₀H₂₀N₂O₅ (M = 368,39 g).

Le rendement correspondant est de 94%.

LC/SM (électrospray positif), m/z : M⁺ = 369.

LC/SM (électrospray négatif), m/z : (M⁻H)⁻ = 367.

RMN du proton, CDCl₃ à 300 MHz, déplacement chimique et multiplicité :
1,32 (t) _{:} CH₃-CH₂O-CO ; 4,27 (qd) : CH₃-CH₂-O-CO ; 3,52 (dd) et 3,67 (d) : N-CH₂-CH ; 4,60 (d) : N-CH₂-CH ; 4,93 et 4,99 (ab) : O-CH₂-φ ; 5,01 (s) : φ-OH ; 5,10 (s) : CH₂-CO-O-CH₂-CH₃ ; 6,69 (d), 6,93 (d) et 7,13 (t) : les H aromatiques, 7,37 (m) et 7,46 (m) : CH₂-φ.

### Exemple 28

### Sel de sodium de trans-1,2,3,5-tétrahydro-3-oxo-9-[[(phénylamino)carbonyl]oxy]-2-(sulfooxy)-1,4-méthano-4H-2,4-benzodiazépine-5-carboxylate d'éthyle.

### Stade A

Dans un ballon placé sous atmosphère d'argon, on dissout 0,1 g du produit obtenu au Stade P de l'Exemple 27 dans 2 ml de dichlorométhane. On ajoute 0,019 ml de triéthyalmine puis on ajoute goutte à goutte à 0°C PhNCO. On agite pendant 30 minutes puis on verse la solution dans le dichlorométhane et on lave la phase organique avec une solution aqueuse de NaH₂PO₄. La phase organique est séchée sur sulfate de magnésium et le solvant est évaporé sous pression réduite. On obtient 0,130 g de produit brut qui est purifié par chromatographie sur silice en éluant avec un mélange éthane/acétate d'éthyle 1/1. On obtient 0,119 g de composé trans-1,2,3,5-tétrahydro-3-oxo-9-[[(phénylamino)carbonyl]oxy]-2-(phénylméthoxy)-1,4-méthano-4*H*-2,4-benzodiazépine-5-carboxylate d'éthyle de formule brute C₂₇H₂₅N₃O₆ (M = 487,52 g).

Le rendement correspondant est de 90%.

### Stade B

On procède comme au Stade M de l'Exemple 1 avec 0,115 g du produit obtenu au stade précédent, 10 ml de THF et 0,027 g de palladium à 10% sur charbon. Le produit obtenu est traité comme au Stade N de l'Exemple 1 avec 2 ml de pyridine, 114 mg du complexe pyridine-SO₃. Le produit obtenu est traité comme au Stade R de l'Exemple 12 en présence de 25 g de résine Dowex. On obtient à l'issue de ces trois étapes, 0,107 g sous forme de cristaux jaune pâle du composé sel de sodium de trans-1,2,3,5-tétrahydro-3-oxo-9-[[(phénylamino)carbonyl]oxy]-2-(sulfooxy)-1,4-méthano-4*H*-2,4-benzodiazépine-5-carboxylate d'éthyle de formule brute C₂₀h₁₈N₃O₉S,Na (M = 476,45 g + 22,99 g).

Le rendement correspondant est de 90%.

LC/SM (électrospray positif) m/z : M+Na⁺ = 522.

RMN du proton, DMSO-d₆, 300 MHz, déplacement chimique et multiplicité :
1,28 (t) : CH₃-CH₂-O ; 4,24 (q) : CH₃-CH₂-O ; 3,52 (d), 3,60 (dd) : CH₂-CH ; 4,98 (d) : CH₂-CH ; 5,05 (s) : CH-CO₂Et ; 7,04, 7,33, 7,50, 7,42, 7,22 : les H aromatiques : 10,08 (1H) mobile.

### Exemple 29

### Sel disodique de trans-1,2,3,5-tétrahydro-3-oxo-2,9-bis(sulfooxy)-1,4-méthano-4H-2,4-benzodiazépine-5-carboxylate de 5-éthyle

### Stade A

On procède comme au Stade M de l'Exemple 1 avec 0,130 g du produit obtenu au Stade P de l'Exemple 27, 13 ml de THF et du palladium sur charbon à 10%. On obtient avec un rendement quantitatif le composé trans-1,2,3,5-tétrahydro-2,9-dihydroxy-3-oxo-1,4-méthano-4*H*-2,4-benzodiazépine-5-carboxylate d'éthyle de formule brute C₁₃H₁₄N₂O₅ (M = 278,27 g).

### Stade B

On procède comme au Stade N de l'Exemple 1 avec 0,1 g du produit obtenu au stade précédent, 0,33 g de complexe pyridine-SO₃ et 2 ml de pyridine. Puis le produit est obtenu et traité de la même façon qu'au Stade R de l'Exemple 12 en présence de 25 g de résine Dowex. On obtient 0,170 g du composé sel disodique de trans-1,2,3,5-tétrahydro-3-oxo-2,9-bis(sulfooxy)-1,4-méthano-4*H*-2,4-benzodiazépine-5-carboxylate de 5-éthyle de formule brute C₁₃H₁₂N₂Na₂O₁₁S₂ (M = 482,35 g).

Le rendement correspondant est quantitatif.

LC/SM (électrospray positif) m/z : M+Na⁺ = 505.

RMN du proton, DMSO-d₆, 300 MHz, déplacement chimique et multiplicité :
3,44 (d) : CH₂-CH ; 3,53 (dd) : CH₂-CH ; 1,26 (t) : CH₃-CH₂-O-CO ; 4,21 (q) : CH₃-CH₂-O-CO ; 4,94 (s) : CH-CH₂Et ; 5,20 (d) : CH-CH₂ ; 7,06 (d), 7,18 (d), 7,27 (d) : les trois H aromatiques.

### Exemple 30

### Sel de sodium de trans-1,2,3,5-tétrahydro-3-oxo-2-(sulfooxy)-9-[[(trifluorométhyl)sulfonyl]oxy]-1,4-méthano-4H-2,4-benzodiazépine-5-carboxylate de 5-éthyle

### Stade A

Dans un ballon placé sous argon à -15°C, on dissout 1 g du produit obtenu au Stade P de l'Exemple 27 dans 15 ml de dichlorométhane. On ajoute 1,17 ml de triéthylamine puis 0,67 ml de (CF₃SO₂)₂O. On agite pendant 30 minutes à -15°C puis une fois le milieu réactionnel revenu à température ambiante, on traite avec une solution de NaHCO₃ et on extrait avec du chlorure de méthylène. La phase organique est ensuite séchée sur sulfate de magnésium et le solvant est évaporé sous pression réduite. On obtient 1,5 g de gomme qui est purifiée par chromatographie sur silice en éluant avec un mélange heptane/acétate d'éthyle 4/1. On obtient 0,8 g sous forme de cristaux blancs du composé trans-1,2,3,5-tétrahydro-3-oxo-2-(phénylméthoxy)-9-[[(trifluorométhyl)sulfonyl]oxy]-1,4-méthano-4*H*-2,4-benzodiazépine-5-carboxylate d'éthyle de formule brute C₂₁H₁₉F₃N₂O₇F (M = 500,4 g).

Le rendement correspondant est de 60%.

### Stade B

On procède comme indiqué au Stade M de l'Exemple 1 avec 0,130 mg du produit obtenu au stade précédent, 13 ml de THF et 25 mg de palladium sur charbon à 10%. On obtient 91 mg du composé trans-1,2,3,5-tétrahydro-2-hydroxy-3-oxo-9-[[(trifluorométhyl)sulfonyl]oxy]-1,4-méthano-4*H*-2,4-benzodiazépine-5-carboxylate d'éthyle de formule brute C₁₄H₁₃N₂O₇S (M = 410,33 g).

Le rendement correspondant est de 90%.

### Stade C

On procède comme indiqué au Stade N de l'Exemple 1 avec 91 mg du produit obtenu au stade précédent, 2 ml de pyridine et 105 mg du complexe pyridine-SO₃. Le produit obtenu est ensuite traité comme indiqué au Stade R de l'Exemple 12 avec 25 g de résine Dowex. On obtient 0,110 g sous forme de cristaux blancs du composé sel de sodium de trans-1,2,3,5-tétrahydro-3-oxo-2-(sulfooxy)-9-[[(trifluorométhyl)sulfonyl]oxy]-1,4-méthano-4*H-*2,4-benzodiazépine-5-carboxylate de 5-éthyle de formule brute C₁₄H₁₂F₃N₂O₁₀S₂Na (M = 489,38 + 22,99 g).

Le rendement correspondant est de 97%.

LC/SM (électrospray positif), m/z : M+Na⁺ = 535.

RMN du proton, DMSO-d₆, 300 MHz, déplacement chimique et multiplicité :
1,26 (t) : CH₃-CH₂-O-CO ; 4,24 (q) : CH₃-CH₂-O-CO ; 3,50 (d) et 3,67 (dd) : CH₂-CH ; 5,02 (d) : CH₂-CH ; 5,17 (s) : CH-CO₂Et ; 7,45 (d), 7,50 (d), 7,59 (t) : les 3 H aromatiques.

### Exemple 31

### Sel de sodium de trans-9-(4-fluorophényl)-1,2,3,5-tétrahydro-3-oxo-2-(sulfooxy)-1,4-méthano-4H-2,4-benzodiazépine-5-carboxylate de 5-éthyle

### Stade A

On procède comme au Stade M de l'Exemple 19 avec 0,35 g du produit obtenu au Stade A de l'Exemple 30, 15 ml de toluène, 0,146 g d'acide 4-fluorophényl boronique, 70 mg de Pd(Pφ₃)₄, 2,8 ml d'une solution de Na₂CO₃ 2M. On obtient 0,280 g de composé trans-9-(4-fluorophényl)-1,2,3,5-tétrahydro-3-oxo-2-(phénylméthoxy)-1,4-méthano-4*H*-2,4-benzodiazépine-5-carboxylate d'éthyle de formule brute C₂₆H₂₃F₂O₄ (M = 446,48 g).

Le rendement correspondant est de 90%.

### Stade B

On procède comme indiqué au Stade M de l'Exemple 1 avec 0,270 g du produit obtenu au stade précédent, 5 ml de THF, et du palladium sur charbon à 10%. Le produit obtenu est traité comme indiqué au Stade N de l'Exemple 1 avec 3 ml de pyridine et 257 mg de complexe pyridine-SO₃. Le produit obtenu est traité comme indiqué au Stade R de l'Exemple 12 avec 25 g de résine Dowex. On obtient 0,28 g sous forme de cristaux blancs du composé sel de sodium de trans-9-(4-fluorophényl)-1,2,3,5-tétrahydro-3-oxo-2-(sulfooxy)-1,4-méthano-4*H*-2,4-benzodiazépine-5-carboxylate de 5-éthyle de formule brute C₁₉H₁₆FN₂NaO₇S (M = 458,40 g).

Le rendement correspondant est quantitatif.

RMN du proton, DMSO-d₆, 300 MHz, déplacement chimique et multiplicité :
1,28 (t) : CH₃-CH₂-O ; 4,23 (q) : CH₃-CH₂-O ; 3,46 (dd) et 3,53 (d) : CH₂-CH ; 4,59 (d) : CH₂-CH ; 5,05 (s) : CH-CO₂ ; 7,14 (m) et 7,52 (m) : les 4 H aromatiques du noyau fluoré ; 7,19 (d), 7,34 (d) et 7,43 (t) : les 3 H aromatiques.

### Exemple 32

### 1,2,3,5-tétrahydro-3-oxo-8-hydroxy-2-(phénylméthoxy)-1,4-méthano-4H-2,4-benzodiazépine-5-carboxylate d'éthyle

### Stade A

### Etape 1

Dans une solution de 48 g (0,253 mole) de chlorhydrate de D,L-Norphenylephrine dans 94 ml de méthanol chauffée à reflux, on introduit à chaud 51,72 g d'une solution de glyoxalate d'éthyle à 50% dans le toluène.

Après 30 minutes de reflux, le chlorhydrate du produit attendu précipite. La suspension est laissée à nouveau 30 minutes à reflux avant d'être refroidie par un bain de glace afin de faire cristalliser le chlorhydrate attendu.

Après avoir ajouté 50 ml d'éther le précipité filtré, lavé à l'éther donne 46 g de 1,2,3,4-tetrahydro-4,6-dihydro-1-isoquinoleinecarboxylate d'éthyle.

### Etape 2

On ajoute 25 ml de TEA à une suspension refroidie à 0°C de 44 g (0,160 mole) du composé obtenu à l'étape précédente dans 500 ml de THF. Puis après changement d'aspect de la suspension, on ajoute 38,7 g (0,177 mole) de (BOC)₂O. On agite ensuite durant 2 heures à 20°C avant de verser le milieu réactionnel sur une solution aqueuse de hydrogénosulfate de sodium à 10%.

Après extraction au THF et à l'acétate d'éthyle, la phase organique est lavée à nouveau par une première solution de hydrogénosulfate de sodium puis une deuxième solution de dihydrogénophosphate de sodium à 1 molaire. On sèche la phase organique sur du sulfate de magnésium puis on filtre et on évapore le solvant sous pression réduite pour obtenir 60,2 g de 3,4-dihydro-4,6-dihydroxy-1,2(1*H*)-isoquinoléinedicarboxylate de 2-(1,1-diméthyléthyle) et de 1-éthyle de formule brute C₁₇H₂₃NO₆ (M = 337,38 g)

### Stade B

On introduit 60 g ( 1,177 mole ) du composé obtenu au stade A2 dans 600 ml d'acétone. Puis on ajoute 49,4 g de carbonate de potassium et goutte à goutte 29 ml de bromure d'allyle. On chauffe à reflux pendant 2 heures 30 puis on filtre les sels, on évapore l'acétone.

Le résidu est dissout dans un mélange heptane/ACOEt. On lave ensuite la phase organique avec une solution saturée de bicarbonate de sodium, on sèche sur sulfate de magnésium, puis on évapore le solvant sous pression réduite.

On obtient 66 g de 3,4-dihydro-4-hydroxy-6-(2-propényloxy)-1,2(1*H*)-isoquinoléinedicarboxylate de 2-(1,1-diméthyléthyle) et de 1-éthyle de formule brute C₂₀H₂₇NO₆ ( M = 377,44 g).

Le rendement correspondant est de 98%.

### Stade C

On introduit 57,5 g de chlorochromate de pyridinium et 120 g de tamis moléculaire dans 1 litre de dichlorométhane. Puis on introduit à 0°C une solution de 65,5 g (0,178 mole) du composé obtenu au stade B dans 300 ml de dichlorométhane. On agite la solution durant 1 heure 30 en la laissant revenir à température ambiante puis on filtre sur 1 kg de Florisil en éluant avec du dichlorométhane.

Après évaporation sous pression réduite du solvant on obtient 49,92 g de 3,4-dihydro-4-oxo-6-(2-propényloxy)-1,2(1*H*)-isoquinoléinedicarboxylate de 2-(1,1-diméthyléthyle) et de 1-éthyle de formule brute C₂₀H₂₅NO₆ (M = 375,40 g).

Le rendement correspondant est de 78%.

### Stade D

On introduit 49,9 g du composé obtenu au stade C dans 500 ml de pyridine puis on ajoute 23,3 g de PhCH₂ONH₂,HCl et on agite le milieu réactionnel pendant 1 heure.

On évapore le solvant sous pression réduite puis le résidu est dissous dans un mélange de solvant heptane/ACOEt-1/2.

On lave la phase organique 3 fois avec une solution de hydrogénosulfate de sodium à 10% puis on sèche sur sulfate de magnésium.

On obtient 57 g sous forme d'huile de 3,4-dihydro-4-[(phénylméthoxy)-imino]-6-(2-propényloxy)-1,2(1*H*)-isoquinoléinedicarboxylate de 2-(1,1-diméthyléthyle) et de 1-éthyle de formule brute C₂₇H₃₂N₂O₆ ( M = 480,57 g).

Le rendement correspondant est de 89%.

### Stade E

On procède comme indiqué au H de l'Exemple 31 avec 56 g du produit obtenu au stade D, 44g de cyanoborohydrure de sodium et 500 ml d'acide acétique glacial.

Dans un ballon placé sous atmosphère d'azote, on introduit 56 g du produit obtenu au stade D et 500 ml d'acide acétique glacial. On refroidit à 10°C et on ajoute environ 44 g de cyanoborohydrure de sodium. On laisse revenir à température ambiante et on laisse réagir pendant 5 heures. On reprend avec 400 ml d'AcOEt, on verse dans 600 ml de soude 1N, on décante, on extrait plusieurs fois à l'AcOEt, on lave à nouveau à la soude 1N, puis à l'eau puis avec une solution de chlorure de sodium. On sèche la phase aqueuse sur du sulfate de magnésium, on filtre et on évapore le solvant sous pression réduite. On purifie le résidu par chromatographie sur silice en éluant avec un mélange dichlorométhane/AcOEt 97/3.

On obtient 22 g de (1S)-3,4-dihydro-4-[(phénylméthoxy)amino]-6-(2-propényloxy)-1,2(1*H*)-isoquinoléinedicarboxylate de 2-(1,1-diméthyléthyle) et de 1-éthyle de formule brute C₂₇H₃₁N₂O₆ (M = 482,58 g).

Le rendement correspondant est de 40%.

### Stade F

On procède comme aux stade I et J de l'exemple 1 avec 22 g du produit obtenu au stade E, 22 ml d'l'acétate d'éthyle, 95 ml d'une solution 4,6M d'acide chlorhydrique dans l'ACOEt.

On obtient 16,5 g de (1S)-1,2,3,4-tétrahydro-4-[(phénylméthoxy)amino]-6-(2-propényloxy)-1-isoquinoléinecarboxylate d'éthyle de formule brute C₂₂H₂₆N₂O₄ (M = 382,46 g).

Le rendement correspondant est quantitatif.

### Stade G

On procède comme au stade K de l'exemple 1 avec 16,4 g du produit obtenu au stade F, 3,5 litres d'acétate d'éthyle, 14,2 ml de TEA, 4,2 g de diphosgène.

On obtient 3,5 g de Trans-1,2,3,5-tétrahydro-3-oxo-2-(phénylméthoxy)-8-(2-propényloxy)-1,4-méthano-4*H*-2,4-benzodiazépine-5-carboxylate d'éthyle de formule brute C₂₃H₂₄N₂O₅ (M = 408 g).

Le rendement correspondant est de 20%.

### Stade H

On dissout 3,5 g du produit obtenu au stade précédent G dans 35 ml de toluène. On introduit sous argon à O°C 0,191 g de Pd(PPh₃)₄ puis 0,52 g d'acide acétique et enfin goutte à goutte 2,89 g de Bu₃SnH.

On agite 45 minutes à 0°C puis on évapore le solvant sous pression réduite et le résidu est purifié par chromatographie sur silice avec un mélange heptane/AcOEt-1/l.

On obtient 2,92 g sous forme de cristaux blancs de 1,2,3,5-tétrahydro-3-oxo-8-hydroxy-2-(phénylméthoxy)-1,4-méthano-4*H-*2,4-benzodiazépine-5-carboxylate d'éthyle de formule brute C₂₀H₂₀N₂O₅ ( M = 368 g).

Le rendement correspondant est de 96%.

### Exemple 33

### Sel de sodium de trans-1,2,3,5-tétrahydro-N-méthoxy-8-[(2-méthoxyéthoxy)méthoxy]-3-oxo-2-(sulfoxy)-1,4-méthano-4H-2,4-benzodiazépine-5-carboxamide

### Stade A

On dissout 3,68 g du produit obtenu au Stade H de l'Exemple 32 dans 40 ml de dichlorométhane. Dans le milieu réactionnel refroidi à 0°C, on ajoute 1,24 ml de C1MEM et 2 ml de DIEA. On agite pendant 30 minutes à 0°C puis on rajoute à nouveau 1,24 ml de C1MEM et 2 ml de DIEA. La réaction est ensuite versée dans de l'eau contenant NaH₂PO₄. La phase organique est extraite et séchée sur sulfate de magnésium et le solvant est évaporé sous pression réduite. Le résidu obtenu est purifié par chromatographie sur silice en éluant avec un mélange dichlorométhane contenant 5% d'acétone. On obtient 2,8 g de composé trans-1,2,3,5-tétrahydro-8-[(2-méthoxyéthoxy)méthoxy]-3-oxo-2-(phénylméthoxy)-1,4-méthano-4*H*-2,4-benzodiazépine-5-carboxylate d'éthyle de formule brute C₂₄H₂₅N₂O₇ (M = 456,50 g).

Le rendement correspondant est de 63%.

### Stade B

On procède comme au Stade A de l'Exemple 7 avec 2,8 g du produit obtenu au stade précédent, 40 ml de dioxane et 3 ml d'eau, 6,13 ml de soude aqueuse 1N. On obtient 2,18 g du composé trans-acide 1,2,3,5-tétrahydro-8-[(2-méthoxyéthoxy)méthoxy]-3-oxo-2-(phénylméthoxy)-1,4-méthano-4*H*-2,4-benzodiazépine-5-carboxylique de formule brute C₂₂H₂₄N₂O₇ (M = 428,35 g).

Le rendement correspondant est de 86%.

### Stade C

On procède comme indiqué au Stade B de l'Exemple 7 avec 0,31 g du produit obtenu au stade précédent, 5 ml de DMF, 0,46 g de BOP, 0,15 g de HOBT, 0,13 g de NH₂OMe,HCl, 0,5 ml de DIEA. On obtient 0,215 g de composé trans-1,2,3,5-tétrahydro-*N*-méthoxy-8-[(2-méthoxyéthoxy)méthoxy]-3-oxo-2-(phénylméthoxy)-1,4-méthano-4H-2,4-benzodiazépine-5-carboxamide de formule brute C₂₃H₂₇N₃O₇ (M = 457,49 g).

Le rendement correspondant est de 64%.

### Stade D

On procède comme indiqué au Stade M de l'Exemple 1 avec 0,2 g du produit obtenu au stade précédent, 3 ml d'éthanol, et 25 mg de palladium sur charbon à 10%. On obtient avec un rendement quantitatif le composé trans-1,2,3,5-tétrahydro-2-hydroxy-*N*-méthoxy-8-[(2-méthoxyéthoxy)méthoxy]-3-oxo-1,4-méthano-4*H*-2,4-benzodiazépine-5-carboxamide de formule brute C₁₆H₂₁N₃O₇ (M = 367,36 g).

### Stade E

On procède comme indiqué au Stade N de l'Exemple 1 avec 0,16 g du produit au stade précédent, 2 ml de pyridine, 240 mg du complexe pyridine-SO₃. On obtient 0,138 g du composé sel de sodium de trans-1,2,3,5-tétrahydro-*N*-méthoxy-8-[(2-méthoxyéthoxy)méthoxy]-3-oxo-2-(sulfoxy)-1,4-méthano-4*H*-2,4-benzodiazépine-5-carboxamide de formule brute C₁₆H₂₀N₃O₁₀S,Na (M = 446,42 + 22,99 g).

Le rendement correspondant est de 68%.

LC/SM (électrospray négatif), m/z : M⁻ = 446,1.

RMN du proton, DMSO-d₆, 300 MHz, déplacement chimique et multiplicité :
3,23 (s) : CH₃-O-(CH₂)₂-O-CH₂-O ; 3,46 (m), 3,72 (m) : CH₃-O-(CH₂)₂-O-CH₂-O ; 5,24 (sl) : CH₃-O-(CH₂)₂-O-CH₂-O ; 3,41 (m), 3,88 (dl) : N-CH₂-CH ; 4,61 (masqué) : N-CH₂-CH ; 4,61 (s) : CH-CO-N-O-CH₃ ; 3,65 (sl) : CH-CO-N-O-CH₃ ; 6,78 (d), 7,03 (dd), 7,09 (dd) : les 3 H aromatiques et 11,78 (s) : 0=C-NH-O.

### Exemple 34

### Sel de sodium de trans-1,2,3,5-tétrahydro-8-hydroxy-N-méthoxy-3-oxo-2-(sulfooxy)-1,4-méthano-4H-2,4-benzodiazépine-5-carboxamide

### Stade A

On dissout 0,06 g du produit obtenu au Stade E de l'Exemple 33 dans une solution contenant 1 ml d'acide trifluoroacétique, 1 ml de dichlorométhane et 0,4 ml d'anisol. On agite pendant 30 minutes puis on évapore le milieu réactionnel sous pression réduite à sec. On ajoute du toluène puis on sèche à nouveau sous pression réduite. Le résidu est repris dans l'éther et on obtient 30 mg sous forme de cristaux jaunes du composé sel de sodium de trans-1,2,3,5-tétrahydro-8-hydroxy-*N*-méthoxy-3-oxo-2-(sulfooxy)-1,4-méthano-4*H*-2,4-benzodiazépine-5-carboxamide.

Le rendement correspondant est de 44%.

LC/SM (électrospray négatif), m/z : M⁻ = 358 g.

### Exemple 35

### Sel de sodium de trans-8-(2-aminoéthoxy)-1,2,3,5-tétrahydro-3-oxo-2-(sulfooxy)-1,4-méthano-4H-2,4-benzodiazépine-5-carboxylate d'éthyle

### Stade A

On dissout 0,5 g du produit obtenu au Stade H de l'Exemple 32 dans 5 ml de dichlorométhane. Puis on ajoute 0,239 g de Boc-NH-CH₂OH et 0,39 g de Pφ₃. On refroidit le milieu réactionnel à 0°C et on ajoute goutte à goutte 258 µl de DEAD. Après agitation une heure à température ambiante, on verse le milieu sur de l'eau, la phase organique est extraite et séchée sur sulfate de sodium. On évapore le solvant sous pression réduite et le résidu est purifié par chromatographie sur silice en éluant avec du dichlorométhane contenant 5% d'acétone. On obtient 320 mg de composé trans-8-[2-[[(1,1-diméthyléthoxy)carbonyl]amino]éthoxy]-1,2,3,5-tétrahydro-3-oxo-2-(phénylméthoxy)-1,4-méthano-4*H*-2,4-benzodiazépine-5-carboxylate d'éthyle de formule brute C₂₇H₃₃N₃O₇ (M = 511,58 g).

Le rendement correspondant est de 47%.

### Stade B

On procède comme indiqué au Stade M de l'Exemple 1 avec 60 mg du produit obtenu au stade précédent, 0,5 ml d'éthanol et 7 mg de palladium sur charbon à 10%. Le produit obtenu est traité comme indiqué au Stade M de l'Exemple 1 avec 0,8 ml de pyridine, et 56 mg du complexe pyridine-SO₃, puis comme indiqué au Stade R de l'Exemple 12 avec 50 g de résine Dowex. On obtient 51 mg du composé sel de sodium de trans-8-[2-[[(1,1-diméthyléthoxy)carbonyl]amino]éthoxy]-1,2,3,5-tétrahydro-3-oxo-2-(sulfooxy)-1,4-méthano-4*H*-2,4-benzodiazépine-5-carboxylate d'éthyle de formule brute C₂₀H₂₆N₃O₁₀S,Na (M = 500,51 + 22,99 g).

Le rendement correspondant est de 84%.

### Stade C

On procède comme indiqué au Stade A de l'Exemple 1 avec 0,048 g du produit obtenu au stade précédent et 0,5 ml d'acide trifluoroacétique. On obtient 30 mg sous forme de cristaux blancs du composé sel de sodium de trans-8-(2-aminoéthoxy)-1,2,3,5-tétrahydro-3-oxo-2-(sulfooxy)-1,4-méthano-4*H*-2,4-benzodiazépine-5-carboxylate d'éthyle de formule brute C₁₅H₁₈N₃O₈S,Na (M = 400,39 + 22,99 g).

Le rendement correspondant est de 79%.

LC/SM (électrospray négatif), m/z : (M-H)⁻ = 400.

RMN du proton, DMSO-d₆, 300 MHz, déplacement chimique et multiplicité :
1,25(t) : CH₃-CH₂-OCO-CH ; 4,21(q) : CH₃-CH₂-OCO-CH ; 4,95(s) ; CH₃-CH₂-OCO-CH ; 2,23(tl):N-CH₂-CH₂-O ; 4,16(m) : N-CH₂-CH₂-O ; 3,52(s) : N-CH₂-CH- ; 4,67(s) : N-CH₂-CH- ; 6,81(d), 7,01(dd), 7,29(d) les 3 H aromatiques ; 7,95(sl) les 2 H mobiles.

### Exemple 36

### Sel de sodium de trans-8-(2-aminoéthoxy)-1,2,3,5-tétrahydro-3-oxo-2-(sulfooxy)-1,4-méthano-4H-2,4-benzodiazépine-5-carboxamide

Stade AOn procède comme indiqué au Stade A de l'Exemple 7 avec 0,327 g du produit obtenu au Stade A de l'Exemple 35, 3 ml de dioxanne, 3 ml d'eau et 0,639 ml de soude aqueuse 1N. On obtient 0,285 g du composé trans-acide 8-[2-[[(1,1-diméthyléthoxy)carbonyl]amino]éthoxy]-1,2,3,5-tétrahydro-3-oxo-2-(phénylméthoxy)-1,4-méthano-4*H*-2,4-benzodiazépine-5-carboxylique de formule brute C₂₅H₂₉N₃O₇ (M = 483,53 g).

Le rendement correspondant est de 95%.

### Stade B

On procède comme indiqué au Stade B de l'Exemple 7 avec 0,28 g du produit obtenu au stade précédent, 4 ml de DMF, 0,368 g de BOP, 0,117 g de HOBt, 0,062 g de NH₄Cl et 0,4 ml de DIEA. On obtient 0,182 mg du composé trans-[2-[[5-(aminocarbonyl)-1,2,3,5-tétrahydro-3-oxo-2-(phénylméthoxy)-1,4-méthano-4*H*-2,4-benzodiazépin-8-yl]oxy]éthyl]-carbamate de 1,1-diméthyléthyle de formule brute C₂₅H₃₀N₄O₆ (M = 482,54 g).

Le rendement correspondant est de 65%.

### Stade D

On procède comme indiqué au Stade M de l'Exemple 1 avec 0,175 g du produit obtenu au stade précédent, 2 ml d'éthanol, 2 ml d'acide acétique et 40 mg de palladium sur charbon à 10%. Le produit obtenu est traité comme indiqué au Stade N de l'Exemple 1 avec 3 ml de pyridine et 250 mg du complexe pyridine-SO₃. Le produit obtenu est traité comme au Stade R de l'Exemple 12 avec 40 g de résine Dowex préparés à la soude. On obtient 0,130 g de composé sel de sodium de trans-[2-[[5-(aminocarbonyl)-1,2,3,5-tétrahydro-3-oxo-2-(sulfooxy)-1,4-méthano-4*H*-2,4-benzodiazépin-8-yl]oxy]éthyl]-carbamate de 1,1-diméthyléthyle de formule brute C₁₈H₂₃N₄O₉S,Na (M = 471,47 + 22,99 g).

Le rendement correspondant est de 73%

### Stade E

On procède comme indiqué au Stade A de l'Exemple 13 avec les 130 mg de produit obtenu au stade précédent en présence de 1 ml d'acide trifluoroacétique. On obtient 80 mg sous forme de cristaux beiges du composé sel de sodium de trans-8-(2-aminoéthoxy)-1,2,3,5-tétrahydro-3-oxo-2-(sulfooxy)-1,4-méthano-4*H*-2,4-benzodiazépine-5-carboxamide de formule brute C₁₃H₁₅N₄O₇S,Na (M = 371,35 + 22,99 g).

Le rendement correspondant est de 77%

LC/SM (électrospray négatif), m/z : M⁻ = 371.

### Exemple 37

### Sel de sodium de trans-1,2,3,5-tétrahydro-3-oxo-8-[[(phénylamino)carbonyl]oxy]-2-(sulfooxy)-1,4-méthano-4H-2,4-benzodiazépine-5-carboxylate d'éthyle

### Stade A

On procède comme indiqué au Stade A de l'Exemple 28 avec 0,2 g du produit obtenu au Stade H de l'Exemple 32, 2 ml de dichlorométhane, 0,04 ml de triéthylamine et 0,06 ml de phénylisocyanate. On obtient 0,170 g du composé trans-1,2,3,5-tétrahydro-3-oxo-8-[[(phénylamino)carbonyl]oxy]-2-(phénylméthoxy)-1,4-méthano-4*H*-2,4-benzodiazépine-5-carboxylate d'éthyle de formule brute C₂₇H₂₅N₃O₆ (M = 487,52 g).

Le rendement correspondant est de 65%.

### Stade B

On procède comme indiqué au Stade M de l'Exemple 1 avec 0,170 g du produit obtenu au stade précédent, 4 ml d'éthanol, 2 ml de THF et 35 mg de palladium sur charbon à 10%. On obtient 0,153 g de composé trans-1,2,3,5-tétrahydro-2-hydroxy-3-oxo-8-[[(phénylamino)carbonyl]oxy]-1,4-méthano-4*H*-2,4-benzodiazépine-5-carboxylate d'éthyle de formule brute C₂₀H₁₉N₃O₆ (M = 397,39 g).

Le rendement correspondant est quantitatif.

### Stade C

On procède comme indiqué au Stade N de l'Exemple 1 avec 0,153 mg du produit obtenu au stade précédent, 2 ml de pyridine et 0,177 g du complexe pyridine-SO₃. Le composé obtenu est ensuite traité comme indiqué au Stade R de l'Exemple 12 avec 50 g de résine Dowex. On obtient le composé sel de sodium de trans-1,2,3,5-tétrahydro-3-oxo-8-[[(phénylamino)carbonyl]oxy]-2-(sulfooxy)-1,4-méthano-4*H*-2,4-benzodiazépine-5-carboxylate d'éthyle de formule brute C₂₀H₁₈N₃O₉Sna ( M = 476,45 + 22,99).

LC/SM (électrospray négatif), m/z : M⁻ (476,1 g)

RMN du proton, DMSO-d₆, 300 MHz, déplacement chimique et multiplicité :
1,27 (t) et 4,24 (q) : CO₂-Et ; 3,55 : N-CH₂-CH ; 4,72 (sl) : N-CH₂-CH ; 5,04 (sl) : N-CH-CO ; 6,99 (d), 7,26 (dd), 7,39 (d) : les 3 H aromatiques, 7,51, 7,32 et 7,05 : les 5 H aromatiques ; 10,29 : NH.

### Exemple 38

### Sel de sodium de trans-8-[[(éthylamino)carbonyl]oxy]-1,2,3,5-tétrahydro-3-oxo-2-(sulfooxy)-1,4-méthano-4H-2,4-benzodiazépine-5-carboxylate d éthyle

### Stade A

On procède comme indiqué au Stade A de l'Exemple 28 avec 0,2 g du produit obtenu au Stade H de l'Exemple 32, 3 ml de dichlorométhane, 0,037 ml de TEA et 0.041 ml d'éthylisocyanate. On obtient 0,170 g du composé trans-8-[[(éthylamino)carbonyl]oxy]-1,2,3,5-tétrahydro-3-oxo-2-(phénylméthoxy)-1,4-méthano-4*H*-2,4-benzodiazépine-5-carboxylate d'éthyle de formule brute C₂₃H₂₅N₃O₆ (M = 439 g).

Le rendement correspondant est de 73%

### Stade B

On procède comme indiqué au Stade M de l'Exemple 1 avec 0,170 g du composé obtenu au stade précédent, 3 ml d'éthanol et 35 mg de palladium sur charbon à 10%. On obtient 0,14 mg du composé trans-8-[[(éthylamino)carbonyl]oxy]-1,2,3,5-tétrahydro-2-hydroxy-3-oxo-1,4-méthano-4*H*-2,4-benzodiazépine-5-carboxylate d'éthyle de formule brute C₁₆H₁₉N₃O₆ (M = 349,35 g).

### Stade C

On procède comme indiqué au Stade N de l'Exemple 1 avec 0,140 mg du produit obtenu au stade précédent, 2 ml de pyridine et 0,180 g du complexe pyridine-SO₃. Le produit obtenu est ensuite traité comme indiqué au Stade E de l'Exemple 12 avec 25 g de résine Dowex. On obtient le composé sel de sodium de trans-8-[[(éthylamino)carbonyl]oxy]-1,2,3,5-tétrahydxo-3-oxo-2-(sulfooxy)-1,4-méthano-4*H*-2,4-benzodiazépine-5-carboxylate d éthyle de formule brute C₁₆H₁₈N₃O₉S,Na (M = 428 g).

LC/SM (électrospray négatif), m/z : M⁻ = 428,1 g.

RMN du proton, DMSO-d₆, 300 MHz, déplacement chimique et multiplicité :
1,08 (t) : CH₃-CH₂-NH ; 3,09 (m) : CH₃-CH₂-NH ; 7,82 (t) : CH₃-CH₂-NH ; 1,26 (t) : CH₃-CH₂-0-CO ; 4,22 (q) : CH₃-CH₂-O-CO ; 3,52 (sl) : N-CH₂-CH ; 4,67 (sl) : N-CH₂-CH ; 5, 00 (s) : N-CH-C=O ; 6,86 (sl), 7,13 (dl), 7,33 (d) : les 3 H aromatiques.

### Exemple 39

### Sel de sodium de trans-8-(4-fluorophényl)-1,2,3,5-tétrahydro-3-oxo-2-(sulfooxy)-1,4-méthano-4H-2,4-benzodiazépine-5-carboxylate d'éthyle

### Stade A

On procède comme indiqué au Stade A de l'Exemple 30 avec l g du composé obtenu au Stade H de l'Exemple 32, 1,17 ml de triéthylamine et 0,67 ml d'anhydride triflique. On obtient 0,9 g de trans-1,2,3,5-tétrahydro-3-oxo-2-(phénylméthoxy)-8-[[(trifluorométhyl)sulfonyl]oxy]-1,4-méthano-4*H*-2,4-benzodiazépine-5-carboxylate d'éthyle de formule brute C₂₁H₁₉F₃N₂O₇ (M = 500,45 g).

### Stade B

On procède comme indiqué au Stade M de l'Exemple 19 avec 0,3 g du produit obtenu au stade précédent, 12 ml de toluène, 60 mg de Pd(Pφ₃)₄, 0,125 g d'acide 4-fluoronylboronique, 2,4 ml d'une solution de Na₂CO₃ 2N. On obtient 0,25 g du composé trans-8-(4-fluorophényl)-1,2,3,5-tétrahydro-3-oxo-2-(phénylméthoxy)-1,4-méthano-4*H*-2,4-benzodiazépine-5-carboxylate d'éthyle de formule brute C₂₆H₂₃FN₂O₄ (M = 446,48 g).

Le rendement correspondant est de 70%.

### Stade C

On procède comme indiqué au Stade M de l'Exemple 1 avec 0,25 g du produit obtenu au stade précédent, 6 ml d'éthanol, 40 mg de palladium sur charbon à 10%. On obtient 0,20 g du composé trans-8-(4-fluorophényl)-1,2,3,5-tétrahydro-2-hydroxy-3-oxo-1,4-méthano-4*H*-2,4-benzodiazépine-5-carboxylate d'éthyle de formule brute C₁₉H₁₇FN₂O₄ (M = 356,36 g).

Le rendement correspondant est quantitatif.

### Stade D

On procède comme indiqué au Stade N de l'Exemple 1 avec 0,20 g du produit obtenu au stade précédent, 2 ml de pyridine et 0,20 g du complexe pyridine-SO₃. Le produit obtenu est traité comme indiqué au Stade E de l'Exemple 12 avec 25 g de résine Dowex. On obtient 0,150 g sous forme de cristaux jaunes du composé sel de sodium de trans-8-(4-fluorophényl)-1,2,3,5-tétrahydro-3-oxo-2-(sulfooxy)-1,4-méthano-4*H*-2,4-benzodiazépine-5-carboxylate d'éthyle de formule brute C₁₉H₁₆FN₂O₅S,NA (M = 458,40 g).

LC/SM (électrospray négatif), m/z : M⁻ = 435,1.

RMN du proton, DMSO-d₆, 300 MHz, déplacement chimique et multiplicité :
1,27 (t) : CH₃-CH₂-O-CO ; 4, 23 (q) : CH₃-CH₂-O-CO ; 3,58 (sl) : N-CH₂-CH ; 4,79 (sl) : N-CH₂-CH ; 5,06 (s) : CH-CO₂-Et ; 7,30 (t) et 7,68 (m) : les H du noyau aromatique fluoré ; 7,38 (d), 7,43 (d), 7,66 (masqué) : les 3 H aromatiques.

### Exemple 40

### Sel de sodium de trans-1,2,3,5-tétrahydro-3-oxo-2-(sulfooxy)-8-[[(trifluorométhyl)sulfonyl]oxy]-1,4-méthano-4H-2,4-benzodiazépine-5-carboxylate d'éthyle

### Stade A

On procède comme indiqué au Stade M de l'Exemple 1 avec 0,12 g du produit obtenu au Stade A de l'Exemple 39. 4 ml d'éthanol et 30 mg de palladium sur charbon à 10%. On obtient 0,105 g du composé trans-1,2,3,5-tétrahydro-2-hydroxy-3-oxo-8-[[(trifluorométhyl)sulfonyl]oxy]-1,4-méthano-4*H*-2,4-benzodiazépine-5-carboxylate d'éthyle de formule brute C₁₄H₁₃F₃N₂O₇S (M = 410,33 g).

Le rendement correspondant est quantitatif.

### Stade B

On procède comme indiqué au Stade N de l'Exemple 1 avec 0,108 g du produit obtenu au stade précédent, 2 ml de pyridine et 120 mg du complexe pyridine-SO₃. Le produit obtenu est traité comme indiqué au Stade R de l'Exemple 12 avec 25 g de résine Dowex. On obtient le composé sel de sodium de trans-1,2,3,5-tétrahydro-3-oxo-2-(sulfooxy)-8-[[(trifluorométhyl)sulfonyl]oxy]-1,4-méthano-4*H*-2,4-benzodiazépine-5-carboxylate d'éthyle de formule brute (à vérifier par le client) C₁₄H₁₂F₃N₂O₁₀S₂ (M = 489 g).

LC/SM (électrospray négatif), m/z : M⁻ = 489.

RMN du proton, DMSO-d₆, 300 MHz, déplacement chimique et multiplicité :
1,26 (t) : CH₃-CH₂-O-CO ; 4,22 (q) : CH₃-CH₂-O-CO ; 3,51 (m) : N-CH₂-CH ; 4,78 : N-CH₂-CH ; 5,15 (sl) : N-CH-CO ; 7,23 (sl) et 7,56 (sl) : les 3 H aromatiques.

### Exemple 41

### Bel disodique de trans-1,2,3,5-tétrahyro-3-oxo-2-(sulfooxy)-8-[2-(aulfooxy)éthoxy]-1,4-méthano-4H-2,4-benzodiazépine-5-carboxamide

### Stade A

On procède comme indiqué au Stade I de l'Exemple 12 avec 3 g du produit obtenu au Stade G de l'Exemple 32, 60 ml de THF, 60 ml de tert-butanol, 30 ml d'eau, 0,054 g de tetroxide d'osmium et 0,99 g de N-oxyde de N-méthyl morpholine. On obtient 3,3 g de produit brut qui sont utilisés tels quels dans l'étape suivante.

### Stade B

3,3 g du produit brut obtenu précédemment sont dissout dans 60 ml de diméthoxypropane et 14 ml d'acétone en présence d'acide para-toluène sulfonique à 0°C. Après 30 minutes d'agitation, on évapore à sec la solution et le résidu est purifié par chromatographie sur silice en éluant avec une mélange heptane/acétate d'éthyle l/1. On obtient 3,075 g de composé trans-8-[(2,2-diméthyl-1,3-dioxolan-4-yl)méthoxy]-1,2,3,5-tétrahydro-3-oxo-2-(phénylméthoxy)-1,4-méthano-4*H*-2,4-benzodiazépine-5-carboxylate d'éthyle de formule brute C₂₆H₃₀N₂O₇ (M = 482,54 g).

Le rendement correspondant est de 90%.

### Stade C

On procède comme indiqué au Stade A de l'Exemple 7 avec 3 g du produit obtenu au stade précédent, 0,261 g de LiOH.H₂O, 15 ml de THF et 15 ml d'eau. On obtient 2 g du composé trans-acide 8-[(2,2-diméthyl-1,3-dioxolan-4-yl)méthoxy]-1,2,3,5-tétrahydro-3-oxo-2-(phénylméthoxy)-1,4-méthano-4*H*-2,4-benzodiazépine-5-carboxylique de formule brute C₂₄H₂₆N₂O₇, (M = 454,48 g).

Le rendement correspondant est de 72%.

### Stade D

On procède comme indiqué au Stade B de l'Exemple 7 avec 2 g du produit obtenu au stade précédent, 32 ml de DMF, 2,79 g de BOP, 10,89 g de HOBt, 0, 47 g de NH₄Cl, 3,06 ml de diisopropyléthylamine. On obtient 1,6 g sous forme de cristaux blancs du composé trans-8-[(2,2-diméthyl-1,3-dioxolan-4-yl)méthoxy]-1,2,3,5-tétrahydro-3-oxo-2-(phénylméthoxy)-1,4-méthano-4*H*-2,4-benzodiazépine-5-carboxamide de formule brute C₂₄H₂₇N₃O₆ (M = 453,50 g).

Le rendement correspondant est de 80%.

### Stade E

On dissout 1 g du produit obtenu au stade précédent dans 10 ml d'un mélange d'acide trifluoroacétique/eau 9/1. On agite pendant 10 minutes puis on évapore à sec le milieu réactionnel tout en effectuant un entraînement au toluène. Le résidu est solubilisé dans 50 ml de chlorure de méthylène auxquels on ajoute 40 ml d'une solution aqueuse saturée en NaHCO₃. On ajoute 100 ml de THF et 40 ml d'une solution aqueuse saturée de NaCl. Après décantation, on lave la phase organique avec une solution saturée de NaCl et on la sèche sur sulfate de magnésium. Après évaporation du solvant sous pression réduite, on obtient des cristaux qui sont repris dans l'éther. Après filtration on obtient 0,82 g du composé trans-8-(2,3-dihydroxypropoxy)-1,2,3,5-tétrahydro-3-oxo-2-(phénylméthoxy)-1,4-méthano-4H-2,4-benzodiazépine-5-carboxamide de formule brute C₂₁H₂₃N₃O₆ (M = 413,43 g).

Le rendement correspondant est de 90%.

### Stade F

On dissout 0,6 g du produit obtenu au stade précédent dans un mélange contenant 13ml de THF 4ml d'eau et 4 ml de méthanol. On ajoute à la solution refroidie à 0°C, 0,62 g de NaIO₄. On agite à 0°C et le produit cristallise. On verse la suspension dans l'acétate d'éthyle, on lave à l'eau et la phase organique est séchée sur sulfate de magnésium. Après évaporation sous pression réduite, on obtient 53 g du composé trans-1,2,3,5-tétrahydro-3-oxo-8-(2-oxoéthoxy)-2-(phénylméthoxy)-1,4-méthano-4H-2,4-benzodiazépine-5-carboxamide de formule brute C₂₀H₁₉N₃O₅.

Le rendement correspondant est de 90%.

### Stade G

On dissout 0,52 g du produit obtenu au stade précédent dans l'éthanol. La solution est refroidie à 0°C et on ajoute quatre équivalents de NaBH₄. On agite pendant deux heures puis on ajoute au milieu réactionnel un mélange heptane/acétate d'éthyle 1/4. La phase organique est lavée avec de l'eau puis avec une solution aqueuse de NaH₂PO₄ puis séchée sur sulfate de magnésium. Après évaporation du solvant, on obtient 0,43 g de produit brut qui est purifié par chromatographie sur silice en éluant avec un mélange chlorure de méthylène contenant 10% de méthanol. Après évaporation des fractions, on obtient 0,187 g de cristaux blancs du composé trans-1,2,3,5-tétrahydro-8-(2-hydroxyéthoxy)-3-oxo-2-(phénylméthoxy)-1,4-méthano-4*H*-2,4-benzodiazépine-5-carboxamide de formule brute C₂₀H₂₁N₃O₅ (M = 383,41 g),

Le rendement correspondant est de 38%.

### Stade H

On procède comme indiqué au Stade M de l'Exemple 1 avec 0,18 g du produit obtenu au stade précédent, 2 ml d'éthanol, 2 ml de THF et 0,043 g de palladium sur charbon à 10%. On obtient 0,12 g du composé trans-1,2,3,5-tétrahydro-2-hydroxy-8-(2-hydroxyéthoxy)-3-oxo-1,4-méthano-4*H*-2,4-benzodiazépine-5-carboxamide de formule brute C₁₃H₁₅N₃O₅.

Le rendement correspondant est de 90%.

### Stade I

On procède comme indiqué au stade N de l'Exemple 1 avec 0,12 g du produit obtenu au stade précédent; 3 ml de pyridine et 159 mg du complexe pyridine-SO₃. Le produit obtenu est traité comme au Stade R de l'Exemple 12 avec 25 g de résine Dowex. On obtient 0,15 g du composé sel disodique de trans-1,2,3,5-tétrahydro-3-oxo-2-(sulfooxy)-8-[2-(sulfooxy)éthoxy]-1,4-méthano-4*H*-2,4-benzodiazépine-5-carboxamide de formule brute C₁₃H₁₃N₃O₁₁S₂,2Na (M = 451,39 + 2 x 22,99 g).

LC/SM (électrospray négatif), m/z : (M²⁻ + H)⁻ = 451,9.

(M²⁻ + H)⁻ = 451,9.

RMN du proton, DMSO-d₆, 300 MHz, déplacement chimique et multiplicité :
3,41 (dd) et 3,77 (d) : N-CH₂-CH ; 4,60 (d) : N-CH₂-CH ; 4,02 (m) et 4,10 (m) : O-CH₂-CH₂ ; 4,72 (s) : CH-CO-NH₂ ; 7,40 (sl) et 7,86 (sl) : CH-CO-NH₂ ; 6,66 (d), 6,93 (dd), 7,16 (d) : les 3 H aromatiques.

### Exemple 42

### Sel de sodium de trans-8-[(2,2-diméthyl-1,3-dioxolan-4-yl)méthoxy]-1,2,3,5-tétrahydro-3-oxo-2-(sulfooxy)-1,4-méthano-4H-2,4-benzodiazépine-5-carboxamide

### Stade A

On procède comme au Stade M de l'Exemple 1 avec 0,6 g du produit obtenu au Stade D de l'Exemple 41, 60 mg de palladium sur charbon à 10% et 20 ml d'éthanol et 3 ml de THF. On obtient 0,46 g du composé trans-8-[(2,2-diméthyl-1,3-dioxolan-4-yl)méthoxy]-1,2,3,5-tétrahydro-2-hydroxy-3-oxo-1,4-méthano-4*H*-2,4-benzodiazépine-5-carboxamide de formule brute C₁₇H₂₁N₃O₆ (M = 363,37 g).

Le rendement correspondant est de 86%.

### Stade B

On procède comme au Stade N de l'Exemple 1 avec 0,46 g du produit obtenu au stade précédent, 3 ml de pyridine et 159 mg du complexe pyridine-SO₃. On obtient un composé qui est traité comme indiqué au Stade R de l'Exemple 12 avec 25 g de résine Dowex. On obtient 0,136 g du composé sel de sodium de trans-8-[(2,2-diméthyl-1,3-dioxolan-4-yl)méthoxy]-1,2,3,5-tétrahydro-3-oxo-2-(sulfooxy)-1,4-méthano-4*H*-2,4-benzodiazépine-5-carboxamide de formule brute C₁₇R₂₀N₃O₉S.Na (M = 442,43 + 22,99 g).

Le rendement correspondant est de 93%.

LC/SM (électrospray négatif), m/z : M⁻ = 442.

RMN du proton, DMSO-d₆ à 300 MHz, déplacement chimique et multiplicité :
1,30 (s), 1,36 (s) : (CH₃)₂-C-C ; 3, 42 (dl), 3,74 (m) : N-CH₂-CH ; 4,60 (dl) : N-CH₂-CH ; 3,76 (m), 4,09 (dd), 3,95 (m), 4,01 (m) : O-CH₂-CH-CH₂-O ; 4,40 (m) : O-CH₂-CH-CH₂-O ; 4,73 (sl) : CH-CO-NH₂ ; 7,41 (sl), 7,87 (sl) : CH-CO-NH₂ ; 6,68 (d), 6,94 (dd), 7,16 (d) : les 3 H aromatiques.

### Exemple 43

### Sel de sodium de trans-8-(2,3-dihydroxypropoxy)-1,2,3,5-tétrahydro-3-oxo-2-(sulfooxy)-1,4-méthano-4H-2,4-benzodiazépine-5-carboxamide

On dissout 0,136 g du produit obtenu au Stade B de l'Exemple 42 dans 1,3 ml d'un mélange acide trifluoroacétique/eau 9/1 à 0°C. On agite pendant 15 minutes, puis on évapore à sec le milieu réactionnel. Le résidu est repris dans l'éther puis filtré. On obtient 0,106 g du composé sel de sodium de trans-8-(2,3-dihydroxypropoxy)-1,2,3,5-tétrahydro-3-oxo-2-(sulfooxy)-1,4-méthano-4*H*-2,4-benzodiazépine-5-carboxamide de formule brute C₁₄H₁₆N₃O₉S, Na (M = 401,37 g + 23 g).

Le rendement correspondant est de 85%.

LC/SM (électrospray négatif), m/z : M⁻ = 402.

RMN du proton, DMSO-d₆, 300 MHz, (déplacement chimique et multiplicité) :
3,43 (m) : CH₂-OH ; 4,67 : CH₂-OH ; 3,99 (m) : O-CH₂-CH-OH ; 3,82 (m) : O-CH₂-CH-OH ; 4,97 (dl) : O-CH₂-CH-OH ; 3,46 et 3,72 : N-CH₂-CH-N ; 4,60 (d) : N-CH₂-CH-N ; 4,73 (s) : φ-CH-N-CO ; 6,67 (d), 6,91 (dd), 7,16 (d) : les 3 H aromatiques ; 7,41 (sl) et 7,87 (sl) : CO-NH₂.

### Exemple 44

### Sel de sodium de trans-3-(4-fluoxophényl)-4,6,7,8-tétrahydro-6-oxo-7-(sulfooxy)-5,8-méthano-5H-thiéno[2,3-e][1,3]diazépine-4-carboxylate d'éthyle

### Stade A

Dans un ballon placé sous atmosphère d'argon, on introduit 100 mg du composé 3,4-dibromo-thiophène dans 300 ml d'éther. On agite et on refroidit le milieu réactionnel à -70°C puis on introduit à l'aide d'une cannule 271 ml d'une solution 1,6M de butyllithium dans l'éther. On agite pendant 45 minutes à -70°C, Cette solution est introduite par cannule dans une solution préalablement refroidie à -70°C sous atmosphère d'argon de 172 mg diéthyloxalate dans 250 ml d'éther contenant un peu de THF. On agite pendant 1 heure à -70°C. Puis une solution revenue à température ambiante, on ajoute une solution aqueuse 1M de NaH₂PO₄, on décante et on extrait plusieurs fois la phase aqueuse avec de l'éther. Les phases organiques sont rassemblées puis séchées sur sulfate de magnésium et filtrées. Après évaporation du solvant sous pression réduite, on obtient 108 g du composé bromé attendu.

Le rendement correspondant est quantitatif.

### Stade B

On dissout 108,6 g du produit obtenu au stade précédent dans 570 ml d'éthanol et 230 ml de THF. On refroidit la solution à -35°C et on ajoute très lentement 15,7 g de NaBH₄. On observe un dégagement gazeux et une fois l'addition terminée, on traite le milieu réactionnel par addition d'un mélange heptane/acétate d'éthyle 4/1. Puis la solution est versée sur une solution glacée de NaH₂PO₄ 1M et la phase aqueuse est extraite avec un mélange heptane/acétate d'éthylc 1/4. Après décantation, on sature la phase aqueuse avec NaCl et on extrait à nouveau la phase aqueuse avec un mélange heptane/acétate d'éthyle 1/42. Les phases organiques sont rassemblées et séchées sur sulfate de magnésium puis concentrées sous pression réduite. Le résidu obtenu après évaporation est traité par 150 ml d'un mélange heptane/acétate d'éthyle 6/1 et 150 ml d'éther. La solution est refroidie à -10°C par un bain méthanol/glace afin de faire précipiter les sels de bore. Après filtration, le filtrat est purifié sur silice en éluant avec un mélange heptane/acétate d'éthyle 4/1. Après évaporation des fractions, on obtient 74,5 g du composé 4-bromo-α-hydroxy-3-thiophèneacétate d'éthyle de formule brute C₈H₉BrO₃S (M = 265,13 g) .

Le rendement correspondant est de 68%.

### Stade C

On procède comme au Stade C de l'Exemple 27 avec 74,5 g du produit obtenu au stade précédent, 97,5 g de (CH₃SO₂)₂O et 80,8 ml de triéthylamine et 395 ml de chlorure de méthylène. On obtient avec un rendement quantitatif le composé 4-bromo-α-[(méthylsulfonyl)oxy]-3-thiophèneacétate d'éthyle de formule brute C₉H₁₁BrO₅S₂ (M = 343,22 g).

### Stade D

On procède comme au Stade D de l'Exemple 27 avec le produit obtenu au stade précédent, 57,8 ml de glycinate de tert-butyle, 50 ml de 2,6-lutidine, on obtient 82,3 g du composé 4-bromo-α-[[2-(1,1-diméthyléthoxy)-2-oxoéthyl]amino]-3-thiophèneacétate d'éthyle de formule brute C₁₄H₂₀BrN₄S (M = 378,29 g) .

Le rendement correspondant est de 77%.

### Stade E

On procède comme au Stade E de l'Exemple 27 avec 48 g du produit obtenu au stade précédent, 24 ml d'anhydride trifluoroacétique et 22 ml de triéthylamine et 250 ml de dichlorométhane. On obtient 32,75 g du composé 4-bromo-α-[[2-(1,1-diméthyléthoxy)-2-oxoéthyl](trifluoroacétyl)amino]-3-thiophèneacétate d'éthyle de formule brute C₁₆H₁₉BrF₃NO₅S (M = 474,30).

Le rendement correspondant est de 53%.

### Stade F

On procède comme au Stade F de l'Exemple 27 avec 40 g du produit obtenu au stade précédent. 234 ml d'acide trifluoroacétique et 230 ml de dichlorométhane. On obtient 37 g du composé 4-bromo-α-[(carboxyméthyl)(trifluoroacétyl)amino]-3-thiophèneacétate d'éthyle de formule brute C₁₂H₁₁BrF₃NO₅S (M = 418,19 g).

Le rendement correspondant est quantitatif.

### Stade G

On procède comme au Stade G de l'Exemple 27 avec 37 g du produit obtenu au stade précédent et 66 ml de SOCl₂. On chauffe à reflux et on agite la solution pendant une heure. Après évaporation à sec, le produit est traité toujours comme au Stade G de l'Exemple 27 par 30 g de AlCl₃ en solution dans 230 ml de nitrométhane. On obtient 9,94 g de 3-bromo-4,5,6,7-tétrahydro-7-oxo-5-(trifluoroacétyl)-thiéno[3,2-c]pyridine-4-carboxylate d'éthyle de formule brute C₁₂H₉BrF₃NO₄S (M = 400,17 g).

Le rendement correspondant est de 28%.

### Stade H

On dissout 9,3 g du produit obtenu au stade précédent dans 100 ml de dichlorométhane et 88 ml de méthanol et on ajoute 0,44 g de NaBH₄. On agite pendant 30 minutes, puis le milieu réactionnel est versé dans une solution aqueuse 1M de NaH₂PO₄ et d'acétate d'éthyle à 0°C. La phase organique est séchée sur sulfate de magnésium et le solvant est évaporé sous pression réduite. On obtient 8,73 g du composé 3-bromo-4,5,6,7-tétrahydro-7-hydroxy-5-(trifluoroacétyl)-thiéno[3,2-*c*]pyridine-4-carboxylate d'éthyle de formule brute C₁₂H₁₁BrF₃NO₃S (M = 402,19 g).

Le rendement correspondant est de 94%.

### Stade I

On procède comme au Stade H de l'Exemple 1 avec 8,73 g du produit obtenu au stade précédent, 5,67 g d'anhydride méthane sulfonique, 3,4 ml de triéthylamine, 17 ml de benzylhydroxylamine et 80 ml de dichlorométhane. On obtient 5,9 g du composé trans-3-bromo-4,5,6,7-tétrahydro-7-[(phénylméthoxy)amino]-5-(trifluoroacétyl)-thiéno[3,2-*c*]pyridine-4-carboxylate d'éthyle de formule brute C₁₉H₁₈BrF₃N₂O₄S (M = 507,33 g).

Le rendement correspondant est de 50%.

### Stade J

Dans un ballon, on dissout 0,05 g du composé obtenu au stade précédent dans 1,25 ml de méthanol et 0,5 ml de THF. La solution est refroidie à 0°C et placée sous argon. On agite et on ajoute 0,007 g de NaBH₄ et 0,013 g de CaCl₂ en poudre. Une fois le dégagement gazeux terminé, le milieu réactionnel est versé sur une solution 1M de NaH₂PO₄ ; la phase aqueuse est extraite par un mélange heptane/acétate d'éthyle 1/2 à 0°C plusieurs fois. Les phases organiques sont rassemblées, séchées sur sulfate de magnésium, puis concentrées sous pression réduite. On obtient 44 mg de produit brut qui sont purifiés par chromatographie sur silice en éluant avec un mélange heptane/acétate d'éthyle 1/ 2. Après évaporation des solvants, on obtient 14,6 mg du composé trans-3-bromo-4,5,6,7-tétrahydro-7-[(phénylméthoxy)amino]-thiéno[3,2-*c*]pyridine-4-carboxylate d'éthyle de formule brute C₁₇H₁₉BrN₂O₃S (M = 411,32 g).

Le rendement correspondant est de 36%.

### Stade K

On procède comme au Stade K de l'Exemple 1 avec 4,5 g du produit obtenu au stade précédent, 2,84 ml de triéthylamine, et 0,6 ml de diphosgène et 1215 ml d'acétonitrile. On obtient 328 mg du composé tans-3-bromo-4,6,7,8-tétrahydro-6-oxo-7-(phénylméthoxy)-5,8-méthano-5*H*-thiéno[2,3-*e*][1,3]diazépine-4-carboxylate d'éthyle de formule brute C₁₈H₁₇BrN₂O₄S (M = 437,32 g) .

Le rendement correspondant est de 7,6%.

### Stade L

On procède comme au Stade M de l'Exemple 19 avec 308 mg du composé obtenu au stade précédent, 2,86 ml d'une solution 2M de Na₂CO₃, 71,8 mg de Pd(Pφ₃)₄ et 148,2 mg d'acide 4-fluorophényl boronique et 15,24 ml de toluène. On obtient 109 mg du composé trans-3-(4-fluorophényl)-4,6,7,8-tétrahydro-6-oxo-7-(phénylméthoxy)- 5,8-méthano-5*H*-thiéno[2,3-*e*][1,3]diazépine-4-carboxylate d'éthyle de formule brute C₂₉H₂₃FN₂O₄S (M = 452,52 g).

Le rendement correspondant est de 34%.

### Stade M

On procède comme indiqué au Stade M de l'Exemple 1 avec 50 mg du produit obtenu au stade précédent, 50 mg de palladium sur charbon à 30% et 0,8 ml de méthanol et 2 ml de THF. On obtient avec un rendement quantitatif le composé Trans-3-(4-fluorophényl)-4,6,7,8-tétrahydro-7-hydroxy-6-oxo-5,8-méthano-5*H*-thiéno[2,3-*e*][1,3]diazépine-4-carboxylate d'éthyle de formule brute C₁₇H₁₅FN₂O₄S (M = 362,38 g).

### Stade N

On procède comme au Stade M de l'Exemple 1 avec le composé obtenu au stade précédent et 53 mg du complexe pyridine-SO₃ et 0,8 ml de pyridine. Le produit obtenu est traité comme au Stade R de l'Exemple 12 avec 15 g de résine Dowex. On obtient 15,4 mg du composé sel de sodium de trans-3-(4-fluorophényl)-4,6,7,8-tétrahydro-6-oxo-7-(sulfooxy)-5,6-méthano-5*H-*thiéno[2,3-*e*][1,3]diazépine-4-carboxylate d'éthyle de formule brute C₁₇H₁₄FN₂O₇S₂, Na (M = 464,43 g).

Le rendement correspondant est de 30%.

LC/SM (électrospray négatif), m/z : M⁻ = 441.

RMN du proton, D₂O, 300 MHz, déplacement chimique et multiplicité :
0,95 (t) : CO-O-CH₂-CH₃ ; 3,97 (m) : CO-O-CH₂-CH₃ ; 3,58 (dl), 3,79 (dl) : N-CH₂-CH-C= ; 5,07 (sl) : N-CH₂-CH-C= ; 5,45 (s) : N-CH-C= ; 7,17 (m) et 7,31 (m) : les 4 H aromatiques du noyau fluoré ; 7,35 (s) : S-CH=.

### Exemple 45

### Sel de sodium de trans-2,5,6,8-tétrahydro-6-oxo-2-phényl-5-(sulfooxy)- 4H-4,7-méthanopyrazolo[3,4-e][1,3]diazépine-8-carboxamide

### Stade A

Dans un ballon, on dissout 54,2 mg du produit obtenu au Stade K de l'Exemple 1 dans 2 ml de dichlorométhane en présence de 40,6 mg d'acide phényl boronique. On obtient une suspension à laquelle on ajoute 45 mg de Cu(OAc)₂ et 2 µl de pyridine et 125 mg de tamis 4 Å. On agite à 20°C pendant 3h30 puis le milieu réactionnel est concentré sous pression réduite. Le résidu obtenu est purifié par chromatographie sur silice en éluant avec un mélange chlorure de méthylène/acétone/triéthylamine 95/5/0,1%. On obtient 33 mg du composé Trans-2,5,6,8-tétrahydro-6-oxo-2-phényl-5-(phénylméthoxy)- 4*H*-4,7-méthanopyrazolo[3,4-*e*][1,3]diazépine-8-carboxylate de méthyle de formule brute C₂₂H₂₀N₄O₄ (M = 404,43 g).

Le rendement correspondant est de 50%.

### Stade B

On procède comme au Stade A de l'Exemple 7 avec 81 mg du produit obtenu au stade précédent, 1,7 ml de dioxanne, 1,7 ml d'eau et 0,22 ml de soude 1N. On obtient 70 mg du composé trans-acide 2,5,6,8-tétrahydro-6-oxo-2-phényl-5-(phénylméthoxy)- 4*H*-4,7-méthanopyrazolo[3,4-*e*][1,3]diazépine-8-carboxylique de formule brute C₂₁H₁₈N₄O₄ (M = 390,4 g).

Le rendement correspondant est de 89,5%.

### Stade C

On procède comme au Stade B de l'Exemple 7 avec 70 mg du produit obtenu au stade précédent, 2,8 ml de DMF, 114 mg de BOP, 37 mg de HOBt, 20 mg de NH₄Cl et 125 µl de DIPEA. On obtient 67,5 mg du composé trans-2,5,6,8-tétrahydro-6-oxo-2-phényl-5-(phénylméthoxy)- 4*H*-4,7-méthanopyrazolo[3,4-*e*][1,3]diazépine-8-carboxamide de formule brute C₂₁H₁₉N₅O₃ (M = 389,42 g).

Le rendement correspondant est de 95%.

### Stade D

On procède comme au Stade M de l'Exemple 1 avec 67,5 mg du produit obtenu au stade précédent, 5 ml de méthanol, 4 ml de THF, et 60 mg de palladium sur charbon à 30%. On obtient 48 mg du composé trans-2,5,6,8-tétrahydro-5-hydroxy-6-oxo-2-phényl-4*H*-4,7-méthanopyrazolo[3,4-*e*][1,3]diazépine-8-carboxamide de formule brute C₁₄H₁₃N₅O₃ (M = 299,29 g).

Le rendement correspondant est de 92%.

### Stade E

On procèce comme au Stade N de l'Exemple 1 avec 48 mg du produit obtenu au stade précédent, 5 ml de pyridine, 77 mg du complexe pyridine-SO₃. Le produit obtenu est purifié par chromatographie sur silice en éluant avec un mélange dichlorométhane/éthanol/ triéthylamine 6/4/0,1%. On obtient 50 mg du composé Sel de triéthylammonium de trans-2,5,6,8-tétrahydro-6-oxo-2-phényl-5-(sulfooxy)- 4*H*-4,7-méthanopyrazolo[3,4-*e*][1,3]diazépine-8-carboxamide de formule brute C₂₀H₂₈N₆O₆S, C₆H₁₅N (M = 480,55 g).

Le rendement correspondant est de 64%.

### Stade F

On procède comme au Stade R de l'Exemple 12 avec 50 mg du produit obtenu au stade précédent et 35 g de résine Dowex. On obtient 34 mg du composé sel de sodium de trans-2,5,6,8-tétrahydro-6-oxo-2-phényl-5-(sulfooxy)- 4H-4,7-méthanopyrazolo[3,4-*e*][1,3]diazépine-8-carboxamide de formule brute C₁₄H₁₂N₅O₆S, Na (M = 401,34 g).

Le rendement correspondant est de 81%.

LC/SM (électrospray négatif), m/z : M⁻ = 378.

RMN du proton, D₂O à 300 MHz, déplacement chimique et multiplicité :
3,45 (d), 3,83 (dd) : N-CH₂-CH-C= ; 5,07 (d) : N-CH₂-CH-C= ; 5,35 (s) : N-CH-C=-C= : 7,43 (tl), 7,54 (tl), 7,65 (dl), 8,28 (s) : les 5 H du noyau aromatique.

### Exemple 46

### Sel de sodium de 1,4,5,8-tétrahydro-1-phényl-5-(sulfooxy)-6H-4,7-méthanopyrazolo[3,4-e][1,3]diazépin-6-one

### Stade A

Dans un ballon, on dissout 5,51 g du produit obtenu au Stade A1 de l'Exemple 2 dans 100 ml d'éthanol. On ajoute 2,23 ml de phényl hydrazine puis on agite la solution pendant une heure à température ambiante. Le milieu réactionnel est ensuite concentré sous vide. On obtient 6,71 g du composé 3,5-dioxo-4-[(2-phénylhydrazino)méthylène]-1-pipéridinecarboxylate de 1,1-diméthyléthyle avec un rendement quantitatif.

### Stade B

On dissout 6,71 g du produit obtenu au stade précédent dans 145 ml d'acide acétique. On chauffe à reflux cette solution pendant une heure puis on évapore l'acide acétique. On rajoute du toluène au résidu et on évapore à nouveau. On obtient 7,3 g de produit brut qui est purifié par chromatographie sur silice en éluant avec un mélange chlorure de méthylène/acétone 95/5. On obtient 1,82 g du composé 1,4,5,7-tétrahydro-4-oxo-1-phényl-6*H*-pyrazolo[3,4-*c*]pyridine-6-carboxylate de 1,1-diméthyléthyle de formule brute C₁₇H₁₉N₃O₃ (M = 313,36 g).

Le rendement correspondant est de 28%.

### Stade C

On mélange 300 mg du produit obtenu au stade précédent dans 10 ml d'éthanol. On ajoute 3 ml de dichlorométhane puis 115 mg de NH₂O-CH₂-CH-CH₂-HCl et 0,23 ml de pyridine. On agite à 20°C pendant 3 heures puis on dilue le milieu réactionnel avec du dichlorométhane. La solution est lavée à l'eau et la phase organique est séchée sur sulfate de magnésium puis concentrée sous pression réduite. On obtient 355 mg du composé 1,4,5,7-tétrahydro-1-phényl-4-[(2-propényloxy)imino]-6*H*-pyrazolo[3,4-*c*]pyridine-6-carboxylate de 1,1-diméthyléthyle de formule brute C₂₀H₂₄N₄O₃ (M = 368,44 g).

Le rendement correspondant est quantitatif.

### Stade D

On dissout 0,355 g du produit obtenu au stade précédent dans 5 ml d'acide acétique. On refroidit la solution à 10°C et on ajoute en plusieurs fois 500 mg de NaBH₃CN. On agite pendant 5 heures à température ambiante puis le milieu réactionnel est dilué avec 150 ml d'acétate d'éthyle. Le milieu est refroidi à 0°C avant d'être neutralisé par 35 ml d'une solution de soude 2N. On agite encore 15 minutes à 0°C et on extrait à l'acétate d'éthyle. Les phases organiques sont lavées par des solutions aqueuses de soude 1N puis séchées sur sulfate de magnésium. Après évaporation sous pression réduite du solvant, on obtient 360 mg de produit brut qui sont purifiés par chromatographie sur silice en éluant avec un mélange chlorure de méthylène/acétone 95/5. Après évaporation des solvants, on obtient 310 mg du composé 1,4,5,7-tétrahydro-1-phényl-4-[(2-propényloxy)amino]-6*H*-pyrazolo[3,4-*c*]pyridine-6-carboxylate de 1,1-diméthyléthyle de formule brute C₂₀H₂₆ N₄O₃ (M = 370,46 g).

Le rendement correspondant est de 86%.

### Stade E

On procède comme au Stade I de l'Exemple 1 avec 305 mg du produit obtenu au stade précédent, 3 ml d'acétate d'éthyle, 3 ml d'une solution d'HCl 5,5M dans l'acétate d'éthyle. On obtient 256 mg du composé Dichlorure de 4,4,6,7-tétrahydro-1-phényl-4-[(2-propényloxy)amino]-1H-pyrazolo[3,4-c]pyridine de formule brute C₁₅H₁₈N₄O, 3HCl (M = 379,72 g).

Le rendement correspondant est de 90%.

### Stade F

On procède comme au Stade K de l'Exemple 1 avec 197 mg du produit obtenu au stade précédent, 32 ml d'acétonitrile, 0,44 ml de triéthylamine, et 40 µl de diphosgène. On obtient 76 mg du composé 1,4,5,8-tétrahydro-1-phényl-5-(2-propényloxy)-6*H*-4,7-méthanopyrazolo[3,4-*e*][1,3]diazépin-6-one de formule brute C₁₆H₁₆N₄O₂ (M = 296,33 g).

Le rendement correspondant est de 49%.

### Stade G

On procède comme au Stade G de l'Exemple 18 avec 71 mg du dérivé obtenu au stade précédent. 7 ml de dichlorométhane, 35 µl d'acide acétique et 139 mg de Pd(Pφ₃)₄. Le produit obtenu est ensuite traité par 8 ml de pyridine et 153 mg du complexe pyridine-SO₃. On obtient 136 mg du composé sel de 1-propényltriphénylphosphonium de 1,4,5,8-tétrahydro-1-phényl-5-(sulfooxy)-6H-4,7-méthanopyrazolo[3,4-*e*][1,3]diazépin-6-one de formule brute C₂₁H₂₀P, C₁₃H₁₁N₄O₅S (M = 638, 69 g).

Le rendement correspondant est de 71,8%.

### Stade H

On procède comme au Stade R de l'Exemple 12 avec 136 mg du produit obtenu au stade précédent et 45 g de résine Dowex. On obtient 67 mg du composé sel de sodium de 1,4,5,8-tétrahydro-1-phényl-5-(sulfooxy)-6*H*-4,7-méthanopyrazolo[3,4-e][1,3]diazépin-6-one de formule brute C₁₃H₁₁N₄NaO₅S (M = 358,31 g).

Le rendement correspondant est de 88%.

LC/SM (électrospray négatif), m/z : M⁻ = 335 et (2M+H)⁻ = 671.

RMN du proton, D₂O, 300 MHz, déplacement chimique et multiplicité :
5,04 (d) : N-CH₂-CH-C= ; 3,48 (d) et 3,84 (dd) : N-CH₂-CH-C= ; 4,48 et 4,74 : N-CH₂-C= ; 7,50 (m), 7,57 (m), 7,45 (m) : les 5 H du noyau aromatique ; 7,85 (s) : N=CH-C=.

### Exemple 47

### Sel de sodium de trans-4,5,6,8-tétrahydro-6-oxo-1-phényl-5-(sulfooxy)-1H-4,7-méthanopyrazolo[3,4-e][1,3]diazépine-8-carboxamide

### Stade A

On procède comme au Stade C de l'Exemple 1 avec 312 mg du produit obtenu au Stade B de l'Exemple 46, 20 ml de méthanol et 38 mg de NaBH₄. On obtient 312 mg du composé 1,4,5,7-tétrahydro-4-hydroxy-1-phényl-6*H*-pyrazolo[3,4-*c*]pyridine-6-carboxylate de 1, 1-diméthyléthyle de formule brute C₁₆H₂₁N₃O₃ (M = 315,38 g).

Le rendement correspondant est de 99%.

### Stade B

On procède comme au Stade G de l'Exemple 1 avec 160 mg du produit obtenu au stade précédent, 4 ml de THF anhydre et 0,89 ml d'une solution 1,7M de tert-butyllithium dans le pentane et en présence d'un courant gazeux de CO₂. Le produit obtenu est ensuite acidifié par HCl 2N puis traité par du diazométhane et l'on obtient 115 mg du composé 1,4,5,7-tétrahydro-4-hydroxy-1-phényl-6*H*-pyrazolo[3,4-*c*]pyridine-6,7-dicarboxylate de 6-(1,1-diméthyléthyle) et de 7-méthyle de formule brute C₁₉H₂₃N₃O₅ (M = 373,41 g).

Le rendement correspondant est de 60%.

### Stade C

On procède comme au Stade H de l'Exemple 1 avec 157 mg du produit obtenu au stade précédent, 5 ml de dichlorométhane, 90 µl de triéthylamine, 110 mg de MS₂O et 156 mg de benzylhydroxylamine. On obtient 102 mg du composé 1,4,5,7-tétrahydro-1-phényl-4-[(phénylméthoxy)amino]-6*H*-pyrazolo[3,4-*c*]pyridine-6,7-dicarboxylate de 6-(1,1-diméthyléthyle) et de 7-méthyle de formule brute C₂₆H₃₀N₄O₅ (M = 478, 55 g).

Le rendement correspondant est de 50,6%.

### Stade D

On procède comme au Stade I de l'Exemple 1 avec 102 mg du produit obtenu au stade précédent, 1 ml d'acétate d'éthyle, 1 ml de méthanol et 1 ml d'une solution HCl 5,5M dans l'acétate d'éthyle. On obtient 91 mg du composé dichlorhydrate de 4,5,6,7-tétrahydro-1-phényl-4-[(phénylméthoxy)amino]-1*H-*pyrazolo[3,4-*c*]pyridine-7-carboxylate de méthyle de formule brute C₂₁H₂₂N₄O₃, 3HCl (M = 487,82 g).

### Stade E

On procède comme au Stade K de l'Exemple 1 avec 90 mg du composé obtenu au stade précédent, 10 ml d'acétonitrile, 150 ml de triéthylamine, 14 µl de diphosgène. On obtient 72 mg du composé trans-4,5,6,8-tétrahydro-6-oxo-1-phényl-5-(phénylméthoxy)-1*H*-4,7-méthanopyrazolo[3,4-*e*][1,3]diazépine-8-carboxylate de méthyle de formule brute C₂₂H₂₀N₄O₄ (M = 404,43 g).

Le rendement correspondant sur les deux stades D et E est de 84%.

### Stade F

On procède comme au Stade A de l'Exemple 7 avec 72 mg du produit obtenu au stade précédent, 1,5 ml de dioxanne, 1,5 ml d'eau et 0,2 ml d'une solution de soude 1N. On obtient 69 mg du composé trans-acide 4,5,6,8-tétrahydro-6-oxo-1-phényl-5-(phénylméthoxy)-1*H*-4,7-méthanopyrazolo[3,4-*e*][1,3]diazépine-8-carboxylique de formule brute C₂₁H₁₈N₄O₄ (M = 390,4 g).

Le rendement correspondant est de 99%.

### Stade G

On procède comme au Stade D de l'Exemple 7 avec 68 mg du produit obtenu au stade précédent, 2 ml de DMF, 112 mg de BOP, 36 mg de HOBt, 20 mg de NH₄Cl et 123 µl de DIPEA. On obtient 50 mg du composé trans-4,5,6,8-tétrahydro-6-oxo-1-phényl-5-(phénylméthoxy)-1*H*-4,7-méthanopyrazolo[3,4-*e*][1,3]diazépine-8-carboxamide de formule brute C₂₁H₁₉N₅O₃ (M = 389,42 g).

Le rendement correspondant est de 72%.

### Stade H

On procède comme au Stade M de l'Exemple 1 avec 50 mg du composé obtenu au stade précédent, 5 ml de méthanol, 95 mg de palladium sur charbon à 10% et 4 ml de THF. On obtient 36,8 mg du composé trans-4,5,6,8-tétrahydro-5-hydroxy-6-oxo-1-phényl-1*H*-4,7-méthanopyrazolo[3,4-*e*][1,3]diazepine-8-carboxamide de formule brute C₁₄H₁₃N₅O₃ (M = 299,29 g).

Le rendement correspondant est de 95%.

### Stade I

On procède comme au Stade N de l'Exemple 1 avec 36,8 mg du produit obtenu au stade précédent, 5 ml de pyridine, 60 mg du complexe pyridine-SO₃. Le produit obtenu est traité comme indiqué au Stade R de l'Exemple 12 avec 25 g de résine Dowex. On obtient 24 mg du composé sel de sodium de trans-4,5,6,8-tétrahydro-6-oxo-1-phényl-5-(sulfooxy)-1*H*-4,7-méthanopyrazolo[3,4-*e*][1,3]diazépine-8-carboxamide de formule brute C₁₄H₁₂N₅O₆S, Na (M = 401,34 g).

Le rendement correspondant est de 48%.

LC/SM (électrospray négatif), m/z : M⁻ = 378. (2M + Na)⁻ = 779.

RMN du proton, D₂O, 300 MHz, déplacement chimique et multiplicité :
3,40 (d), 3,76 (dd) : N-CH₂-CH=C= ; 5,07 (d) : N-CH₂-CH=C= ; 5,62 (s) : N-CH-C= ; 7,50 (m), 7,55 (m), 7,44 (m) : les 5 H du noyau aromatique.

### ETUDE PHARMACOLOGIQUE DES PRODUITS DE L'INVENTION

### Activité in vitro, méthode des dilutions en milieu liquide

On prépare une série de tubes dans lesquels on répartit la même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination, ce qui permet de déterminer les concentrations minimales inhibitrices (C.M.I.) exprimées en µg/ml.

On effectue ainsi des tests avec les produits de l'invention suivants :
- le sel de triéthylammonium de 5,6-dihydro-6-oxo-N²-phényl-5(sulfooxy)-4*H*-4,7-méthanopyrazolo[3,4-e][1,3]diazépine-2,8(8*H*)-dicarboxamide (A),
- le sel de sodium de 4,5,6,8-tétrahydro-6-oxo-5-(sulfooxy)-1*H*-4,7-méthanopyrazolo[3,4-e][1,3]diazépine - 1-carboxamide (B),
- le sel de sodium de 1,4,5,8-tétrahydro-1-[(4-méthoxyphényl)méthyl]-5-(sulfooxy)-6*H*-4,7-méthanopyrazolo[3,4-e][1,3]diazépin-6-one (C),
- le sel de sodium de trans-4,5,6,8-tétrahydro-2-(2-méthylphényl)-6-oxo-5-(sulfooxy)-4,7-méthano-7*H-*thiéno[2,3-e][1,3]diazépine-8-carboxamide (D),
- le sel de sodium de trans-4,5,6,8-tétrahydro-6-oxo-5-(sulfooxy)-2-[2-(trifluorométhyl)phényl]-4,7-méthano-7*H-*thiéno[2,3-e][1,3]diazépine-8-carboxamide (E),
- le sel de sodium de trans-2-(2-éthylphényl)-4,5,6,8-tétrahydro-6-oxo-5-(sulfooxy)-4,7-méthano-7*H*-thiéno[2,3-e][1,3]diazépine-8-carboxamide (F),
- le sel de sodium de trans-8-(2,3-dihydroxypropoxy)-1,2,3,5-tétrahydro-3-oxo-2-(sulfooxy)-1,4-méthano-4*H*-2,4-benzodiazépine-5-carboxamide (G),
- le sel de sodium de trans-3-(4-fluorophényl)-4,6,7,8-tétrahydro-6-oxo-7-(sulfooxy)-5,8-méthano-5*H*-thiéno [2,3-e][1,3]diazépine-4-carboxylate d'éthyle (H),
- le sel de sodium de trans-2,5,6,8-tétrahydro-6-oxo-2-phényl-5-(sulfooxy)-4*H*-4,7-méthanopyrazolo[3,4-e][1,3]diazépine-8-carboxamide (I),
- le sel de sodium de 1,4,5,8-tétrahydro-l-phényl-5-(sulfooxy)-6*H*-4,7-méthanopyrazolo[3,4-e][1,3]diazépin-6-one (J),
- le sel de sodium de trans-4,5,6,8-tétrahydro-6-oxo-1-phényl-5(sulfooxy)-1*H*-4,7-méthanopyrazolo[3,4-e][1,3]diazépine-8-carboxamide (K).

Ces composés ont les activités regroupées dans le tableau suivant :

| **Gram-positif CMI** µ**g/ml à 24 heures** | | **Composés** |
|---|---|---|
| S. aureus SG511 | 0,3 - 40 | A à K |
| E. faecium M 78 L | 2,5 - 80 | A et C à K |
| S. pyogenes A561 | < 0,15 - 2,5 | A à K |

| **Gram-négatif GMI** µ**g/ml à 24 heures** | | |
|---|---|---|
| E. coli UC1894 | < 0,15 - 2,5 | B à G |
| E. coli 250HT7 | 0,3 - 80 | B,C,F,G |
| E. cloacae 1321E | < 0,15 - 10 | B et D à G |
| E. coli K 12 | 1,2 - 10 | A et H à K |
| E. coli DB 10 | < 0,15 - 2,5 | A et H à K |

Les composés selon l'invention montrent donc une activité anti-bactérienne.

### II/ ACTIVITE INHIBITRICE DE β-LACTAMASES

Les composés de formule (I) et leurs sels pharmaceutiquement acceptables présentent des activités inhibitrices marquées contre les β-lactamases de diverses souches bactériennes et ces propriétés thérapeutiquement intéressantes peuvent être déterminées in vitro sur des β-lactamases isolées :

### A. Préparation des β-lactamases Tem-1 et P99

Les β-lactamases sont isolées à partir de souches bactériennes résistantes aux pénicillines et aux céphalosporines (Tem1 et P99 sont respectivement produites par *E.coli* 250HT21 et *E.Cloacae* 293HT6).

Les bactéries sont cultivées dans du bouillon coeur-cervelle à 37 g/l(DIFCO), à 37°C. Elles sont récoltées en fin de phase exponentielle, refroidies et centrifugées. Les culots bactériens sont repris dans du tampon Phosphate de sodium 50 mM, pH 7.0 et à nouveau centrifugés. Les bactéries sont reprises dans deux volumes de ce même tampon et lysées au moyen d'une French-Press maintenue à 4°C. Après une centrifugation 1h à 100000G, à 4°C, les surnageants contenant la fraction soluble des extraits bactériens sont récupérés et congelés à -80°C.

### B. Détermination de l'activité β-lactamases

La méthode utilise comme substrat la Nitrocéfine (OXOID), céphalosporine chromogène, dont le produit d'hydrolyse par les Beta-lactamases est rouge et absorbe à 485 nm. L'activité β-lactamase est déterminée en cinétique par la mesure de la variation d'absorbance à 485 nm résultant de l'hydrolyse du substrat sur un spectrophotomètre de plaques (Spectra Max Plus de Molecular Devices). Les expériences se font à 37°C. La quantité d'enzyme a été normalisée et les mesures se font en vitesse initiale.

### C. Détermination de l'activité inhibitrice des β-lactamases

Deux mesures sont effectuées, sans préincubation et avec préincubation de l'enzyme et de l'inhibiteur (5 mn), afin de tester l'irréversibilité de la réaction. Les produits sont testés à 6 ou 8 concentrations en duplicate. Le mélange réactionnel contient 100 µM de Nitrocéfine et du tampon phosphate de sodium 50 mM pH 7.0.

### D. Calculs des CI50

Les Vitesses d'hydrolyse sont mesurées avec et sans inhibiteur. On détermine la concentration d'inhibiteur qui inhibe de 50% la réaction d'hydrolyse de la Nitrocéfine par l'enzyme (CI50). Le traitement des données est réalisé à l'aide du logiciel GraFit (Erathycus Software).

| **EXEMPLE n°** | **CI₅₀ nM/TEM1** | **CI₅₀ nM/P99** |
|---|---|---|
| 28 | 33 | 25 |
| 38 | 59 | 19 |
| 37 | 41 | 21 |
| 40 | 11 | 12 |
| 42 | 15 | 44 |
| 18 | 5 | 13 |
| 41 | 9 | 29 |
| 19 | 5 | 16 |
| 21 | 7 | 69 |
| 33 | 56 | 17 |
| 24 | 2 | 10 |
| 25 | 2 | 6 |
| 26 | 1 | 6 |
| 46 | 5 | 80 |
| 7 | 1 | 14 |
| 44 | 34 | 2 |
| 11 | 11 | 39 |
| 10 | 12 | 18 |
| 47 | 12 | 3 |
| 5 | 13 | 17 |
| 4 | 34 | 7 |
| 45 | 1 | 12 |
| 2 | 16 | 20 |
| 3 | 2 | 11 |
| 1 | 3 | 5 |
| 9 | 60 | 50 |

| | | |
|---|---|---|
| CI₅₀ après 5 mn de pré incubation avec l'enzyme. | | |

### Exemples de composition pharmaceutique :

1) On a préparé une composition pharmaceutique pour injection dont les ingrédients sont les suivants :
   - composé de l'exemple ..... 500 mg
   - excipient aqueux stérile ..... q.s.p. 10 ml
2) On a préparé une composition pharmaceutique (lyophilisat) pour injection renfermant :
   - d'une part : composé de l'exemple ..... 500 mg
   - d'autre part : Céfotaxime ..... 1 g
   - excipient aqueux stérile ..... q.s.p 5 ml

Les deux principes actifs peuvent, si désiré, être introduits séparément dans deux ampoules ou flacons distincts.

## Revendications

1. Composé de formule générale, ou l'un de ses sels avec une base ou un acide : 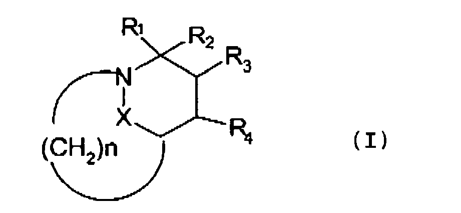 dans laquelle :
R1 représente un atome d'hydrogène, un radical COOH, COOR, CN, (CH₂)ₙ·R₅, CONR₆R₇ ou 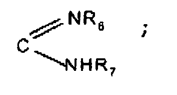
R est choisi dans le groupe constitué par un radical alkyle renfermant de 1 à 6 atomes de carbone, éventuellement substitué par un ou plusieurs atomes d'halogène ou par un radical pyridyle, un radical -CH₂-alkényle renfermant au total de 3 à 9 atomes de carbone, un groupe (poly)alkoxyalkyle renfermant 1 à 4 atomes d'oxygène et 3 à 10 atomes de carbone, un radical aryle renfermant de 6 à 10 atomes de carbone ou aralkyle renfermant de 7 à 11 atomes de carbone, le noyau du radical aryle ou aralkyle étant éventuellement substitué par un radical OH, NH₂, NO₂, alkyle renfermant de 1 à 6 atomes de carbone, alkoxy renfermant de 1 à 6 atomes de carbone ou par un ou plusieurs atomes d'halogène,
R₅ est choisi dans le groupe constitué par un radical COOH, CN, OH, NH₂, CO-NR₆R₇, COOR, OR, R étant défini comme ci-dessus,
R₆ et R₇ sont individuellement choisis dans le groupe constitué par un atome d'hydrogène, un radical alkyle renfermant de 1 à 6 atomes de carbone, alkoxy renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle,
n' est égal à 1 ou 2,
R₃ et R₄ forment ensemble un phényl ou un hétérocycle à caractère aromatique à 5 sommets renfermant 1 ou 2 héréroatomes choisis parmi l'azote, l'oxygène et le soufre, tel que défini ci-après:: 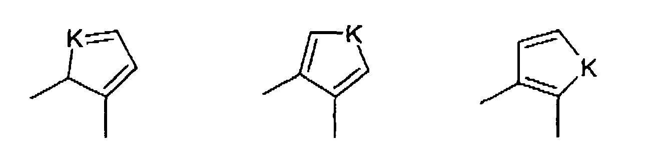 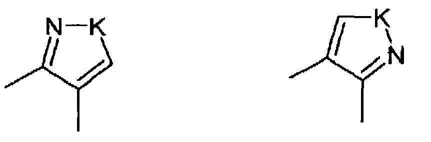 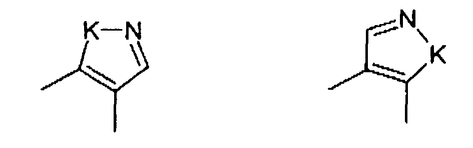 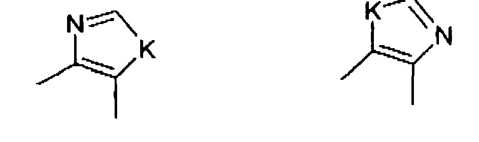 avec K = NH, S, O
substitué par un groupement R', R' étant choisi dans le groupe constitué par les radicaux
-(O)ₐ-(CH₂)_{b}-(O)ₐ-CONR₆R₇, -(O)ₐ-(CH₂)_{b}-OSO₃H, -(O)ₐ-(CH₂)_{b}-SO₃H,
-(O)ₐ-SO₂R, -(O)ₐ-SO₂-CHal₃ -(O)ₐ-(CH₂)_{b}-NR₆R₇,
(O)ₐ-(CH₂)_{b}-NH-COOR, -(CH₂)_{b}-COOH, -(CH₂)_{b}-COOR, -OR", OH,
-(CH₂)_{b}- phényle et -(CH₂)_{b}- hétérocycle à caractère aromatique à 5 ou 6 sommets renfermant de 1 à 4 hétéroatomes choisis parmi l'azote, l'oxygène et le soufre, le phényle et l'hétérocycle étant éventuellement substitués par un ou plusieurs halogènes, alkyle renfermant de 1 à 6 atomes de carbone, alkoxy renfermant de 1 à 6 atomes de carbone ou CF₃, R, R₆ et R₇ étant tels que définis précédemment, R" étant choisi dans le groupe constitué par les radicaux alkyles renfermant de 1 à 6 atomes de carbone substitués par un ou plusieurs radicaux hydroxy, oxo, halogène ou cyano, a étant égal à O ou 1 et b étant un entier de 0 à 6, étant entendu que lorsque R' est OH, R₁ représente le radical CONR₆R₇ dans lequel R₆ ou R₇ est un alkoxy renfermant de 1 à 6 atomes de carbone, et étant entendu que lorsque R' est -(O)ₐ-(CH₂)_{b}-NR₆R₇ avec R₆ et R₇ étant H, ou -(O)ₐ-(CH₂)_{b}-NH-COOR, a et b ne prennent pas en même temps la valeur 0, et lorsque R' est -(CH₂)_{b}-COOH ou - (CH₂)_{b}-COOR b ne vaut pas 0,
R₂ représente un atome d'hydrogène,
X représente un groupement divalent -C(O)-B- relié à l'atome d'azote par l'atome de carbone,
B représente un groupement divalent -NR₈-(CH₂)_{n"}- relié au carbonyle par l'atome d'azote, n" est égal à 0 ou 1 et R₈ est choisi dans le groupe constitué par un atome d'hydrogène, un radical OH, R, OR, Y, OY, Y₁ et OY₁, égal à 0, 1 ou 2,
Y est choisi dans le groupe constitué par les radicaux COH, COR, COOR, CONH₂, CONHR, CONHOH, CONHSO₂R, CH₂COOH, CH₂COOR, CHF-COOH, CHF-COOR, CF₂-COOH, CF₂-COOR, CN, CH₂CN, CH₂CONHOH, CH₂CONHCN, CH₂-tétrazole, CH₂SO₃H, CH₂SO₂R, CH₂PO(OR)₂, CH₂PO(OR)(OH), CH₂PO(R) (OH) et CH₂PO(OH)₂,
Y₁ est choisi dans le groupe constitué par les radicaux SO₂R, SO₂NHCOH, SO₂NHCOR, SO₂NHCOOR, SO₂NHCONHR, SO₂NHCONH₂ et SO₃H,
n est égal à 1.

2. Composé de formule générale selon la revendication 1, dans laquelle R' est choisi dans le groupe constitué par les radicaux -(O)ₐ-(CH₂)_{b}-(O)ₐ-CONR₆R₇, -(O)ₐ-(CH₂)_{b}-OSO₃H, -(O)ₐ-(CH₂)_{b}-SO₃H,
-(O)ₐ-SO₂R, -(O)ₐ-SO₂-CHal₃, -(O)ₐ-(CH₂)_{b}-NR₆R₇, (O)ₐ-(CH₂)_{b}-NH-COOR,
-(CH₂)_{b}-COOH, -(CH₂)_{b}-COOR, -OR", OH,
-(CH₂)_{b}- phényle et -(CH₂)_{b} - hétérocycle à caractère aromatique à 5 ou 6 sommets renfermant de 1 à 4 hétéroatomes choisis parmi l'azote, l'oxygène et le soufre, le phényle et l'hétérocycle étant éventuellement substitués par un ou plusieurs halogènes, alkyle renfermant de 1 à 6 atomes de carbone, alkoxy renfermant de 1 à 6 atomes de carbone ou CF₃, R, R₆ et R₇ étant tels que définis à la revendication 1, R" étant choisi dans le groupe constitué par les radicaux alkyles renfermant de 1 à 6 atomes de carbone substitués par un ou plusieurs radicaux hydroxy, oxo, halogène ou cyano, a étant égal à O ou 1 et b étant un entier de 0 à 6, étant entendu que
(i) lorsque R' est OH ou OR'' avec R'' étant un radical alkyle renfermant de 1 à 6 atomes de carbone substitués par un ou plusieurs radicaux oxo, alors R₁ représente le radical CONR₆R₇ dans lequel R₆ ou R₇ est un alkoxy renfermant de 1 à 6 atomes de carbone, ou bien
(j) lorsque R' est un radical (O)ₐ-(CH₂)_{b}-NR₆R₇, dans lequel R₆ et R₇ sont tels que définis à la revendication 1 alors a est égal à 0 ou 1 et b est un entier de 1 à 6 ou bien
(k) a est égal à 0 ou 1 et b est un entier de 0 à 6 lorsque R' est un radical -(O)ₐ-(CH₂)_{b}-NR₆R₇, dans lequel R₆ et R₇ sont individuellement choisis dans le groupe constitué par un radical alkyle renfermant de 1 à 6 atomes de carbone, alkoxy renfermant de 1 à 6 atomes de carbone, aryle renfermant de 6 à 10 atomes de carbone et aralkyle renfermant de 7 à 11 atomes de carbone et un radical alkyle renfermant de 1 à 6 atomes de carbone substitué par un radical pyridyle, ou bien encore
(l) lorsque R' est choisi parmi le groupe constitué par les radicaux -(O)ₐ-(CH₂)_{b}-NH-COOR, -(CH₂)_{b}-COOH, -(CH₂)_{b}-COOR alors b est un entier de 1 à 6 et a vaut 0 ou 1 ; Les autres valeurs étant définies comme à la revendication 1.

3. Composé selon la revendication 1, **caractérisé en ce que** R₃ et R₄ forment ensemble un thiényle ou un pyrazolyle substitué par un substituant R' défini dans la revendication 1.

4. Composé selon l'une des revendications 1 à 3, **caractérisé en ce que** R₁ est choisi dans le groupe constitué par l'atome d'hydrogène et les groupements COOCH₃, COOC₂H₅, CONH₂, CONHCH₃ et CONHOCH₃.

5. Composé selon l'une des revendications 1 à 4, **caractérisé en ce que** R₈ est un groupement OY dans lequel Y est choisi parmi les groupements CH₂COOH, CH₂COOR, CHF-COOH, CHF-COOR, CF₂-COOH, CF₂-COOR, CN, CH₂CN, CH₂CONHOH, CH₂CONHCN, CH₂-tétrazole, CH₂SO₃H, CH₂SO₂R, CH₂PO(OR)₂, CH₂PO(OR)(OH), CH₂PO(R)(OH) et CH₂PO(OH)₂ ou OY₁ dans lequel Y₁ est choisi parmi les groupements SO₂R, SO₂NHCOR, SO₂NHCOOR, SO₂NHCONHR et SO₃H, R étant tel que défini à la revendication 1.

6. Composé selon l'une quelconque des revendications 1 à 5, **caractérisé en ce que** R' est choisi dans le groupe constitué par -O-CH₂-CHOH-CH₂OH, -CH₂-CH₂-NH₂, -CH₂-COOC₂H₅, - CH₂-CH₂-Phényl, -CH₂-Phényl, -O-CO-NHPhényl, -O-CO-NHC₂H₅, -O-SO₂-CF₃, -O-(CH₂)₂-O-SO₃H, -CH₂-COOH, -CO-NH₂, -CO-NH Phenyl, -CH₂-(p-OCH₃ Phényl) et Phényl éventuellement substitué par CH₃, C₂H₅, F et CF₃.

7. Composés de formule (I), telle que définie à la revendication 1, dont les noms suivent :
- le sel de triéthylammonium de 5,6-dihydro-6-oxo-N²-phényl-5(sulfooxy)-4*H*-4,7-méthanopyrazolo[3,4-e][1,3]diazépine-2,8(8*H*)-dicarboxamide,
- le sel de sodium de 4,5,6,8-tétrahydro-6-oxo-5-(sulfooxy)-1*H*-4,7-méthanopyrazolo[3,4-e][1,3]diazépine -1-carboxamide,
- le sel de sodium de 1,4,5,8-tétrahydro-1-[(4-méthoxyphényl)méthyl]-5-(sulfooxy)-6*H*-4,7-méthanopyrazolo[3,4-e][1,3]diazépin-6-one,
- le sel de sodium de trans-4,5,6,8-tétrahydro-2-(2-méthylphényl)-6-oxo-5-(sulfooxy)-4,7-méthano-7*H-*thiéno[2,3-e][1,3]diazépine-8-carboxamide,
- le sel de sodium de trans-4,5,6,8-tétrahydro-6-oxo-5-(sulfooxy)-2-[2-(trifluorométhyl)phényl]-4,7-méthano-7*H*-thiéno[2,3-e][1,3]diazépine-8-carboxamide,
- le sel de sodium de trans-2-(2-éthylphényl)-4,5,6,8-tétrahydro-6-oxo-5-(sulfooxy)-4,7-méthano-7*H-*thiéno[2,3-e][1,3]diazépine-8-carboxamide,
- le sel de sodium de trans-8-(2,3-dihydroxypropoxy)-1,2,3,5-tétrahydro-3-oxo-2-(sulfooxy)-1,4-méthano-4*H-*2,4-benzodiazépine-5-carboxamide,
- le sel de sodium de trans-3-(4-fluorophényl)-4,6,7,8-tétrahydro-6-oxo-7-(sulfooxy)-5,8-méthano-5*H-*thiéno[2,3-e][1,3]diazépine-4-carboxylate d'éthyle,
- le sel de sodium de trans-2,5,6,8-tétrahydro-6-oxo-2-phényl-5-(sulfooxy)-4*H*-4,7-méthanopyrazolo[3,4-e][1,3]diazépine-8-carboxamide,
- le sel de sodium de 1,4,5,8-tétrahydro-1-phényl-5-(sulfooxy)-6*H*-4,7-méthanopyrazolo[3,4-e][1,3]diazépin-6-one,
- le sel de sodium de trans-4,5,6,8-tétrahydro-6-oxo-1-phényl-5(sulfooxy)-1*H*-4,7-méthanopyrazolo[3,4-e][1,3]diazépine-8-carboxamide,
- le sel de triéthylammonium de trans-2,5,6,8-tétrahydro-6-oxo-2-(phénylméthyl)-5-(sulfooxy)-4H-4,7-méthanopyrazolo[3,4-e][1,3]diazépine-8-carboxylate de méthyle,
- le sel de triéthylammonium de trans-4,5,6,8-tétrahydro-6-oxo-1-(2-phényléthyl)-5-(sulfooxy)-1H-4,7-méthanopyrazolo[3,4-e][1,3]diazépine-8-carboxylate de méthyle,
- le sel de triéthylammonium de trans-4,5,6,8-tétrahydro-8-(méthoxycarbonyl)-6-oxo-5-(sulfooxy)-1H-4,7-méthanopyrazolo[3,4-e][1,3]diazépine-1-acétate d'éthyle,
- le sel de triéthylammonium de trans-5,6-dihydro-8-(méthoxycarbonyl)-6-oxo-5-(sulfooxy)-4H-4,7-méthanopyrazolo[3,4-e][1,3]diazépine-2(8H)-acétate d'éthyle,
- le sel de di-(triéthylammonium) de l'acide trans-5,6-dihydro-8-(méthoxycarbonyl)-6-oxo-5-sulfooxy-4H-4,7-méthanopyrazolo[3,4-e][1,3]diazépine-2(8H)-acétique,
- le sel de pyridinium de trans-1-(aminocarbonyl)-4,5,6,8-tétrahydro-6-oxo-5-(sulfooxy)-1H-4,7-méthanopyrazolo[3,4-e][1,3]diazépine-8-carboxylate de méthyle,
- le sel de pyridinium de trans-2-(aminocarbonyl)-2,5,6,8-tétrahydro-6-oxo-5-(sulfooxy)-4H-4,7-méthanopyrazolo[3,4-e][diazépine-8-carboxylate de méthyle,
- le sel de sodium de trans-2-(4-fluorophényl)-4,5,6,8-tétrahydro-6-oxo-5-(sulfooxy)-4,7-méthano-7H-thiéno[2,3-e][1,3]diazépine-8-carboxylate de méthyle,
- le sel de sodium de trans-2(aminocarbonyl)-4,5,6,8-tétrahydro-6-oxo-5-(sulfooxy)-4,7-méthano-7H-thiéno[2,3-e][1,3]diazépine-8-carboxylate de méthyle,
- le sel de sodium de trans-1,2,3,5-tétrahydro-3-oxo-9-[[(phénylamino)carbonyl]oxy]-2-(sulfooxy)-1,4-méthano-4H-2,4-benzodiazépine-5-carboxylate d'éthyle,
- le sel de sodium de trans-1,2,3,5-tétrahydro-N-méthoxy-8-[(2 méthoxyéthoxy)méthoxy]-3-oxo-2-(sulfoxy)-1,4-méthano-4H-2,4-benzodiazépine-5-carboxamide,
- le sel de sodium de trans-1,2,3,5-tétrahydro-3-oxo-8-[[(phénylamino)carbonyl]oxy]-2-(sulfooxy)-1,4-méthano-4H-2,4-benzodiazépine-5-carboxylate d'éthyle,
- le sel de sodium de trans-8-[[(éthylamino)carbonyl]oxy]-1,2,3,5-tétrahydro-3-oxo-2-(sulfooxy)-1,4-méthano-4H-2,4-benzodiazépine-5-carboxylate d'éthyle,
- le sel de sodium de trans-1,2,3,5-tétrahydro-3-oxo-2-(sulfooxy)-8-[[trifluorométhyl)sulfonyl]oxy]-1,4-méthano-4H-2,4-benzodiazépine-5-carboxylate d'éthyle,
- le sel disodique de trans-1,2,3,5-tétrahydro-3-oxo-2-(sulfooxy)-8-[2-(sulfooxy)éthoxy]-1,4-méthano-4H-2,4-benzodiazépine-5-carboxamide,
- le sel de sodium de trans-8-[(2,2-diméthyl-1,3-dioxolan-4-yl)méthoxy]-1,2,3,5-tétrahydro-3-oxo-2-(sulfooxy)-1,4-méthano-4H-2,4-benzodiazépine-5-carboxamide.
- Sel de triéthylammonium de trans-2,5,6,8-tétrahydro-6-oxo-2-(2-phényléthyl)-5-(sulfooxy)-4*H*-4,7-méthanopyrazolo[3,4-e][1,3]diazépine-8-carboxylate de méthyle.

8. Procédé de préparation d'un composé selon l'une des revendications 1 à 7, **caractérisé en ce qu'**il comporte :
a) une étape au cours de laquelle on fait réagir, avec un agent de carbonylation choisi dans le groupe constitué par le phosgène, le diphosgène, le triphosgène, les chloro-formiates d'aryle, d'aralkyle, d'alkyle et d'alkényle, les dicarbonates d'alkyle, le carbonyl-diimidazole et leurs mélanges,, le cas échéant en présence d'une base, un composé de formule (II) : 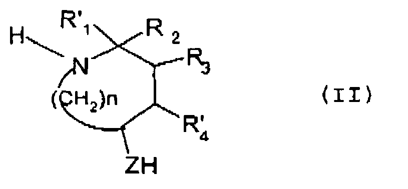 dans laquelle :
R'₁ représente un atome d'hydrogène, un radical CN, COOH protégé, COOR₉, (CH₂)_{n'}R'₅, CONR₆R₇, 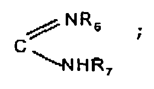
R₉ est choisi dans le groupe constitué par un radical alkyle renfermant de 1 à 6 atomes de carbone, éventuellement substitué par un ou plusieurs atomes d'halogène ou par un radical pyridyle, un radical -CH₂-alkényle renfermant au total de 3 à 9 atomes de carbone, aryle renfermant de 6 à 10 atomes de carbone ou aralkyle renfermant de 7 à 11 atomes de carbone, le noyau du radical aryle ou aralkyle étant éventuellement substitué par un radical NO₂, OH protégé, NH₂ protégé, alkyle renfermant de 1 à 6 atomes de carbone, alkoxy renfermant de 1 à 6 atomes de carbone ou par un ou plusieurs atomes d'halogène,
R'₅ est choisi dans le groupe constitué par un radical OH protégé, CN, NH₂ protégé, CO-NR₆R₇, COOH protégé, COOR₉, OR₉, R₉ étant défini comme ci-dessus,
n', R₆ et R₇ sont tels que définis à la revendication 1,
R₃ et R'₄ forment ensemble un phényl ou un hétérocycle à caractère aromatique à 5 sommets tel que défini à la revendication 1, substitué par un groupement R₁₀, R₁₀ étant choisi dans le groupe constitué par un atome d'hydrogène et les radicaux alkyle renfermant de 1 à 6 atomes de carbone substitué par un ou plusieurs radicaux hydroxy, oxo, halogène ou cyano, ou par un radical alkényle renfermant de 2 à 6 atomes de carbone, halogèno, OH protégé, -OR, OR", R" étant tel que défini précédemment, (CH₂)_{b}-phényle ou -(CH₂)_{b}-hétérocycle, éventuellement substitué, tel que défini dans les revendications 1 à 6,
R₂ représente un atome d'hydrogène,
ZH représente un groupement HNR'₈-(CH₂)_{n"}-, n" est tel que défini à la revendication 1, R'₆ représente un atome d'hydrogène, un radical R₉, OH protégé, OR₉, Y', OY', Y'₁, OY'₁, R₉ étant défini comme précédemment, et m étant égal à 0, 1 ou 2,
Y' est choisi dans le groupe constitué par les radicaux COH, COR₉, COOR₉, CONH₂, CONHR₉, CONHSO₂R₉, CH₂COOR₉, CH₂₋tétrazole protégé, CH₂SO₂R₉, CH₂PO(OR₉)₂, CONHOH protégé, CH₂COOH protégé, CH₂CONHOH protégé, CH₂SO₃ protégé, CH₂PO(OR) (OH) protégé, CH₂PO(R) (OH) protégé et CH₂PO(OH)₂ protégé,
Y'₁ est choisi dans le groupe constitué par les radicaux SO₂R₉, SO₂NHCOH, SO₂NHCOR₉, SO₂NHCOOR₉, SO₂NHCONH₂, SO₂NHCONHR₉ et SO₃H protégé,
et n est égal à 1 ;
en vue d'obtenir un composé intermédiaire de formule (III):
dans laquelle 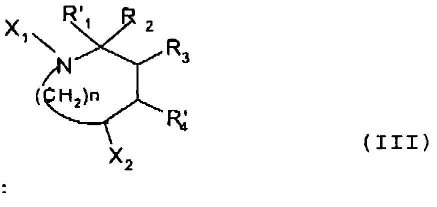
R'₁, R₂, R₃, R'₄ et n ont les mêmes significations que ci-dessus et soit X₁ est un atome d'hydrogène et X₂ représente un groupement -Z-CO-X₃, X₃ représentant le reste de l'agent de carbonylation, à savoir un reste Cl, OCCl₃, OAr, OCH₂Ar, OAlk, OAlkenyl, OCOOAlk et imidazolyl, soit X₂ est un groupement -ZH et X₁ représente un groupement CO-X₃, X₃ étant défini comme ci-dessus ;
b) une étape au cours de laquelle on cyclise l'intermédiaire obtenu précédemment, en présence d'une base ;
et **en ce que** :
c) le cas échéant, l'étape a) est précédée et/ou l'étape b) est suivie de l'une ou de plusieurs des réactions suivantes, dans un ordre approprié :
- protection des fonctions réactives,
- déprotection des fonctions réactives,
- estérification,
- saponification,
- sulfatation,
- phosphatation,
- amidification,
- acylation,
- sulfonylation ;
- alkylation ;
- formation d'un groupe urée ;
- réduction d'acides carboxyliques ;
- réduction de cétones et d'aldéhydes en alcools;
- salification ;
- échange d'ions ;
- dédoublement ou séparation de diastéréoisomères;
- oxydation de sulfure en sulfoxyde et/ou sulfone;
- oxydation d'aldéhyde en acide ;
- oxydation d'alcool en cétone ;
- halogénation ou déshalogénation ;
- carbamoylation ;
- carboxylation ;
- introduction d'un groupe azido ;
- réduction d'un azido en amine ;
- réactions de couplage d'halogénures ou de triflates aromatiques ou hétéroaromatiques ou d'azotes hétérocycliques avec des acides aryl ou hétéroaryl boroniques ;
- réactions de couplage d'halogénures ou de triflates aromatiques ou hétéroaromatiques avec des réactifs stannylés ;
- hydrogénation de doubles liaisons ;
- dihydroxylation de doubles liaisons ;
- cyanuration.

9. Procédé selon la revendication 8 **caractérisé en ce que** la réaction de carbonylation a lieu en présence d'une base.

10. Procédé selon l'une quelconque des revendications 8 et 9, **caractérisé en ce que** dans l'étape b) la base est choisie dans le groupe constitué par les amines, les hydrures, alcoolates, amidures ou carbonates de métaux alcalins ou alcalino-terreux.

11. Procédé selon la revendication 10, **caractérisé en ce que** la base est une amine.

12. Procédé selon l'une quelconque des revendications 8 à 11, **caractérisé en ce que** le composé de formule (II) dans laquelle ZH représente un groupement HNR'₈-(CH₂)_{n"}- dans lequel n" est égal à O, R'₈ étant défini comme à la revendication 8, est obtenu par un procédé dans lequel on traite un composé de formule (IV) : 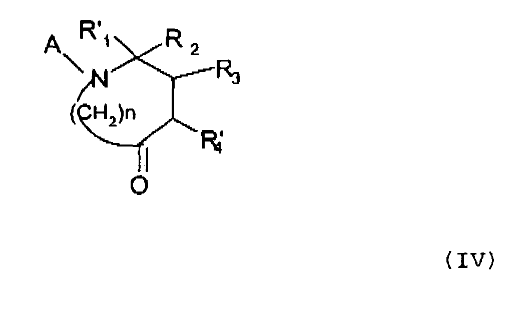 dans laquelle R'₁, R₂ et n sont définis comme à la revendication 8, R₃ et R'₄ ont les valeurs définies à la revendication 8 ou des valeurs précurseurs de celles définies précédemment et A représente un atome d'hydrogène ou un groupement protecteur de l'azote,
par un agent de réduction, pour obtenir un composé de formule (V) : 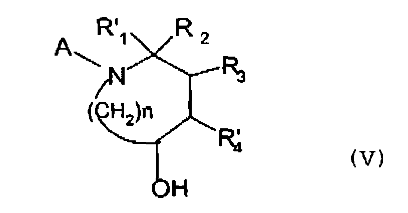 dans laquelle A représente un atome d'hydrogène ou un groupement protecteur de l'azote, R'₁, R₂, R₃, R'₄ et n conservent leur signification citée dans la revendication 8, dans lequel le cas échéant, l'on remplace le groupement OH par un groupe partant,
pour obtenir un composé de formule (VI) : 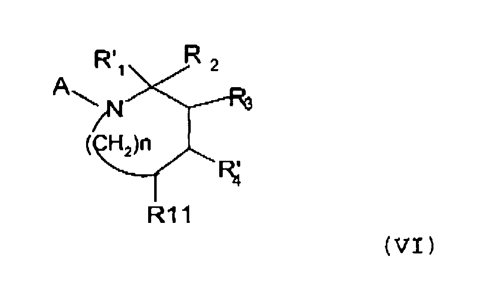 dans laquelle A représente un atome d'hydrogène ou un groupement protecteur de l'azote, R'₁, R₂, R₃, R'₄ et n conservent leur signification citée dans la revendication 8 et R₁₁ représente un groupe partant, que l'on traite par un composé de formule Z₁H₂ dans laquelle Z₁ représente un groupement divalent -NR'₈, R'₅ conservant la signification citée dans la revendication 8, pour obtenir un composé de formule (VIII) ou (VIII') : 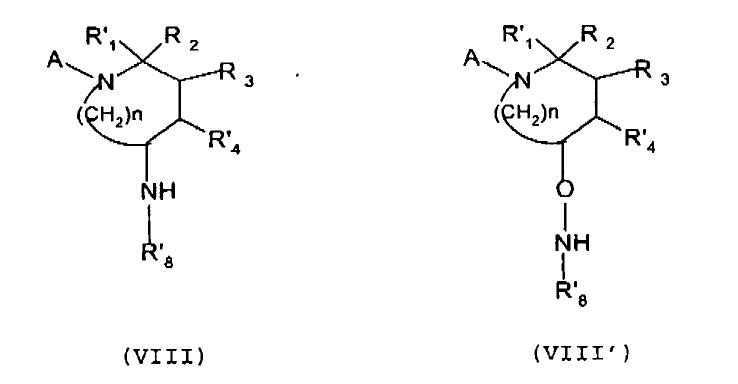 dans laquelle A représente un atome d'hydrogène ou un groupement protecteur de l'azote, R'₁, R₂, R₃, R'₄, n et R'₈ sont définis comme citée dans la revendication 8, puis, le cas échéant, par un agent de déprotection de l'atome d'azote approprié, et **en ce que**, le cas échéant, l'on soumet l'intermédiaire de formule (IV), (V) (VIII) ou VIII'), à une ou plusieurs des réactions décrites à l'étape c) du procédé de la revendication 8, dans un ordre approprié.

13. Procédé selon l'une quelconque des revendications 8 à 11, selon lequel le composé de formule (II) dans laquelle ZH représente un groupement HNR'₈-(CH₂)_{n"}- dans lequel n" est égal à O est obtenu par un procédé **caractérisé en ce que** l'on traite un composé de formule (IV) tel que définie précédemment, par un composé de formule H₂NR'₈, pour obtenir un composé de formule (VII): 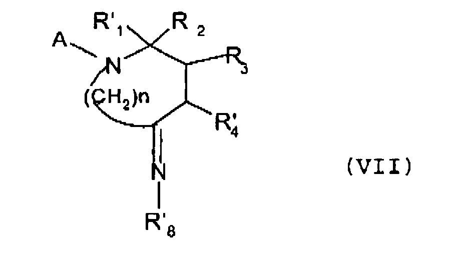 dans laquelle A représente un atome d'hydrogène ou un groupement protecteur de l'azote, R'₁, R₂, R₃, R'₄, n et R'₈ sont définis comme à la revendication 8, que l'on fait réagir avec un agent de réduction pour obtenir un composé de formule (VIII) 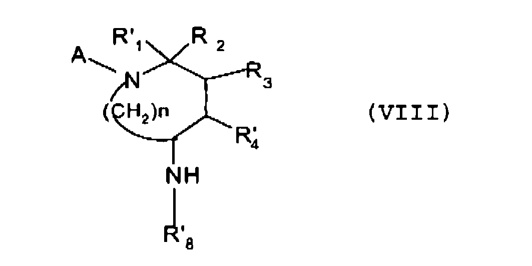 dans laquelle A représente un atome d'hydrogène ou un groupement protecteur de l'azote, R'₁, R₂, R₃, R'₄, n et R'₈ sont définis comme à la revendication 8, que l'on traite, le cas échéant, par un agent de déprotection de l'atome d'azote approprié, et **en ce que**, le cas échéant, l'on soumet l'intermédiaire de formule (VII) ou (VIII) à une ou plusieurs des réactions décrites à l'étape c) du procédé de la revendication 8, dans un ordre approprié.

14. A titre de médicaments, les produits tels que définis à l'une quelconque des revendications 1 à 6 ainsi que leurs sels avec les acides et les bases pharmaceutiquement acceptables.

15. A titre de médicaments, les produits tels que définis à la revendication 7.

16. Compositions pharmaceutiques contenant, à titre de principe actif, au moins un médicament selon l'une des revendications 14 ou 15.

17. Compositions pharmaceutiques contenant, à titre de principe actif, au moins un médicament inhibiteur de β-lactamase tel que défini à la revendication 14 ou 15 et au moins un médicament de type β-lactamines.

18. Composé de formule générale (III) ou l'un de ses sels avec un acide, notamment son chlorhydrate et son trifluoroacétate : 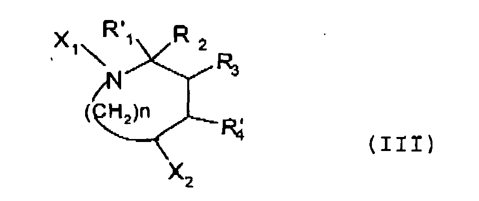 dans laquelle R₃ et R'₄ forment ensemble un phényl ou un hétérocycle à caractère aromatique, substitué par un radical -(CH₂)_{b}-phényle ou -(CH₂)_{b}-hétérocycle à caractère aromatique, éventuellement substitué, tel que défini à la revendication 1, les autres valeurs étant définies comme à la revendication 8, les groupements protecteurs apparaissant dans les définitions ci-dessus étant choisis comme suit:
- pour une fonction acide, les restes d'esters d'alkyle, d'allyle, de benzyle, de benzhydryle et de p-nitrobenzyle,
- pour OH, les restes d'éthers d'alkyle, d'alkoxyalkyle, d'aryle et d'aralkyle, les restes d'éthers silylés choisis parmi terbutyldiméthyl, triméthyl, triphényl et diphénylterbutyl-silyle, les restes d'esters clivables choisis parmi l'acétate, le propionate, le benzoate et le p-nitrobenzoate, les restes de carbonates de méthyle, tertbutyle, allyle, benzyle et p-nitrobenzyle,
- pour NH₂, les dérivés benzylés et tritylés, les carbamates d'allyle, de benzyle, phényle et terbutyle, les dérivés silylés choisis parmi tertbutyle diméthyl, triméthyl, triphényl et diphényl terbutyl-silyle, et les dérivés phénylsulfonylalkyle et cyanoalkyle,
- pour les hydroxylamines, les éthers de benzyle et d'allyle.

19. Composé de formule générale (III) ou l'un de ses sels avec acide, notamment son chlorhydrate et son trifluoroacétate 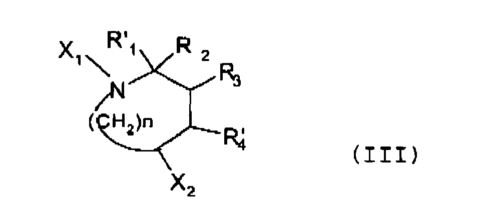 dans laquelle R'₁ représente un radical CONR₆R₇ dans lequel R₆ ou R₇ représente un radical alcoxy renfermant de 1 à 6 atomes de carbone, les autres valeurs étant définies comme à la revendication 8 et à la revendication 18.

20. Composés de formules (II) ou l'un de leurs sels avec un acide, notamment son chlorhydrate et son trifluoroacétate : 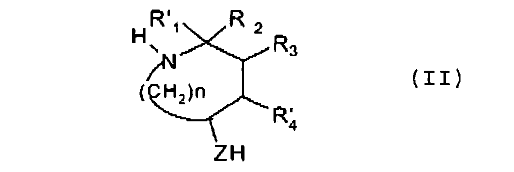 dans laquelle R₃ et R'₄ forment ensemble un phényl ou un hétérocycle à caractère aromatique, substitué par un radical - (CH₂)_{b}-phényle ou -(CH₂)_{b}-hétérocycle à caractère aromatique, éventuellement substitué, tel que défini à la revendication 1, les autres valeurs étant définies comme à la revendication 8 et à la revendication 18.

21. Composés de formules (II) ou l'un de leurs sels avec un acide, notamment son chlorhydrate et son trifluoroacétate : 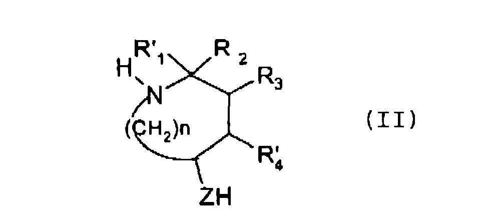 dans laquelle R'₁ représente un radical CONR₆R₇ dans lequel R₆ ou R₇ représente un radical alkoxy renfermant de 1 à 6 atomes de carbone, les autres valeurs étant définies comme à la revendication 8 ou à la revendication 18.

22. Composés de formules (VI) ou l'un de leurs sels avec un acide, notamment leurs chlorhydrates et leurs trifluoroacétates : 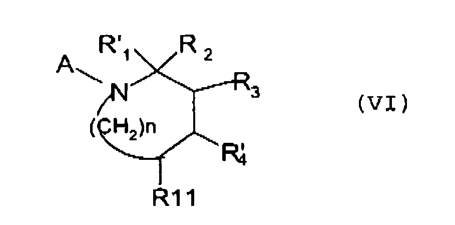 dans laquelle A représente un atome d'hydrogène ou un groupement protecteur de l'azote choisi parmi les dérivés benzylés ou tritylés, les carbamates d'allyle, de benzyle, phényle ou tertbutyle, ou encore les dérivés silylés tels que les dérivés tertbutyle diméthyl, triméthyl, triphényl ou encore diphényl tertbutyl-silyle, ou les dérivés phénylsulfonylalkyle ou cyanoalkyle, R₁₁ représente un groupe partant choisi parmi le groupe des phosphate, sulfonate, ou halogène et R₃ et R'₄ forment ensemble un phényl ou un hétérocycle à caractère aromatique, substitué par un radical - (CH₂)_{b}-phényle ou -(CH₂)_{b}-hétérocycle à caractère aromatique, éventuellement substitué, tel que défini à la revendication 1, les autres valeurs étant définies comme à la revendication 8 ou à la revendication 18.

23. Composés de formules (VI) 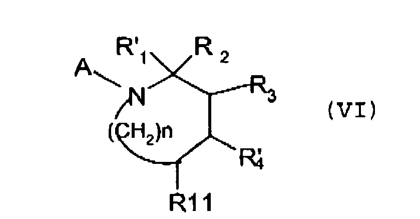 ou l'un de leurs sels avec un acide, notamment leurs chlorhydrates et leurs trifluoroacétates dans laquelle A représente un atome d'hydrogène ou un groupement protecteur de l'azote tel que défini dans la revendication 22, R'₁ représente un radical CONR₆R₇ dans lequel R₆ ou R₇ représente un radical alkoxy renfermant de 1 à 6 atomes de carbone, R₁₁ est défini comme à la revendication 22, et les autres valeurs sont définies comme à la revendication 8 ou à la revendication 18.

24. Composés de formules (VII), (VIII) et (VIII') ou l'un de leurs sels avec un acide, notamment leurs chlorhydrates et leurs trifluoroacétates : 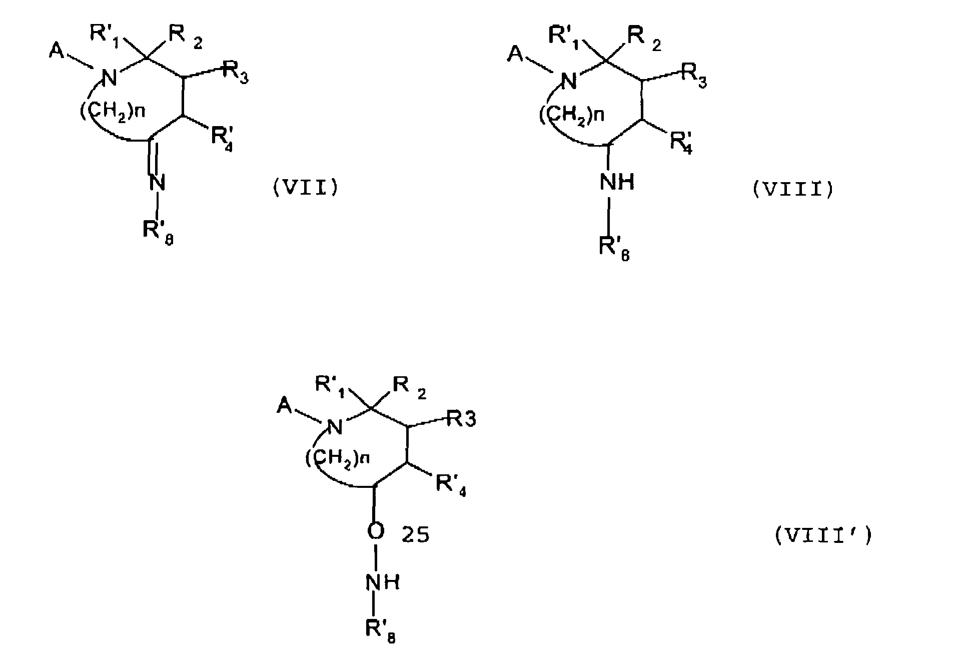 dans lesquelles A représente un atome d'hydrogène ou un groupement protecteur de l'azote tel que défini dans la revendication 22 et les autres valeurs sont définies comme à la revendication 18.

25. Composés de formules (VII) et (VIII) 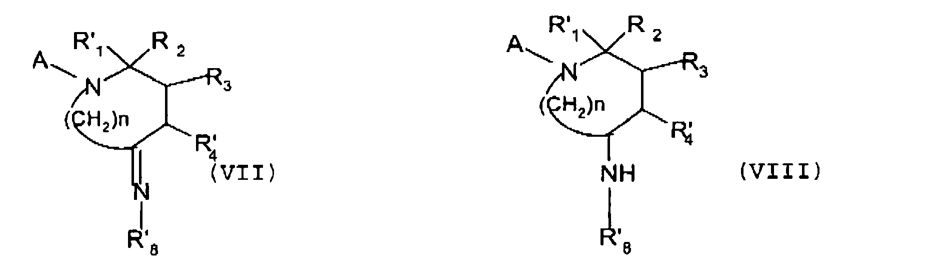 ou l'un de leurs sels avec un acide, notamment leurs chlorhydrates et leurs trifluoroacétates, dans lesquelles A représente un atome d'hydrogène ou un groupement protecteur de l'azote tel que défini dans la revendication 22, R'₁ représente un radical CONR₆R₇ dans lequel R₆ ou R₇ représente un radical alkoxy renfermant de 1 à 6 atomes de carbone et les autres valeurs sont définies comme à la revendication 8 ou à la revendication 18.

## Claims

1. Compound of general formula, or one of its salts with a base or an acid: 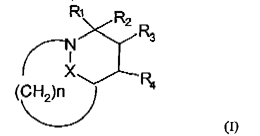 in which:
R₁ represents a hydrogen atom, a radical COOH, COOR, CN, (CH₂)ₙR₅, CONR₆R₇ or the radical 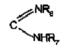
- R is chosen from the group consisting of: an alkyl radical containing from I to 6 carbon atoms, optionally substituted with one or more halogen atoms or with a pyridyl radical, a -CH₂-alkenyl radical containing in total from 3 to 9 carbon atoms, a (poly)-alkoxyalkyl group containing 1 to 4 oxygen atoms and 3 to 10 carbon atoms, an aryl radical containing from 6 to 10 carbon atoms or an aralkyl radical containing from 7 to 11 carbon atoms, the nucleus of the aryl or aralkyl radical being optionally substituted with an OH, NH₂, NO₂, an alkyl radical containing from 1 to 6 carbon atoms, an alkoxy radical containing from 1 to 6 carbon atoms or with one or more halogen atoms,
- R₅ is chosen from the group consisting of the COOH, CN, OH, NH₂, CO, NR₆R₇, COOR and OR radicals, R being as defined above,
- R₆ and R₇ are individually chosen from the group consisting of the hydrogen atom, an alkyl radical containing from I to 6 carbon atoms, an alkoxy radical containing from 1 to 6 carbon atoms, an aryl radical containing from 6 to 10 carbon atoms and an aralkyl radical containing from 7 to 11 carbon atoms and an alkyl radical containing from 1 to 6 carbon atoms which is substituted with a pyridyl radical,
- n' is equal to 1 or 2,
- R₃ and R₄ form together a phenyl or a heterocycle with an aromatic character containing 5 or 6 members among which 1 to 4 heteroatoms chosen from nitrogen, oxygen and sulphur, such as defined here under 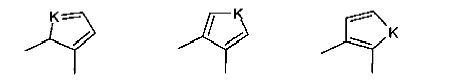 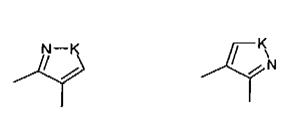 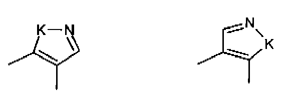 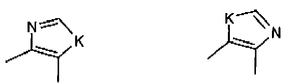
where K = NH, S, O,
which is substituted with one or more R' groups, R' being chosen from the group consisting of -(O)ₐ-(CH₂)_{b}-(O)ₐ-CONR₆R₇, -(O)ₐ-(CH₂)_{b}-OSO₃H, (O)ₐ-(CH₂)_{b}-SO₃H, -(O)ₐ-SO₂R, -(O)ₐ-SO₂-CHal₃, -(O)ₐ-(CH₂)_{b}-NR₆R₇, -(O)ₐ-(CH₂)_{b}-NH-COOR, -(CH₂)_{b}-COOH, -(CH₂)_{b}-COOR, -OR", -OH, -(CH₂)_{b}- phenyl radicals and -(CH₂)_{b}- heterocycle presenting an aromatic character containing 5 or 6 members among which I to 4 heteroatoms chosen from nitrogen, oxygen and sulphur, the phenyl and the heterocycle being optionally substituted with one or more halogens, alkyl containing from 1 to 6 carbon atoms, alkoxy containing from I to 6 carbon atoms or CF₃,
R, R₆ and R₇ being as defined above,
R" being chosen from the group consisting of alkyl radicals containing from 1 to 6 carbon atoms substituted with one or more hydroxy, oxo, halogen or cyano radicals,
a being equal to 0 or I and
b being an integer from 0 to 6,
- it being understood that, when R' is OH, R₁ represents the CONR₆R₇ radical in which R₆ or R₇ is an alkoxy containing from 1 to 6 carbon atoms,
- it being understood that when R' is -(O)ₐ-(CH₂)_{b}-NR₆R₇, with R₆ and R₇ being H, or -(O)ₐ-(CH₂)_{b}-NH-COOR, a and b do not take at the same time the value 0, and when R' is -(CH₂)_{b}-COOH or -(CH₂)_{b}-COOR, b is not equal to 0.
R₂ is a hydrogen atom,
X represents a divalent -C(O)-B- group linked to the nitrogen atom by the carbon atom,
B represents a divalent -NR₈-(CH₂)ₙ" group linked to the carbonyl by the nitrogen atom,
n" is equal to 0 or I and
R₈ is chosen from the group consisting of a hydrogen atom, an OH, R, OR, Y, OY, Y₁, OY radical, n" being equal to 0, 1 or 2,
- Y is chosen from the group consisting of the COH, COR, COOR, CONH₂, CONHR, CONHOH, CONHSO₂R, CH₂COOH, CH₂COOR, CHF-COOH, CHF-COOR, CF₂-COOH, CF₂-COOR, CN, CH₂CN, CH₂CONHOH, CH₂CONHCN, CH₂tetrazole, CH₂SO₃H, CH₂SO₂R, CH₂PO(OR)₂, CH₂PO(OR)(OH), CH₂PO(R)(OH) and CH₂PO(OH)₂ radicals,
- Y₁ is chosen from the group consisting of the SO₂R, SO₂NHCOH, SO₂NHCOR, SO₂NHCOOR, SO₂NHCONHR, SO₂NHCONH₂ and SO₃H radicals,
- n is equal to 1.

2. Compound of general formula according to claim 1, in which
R' is chosen from the group consisting of the -(O)ₐ-(CH₂)_{b}-(O)ₐ-CONR₆R₇, -(O)ₐ-(CH₂)_{b}-OSO₃H, -(O)ₐ-(CH₂)_{b}-SO₃H, -(O)ₐ-SO₂R, -(O)ₐ-SO₂-CHal₃, -(O)ₐ-(CH₂)_{b}-NR₆R₇, -(O)ₐ-(CH₂)_{b}-NHCOOR, -(CH₂)_{b}-COOH, - (CH₂)_{b}-COOR, -OR", OH, -(CH₂)_{b}-phenyl and -(CH₂)_{b}-heterocycle radicals with an aromatic character containing 5 or 6 members among which 1 to 4 heteroatoms chosen from nitrogen, oxygen and sulphur, the phenyl and the heterocycle being optionally substituted with one or more halogens, alkyl containing from I to 6 carbon atoms, alkoxy containing from I to 6 carbon atoms or CF₃,
- R, R₆ and R₇ being as defined in claim 1,
- R" being chosen from the group consisting of the alkyl radicals containing from 1 to 6 carbon atoms substituted with one or more hydroxy, oxo, halogen or cyano radicals,
- a being equal to 0 or 1 and
b being an integer from 0 to 6,
(i) it being understood that, when R' is OH or OR" with R" being an alkyl radical containing from 1 to 6 carbon atoms substituted with several oxo radicals, then R₁ represents the CONR₆R₇ radical in which R₆ or R₇ is an alkoxy containing from 1 to 6 carbon atoms, or
(j) R' is an -(O)ₐ-(CH₂)_{b}-NR₆R₇ radical, in which R₆ and R₇ are as defined in claim 1 and then a is equal to 0 or 1 and b is an integer from 0 to 6, or
(k) a is equal to 0 or 1 and b is an integer from 0 to 6 when R' is a -(O)ₐ-(CH₂)_{b}-NR₆R₇ radical, where R₆ and R₇ are individually chosen from the group consisting of an alkyl radical containing from 1 to 6 carbon atoms, an alkoxy radical containing from 1 to 6 carbon atoms, an aryl radical containing from 6 to 10 carbon atoms and an aralkyl radical containing from 7 to 11 carbon atoms and an alkyl radical containing from I to 6 carbon atoms substituted with a pyridyl radical, or further
(l) when R' is chosen from the group consisting of the -(O)ₐ-(CH₂)_{b}-NH-COOR, -(CH₂)_{b}-COOH,-(CH₂)_{b}-COOR radicals then b is an integer from 1 to 6, and a is equal to 0,
all of the other values being as defined in claim 1.

3. Compound according to claim 1, **characterized in that** R₃ and R₄ form together a thienyl or a pyrazolyl substituted with a substituent R' such as defined in claim 1.

4. Compound according to one of claims 1 to 3, **characterized in that** R₁ is chosen from the group consisting of the hydrogen atom and the COOCH₃, COOC₂H₅, CONH₂, CONHCH₃ and CONHOCH₃ groups.

5. Compound according to one of claims 1 to 4, **characterized in that** R₈ is an OY group in which Y is chosen from the CH₂COOH, CH₂COOR, CHF-COOH, CHF-COOR, CF₂-COOH, CF₂-COOR, CN, CH₂CN, CH₂CONHOH, CH₂CONHCN, CH₂tetrazole, CH₂SO₃H, CH₂SO₂R, CH₂PO(OR)₂, CH₂PO(OR)(OH), CH₂PO(R)(OH) and CH₂PO(OH)₂ groups or OY₁ in which Y₁ is chosen from the SO₂R, SO₂NHCOR, SO₂NHCOOR, SO₂NHCONHR and SO₃H groups, R being as defined in claim 1.

6. Compound according to any one of claims 1 to 5, **characterized in that** R' is chosen from the group consisting of -O-CH₂-CHOH-CH₂OH, -CH₂-CH₂-NH₂, -CH₂-COOC₂H₅, -CH₂-CH₂-phenyl, -CH₂-phenyl, -O-CO-NHphenyl, -O-CO-NHC₂H₅, -O-SO₂-CF₃, -O-(CH₂)₂-O-SO₃H, -O-(CH₂)₂-O-CH₃, -CH₂-COOH, -CO-NH₂, -CO-NH phenyl, -CH₂-(p-OCH₃ phenyl) and phenyl optionally substituted with CH₃, C₂H₅, F and CF₃.

7. Compounds of formula (I), as defined in claim 1, the names of which are as follows:
- the triethylammonium salt of 5,6-dihydro-6-oxo-N²-phenyl-5-(sulphoxy)-4*H*-4,7-methanopyrazolo[3,4-e][1,3]diazepine-2,8(8*H*)-dicarboxamide,
- the sodium salt of 4,5,6,8-tetrahydro-6-oxo-5-(sulphoxy)-1*H*-4,7-methanopyrazolo[3,4-e][1,3]diazepine-1-carboxamide,
- the sodium salt of 1,4,5,8-tetrahydro-1-[(4-methoxyphenyl)methyl]-5-(sulphoxy)-6*H*-4,7-methanopyrazolo[3,4-e][1,3]diazepin-6-one,
- the sodium salt of trans-4,5,6,8-tetrahydro-2-(2-methylphenyl)-6-oxo-5-(sulphoxy)-4,7-methano-7*H*-thieno[2,3-e][1,3]diazepine-8-carboxamide,
- the sodium salt of trans-4,5,6,8-tetrahydro-6-oxo-5-(sulphoxy)-2-[2-(trifluoromethyl)phenyl]-4,7-methano-7*H*-thieno[2,3-e][1,3]diazepine-8-carboxamide,
- the sodium salt of trans-2-(2-ethylphenyl)-4,5,6,8-tetrahydro-6-oxo-5-(sulphoxy)-4,7-methano-7*H*-thieno[2,3-e][1,3]diazepine-8-carboxamide,
- the sodium salt of trans-8-(2,3-dihydroxypropoxy)-1,2,3,5-tetrahydro-3-oxo-2-(sulphoxy)-1,4-methano-4*H*-2,4-benzodiazepine-5-carboxamide,
- the sodium salt of ethyl trans-3-(4-fluorophenyl)-4,6,7,8-tetrahydro-6-oxo-7-(sulphoxy)-5,8-methano-5*H*-thieno[2,3-e][1,3]diazepine-4-carboxylate,
- the sodium salt of trans-2,5,6,8-tetrahydro-6-oxo-2-phenyl-5-(sulphoxy)-4*H*-4,7-methanopyrazolo[3,4-e][1,3]diazepine-8-carboxamide,
- the sodium salt of 1,4,5,8-tetrahydro-1-phenyl-5-(sulphoxy)-6*H*-4,7-methano-pyrazolo[3,4-e][1,3]diazepin-6-one,
- the sodium salt of trans-4,5,6,8-tetrahydro-6-oxo-1-phenyl-5-(sulphoxy)-1*H*-4,7-methanopyrazolo[3,4-e][1,3] diazepine-8-carboxamide,
- the triethylammonium salt of methyl trans-2,5,6,8-tetrahydro-6-oxo-2-(phenylmethyl)-5-(sulphoxy)-4H-4,7-methanopyrazolo[3,4-e][1,3]diazepine-8-carboxylate,
- the triethylammonium salt of methyl trans-4,5,6,8-tetrahydro-6-oxo-1-(2-phenylethyl)-5-(sulphoxy)-1H-4,7-methanopyrazolo[3,4-e][1,3]diazepine-8-carboxylate,
- the triethylammonium salt of ethyl trans-4,5,6,8-tetrahydro-8-(methoxycarbonyl)-6-oxo-5-(sulphoxy)-1*H*-4,7-methanopyrazolo[3,4-e][1,3]diazepine-1-acetate,
- the triethylammonium salt of ethyl trans-5,6-dihydro-8-(methoxycarbonyl)-6-oxo-5-(sulphoxy)-4*H*-4,7-methanopyrazolo[3,4-e][1,3]diazepine-2(8*H*)-acetate,
- the di(triethylammonium) salt of trans-5,6-dihydro-8-(methoxycarbonyl)-6-oxo-5-sulphoxy-4*H*-4,7-methanopyrazolo[3,4-e][1,3]diazepine-2(8*H*)-acetic acid,
- the pyridinium salt of methyl trans-1-(aminocarbonyl)-4,5,6,8-tetrahydro-6-oxo-5-(sulphoxy)-1*H*-4,7-methanopyrazolo[3,4-e][1,3]diazepine-8-carboxylate,
- the pyridinium salt of methyl trans-2-(aminocarbonyl)-2,5,6,8-tetrahydro-6-oxo-5-(sulphoxy)-4*H*-4,7-methanopyrazolo[3,4-e]diazepine-8-carboxylate,
- the sodium salt of methyl trans-2-(4-fluorophenyl)-4,5,6,8-tetrahydro-6-oxo-5-(sulphoxy)-4,7-methano-7*H*-thieno[2,3-e][1,3]diazepine-8-carboxylate,
- the sodium salt of methyl trans-2(aminocarbonyl)-4,5,6,8-tetrahydro-6-oxo-5-(sulphoxy)-4,7methano-7*H*-thieno[2,3-e][1,3]diazepine-8-carboxylate,
- the sodium salt of ethyl trans-1,2,3,5-tetrahydro-3-oxo-9-[[(phenylamino)-carbonyl]oxy]-2-(sulphoxy)-1,4-methano-4*H*-2,4-benzodi-azepine-5-carboxylate,
- the sodium salt of trans-1,2,3,5-tetrahydro-N-methoxy-8-[(2-methoxyethoxy)-methoxy]-3-oxo-2-(sulphoxy)-1,4-methano-4*H*-2,4-benzo-diazepine-5carboxamide,
- the sodium salt of ethyl trans-1,2,3,5-tetrahydro-3-oxo-8-[[(phenyl-amino)-carbonyl]oxy]-2-(sulphoxy)-1,4-methano-4*H*-2,4-benzodiazepine-5-carboxylate,
- the sodium salt of ethyl trans-8-[[(ethylamino)carbonyl]oxy]-1,2,3,5-tetrahydro-3-oxo-2-(sulphoxy)-1,4-methano-4*H*-2,4-benzodiazepine-5-carboxylate,
- the sodium salt of ethyl trans-1,2,3,5-tetrahydro-3-oxo-2-(sulphoxy)-8-{[trifluoromethyl)sulphonyl]oxy}-1,4-methano-4*H*-2,4-benzodiazepine-5-carboxylate,
- the disodium salt of trans-1,2,3,5-tetrahydro-3-oxo-2-(sulphoxy)-8-[2-(sulphoxy)ethoxy]-1,4-methano-4*H*-2,4-benzodiazepine-5-carboxamide,
- the sodium salt of trans-8-[(2,2-dimethyl-1,3-dioxolan-4-yl)methoxy]-1,2,3,5-tetrahydro-3-oxo-2-(sulphoxy)-1,4-methano-4*H*-2,4-benzodiazepine-5-carboxamide,
- the triethylammonium salt of methyl trans-2,5,6,8-tetrahydro-6-oxo-(2-phenylethyl)-5-(sulphoxy)-4*H*-4,7-methanopyrazolo[3,4-*e*][1,3]diazepine-8-carboxylate.

8. Process for preparing a compound according to one of claims 1 to 11, **characterized in that** it comprises:
a) a stage during which a reaction is brought about between a carbonylating agent chosen from the group consisting of phosgene, diphosgene, triphosgene, aryl, aralkyl, alkyl and alkenyl chloroformates, alkyl dicarbonates, carbonyldiimidazole and their mixtures, where appropriate in the presence of a base, and a compound of formula (II): 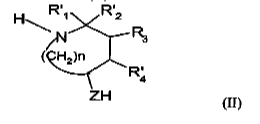 in which:
R'₁ represents a hydrogen atom, a CN, protected COOH, COOR₉, (CH₂)ₙR'₅, CONR₆R₇, or a radical 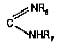 R₉ is chosen from the group consisting of an alkyl radical containing from I to 6 carbon atoms, optionally substituted with one or more halogen atoms or with a pyridyl radical, a -CH₂-alkenyl radical containing in total from 3 to 9 carbon atoms, an aryl radical containing from 6 to 10 carbon atoms or an aralkyl radical containing from 7 to 11 carbon atoms, the nucleus of the aryl or aralkyl radical being optionally substituted with an NO₂, protected OH, protected NH₂, alkyl radical containing from 1 to 6 carbon atoms, an alkoxy radical containing from 1 to 6 carbon atoms or with one or more halogen atoms,
R'₅ is chosen from the group consisting of a protected OH, CN, protected NH₂, CO-NR₆R₇, protected COOH, COOR₉, OR₉ radical, R₉ being as defined above,
n', R₆ and R₇ are as defined in claim 1,
R₃ and R'₄ form together a phenyl or a heterocycle with an aromatic character with 5 members such as defined in claim 1, substituted with a R₁₀ group, R₁₀ being chosen from the group consisting of a hydrogen atom and the alkyl radicals containing from 1 to 6 carbon atoms substituted with one or more hydroxy, oxo, halogen or cyano radicals, or with an alkenyl radical containing from 2 to 6 carbon atoms, halo, protected OH, -OR, OR", R" being as defined above, -(CH₂)_{b}-phenyl or -(CH₂)_{b}-heterocycle, which is optionally substituted, as defined in claims 1 to 6;
R₂ is a hydrogen atom,
ZH represents an HNR'₈-(CH₂)_{n"}wherein
- n" is as defined in claim 1 and
- R'₈ represents a hydrogen atom, a R₉ radical, a protected OH, a radical OR₉, Y', OY', Y'₁, OY'₁, R₉ and m being as defined above, and m being equal to 0, 1 or 2
- Y' is chosen from the group consisting of the COH, COR₉, COOR₉, CONH₂, CONHR_{9,} CONHSO₂R₉, CH₂COOR₉, CH₂SO₂R₉, CH₂PO(OR₉)₂, protected CONHOH, protected CH₂COOH, protected CH₂CONHOH, protected CH₂SO₃, protected CH₂PO(OR)(OH), protected CH₂PO(R)(OH) and protected CH₂PO(OH)₂ radicals,
- Y'₁ is chosen from the group consisting of the SO₂R₉, SO₂NHCOH, SO₂NHCOR₉, SO₂NHCOOR₉, SO₂NHCONH₂, SO₂NHCONHR₉ and protected SO₃H radicals,
and n is equal to 1;
in order to obtain an intermediate compound of formula (III): 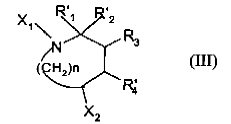
in which:
R'₁, R'₂, R₃, R'₄ and n have the same meanings as above
and either X₁ is a hydrogen atom and X₂ represents a -Z-CO-X₃ group, X₃ representing the residue of the carbonylating agent, i. e. a rest Cl, OCCl₃, OAr, OCH₂Ar, OAlk, OAlkenyl, OCOOAlk and imidazolyl,
or X₂ is a -ZH group and X₁ represents a CO-X₃ group, X₃ being as defined above;
b) a stage in which the intermediate obtained above is cyclized, in the presence of a base;
and **in that**:
c) where appropriate, Stage a) is preceded and/or Stage b) is followed by one or more of the following reactions, in an appropriate order:
- protection of the reactive functional groups;
- deprotection of the reactive functional groups;
- esterification;
- saponification;
- sulphation;
- phosphatization;
- amidation;
- acylation;
- sulphonylation;
- alkylation;
- formation of a urea group;
- reduction of carboxylic acids;
- reduction of ketones and aldehydes to alcohols;
- salification;
- ion exchange;
- resolution or separation of diastereoisomers;
- oxidation of sulphide to sulphoxide and/or sulphone;
- oxidation of aldehyde to acid;
- oxidation of alcohol to ketone;
- halogenation or dehalogenation;
- carbamoylation;
- carboxylation;
- introduction of an azido group;
- reduction of an azido to amine;
- reactions of coupling of aromatic or heteroaromatic halides or triflates or of heterocyclic nitrogens with aryl- or heteroarylboronic acids;
- reactions of coupling of aromatic or heteroaromatic halides or triflates with stannyl reagents;
- hydrogenation of double bonds;
- dihydroxylation of double bonds;
- cyanidation.

9. Process according to claim 8, **characterized in that** the carbonylation reaction occurs in the presence of a base.

10. Process according to any one of claims 8 or 9, **characterized in that,** in Stage b), the base is chosen from the group consisting of amines, hydrides, alcoholates, amides or carbonates of alkali or alkaline earth metals.

11. Process according to claim 10, **characterized in that** the base is an amine.

12. Process according to any one of claims 8 to 11, **characterized in that** the compound of formula (II) in which ZH represents an HO-(CH₂)_{n"}group in which n" is equal to 0, R'₈ being as defined in claim 11, is obtained by a process in which a compound of formula (IV): 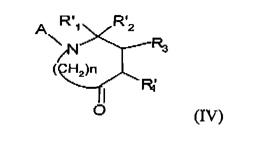 in which R'₁, R'₂ and n are as defined in claim 8, R₃ and R'₄ have the values defined in claim 8 or values which are precursors of those defined above and A represents a hydrogen atom or a protective group of the nitrogen,
is treated with a reducing agent, in order to obtain a compound of formula (V): 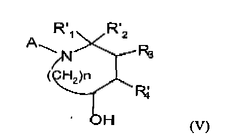 in which A represents a hydrogen atom or a protective group of the nitrogen, R'₁, R'₂, R₃, R'₄ and n retain their meaning mentioned in claim 8, in which, where appropriate, the OH group is replaced by a leaving group,
in order to obtain a compound of formula (VI): 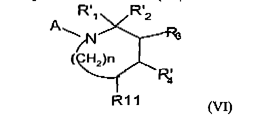 in which A represents a hydrogen atom or a protective group of the nitrogen, R'₁, R'₂, R₃, R'₄ and n retain their meaning mentioned in claim 8 and R₁₁ represents a leaving group, which is then treated with a compound of formula Z₁H₂ in which Z₁ represents a divalent -NR'₈ or -O-NR'₈ group, R'₈ retaining the meaning mentioned in claim 8, in order to obtain a compound of formula (VIII) or (VIII'); 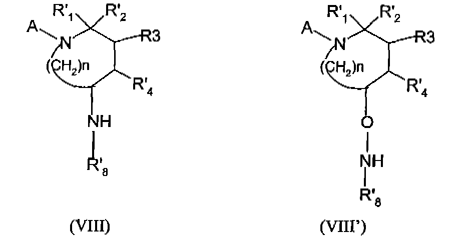 in which A represents a hydrogen atom or a protective group of the nitrogen, R'₁, R'₂, R₃, R'₄, n and R'₈ are defined as mentioned in claim 8, then, where appropriate, with an appropriate agent for deprotecting the nitrogen atom, and **in that**, where appropriate, the intermediate of formula (IV), (V), (VIII) or (VIII') is subjected to one or more of the reactions described in Stage c) of the process of claim 8, in an appropriate order.

13. Process according to any one of claims 8 to 11, according to which the compound of formula (II) in which ZH represents an NHR'₈-(CH₂)_{n"}- group in which n" is equal to 0 is obtained by a process **characterized in that** a compound of formula (IV) as defined above is treated with a compound of formula H₂NR'₈, in order to obtain a compound of formula (VII): 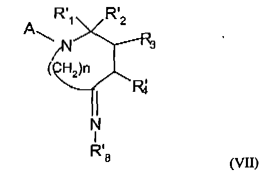 in which A represents a hydrogen atom or a protective group of the nitrogen, R'₁, R'₂, R₃, R'₄, n and R'₈ are as defined in claim 8 which is reacted with a reducing agent in order to obtain a compound of formula (VIII); 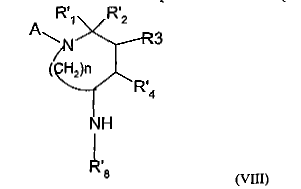 in which A represents a hydrogen atom or a protective group of the nitrogen, R'₁, R'₂, R₃, R'₄, n and R'₈ are as defined in claim 8, which is treated, where appropriate, with an appropriate agent for deprotecting the nitrogen atom, and **in that**, where appropriate, the intermediate of formula (VII) or (VIII) is subjected to one or more of the reactions described in Stage c) of the process of claim 8, in an appropriate order.

14. As medicaments, the products as defined in any one of claims 1 to 6 and their salts with pharmaceutically acceptable acids and bases.

15. As medicaments, the products as defined in claim 7.

16. Pharmaceutical compositions containing, as active ingredient, at least one medicament according to either of claims 14 or 15.

17. Pharmaceutical compositions containing, as active ingredient, at least one β-lactamase inhibiting medicament as defined in claim 14 or 15 and at least one β-lactamine type medicament.

18. Compound of general formula (III) or one of its salts with an acid, in particular its hydrochloride and its trifluoroacetate: 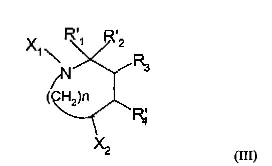 in which R₃ and R'₄ form together a phenyl or a heterocycle with an aromatic character, which is substituted with a -(CH₂)_{b}-phenyl radical or-(CH₂)_{b}-heterocycle with an aromatic character, which is optionally substituted, as in claim 1, the other groups being defined in claim 8, the protecting groups such as defined in the above definition being chosen as shown hereunder:
- for an acid function: the rests of alkyl, allyl, benzyl benzhydryl and p-nitrobenzyl esters;
- for OH: the rests of rests of alkyl, alkoxyalkyl, aryl and aralkyl ethers, the rests of silyl ethers chosen from the group formed of terbutyldimethyl, trimethyl, triphenyl and diphenyl-terbutyl-silyle, the rests of cleavable esters chosen from the group comprising acetate, propionate, benzoate and p-nitrobenzoate, the rests of methyl, terbutyl, allyl, benzyl and p-nitrobenzyl carbonates,
- for NH₂: benzyl and trityl derivatives, the allyl, benzyl, phenyl and terbutyl carbamates, the silyl derivatives chosen from the group formed of terbutyldimethyl, trimethyl, triphenyl and diphenylterbutyl-silyle and the cyanoalkyl and phenylsulfonylalkyl derivatives;
- for hydroxylamines: the allyl and benzyl ethers.

19. Compound of general formula (III) or one of its salts with acid, in particular its hydrochloride and its trifluoroacetate: 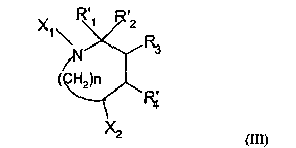 in which R'₁ represents a CONR₆R₇ radical with R₆ or R₇ representing an alkoxy radical containing from I to 6 carbon atoms, the other values being as defined in claim 8 or in claim 18.

20. Compounds of formula (II) or one of their salts with an acid, in particular its hydrochloride and its trifluoroacetate; 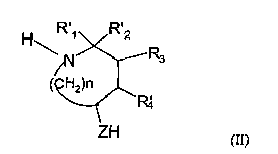 in which R₃ and R'₄ form together a phenyl or a heterocycle with an aromatic character, which is substituted with a -(CH₂)_{b}-phenyl or -(CH₂)_{b}-heterocycle radical with an aromatic character, which is optionally substituted, as defined in claim 1, the other values being as defined in claim 8 and in claim 18.

21. Compounds of formula (II) or one of their salts with an acid, in particular its hydrochloride and its trifluoroacetate 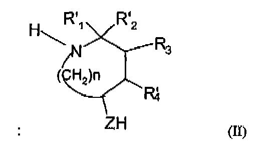 in which R'₁ represents a CONR₆R₇ radical in which R₆ or R₇ represents an alkoxy radical containing from I to 6 carbon atoms, the other values being as defined in claim 8 or in claim 18.

22. Compounds of formulae ((VI) or one of their salts with an acid, in particular their hydrochlorides and their trifluoroacetates: 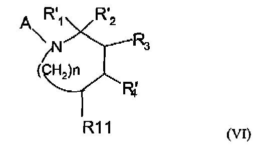 in which
- A represents a hydrogen atom or a protective group of the nitrogen chosen in the group consisting in benzyl and trityl derivatives, the allyl, benzyl, phenyl and terbutyl-carbamates, or further the silyl derivatives chosen from the group formed of terbutyl-dimethyl, trimethyl, triphenyl and diphenylterbutyl-silyle or the cyanoalkyl and phenylsulfonylalkyl derivatives;
- R₁₁ represents a leaving group chosen in the group consisting in phosphate, sulfonate or halogene
- and R₃ and R'₄ form together a phenyl or a heterocycle with an aromatic character, which is substituted with a -(CH₂)_{b}-phenyl radical or -(CH₂)_{b}-heterocycle radical with an aromatic character, which is optionally substituted, as defined in claim 1, the other values being as defined in claim 8 or in claim 18.

23. Compounds of formula (VI) 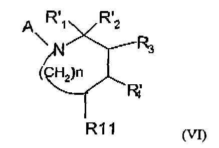 or one of their salts with an acid, in particular their hydrochlorides and their trifluoroacetates in which
- A represents a hydrogen atom or a protective group of the nitrogen such as defined in claim 22,
- R'₁ represents a CONR₆R₇ radical in which R₆ or R₇ represents an alkoxy radical containing from I to 6 carbon atoms,
- R₁₁ is such as defined in claim22
- and the other values being as defined in claim 8 or in claim 18.

24. Compounds of formulae (VII), (VIII) and (VIII') or one of their salts with an acid, in particular their hydrochlorides and their trifluoroacetates: 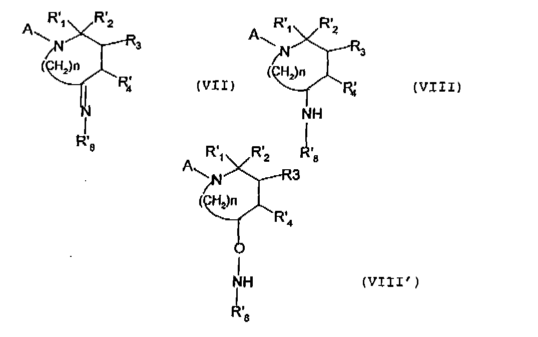 in which A represents a hydrogen atom or a protective group of the nitrogen such as defined in claim 22, and the other values being as defined in claim 18.

25. Compounds of formulae (VII) and (VIII) 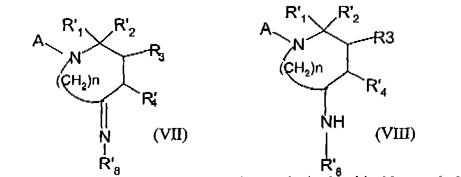 or one of their salts with an acid, in particular their hydrochlorides and their trifluoroacetates in which
- A represents a hydrogen atom or a protective group of the nitrogen such as defined in claim 22,
- R'₁ represents a CONR₆R₇ radical in which R₆ or R₇ represents an alkoxy radical containing from I to 6 carbon atoms,
- and the other values are as defined in claim 8 or in claim 18.

## Patentansprüche

1. Verbindung der allgemeinen Formel oder eines ihrer Salze mit einer Base oder einer Säure: 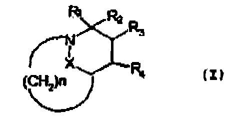 worin:
- R₁ für ein Wasserstoffatom, ein COOH-, CN-, COOR-, (CH₂)_{n'}R₅-, CONR₆R₇-Radikal oder 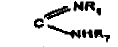 steht;
■ R ausgewählt ist aus der Gruppe bestehend aus einem Alkylradikal, das 1 bis 6 Kohlenstoffatome aufweist, das optional mit einem oder mehreren Halogenatomen oder mit einem Pyridylradikal substituiert ist, einem - CH₂-Alkenylradikal, das insgesamt 3 bis 9 Kohlenstoffatome aufweist, einer (Poly)alkoxyalkylgruppe, die 1 bis 4 Sauerstoffatome und 3 bis 10 Kohlenstoffatome aufweist, einem Arylradikal, das 6 bis 10 Kohlenstoffatome aufweist, oder einem Aralkylradikal, das 7 bis 11 Kohlenstoffatome aufweist, wobei der Kern des Aryl- oder Aralkylradikals optional mit einem OH-, NH₂-, NO₂-, Alkylradikal, das 1 bis 6 Kohlenstoffatome aufweist, Alkoxradikal, das 1 bis 6 Kohlenstoffatome aufweist, oder mit einem oder mehreren Halogenatomen substituiert ist,
■ R₅ ausgewählt ist aus der Gruppe bestehend aus einem COOH-, CN-, OH-, NH₂-, CO-NR₆R₇-, COOR-, OR-Radikal, wobei R ist, wie oben definiert,
■ R₆ und R₇ unabhängig ausgewählt sind aus der Gruppe bestehend aus einem Wasserstoffatom, einem Alkylradikal, das 1 bis 6 Kohlenstoffatome aufweist, einem Alkoxyradikal, das 1 bis 6 Kohlenstoffatome aufweist, einem Arylradikal, das 6 bis 10 Kohlenstoffatome aufweist und einem Aralkylradikal, das 7 bis 11 Kohlenstoffatome aufweist und einem Alkylradikal, das 1 bis 6 Kohlenstoffatome aufweist, das mit einem Pyridylradikal substituiert ist,
■ n' gleich 1 oder 2 ist,
- R₃ und R₄ gemeinsam ein Phenyl oder einen Heteroring mit aromatischem Charakter mit 5 Gliedern bilden, der 1 oder 2 Heteroatome aufweist, die ausgewählt sind aus Stickstoff, Sauerstoff und Schwefel, wie nachfolgend definiert: 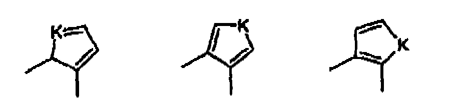 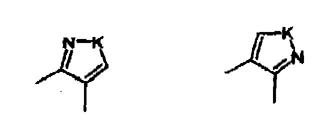 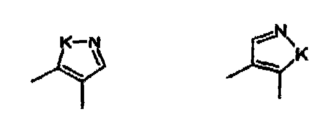 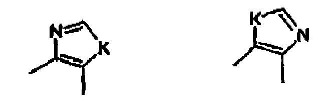 mit K = NH, S, O
substituiert mit einer R-Gruppe, wobei R' ausgewählt ist aus der Gruppe bestehend aus den folgenden Radikalen
-(O)ₐ-(CH₂)_{b}-(O)ₐ-CONR₆R₇, -(O)ₐ-(CH₂)_{b}-OSO₃H, -(O)ₐ-(CH₂)_{b}-SO₃H,
-(O)ₐ-SO₂R, -(O)ₐ-SO₂-CHal₃, -(O)ₐ-(CH₂)_{b}-NR₆R₇, -(O)ₐ-(CH₂)_{b}-NH-COOR, -(CH₂)_{b}-COOH, -(CH₂)_{b}-COOR, -OR", OH, -(CH₂)_{b}-Phenyl und einem -
(CH₂)_{b}-Heteroring mit aromatischem Charakter mit 5 oder 6 Gliedern, der 1 bis 4 Heteroatome aufweist, ausgewählt aus Stickstoff, Sauerstoff und Schwefel,
wobei das Phenyl und der Heteroring optional mit einem oder mehreren Halogenen substituiert ist, einem Alkylradikal, das 1 bis 6 Kohlenstoffatome aufweist, einem Alkoxyradikal, das 1 bis 6 Kohlenstoffatome aufweist, oder CF₃, wobei R, P₆ und R₇ sind, wie zuvor definiert, R" ausgewählt ist aus der Gruppe bestehend aus den Alkylradikalen, die 1 bis 6 Kohlenstoffatome aufweisen, die mit einem oder mehreren Hydroxy-, Oxo-, Halogen- oder Cyanoradikalen substituiert sind, a gleich 0 oder 1 ist und b eine ganze Zahl von 0 bis 6 ist, wobei es sich versteht, dass wenn R' gleich OH ist, R₁ für das CONR₆R₇-Radikal steht, worin R₆ oder R₇ ein Alkoxyradikal ist, das 1 bis 6 Kohlenstoffatome aufweist, und wobei es sich versteht, dass, wenn R' -(O)ₐ-(CH₂)_{b}-NR-₆R₇ ist, wobei R₆ und R₇ gleich H sind, oder -(O)ₐ-(CH₂)_{b}-NH-COOR, a und b nicht gleichzeitig den Wert 0 annehmen, und wenn R' gleich - (CH₂)_{b}-COOH oder -(CH₂)_{b}-NH-COOR ist, b nicht gleich 0 ist,
- R₂ für ein Wasserstoffatom steht,
- X für eine bivalente -C(O)-B-Gruppe steht, die mit dem Stickstoffatom über das Kohlenstoffatom verbunden ist,
B für eine bivalente -NR₈-(CH₂)_{n"}-Gruppe steht, die mit dem Carbonyl über das Stickstoffatom verbunden ist, n" gleich 0 oder 1 ist, und R₈ ausgewählt ist aus der Gruppe bestehend aus einem Wasserstoffatom, einem OH-, R-, OR-, Y-, OY-, Y₁- und OY₁-Radikal, gleich 0, 1 oder 2 ist,
Y ausgewählt ist aus der Gruppe bestehend aus den Radikalen COH, COR, COOR, CONH₂, CONHR, CONHOH, CONHSO₂R, CH₂COOH, CH₂COOR, CHF-COOH, CHF-COOR, CF₂-COOH, CF₂-COOR, CN, CH₂CN, CH₂CONHOH, CH₂CONHCN, CH₂-Tetrazol, CH₂SO₃H, CH₂SO₂R, CH₂PO(OR)₃, CH₂PO(OR)(OH), CH₂PO(R)(OH) und CH₂PO(OH)₂,
Y₁ ausgewählt ist aus der Gruppe bestehend aus den Radikalen SO₂R, SO₂NHCOH, SO₂NHCOR, SO₂NHCOOR, SO₂NHCONHR, SO₂NHCONH₂ und SO₃H,
- n gleich 1 ist.

2. Verbindung der allgemeinen Formel nach Anspruch 1, worin R' ausgewählt ist aus der Gruppe bestehend aus den Radikalen
-(O)ₐ-(CH₂)_{b}-(O)ₐ-CONR₆R₇, -(O)ₐ-(CH₂)_{b}-OSO₃H, -(O)ₐ-(CH₂)_{b}-SO₃H, - (O)ₐ-SO₂R, -(O)ₐ-SO₂-CHal₃, -(O)ₐ-(CH₂)_{b}-NR₆R₇, -(O)ₐ-(CH₂)_{b}-NH-COOR, - (CH₂)_{b}-COOH, -(CH₂)_{b}-COOR, -OR", OH, -(CH₂)_{b}-Phenyl und einem - (CH₂)_{b}-Heteroring mit aromatischem Charakter mit 5 oder 6 Gliedern, der 1 bis 4 Heteroatome aufweist, ausgewählt aus Stickstoff, Sauerstoff und Schwefel, wobei das Phenyl und der Heteroring optional substituiert sind mit einem oder mehreren Halogenen, einem Alkylradikal, das 1 bis 6 Kohlenstoffatome aufweist, einem Alkoxyradikal, das 1 bis 6 Kohlenstoffatome aufweist, oder CF₃, wobei R, R₆ und R₇ sind, wie zuvor definiert, R" ausgewählt ist aus der Gruppe bestehend aus den Alkylradikalen, die 1 bis 6 Kohlenstoffatome aufweisen, die mit einem oder mehreren Hydroxy-, Oxo-, Halogen- oder Cyanoradikalen substituiert sind, a gleich 0 oder 1 ist und b eine ganze Zahl von 0 bis 6 ist, wobei es sich versteht, dass wenn R' gleich OH ist, R₁ für das CONR₆R₇-Radikal steht, worin R₆ oder R₇ ein Alkoxyradikal sind, das 1 bis 6 Kohlenstoffatome aufweist, und wobei es sich versteht, dass
(i) wenn R' gleich OH oder OR" ist, wobei R" ein Alkylradikal ist, das 1 bis 6 Kohlenstoffatome aufweist, das mit einem oder mehreren Oxoradikalen substituiert ist, R₁ dann für das Radikal CONR₆R₇ steht, worin R₆ oder R₇ ein Alkoxyradikal ist, das 1 bis 6 Kohlenstoffatome aufweist, oder auch
(j) wenn R' ein -(O)ₐ-(CH₂)_{b}-NR₆R₇-Radikal ist, worin R₆ und R₇ sind, wie in Anspruch 1 definiert, dann ist a gleich 0 oder 1 und b gleich einer ganzen Zahl von 1 bis 6, oder auch
(k) a gleich 0 oder 1 oder b gleich einer ganzen Zahl von 1 bis 6 ist, wenn R' ein -(O)ₐ-(CH₂)_{b}-NR₄R₇-Radikal ist, worin R₆ und R₇ unabhängig ausgewählt sind aus der Gruppe bestehend aus einem Alkylradikal, das 1 bis 6 Kohlenstoffatome aufweist, einem Alkoxyradikal, das 1 bis 6 Kohlenstoffatome aufweist, einem Arylradikal, das 6 bis 10 Kohlenstoffatome aufweist und einem Aralkylradikal, das 7 bis 11 Kohlenstoffatome aufweist, und einem Alkylradikal, das 1 bis 6 Kohlenstoffatome ausweist, das mit einem Pyridylradikal substituiert ist, oder auch
(l) wenn R' ausgewählt ist aus der Gruppe bestehend aus den Radikalen - (O)ₐ-(CH₂)_{b}-NH-COOR, -(CH₂)_{b}-COOH, -(CH₂)_{b}-COOR, b dann eine ganze Zahl von 1 bis 6 ist und a gleich 0 oder 1 ist; wobei die anderen Werte sind, wie in Anspruch 1 definiert.

3. Verbindung nach Anspruch 1, **dadurch gekennzeichnet, dass** R₃ und R₄ gemeinsam ein Thienyl oder ein Pyrazolyl bilden, das mit einem Substituenten R' substituiert ist, der in Anspruch 1 definiert wurde.

4. Verbindung nach einem der Ansprüche 1 bis 3, **dadurch gekennzeichnet, dass** R₁ ausgewählt ist aus der Gruppe bestehend aus einem Wasserstoffatom und den Gruppen COOCH₃, COOC₂H₅, CONH₂, CONHCH₃ und CONHOCH₃.

5. Verbindungen nach einem der Ansprüche 1 bis 4, **dadurch gekennzeichnet, dass** R₈ eine OY-Gruppe ist, worin Y ausgewählt ist aus den Gruppen CH₂COOH, CH₂COOR, CHF-COOH, CHF-COOR, CF₂-COOH, CF₂-COOR, CN, CH₂CN, CH₂CONHOH, CH₂CONHCN, CH₂tetrazole, CH₂SO₃H, CH₂SO₂R, CH₂PO(OR)₂, CH₂PO(OR)(OH), CH₂PO(R)(OH) und CH₂PO(OH)₂, oder OY₁, worin Y₁ ausgewählt ist aus den Gruppen SO₂R, SO₂NHCOR, SO₂NHCOOR, SO₂NHCONHR und SO₃H, wobei R ist, wie in Anspruch 1 definiert.

6. Verbindung nach einem der Ansprüche 1 bis 5, **dadurch gekennzeichnet, dass** R' ausgewählt ist aus der Gruppe bestehend aus O-CH₂-CHOH-CH₂OH, -CH₂-CH₂-NH₂, CH₂-COOC₂H₅, CH₂-CH₂-Phenyl, CH₂-Phenyl, - O-CO-NHPhenyl, -O-NHC₂H₅, -O-SO2-CF3, -O-(CH₂)₂-O-SO₃H, -CH₂-COOH, - CO-NH₂, -CO-NHPhenyl, -CH₂-(p-OCH₃ Phenyl) und Phenyl optional mit CH₂, C₂H₅, F und CF₃ substituiert ist.

7. Verbindungen der Formel (I), wie in Anspruch 1 definiert, mit folgenden Bezeichnungen:
- 5,6-Dihydro-6-oxo-N²-phenyl-5(sulfooxy)-4*H*-4,7-methanopyrazolo[3,4-e][1,3]diazepin-2,8(8*H*)-dicarboxamid-Triethylammoniumsalz,
- 4,5,6,8.Tetrahydro-6-oxo-5-(sulfooxy)-1*H*-4,7-methanopyrazolo[3,4-e][1,3]diazepin-1-carboxamid-Natriumsalz,
- 1,4,5,8-Tetrahydro-1-[(4-methoxyphenyl)methyl]-5-(sulfooxy)-6*H*-4,7-methanopyrazolo[3,4-e][1,3]diazepin-6-on-Natriumsalz,
- trans-4,5,6,8-Tetrahydro-2-(2-methylphenyl)-6-oxo-5-(sulfooxy)-4,7-methano-7*H*-thieno[2,3-e][1,3]diazepin-8-carboxamid-Natriumsalz,
- trans-4,5,6,8-Tetrahydro-6-oxo-5-(sulfooxy)-2-[2-(trifluormethyl)phenyl]-4,7-methano-7*H*-thieno[2,3-e][1,3]diazepin-8-carboxamid-Natriumsalz,
- trans-2-(2-Ethylphenyl-4,5,6,8-tetrahydro-6-oxo-5-(sulfooxy)-4,7-methano-7*H*-thieno[2,3-e][1,3]diazepin-8-carboxamid-Natriumsalz,
- trans-8-(2,3-Dihydroxypropoxy)-1,2,3,5-tetrahydro-3-oxo-2-(sulfooxy)-1,4-methano-4*H*-2,4-benzodiazepin-5-carboxamid-Natriumsalz,
- trans-3-(4-Fluorphenyl)-4,6,7,8-tetrahydro-6-oxo-7-(sulfooxy)-5,8-methano-5*H*-thieno[2,3-e][1,3]diazepin-4-ethylcarboxylat-Natriumsalz,
- trans-2,5,6,8-Tetrahydro-6-oxo-2-phenyl-5-(sulfooxy)-4*H*-4,7-methanopyra-zolo[3,4-e][1,3]diazepin-8-carboxamid-Natriumsalz,
- 1,4,5,8-Tetrahydro-1-phenyl-5-(sulfooxy)-6*H*-4,7-methanopyrazolo[3,4-e]-[1,3]-diazepin-6-on-Natriumsalz,
- Trans-4,5,6,8-tetrahydro-6-oxo-1-phenyl-5(sulfooxy)-1*H*-4,7-methanopyra-zolo[3,4-e][1,3]diazepin-8-carboxamid-Natriumsalz,
- Trans-2,5,6,8-tetrahydro-6-oxo-2-(phenylmethyl)-5-(sulfooxy)-4*H*-4,7-methanopyrazolo[3,4-e][1,3]diazepin-8-methylcarboxylat-Triethylammoniumsalz,
- Trans-4,5,6,8-tetrahydro-6-oxo-1-(2-phenylethyl)-5-(sulfooxy)-1*H*-4,7-methanopyrazolo[3,4-e][1,3]diazepin-8-methylcarboxylat-Triethylammoniumsalz,
- Trans-4,5,6,8-tetrahydro-8-(methoxycarbonyl)-6-oxo-5-(sulfooxy)-1*H*-4,7-methanopyrazolo[3,4-e][1,3]diazepin-1-ethylacetat-Triethylammoniumsalz,
- Trans-5,6-dihydro-8-(methoxycarbonyl)-6-oxo-5-(sulfooxy)-4*H*-4,7-methanopyrazolo[3,4-e][1,3]diazepin-2(8*H*)-ethylacetat-Triethylammoniumsalz,
- Trans-5,6-dihydro-8-(methoxycarbonyl)-6-oxo-5-sulfooxy-4*H*-4,7-methanopyrazolo[3,4-e][1,3]diazepin-2(8*H*)-essigsäure-Di-(triethylammonium)salz,
- Trans-1-(aminocarbonyl)-4,5,6,8-tetrahydro-6-oxo-5-(sulfooxy)-1*H*-4,7-methanopyrazolo[3,4-e][1,3]diazepin-8-methylcarboxylat-Pyridiniumsalz,
- Trans-2-(aminocarbonyl)-2,5,6,8-tetrahydro-6-oxo-5-(sulfooxy)-4*H*-4,7-methanopyrazolo[3,4-e][diazepin-8-methylcarboxylat-Pyridiniumsalz,
- Trans-2-(4-fluorophenyl)-4,5,6,8-tetrahydro-6-oxo-5-(sulfooxy)-4,7-methano-7*H*-thieno[2,3-e][1,3]diazepin-8-methylcarboxylat-Natriumsalz,
- trans-2(Aminocarbonyl)-4,5,6,8-tetrahydro-6-oxo-5-(sulfooxy)-4,7-methano-7*H*-thieno[2,3-e][1,3]diazepin-8-methylcarboxylat-Natriumsalz,
- trans-1,2,3,5-Tetrahydro-3-oxo-9-[[(phenylamino)carbonyl]oxy3-2-(sulfooxy)-1,4-methano-4*H*-2,4-benzodiazepin-5-ethylcarboxylat-Natriumsalz,
- trans-1,2,3,5-Tetrahydro-N-methoxy-8-[(2-methoxyethoxy)methoxy]-3-oxo-2-(sulfoxy)-1,4-methano-4*H*-2,4-benzodiazepin-5-carboxamid-Natriumsalz,
- trans-1,2,3,5-Tetrahydro-3-oxo-8-[[(phenylamino)carbonyl]oxy]-2-(sulfooxy)-1,4-methano-4*H*-2,4-benzodiazepin-5-ethylcarboxylat-Natriumsalz,
- trans-8-[[(Ethylamino)carbonyl]oxy]-1,2,3,5-tetrahydro-3-oxo-2-(sulfooxy)-1,4-methano-4*H*-2,4-benzodiazepine-5-ethylearboxylat-Natriumsalz,
- trans-1,2,3,5-Tetrahydro-3-oxo-2-(sulfooxy)-8-[[trifluormethyl)sulfonyl]-oxy]-1,4-methano-4*H*-2,4-benzodiazepin-5-ethylcarboxylat-Natriumsalz,
- trans-1,2,3,5-Tetrahydro-3-oxo-2-(sulfooxy)-8-[2-(sulfooxy)ethoxy]-1,4-methano-4*H*-2,4-benzodiazepin-5-carboxamid-Dinatriumsalz,
- trans-8-[(2,2-Dimethyl-1,3-dioxolan-4-yl)methoxy]-1,2,3,5-tetrahydro-3-oxo-2-(sulfooxy)-1,4-methano-4*H*-2,4-benzodiazepine-5-carboxamid-Natriumsalz,
- trans-2,5,6,8-Tetrahydro-6-oxo-2-(2-phenylethyl)-5- (sulfooxy)-4*H*-4,7-methano-pyrazolo[3,4-e][1,3]diazepin-8-methylcarboxylat-Triethylammoniumsalz.

8. Verfahren zur Herstellung einer Verbindung nach einem der Ansprüche 1 bis 7, **dadurch gekennzeichnet, dass** es umfasst:
a) einen Schritt, in dessen Verlauf, mit einem Carbonylierungsmittel, das ausgewählt ist aus der Gruppe bestehend aus Phosgen, Diphosgen, Triphosgen, Aryl-, Aralkyl-, Alkyl- und Alkenylchlorformiaten, Alkyldicarbonaten, Carbonyl-diimidazol und deren Mischungen, und gegebenenfalls in Gegenwart einer Base, eine Verbindung der Formel (II) umgesetzt wird: 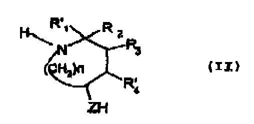 worin:
R'₁ für H, ein CN-, geschütztes COOH-, COOR₉-, (CH₂)ₙ-R'₅-, CONR₅R₇-Radikal 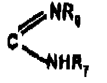 steht;
R₉ ausgewählt ist aus der Gruppe bestehend aus einem Alkylradikal, das 1 bis 6 Kohlenstoffatome aufweist, das optional mit einem oder mehreren Halogenatomen oder mit einem Pyridylradikal substituiert ist, einem -CH₂-Alkenylradikal, das insgesamt 3 bis 9 Kohlenstoffatome aufweist, einem Arylradikal, das 6 bis 10 Kohlenstoffatome aufweist, oder einem Aralkylradikal, das 7 bis 11 Kohlenstoffatome aufweist, wobei der Kern des Aryl- oder Aralkylradikals optional mit einem NO₂-, geschützten OH-, NH₂-Radikal, Alkylradikal, das 1 bis 6 Kohlenstoffatome aufweist, Alkoxradikal, das 1 bis 6 Kohlenstoffatome aufweist, oder mit einem oder mehreren Halogenatomen substituiert ist,
R'₉ ausgewählt ist aus der Gruppe bestehend aus einem geschützten OH-, CN-, geschütztes NH₂-, CO-NR₆R₇-, geschützten COOH-, COOR₉-, OR₉-Radikal,
wobei R₉ ist, wie oben definiert,
n', R₆ und R₇ sind, wie in Anspruch 1 definiert,
R₃ und R'₄ gemeinsam ein Phenyl oder einen Heteroring mit aromatischem Charakter mit 5 oder 6 Gliedern bilden, wie in Anspruch 1 definiert, der mit einer Gruppe R₁₀ substituiert ist, wobei R₁₀ ausgewählt ist aus der Gruppe bestehend aus einem Wasserstoffatom und den Alkylradikalen, die 1 bis 6 Kohlenstoffatome aufweisen, die mit einem oder mehreren Hydroxy-, Oxo-, Halogen- oder Cyanoradikalen substituiert sind, oder einem Alkenylradikal, das 2 bis 6 Kohlenstoffatome aufweist, einem Halogenoradikal, einem geschützten OH-, -OR-, OR"-Radikal, wobei R" ist, wie zuvor definiert, -(CH₂)_{b}-Phenyl oder -(CH₂)_{b}-Heteroring, der optional substituiert ist, wie in den Ansprüchen 1 bis 6 definiert,
- R₂ für ein Wasserstoffatom steht,
- ZH für eine HNR'₈-(CH₂)_{n"}-Gruppe steht, n" ist, wie in Anspruch 1 definiert, R'₈ für ein Wasserstoffatom, ein R₉-, geschütztes OH-, OR₉₋, Y'-, OY'-, Y'₁₋, OY'₁-Radikal steht,
wobei R₉ ist, wie zuvor definiert, und m gleich 0, 1 oder 2 ist,
- Y' ausgewählt ist aus der Gruppe bestehend aus den Radikalen COH, COR₉, COOR₉, CONH₂, CONHR₉, CONHSO₂R₉, CH₂COOR₉, geschütztem CH₂-Tetrazol, CH₂SO₂R₉, CH₂PO(OR₉)₂, geschütztem CONHOH, geschütztem HC₂COOH, geschütztem CH₂CONHOH, geschütztem CH₂SO₃, geschütztem CH₂PO(OR)(OH), geschütztem CH₂PO(R)(OH) und geschütztem CH₂PO(OH)₂,
- Y'₁ ausgewählt ist aus der Gruppe bestehend aus den Radikalen SO₂R₉, SO₂NHCOH, SO₂NHCOR₉, SO₂NHCOOR₉, SO₂NHCONH₂, SO₂NHCONHR₉ und geschütztem SO₃H,
- und n gleich 1 ist;
um eine Zwischenverbindung der Formel (III) zu erhalten: 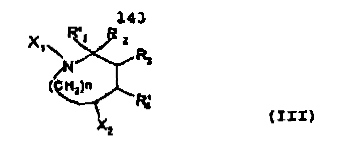
worin:
- R'₁, R₂, R₃, R'₄ und n die gleichen Bedeutungen haben wie oben, und X₁ entweder ein Wasserstoffatom ist und X₂ für eine -Z-CO-X₃-Gruppe steht, wobei X₃ für den Rest des Carbonylierungsmittel steht, nämlich einem Cl-, OCCl₃-, OAr-, OCH₂Ar-, OAlk-, Oalkenyl-, OCOOAlk- und Imidazolylrest, oder X₂ eine -ZH-Gruppe ist und X₁ für eine CO-X₃-Gruppe steht, wobei X₃ ist, wie oben definiert;
b) einen Schritt, in dessen Verlauf das zuvor erhaltene Zwischenprodukt in Gegenwart einer Base cyclisiert wird;
und **dadurch**, dass:
c) gegebenenfalls eine oder mehrere der folgenden Reaktionen, in einer geeigneten Reihenfolge Schritt a) vorausgehen und/oder auf Schritt b) folgen:
- Schutz der reaktiven Funktionen,
- Entschützung der reaktiven Funktionen,
- Veresterung,
- Verseifung,
- Sulfatierung,
- Phosphatierung,
- Amidierung,
- Acylierung,
- Sulfonylierung;
- Alkylierung;
- Bildung einer Hamstoffgruppe
- Carbonsäurereduktion
- Reduktion von Ketonen und Aldehyden zu Alkoholen;
- Salzbildung;
- Ionenaustausch;
- Spaltung oder Trennung von Diastereoisomeren;
- Oxidation von Sulfid zu Sulfoxid und/oder Sulfon;
- Oxidation eines Aldehyds zu Säure;
- Oxidation eines Alkohols zu Keton;
- Halogenierung oder Dehalogenierung
- Carbamylation;
- Carboxylierung;
- Einführung einer Azidogruppe
- Reduktion einer Azidogruppe zu einem Amin
- Kupplungsreaktionen von Halogeniden oder aromatischen oder heteroaromatischen Triflaten oder heterocyclischen Stickstoffen mit Aryl-oder Heteroarylborsäuren;
- Kupplungsreaktionen von Halogeniden oder aromatischen oder heteroaromatischen Triflaten mit Verbindungen, die eine Stannylgruppe aufweisen;
- Hydrierung von Doppelbindungen;
- Dihydroxylierung von Doppelbindungen;
- Einführung einer Cyanidgruppe.

9. Verfahren nach Anspruch 8, **dadurch gekennzeichnet, dass** die Carbonylierungsreaktion in Gegenwart einer Base abläuft.

10. Verfahren nach einem der Ansprüche 8 bis 9, **dadurch gekennzeichnet, dass** in Schritt b) die Base ausgewählt ist aus der Gruppe bestehend aus Aminen, Hydriden, Alkoholaten, Amiden oder Carbonaten von Alkali- oder Erdalkalimetallen.

11. Verfahren nach Anspruch 10, **dadurch gekennzeichnet, dass** die Base ein Amin ist.

12. Verfahren nach einem der Ansprüche 8 bis 11, **dadurch gekennzeichnet, dass** die Verbindung der Formel (II), worin ZH für eine HNR'₈-(CH₂)ₙ-Gruppe steht, worin n" gleich 0 ist, wobei R'₈ ist, wie in Anspruch 8 definiert, mittels eines Verfahrens erhalten wird, worin eine Verbindung der Formel (IV): 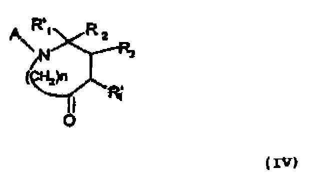 worin R'₁, R₂ und n definiert sind wie in Anspruch 8, R₃ und R'₄ die Werte haben, die in Anspruch 8 definiert wurden, oder Vorläuferwerte jener, die zuvor definiert wurden, und A für ein Wasserstoffatom oder eine Stickstoffschutzgruppe steht,
mit einem Reduktionsmittel behandelt wird, um eine Verbindung der Formel (V) zu erhalten: 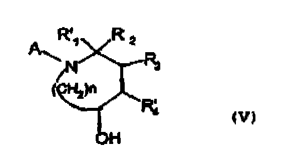 worin A für ein Wasserstoffatom oder eine Stickstoffschutzgruppe steht, R'₁, R₂, R₃, R'₄ und n ihre in Anspruch 8 zitierte Bedeutung beibehalten, worin gegebenenfalls die OH-Gruppe durch eine abgehende Gruppe ersetzt wird,
um eine Verbindung der Formel (VI) zu erhalten: 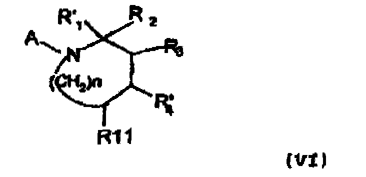 worin A für ein Wasserstoffatom oder eine Stickstoffschutzguppe steht, R'₁, R₂, R₃, R'₄ und n ihre in Anspruch 8 zitierte Bedeutung beibehalten und R₁₁ für eine abgehende Gruppe steht, die mit einer Verbindung der Formel Z₁H₂ behandelt wird, worin Z₁ für eine bivalente -NR'₈-Gruppe steht, wobei R'₈ die in Anspruch 8 zitierte Bedeutung beibehält,
um eine Verbindung der Formel (VIII) oder (VIII') zu erhalten: 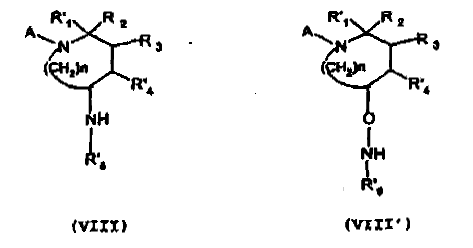 worin A für ein Wasserstoffatom oder eine Stickstoffschutzgruppe steht, R'₁, R₂, R₃, R'₄, n und R'₈ sind, wie in Anspruch 8 definiert, dann gegebenenfalls mit einem geeigneten Entschützungsmittel des Stickstoffatoms,
und **dadurch**, dass das Zwischenprodukt der Formel (IV), (V) (VIII) oder VIII') gegebenenfalls einer oder mehreren der in Schritt c) des Verfahrens aus Anspruch 11 beschriebenen Reaktion(en) in einer geeigneten Reihenfolge unterzogen wird.

13. Verfahren nach einem der Ansprüche 8 bis 11, nach dem die Verbindung der Formel (II), worin ZH für eine HNR'₈-(CH₂)ₙ"-Gruppe steht, worin n" gleich 0 ist, durch ein Verfahren erhalten wird, dass **dadurch gekennzeichnet ist, dass** eine Verbindung der Formel (IV) mit einer Verbindung der Formel H₂NR'₈ behandelt wird, wie zuvor definiert, um eine Verbindung der Formel (VII) zu erhalten: 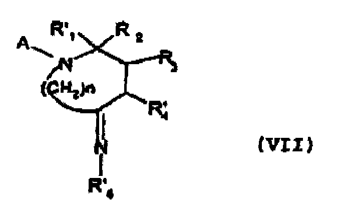 worin A für ein Wasserstoffatom oder eine Stickstoffschutzgruppe steht, R'₁, R₂, R₃, R'₄, n und R'₈ sind, wie in Anspruch 8 definiert, die mit einem Reduktionsmittel umgesetzt wird,
um eine Verbindung der Formel (VIII) zu erhalten: 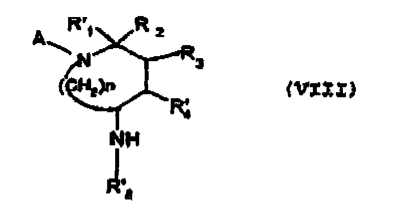 worin A für ein Wasserstoffatom oder eine Stickstoffschutzgruppe steht, R'₁, R₂, R₃, R'₄, n und R'₈ wie in Anspruch 8 definiert sind, die gegebenenfalls mit einem geeigneten Entschützungsmittel des Stickstoffatoms behandelt wird,
und **dadurch**, dass das Zwischenprodukt der Formel (IV) oder (VIII) gegebenenfalls einer oder mehreren der in Schritt c) des Verfahrens aus Anspruch 8 beschriebenen Reaktion(en) in einer geeigneten Reihenfolge unterzogen wird.

14. Verwendung der Produkte, wie in einem der Ansprüche 1 bis 6 definiert, sowie deren Salze mit pharmazeutisch unbedenklichen Säuren und Basen als Medikamente.

15. Verwendung der Produkte, wie in Anspruch 7 definiert, als Medikamente.

16. Pharmazeutische Zusammensetzungen, die als Wirkstoff mindestens ein Medikament nach einem der Ansprüche 14 oder 15 enthalten.

17. Pharmazeutische Zusammensetzungen die als Wirkstoff mindestens ein β-Lactamase hemmendes Medikament enthalten, wie in Anspruch 14 oder 15 definiert, und mindestens ein Medikament vom β-Lactamin-Typ.

18. Verbindung der allgemeinen Formel (III) oder eines ihrer Salze mit einer Säure, insbesondere ihr Hydrochlorid oder ihr Trifluoracetat: 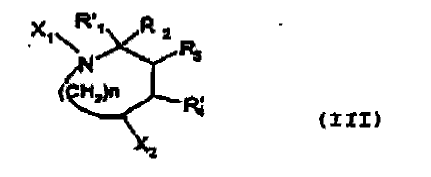 worin R₃ und R'₄ gemeinsam ein Phenyl oder einen Heteroring mit aromatischem Charakter bilden, der mit einem -(CH₂)_{b}-Phonyl oder einem -(CH₂)_{b}-Heteroringradikal mit aromatischem Charakter substituiert ist, der optional substituiert ist, wie in Anspruch 1 definiert, wobei die anderen Werte sind, wie in Anspruch 8 definiert, wobei die Schutzgruppen in den nachfolgenden Definitionen erscheinen, die wie folgt ausgewählt sind:
- für eine Säurefunktion, Alkyl-, Allyl-, Benzyl-, Benzhydryl- und p-Nitrobenzylesterreste,
- für OH, Alkyl-, Alkoxyalkyl-, Aryl- und Aralkyletherreste, Silyletherreste, ausgewählt aus tert-Butyldimethyl, Trimethyl, Triphenyl und Diphenyltert-butylsilyl, Reste spaltbarer Ester, ausgewählt aus Acetat, Propionat, Benzoat und p-Nitrobenzoat, Methyl-, tert-Butyl-, Allyl-, Benzyl- und p-Nitrobenzylcarbonatreste,
- für NH₂, benzylierte und tritylierte Derivate, Allyl-, Benzyl-, Phenyl- und tert-Butylcarbate, silylierte Derivate, ausgewählt aus tert-Butyldimethyl, Trimethyl, Triphenyl- und Diphenyltert-butylsilyl, und Phenylsulfonylalkyl- und Cyanoalkylderivate,
- für Hydroxylamine, Benzyl- und Allylether.

19. Verbindung der allgemeinen Formel (III) oder eines ihrer Salze mit Säure, insbesondere ihr Hydrochlorid oder ihr Trifluoracetat: 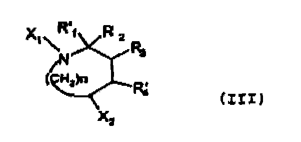 worin R'₁ für ein CONR₆R₇-Radikal steht, worin R₆ oder R₇ für ein Alkoxyradikal stehen, das 1 bis 6 Kohlenstoffatome aufweist, wobei die anderen Werte sind, wie in Anspruch 8 und in Anspruch 18 definiert.

20. Verbindungen der Formeln (III) oder eines ihrer Salze mit einer Säure, insbesondere ihr Hydrochlorid oder ihr Trifluoracetat: 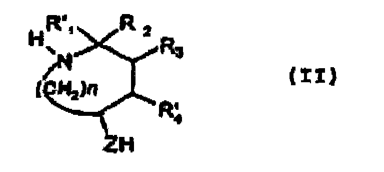 worin R₃ und R'₄ gemeinsam ein Phenyl oder einen Heteroring mit aromatischem Charakter bilden, der mit einem -(CH₂)_{b}-Phenyl oder einem -(CH₂)_{b}-Heteroringradikal mit aromatischem Charakter substituiert ist, das optional substituiert ist, wie in Anspruch 1 definiert, wobei die anderen Werte sind, wie in Anspruch 8 und in Anspruch 18 definiert.

21. Verbindungen der Formeln (III) oder eines ihrer Salze mit einer Säure, insbesondere ihr Hydrochlorid oder ihr Trifluoracetat: 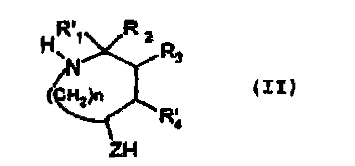 worin R'₁ für ein CONR₆R₇-Radikal steht, worin R₆ oder R₇ für ein Alkoxyradikal stehen, das 1 bis 6 Kohlenstoffatome aufweist, wobei die anderen Werte sind, wie in Anspruch 8 oder in Anspruch 18 definiert.

22. Verbindungen der Formeln (IV) oder eines ihrer Salze mit einer Säure, insbesondere ihre Hydrochloride oder ihre Trifluoracetate: 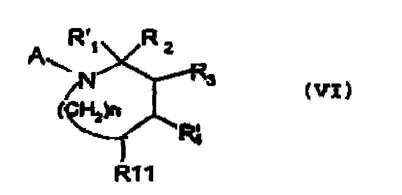 worin A für ein Wasserstoffatom oder eine Stickstoffschutzgruppe steht, ausgewählt aus benzylierten oder tritylierten Derivaten, Allyl-, Benzyl-, Phenyl- und tert-Butylcarbamaten, oder auch silylierten Derivaten, wie etwa tert-Butyldimethyl-, Trimethyl-, Triphenyl- oder auch Diphenyl-tert-Butyl-silylderivaten oder Phenylsulfonylalkyl- und Cyanoalkylderivaten, R₁₁ für eine abgehende Gruppe steht, ausgewählt aus der Gruppe Phosphat, Sulfonat oder Halogen, und R₃ und R'₄ gemeinsam ein Phenyl oder einen Heteroring mit aromatischem Charakter bilden, der mit einem -(CH₂)_{b}-Phenyl oder -(CH₂)_{b}-Heteroringradikal mit aromatischem Charakter substituiert ist, das optional substituiert ist, wie in Anspruch 1 definiert, wobei die anderen Werte sind, wie in Anspruch 8 oder in Anspruch 18 definiert.

23. Verbindungen der Formeln (IV) 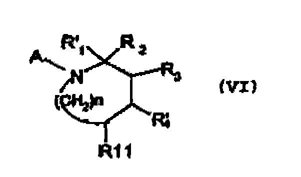 oder eines ihrer Salze mit einer Säure, insbesondere ihre Hydrochloride und ihre Trifluoracetate, worin A für ein Wasserstoffatom oder eine Stickstoffschutzgruppe steht, wie in Anspruch 22 definiert, R'₁ für ein CONR₆R₇-Radikal steht, worin R₆ oder R₇ für ein Alkoxyradikal stehen, das 1 bis 6 Kohlenstoffatome aufweist, R₁₁ ist, wie in Anspruch 22 definiert, und die anderen Wert sind, wie in Anspruch 8 oder in Anspruch 18 definiert.

24. Verbindungen der Formeln (VII), (VIII) und (VIII') oder eines ihrer Salze mit einer Säure, insbesondere ihre Hydrochloride oder ihre Trifluoracetate; 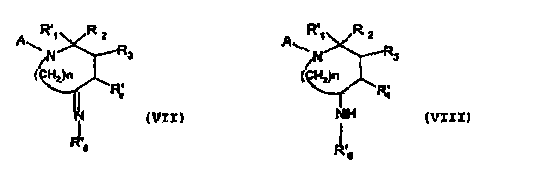 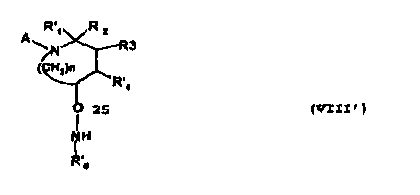 worin A für ein Wasserstoffatom oder eine Stickstoffschutzgruppe steht, wie in Anspruch 22 definiert, und die anderen Werte sind, wie in Anspruch 18 definiert.

25. Verbindungen der Formeln (VII) und (VIII) 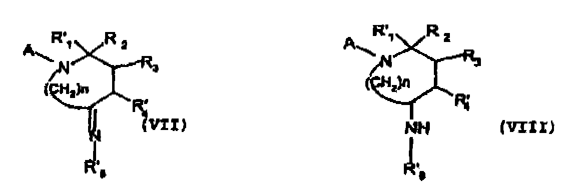 oder eines ihrer Salze mit einer Säure, insbesondere ihre Hydrochloride und ihre Trifluoracetate, worin A für ein Wasserstoffatom oder eine Stickstoffschutzgruppe steht, wie in Anspruch 22 definiert, R'₁ für ein CONR₆R₇-Radikal steht, worin R₆ oder R₇ für ein Alkoxyradikal steht, das 1 bis 6 Kohlenstoffatome aufweist, und die anderen Wert sind, wie in Anspruch 8 oder in Anspruch 18 definiert.
